(19) Europäisches Patentamt
European Patent Office
Office européen des brevets

(11) EP 3 037 103 A1

(12) EUROPEAN PATENT APPLICATION

(43) Date of publication:
**29.06.2016 Bulletin 2016/26**

(21) Application number: **14307153.8**

(22) Date of filing: **23.12.2014**

(51) Int Cl.:
***A61K 39/12*** *(2006.01)* ***C07K 14/005*** *(2006.01)*
***C07K 14/235*** *(2006.01)* ***A61K 39/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **GENTICEL**
**31670 Labege (FR)**

(72) Inventors:
- **MARUSSIG, Myriam**
**94400 Vitry Sur Seine (FR)**
- **TIMMERMAN, Benedikt**
**31120 Goyrans (FR)**

(74) Representative: **Desaix, Anne et al**
**Ernest Gutmann - Yves Plasseraud SAS**
**3, rue Auber**
**75009 Paris (FR)**

# (54) Immunotherapeutic vaccine comprising E7 proteins of HPV16 and HPV18 fused with CyaA, for use in subjects infected with HPV

(57) The invention relates to an immunotherapeutic vaccine for the treatment of infections by HPV16 or HPV 18 or both HPV16 and HPV 18 when the treated subject is selected from the group of human subjects infected with HPV16 or HPV18 or both (HPV16 and/or HPV18) and having no premalignant genital lesions related to said HPV(s), in particular no premalignant cervical lesions, and especially having no cancer related to said HPV infections, in particular no carcinomas related to said HPV infection.

EP 3 037 103 A1

SEQUENCE LISTING

<110> GENTICEL

<120> IMMUNOTHERAPEUTIC VACCINE COMPRISING E7 PROTEINS OF HPV16 AND HPV18 FUSED WITH CyaA, FOR USE IN SUBJECTS INFECTED WITH HPV

<130> B11000 - AD/KN

<140> EPXXXXXX
<141> 2014-12-23

<160> 99

<170> PatentIn version 3.5

<210> 1
<211> 17
<212> DNA
<213> Artificial Sequence

<220>
<223> Synthetic construct

<400> 1
cgtcgcaggc gtaaacg 17

<210> 2
<211> 17
<212> DNA
<213> Artificial Sequence

<220>
<223> Synthetic construct

<400> 2
acaggaggca ggtacac 17

<210> 3
<211> 17
<212> DNA
<213> Artificial Sequence

<220>
<223> Synthetic construct

<400> 3
agatgtccgt gtggcgg 17

<210> 4
<211> 23
<212> DNA
<213> Artificial Sequence

<220>
<223> Synthetic construct

<400> 4
cgagcaatta ggagagtcag agg 23

<210> 5
<211> 20
<212> DNA
<213> Artificial Sequence

<220>
<223> Synthetic construct

<400> 5
tgtgtgacgc tgtggttcat 20

<210> 6
<211> 17
<212> DNA
<213> Artificial Sequence

<220>
<223> Synthetic construct

<400> 6
aacccgacca tgcagtt 17

<210> 7
<211> 22
<212> DNA
<213> Artificial Sequence

<220>
<223> Synthetic construct

<400> 7
acgattccac aacataggag ga 22

<210> 8
<211> 24
<212> DNA
<213> Artificial Sequence

<220>
<223> Synthetic construct

<400> 8
tacacttggg tttcagtacg aggt 24

<210> 9
<211> 23
<212> DNA
<213> Artificial Sequence

<220>
<223> Synthetic construct

<400> 9
ctccaacatg ctatgcaacg tcc 23

<210> 10
<211> 21

<212> DNA
<213> Artificial Sequence

<220>
<223> Synthetic construct

<400> 10
agctcagagg aggaggatga a 21

<210> 11
<211> 23
<212> DNA
<213> Artificial Sequence

<220>
<223> Synthetic construct

<400> 11
ggttacaata ttgtaatggg ctc 23

<210> 12
<211> 16
<212> DNA
<213> Artificial Sequence

<220>
<223> Synthetic construct

<400> 12
cagctggaca agcaga 16

<210> 13
<211> 16
<212> DNA
<213> Artificial Sequence

<220>
<223> Synthetic construct

<400> 13
ccgacgagcc gaacca 16

<210> 14
<211> 25
<212> DNA
<213> Artificial Sequence

<220>
<223> Synthetic construct

<400> 14
ctcaattctg gcttcacact tacaa 25

<210> 15
<211> 16
<212> DNA
<213> Artificial Sequence

<220>
<223> Synthetic construct

<400> 15
aacgtcacac aatgtt 16

<210> 16
<211> 22
<212> DNA
<213> Artificial Sequence

<220>
<223> Synthetic construct

<400> 16
tgcatttgac tcctgaggag aa 22

<210> 17
<211> 19
<212> DNA
<213> Artificial Sequence

<220>
<223> Synthetic construct

<400> 17
gggcctcacc accaacttc 19

<210> 18
<211> 16
<212> DNA
<213> Artificial Sequence

<220>
<223> Synthetic construct

<400> 18
ctgccgttac tgccct 16

<210> 19
<211> 19
<212> DNA
<213> Artificial Sequence

<220>
<223> Synthetic construct

<400> 19
cgtcgggctg gtagttgtg 19

<210> 20
<211> 23
<212> DNA
<213> Artificial Sequence

<220>
<223> Synthetic construct

<400> 20
attgcatttg gaacctcaga atg 23

<210> 21
<211> 18
<212> DNA
<213> Artificial Sequence

<220>
<223> Synthetic construct

<400> 21
atgactaact ccatctgc 18

<210> 22
<211> 20
<212> DNA
<213> Artificial Sequence

<220>
<223> Synthetic construct

<400> 22
ccaaagagga cctgcgtgtt 20

<210> 23
<211> 20
<212> DNA
<213> Artificial Sequence

<220>
<223> Synthetic construct

<400> 23
tacttgatgc gcagagtggg 20

<210> 24
<211> 18
<212> DNA
<213> Artificial Sequence

<220>
<223> Synthetic construct

<400> 24
tacaacagct gcttatgg 18

<210> 25
<211> 23
<212> DNA
<213> Artificial Sequence

<220>
<223> Synthetic construct

<400> 25
acaacagcgt cacacaattc agt 23

<210> 26
<211> 21
<212> DNA
<213> Artificial Sequence

<220>
<223> Synthetic construct

<400> 26
cagttctacg ttccgcaggt t 21

<210> 27
<211> 29
<212> DNA
<213> Artificial Sequence

<220>
<223> Synthetic construct

<400> 27
actgcaacta gtagtagaag cgtcgcggg 29

<210> 28
<211> 16
<212> DNA
<213> Artificial Sequence

<220>
<223> Synthetic construct

<400> 28
tgtgcggcgt gttgga 16

<210> 29
<211> 22
<212> DNA
<213> Artificial Sequence

<220>
<223> Synthetic construct

<400> 29
tggcgttttt acacgtcaca gt 22

<210> 30
<211> 15
<212> DNA
<213> Artificial Sequence

<220>
<223> Synthetic construct

<400> 30
cccgacgtag agaaa 15

<210> 31
<211> 18

<212> DNA
<213> Artificial Sequence

<220>
<223> Synthetic construct

<400> 31
gtgtggaccg gccagatg 18

<210> 32
<211> 20
<212> DNA
<213> Artificial Sequence

<220>
<223> Synthetic construct

<400> 32
cgtcgcagtg ctatgaatgc 20

<210> 33
<211> 16
<212> DNA
<213> Artificial Sequence

<220>
<223> Synthetic construct

<400> 33
acaagcagaa caagcc 16

<210> 34
<211> 21
<212> DNA
<213> Artificial Sequence

<220>
<223> Synthetic construct

<400> 34
ccacggacat tgcatgattt g 21

<210> 35
<211> 23
<212> DNA
<213> Artificial Sequence

<220>
<223> Synthetic construct

<400> 35
ctttttgcat tcaacgcatt tca 23

<210> 36
<211> 26
<212> DNA
<213> Artificial Sequence

<220>
<223> Synthetic construct

<400> 36
tggagacatc tgtgcatgaa atcgaa 26

<210> 37
<211> 24
<212> DNA
<213> Artificial Sequence

<220>
<223> Synthetic construct

<400> 37
tgtgctacga gcaattacct gact 24

<210> 38
<211> 26
<212> DNA
<213> Artificial Sequence

<220>
<223> Synthetic construct

<400> 38
tgattaactc catctggttc atcttt 26

<210> 39
<211> 15
<212> DNA
<213> Artificial Sequence

<220>
<223> Synthetic construct

<400> 39
cgactccgag aatga 15

<210> 40
<211> 21
<212> DNA
<213> Artificial Sequence

<220>
<223> Synthetic construct

<400> 40
aaaaaccgct gtgtccagtt g 21

<210> 41
<211> 23
<212> DNA
<213> Artificial Sequence

<220>
<223> Synthetic construct

<400> 41
cacctcggtt tctctacgtg ttg 23

<210> 42
<211> 23
<212> DNA
<213> Artificial Sequence

<220>
<223> Synthetic construct

<400> 42
attccataac atcggtggac ggt 23

<210> 43
<211> 27
<212> DNA
<213> Artificial Sequence

<220>
<223> Synthetic construct

<400> 43
gttcataaag ctaaattgta cgtggaa 27

<210> 44
<211> 22
<212> DNA
<213> Artificial Sequence

<220>
<223> Synthetic construct

<400> 44
tgtgaatctt gtccgtccac tt 22

<210> 45
<211> 36
<212> DNA
<213> Artificial Sequence

<220>
<223> Synthetic construct

<400> 45
acaatatcct ttagggtaac atgtcttcca tgcatg 36

<210> 46
<211> 21
<212> DNA
<213> Artificial Sequence

<220>
<223> Synthetic construct

<400> 46
aaagcaaaaa ttggtggacg a 21

<210> 47
<211> 19
<212> DNA
<213> Artificial Sequence

<220>
<223> Synthetic construct

<400> 47
tgccagcaat tagcgcatt 19

<210> 48
<211> 24
<212> DNA
<213> Artificial Sequence

<220>
<223> Synthetic construct

<400> 48
catgaaatag cgggacgttg gacg 24

<210> 49
<211> 26
<212> DNA
<213> Artificial Sequence

<220>
<223> Synthetic construct

<400> 49
aacggtttca caaaatttca catatg 26

<210> 50
<211> 24
<212> DNA
<213> Artificial Sequence

<220>
<223> Synthetic construct

<400> 50
tgattcagtt gctgttgtgt gtct 24

<210> 51
<211> 21
<212> DNA
<213> Artificial Sequence

<220>
<223> Synthetic construct

<400> 51
accgggtcgt gcctgacatg c 21

<210> 52
<211> 20

<212> DNA
<213> Artificial Sequence

<220>
<223> Synthetic construct

<400> 52
gtccgttaac accggaggaa 20

<210> 53
<211> 18
<212> DNA
<213> Artificial Sequence

<220>
<223> Synthetic construct

<400> 53
cccggtccat gcatatgc 18

<210> 54
<211> 36
<212> DNA
<213> Artificial Sequence

<220>
<223> Synthetic construct

<400> 54
aacaattgca ctgtgaacat aaaagacgat ttcatt 36

<210> 55
<211> 28
<212> DNA
<213> Artificial Sequence

<220>
<223> Synthetic construct

<400> 55
gcttcataaa actaaataac cagtggaa 28

<210> 56
<211> 23
<212> DNA
<213> Artificial Sequence

<220>
<223> Synthetic construct

<400> 56
tcaggaggct gcaggtctag tac 23

<210> 57
<211> 17
<212> DNA
<213> Artificial Sequence

<220>
<223> Synthetic construct

<400> 57
cgttgcttac actgctg 17

<210> 58
<211> 22
<212> DNA
<213> Artificial Sequence

<220>
<223> Synthetic construct

<400> 58
cattgaccac acggacaaaa ag 22

<210> 59
<211> 18
<212> DNA
<213> Artificial Sequence

<220>
<223> Synthetic construct

<400> 59
cgaagtgctc gaatgcga 18

<210> 60
<211> 24
<212> DNA
<213> Artificial Sequence

<220>
<223> Synthetic construct

<400> 60
tcatttcgga tggtcaagca gcca 24

<210> 61
<211> 98
<212> PRT
<213> Artificial Sequence

<220>
<223> Protein sequence of E7 of HPV16

<400> 61

```
Met His Gly Asp Thr Pro Thr Leu His Glu Tyr Met Leu Asp Leu Gln
1               5                   10                  15

Pro Glu Thr Thr Asp Leu Tyr Cys Tyr Glu Gln Leu Asn Asp Ser Ser
            20                  25                  30

Glu Glu Glu Asp Glu Ile Asp Gly Pro Ala Gly Gln Ala Glu Pro Asp
        35                  40                  45
```

```
Arg Ala His Tyr Asn Ile Val Thr Phe Cys Cys Lys Cys Asp Ser Thr
    50                  55                  60

Leu Arg Leu Cys Val Gln Ser Thr His Val Asp Ile Arg Thr Leu Glu
65                  70                  75                  80

Asp Leu Leu Met Gly Thr Leu Gly Ile Val Cys Pro Ile Cys Ser Gln
                85                  90                  95

Lys Pro


<210>  62
<211>  105
<212>  PRT
<213>  Artificial Sequence

<220>
<223>  Protein sequence of E7 of HPV18

<400>  62

Met His Gly Pro Lys Ala Thr Leu Gln Asp Ile Val Leu His Leu Glu
1               5                   10                  15

Pro Gln Asn Glu Ile Pro Val Asp Leu Leu Cys His Glu Gln Leu Ser
            20                  25                  30

Asp Ser Glu Glu Glu Asn Asp Glu Ile Asp Gly Val Asn His Gln His
        35                  40                  45

Leu Pro Ala Arg Arg Ala Glu Pro Gln Arg His Thr Met Leu Cys Met
    50                  55                  60

Cys Cys Lys Cys Glu Ala Arg Ile Glu Leu Val Val Glu Ser Ser Ala
65                  70                  75                  80

Asp Asp Leu Arg Ala Phe Gln Gln Leu Phe Leu Asn Thr Leu Ser Phe
                85                  90                  95

Val Cys Pro Trp Cys Ala Ser Gln Gln
            100                 105


<210>  63
<211>  5121
<212>  DNA
<213>  Bordetella pertussis (wild type CyaA)

<220>
```

<221> CDS
<222> (1)..(5121)

<400> 63
```
atg cag caa tcg cat cag gct ggt tac gca aac gcc gcc gac cgg gag      48
Met Gln Gln Ser His Gln Ala Gly Tyr Ala Asn Ala Ala Asp Arg Glu
1               5                   10                  15

tct ggc atc ccc gca gcc gta ctc gat ggc atc aag gcc gtg gcg aag      96
Ser Gly Ile Pro Ala Ala Val Leu Asp Gly Ile Lys Ala Val Ala Lys
            20                  25                  30

gaa aaa aac gcc aca ttg atg ttc cgc ctg gtc aac ccc cat tcc acc      144
Glu Lys Asn Ala Thr Leu Met Phe Arg Leu Val Asn Pro His Ser Thr
        35                  40                  45

agc ctg att gcc gaa ggg gtg gcc acc aaa gga ttg ggc gtg cac gcc      192
Ser Leu Ile Ala Glu Gly Val Ala Thr Lys Gly Leu Gly Val His Ala
    50                  55                  60

aag tcg tcc gat tgg ggg ttg cag gcg ggc tac att ccc gtc aac ccg      240
Lys Ser Ser Asp Trp Gly Leu Gln Ala Gly Tyr Ile Pro Val Asn Pro
65                  70                  75                  80

aat ctt tcc aaa ctg ttc ggc cgt gcg ccc gag gtg atc gcg cgg gcc      288
Asn Leu Ser Lys Leu Phe Gly Arg Ala Pro Glu Val Ile Ala Arg Ala
                85                  90                  95

gac aac gac gtc aac agc agc ctg gcg cat ggc cat acc gcg gtc gac      336
Asp Asn Asp Val Asn Ser Ser Leu Ala His Gly His Thr Ala Val Asp
            100                 105                 110

ctg acg ctg tcg aaa gag cgg ctt gac tat ctg cgg caa gcg ggc ctg      384
Leu Thr Leu Ser Lys Glu Arg Leu Asp Tyr Leu Arg Gln Ala Gly Leu
        115                 120                 125

gtc acc ggc atg gcc gat ggc gtg gtc gcg agc aac cac gca ggc tac      432
Val Thr Gly Met Ala Asp Gly Val Val Ala Ser Asn His Ala Gly Tyr
    130                 135                 140

gag cag ttc gag ttt cgc gtg aag gaa acc tcg gac ggg cgc tat gcc      480
Glu Gln Phe Glu Phe Arg Val Lys Glu Thr Ser Asp Gly Arg Tyr Ala
145                 150                 155                 160

gtg cag tat cgc cgc aag ggc ggc gac gat ttc gag gcg gtc aag gtg      528
Val Gln Tyr Arg Arg Lys Gly Gly Asp Asp Phe Glu Ala Val Lys Val
                165                 170                 175

atc ggc aat gcc gcc ggt att cca ctg acg gcg gat atc gac atg ttc      576
Ile Gly Asn Ala Ala Gly Ile Pro Leu Thr Ala Asp Ile Asp Met Phe
            180                 185                 190

gcc att atg ccg cat ctg tcc aac ttc cgc gac tcg gcg cgc agt tcg      624
Ala Ile Met Pro His Leu Ser Asn Phe Arg Asp Ser Ala Arg Ser Ser
        195                 200                 205

gtg acc agc ggc gat tcg gtg acc gat tac ctg gcg cgc acg cgg cgg      672
Val Thr Ser Gly Asp Ser Val Thr Asp Tyr Leu Ala Arg Thr Arg Arg
    210                 215                 220

gcc gcc agc gag gcc acg ggc ggc ctg gat cgc gaa cgc atc gac ttg      720
Ala Ala Ser Glu Ala Thr Gly Gly Leu Asp Arg Glu Arg Ile Asp Leu
225                 230                 235                 240
```

```
ttg tgg aaa atc gct cgc gcc ggc gcc cgt tcc gca gtg ggc acc gag      768
Leu Trp Lys Ile Ala Arg Ala Gly Ala Arg Ser Ala Val Gly Thr Glu
                245                 250                 255

gcg cgt cgc cag ttc cgc tac gac ggc gac atg aat atc ggc gtg atc      816
Ala Arg Arg Gln Phe Arg Tyr Asp Gly Asp Met Asn Ile Gly Val Ile
            260                 265                 270

acc gat ttc gag ctg gaa gtg cgc aat gcg ctg aac agg cgg gcg cac      864
Thr Asp Phe Glu Leu Glu Val Arg Asn Ala Leu Asn Arg Arg Ala His
        275                 280                 285

gcc gtc ggc gcg cag gac gtg gtc cag cat ggc act gag cag aac aat      912
Ala Val Gly Ala Gln Asp Val Val Gln His Gly Thr Glu Gln Asn Asn
    290                 295                 300

cct ttc ccg gag gca gat gag aag att ttc gtc gta tcg gcc acc ggt      960
Pro Phe Pro Glu Ala Asp Glu Lys Ile Phe Val Val Ser Ala Thr Gly
305                 310                 315                 320

gaa agc cag atg ctc acg cgc ggg caa ctg aag gaa tac att ggc cag     1008
Glu Ser Gln Met Leu Thr Arg Gly Gln Leu Lys Glu Tyr Ile Gly Gln
                325                 330                 335

cag cgc ggc gag ggc tat gtc ttc tac gag aac cgt gca tac ggc gtg     1056
Gln Arg Gly Glu Gly Tyr Val Phe Tyr Glu Asn Arg Ala Tyr Gly Val
            340                 345                 350

gcg ggg aaa agc ctg ttc gac gat ggg ctg gga gcc gcg ccc ggc gtg     1104
Ala Gly Lys Ser Leu Phe Asp Asp Gly Leu Gly Ala Ala Pro Gly Val
        355                 360                 365

ccg agc gga cgt tcg aag ttc tcg ccg gat gta ctg gaa acg gtg ccg     1152
Pro Ser Gly Arg Ser Lys Phe Ser Pro Asp Val Leu Glu Thr Val Pro
    370                 375                 380

gcg tca ccc gga ttg cgg cgg ccg tcg ctg ggc gca gtg gaa cgc cag     1200
Ala Ser Pro Gly Leu Arg Arg Pro Ser Leu Gly Ala Val Glu Arg Gln
385                 390                 395                 400

gat tcc ggc tat gac agc ctt gat ggg gtg gga tcg cga tcg ttc tcg     1248
Asp Ser Gly Tyr Asp Ser Leu Asp Gly Val Gly Ser Arg Ser Phe Ser
                405                 410                 415

ttg ggc gag gtg tcc gac atg gcc gcc gtg gaa gcg gcg gaa ctg gaa     1296
Leu Gly Glu Val Ser Asp Met Ala Ala Val Glu Ala Ala Glu Leu Glu
            420                 425                 430

atg acc cgg caa gtc ttg cac gcc ggg gcg cgg cag gac gat gcc gag     1344
Met Thr Arg Gln Val Leu His Ala Gly Ala Arg Gln Asp Asp Ala Glu
        435                 440                 445

ccg ggc gtg agc ggt gcg tcg gcg cac tgg ggg cag cgg gcg ctg cag     1392
Pro Gly Val Ser Gly Ala Ser Ala His Trp Gly Gln Arg Ala Leu Gln
    450                 455                 460

ggc gcc cag gcg gtg gcg gcg gcg cag cgg ctg gtt cat gcc att gcc     1440
Gly Ala Gln Ala Val Ala Ala Ala Gln Arg Leu Val His Ala Ile Ala
465                 470                 475                 480

ctg atg acg caa ttc ggc cgg gcc ggt tcc acc aac acg ccg cag gaa     1488
Leu Met Thr Gln Phe Gly Arg Ala Gly Ser Thr Asn Thr Pro Gln Glu
```

```
                485                 490                 495

gcg gcc tcg ttg tcg gcg gcc gtg ttc ggc ttg ggc gag gcc agc agc      1536
Ala Ala Ser Leu Ser Ala Ala Val Phe Gly Leu Gly Glu Ala Ser Ser
            500                 505                 510

gcc gtg gcc gaa acc gtg agc ggt ttt ttc cgc ggg tct tcg cgc tgg      1584
Ala Val Ala Glu Thr Val Ser Gly Phe Phe Arg Gly Ser Ser Arg Trp
        515                 520                 525

gcc ggc ggt ttc ggc gtg gct ggc ggc gcg atg gcg ctg gga ggc ggc      1632
Ala Gly Gly Phe Gly Val Ala Gly Gly Ala Met Ala Leu Gly Gly Gly
    530                 535                 540

atc gcc gcg gcc gtt ggc gcc ggg atg tcg ttg acc gat gac gcg ccg      1680
Ile Ala Ala Ala Val Gly Ala Gly Met Ser Leu Thr Asp Asp Ala Pro
545                 550                 555                 560

gcc gga cag aag gcc gcc gcc ggc gcc gag atc gcg ctg cag ttg aca      1728
Ala Gly Gln Lys Ala Ala Ala Gly Ala Glu Ile Ala Leu Gln Leu Thr
                565                 570                 575

ggt gga acg gtc gag ctg gct tct tcc atc gcg ttg gcg ctg gcc gcg      1776
Gly Gly Thr Val Glu Leu Ala Ser Ser Ile Ala Leu Ala Leu Ala Ala
            580                 585                 590

gcg cgc ggc gtg acc agc ggc ttg cag gtg gcc ggg gcg tcg gcc ggg      1824
Ala Arg Gly Val Thr Ser Gly Leu Gln Val Ala Gly Ala Ser Ala Gly
        595                 600                 605

gcg gct gcc ggc gca ttg gcc gcg gcg ctc agt ccc atg gag atc tac      1872
Ala Ala Ala Gly Ala Leu Ala Ala Ala Leu Ser Pro Met Glu Ile Tyr
    610                 615                 620

ggc ctg gtg cag caa tcg cac tat gcg gat cag ctg gac aag ctg gcg      1920
Gly Leu Val Gln Gln Ser His Tyr Ala Asp Gln Leu Asp Lys Leu Ala
625                 630                 635                 640

cag gaa tcg agc gca tac ggt tac gag ggc gac gcc ttg ctg gcc cag      1968
Gln Glu Ser Ser Ala Tyr Gly Tyr Glu Gly Asp Ala Leu Leu Ala Gln
                645                 650                 655

ctg tat cgc gac aag acg gcc gcc gag ggc gcc gtc gcc ggc gtc tcc      2016
Leu Tyr Arg Asp Lys Thr Ala Ala Glu Gly Ala Val Ala Gly Val Ser
            660                 665                 670

gcc gtc ctg agc acg gtg ggg gcg gcg gtg tcg atc gcc gcg gcg gcc      2064
Ala Val Leu Ser Thr Val Gly Ala Ala Val Ser Ile Ala Ala Ala Ala
        675                 680                 685

agc gtg gta ggg gcc ccg gtg gcg gtg gtc act tcc ttg ctg acc ggg      2112
Ser Val Val Gly Ala Pro Val Ala Val Val Thr Ser Leu Leu Thr Gly
    690                 695                 700

gct ctc aac ggc atc ctg cgc ggc gtg cag cag ccc atc atc gaa aag      2160
Ala Leu Asn Gly Ile Leu Arg Gly Val Gln Gln Pro Ile Ile Glu Lys
705                 710                 715                 720

ctg gcc aac gat tac gct cgc aag atc gac gag ctg ggc ggg ccg caa      2208
Leu Ala Asn Asp Tyr Ala Arg Lys Ile Asp Glu Leu Gly Gly Pro Gln
                725                 730                 735

gcg tac ttc gag aaa aac ctg cag gcg cgt cac gaa caa ctg gcc aat      2256
```

```
Ala Tyr Phe Glu Lys Asn Leu Gln Ala Arg His Glu Gln Leu Ala Asn
            740                 745                 750

tcg gac ggc cta cgg aaa atg ctg gcc gac ctg cag gcc ggt tgg aac      2304
Ser Asp Gly Leu Arg Lys Met Leu Ala Asp Leu Gln Ala Gly Trp Asn
        755                 760                 765

gcc agc agc gtg atc ggg gtg cag acg aca gag atc tcc aag tcg gcg      2352
Ala Ser Ser Val Ile Gly Val Gln Thr Thr Glu Ile Ser Lys Ser Ala
    770                 775                 780

ctc gaa ctg gcc gcc att acc ggc aac gcg gac aac ctg aaa tcc gtc      2400
Leu Glu Leu Ala Ala Ile Thr Gly Asn Ala Asp Asn Leu Lys Ser Val
785                 790                 795                 800

gac gtg ttc gtg gac cgc ttc gtc cag ggc gag cgg gtg gcc ggc cag      2448
Asp Val Phe Val Asp Arg Phe Val Gln Gly Glu Arg Val Ala Gly Gln
                805                 810                 815

ccg gtg gtc ctc gac gtc gcc gcc ggc ggc atc gat atc gcc agc cgc      2496
Pro Val Val Leu Asp Val Ala Ala Gly Gly Ile Asp Ile Ala Ser Arg
            820                 825                 830

aag ggc gag cgg ccg gcg ctg acg ttc atc acg ccg ctg gcc gcg cca      2544
Lys Gly Glu Arg Pro Ala Leu Thr Phe Ile Thr Pro Leu Ala Ala Pro
        835                 840                 845

gga gaa gag cag cgc cgg cgc acg aaa acg ggc aag agc gaa ttc acc      2592
Gly Glu Glu Gln Arg Arg Arg Thr Lys Thr Gly Lys Ser Glu Phe Thr
    850                 855                 860

aca ttc gtc gag atc gtg ggc aag cag gac cgc tgg cgc atc cgg gac      2640
Thr Phe Val Glu Ile Val Gly Lys Gln Asp Arg Trp Arg Ile Arg Asp
865                 870                 875                 880

ggc gcg gcc gac acc acc atc gat ctg gcc aag gtg gtg tcg caa ctg      2688
Gly Ala Ala Asp Thr Thr Ile Asp Leu Ala Lys Val Val Ser Gln Leu
                885                 890                 895

gtc gac gcc aat ggc gtg ctc aag cac agc atc aaa ctg gat gtg atc      2736
Val Asp Ala Asn Gly Val Leu Lys His Ser Ile Lys Leu Asp Val Ile
            900                 905                 910

ggc gga gat ggc gat gac gtc gtg ctt gcc aat gct tcg cgc atc cat      2784
Gly Gly Asp Gly Asp Asp Val Val Leu Ala Asn Ala Ser Arg Ile His
        915                 920                 925

tat gac ggc ggc gcg ggc acc aac acg gtc agc tat gcc gcc ctg ggt      2832
Tyr Asp Gly Gly Ala Gly Thr Asn Thr Val Ser Tyr Ala Ala Leu Gly
    930                 935                 940

cga cag gat tcc att acc gtg tcc gcc gac ggg gaa cgt ttc aac gtg      2880
Arg Gln Asp Ser Ile Thr Val Ser Ala Asp Gly Glu Arg Phe Asn Val
945                 950                 955                 960

cgc aag cag ttg aac aac gcc aac gtg tat cgc gaa ggc gtg gct acc      2928
Arg Lys Gln Leu Asn Asn Ala Asn Val Tyr Arg Glu Gly Val Ala Thr
                965                 970                 975

cag aca acc gcc tac ggc aag cgc acg gag aat gtc caa tac cgc cat      2976
Gln Thr Thr Ala Tyr Gly Lys Arg Thr Glu Asn Val Gln Tyr Arg His
            980                 985                 990
```

```
gtc gag ctg gcc cgt gtc ggg caa  ctg gtg gag gtc gac  acg ctc gag     3024
Val Glu Leu Ala Arg Val Gly Gln  Leu Val Glu Val Asp  Thr Leu Glu
        995                1000                 1005

cat gtg  cag cac atc atc ggc  ggg gcc ggc aac gat  tcg atc acc       3069
His Val  Gln His Ile Ile Gly  Gly Ala Gly Asn Asp  Ser Ile Thr
    1010                 1015                 1020

ggc aat  gcg cac gac aac ttc  cta gcc ggc ggg tcg  ggc gac gac       3114
Gly Asn  Ala His Asp Asn Phe  Leu Ala Gly Gly Ser  Gly Asp Asp
    1025                 1030                 1035

agg ctg  gat ggc ggc gcc ggc  aac gac acc ctg gtt  ggc ggc gag       3159
Arg Leu  Asp Gly Gly Ala Gly  Asn Asp Thr Leu Val  Gly Gly Glu
    1040                 1045                 1050

ggc caa  aac acg gtc atc ggc  ggc gcc ggc gac gac  gta ttc ctg       3204
Gly Gln  Asn Thr Val Ile Gly  Gly Ala Gly Asp Asp  Val Phe Leu
    1055                 1060                 1065

cag gac  ctg ggg gta tgg agc  aac cag ctc gat ggc  ggc gcg ggc       3249
Gln Asp  Leu Gly Val Trp Ser  Asn Gln Leu Asp Gly  Gly Ala Gly
    1070                 1075                 1080

gtc gat  acc gtg aag tac aac  gtg cac cag cct tcc  gag gag cgc       3294
Val Asp  Thr Val Lys Tyr Asn  Val His Gln Pro Ser  Glu Glu Arg
    1085                 1090                 1095

ctc gaa  cgc atg ggc gac acg  ggc atc cat gcc gat  ctt caa aag       3339
Leu Glu  Arg Met Gly Asp Thr  Gly Ile His Ala Asp  Leu Gln Lys
    1100                 1105                 1110

ggc acg  gtc gag aag tgg ccg  gcc ctg aac ctg ttc  agc gtc gac       3384
Gly Thr  Val Glu Lys Trp Pro  Ala Leu Asn Leu Phe  Ser Val Asp
    1115                 1120                 1125

cat gtc  aag aat atc gag aat  ctg cac ggc tcc cgc  ctg aac gac       3429
His Val  Lys Asn Ile Glu Asn  Leu His Gly Ser Arg  Leu Asn Asp
    1130                 1135                 1140

cgc atc  gcc ggc gac gac cag  gac aac gag ctc tgg  ggc cac gat       3474
Arg Ile  Ala Gly Asp Asp Gln  Asp Asn Glu Leu Trp  Gly His Asp
    1145                 1150                 1155

ggc aac  gac acg ata cgc ggc  cgg ggc ggc gac gac  atc ctg cgc       3519
Gly Asn  Asp Thr Ile Arg Gly  Arg Gly Gly Asp Asp  Ile Leu Arg
    1160                 1165                 1170

ggc ggc  ctg ggc ctg gac acg  ctg tat ggc gag gac  ggc aac gac       3564
Gly Gly  Leu Gly Leu Asp Thr  Leu Tyr Gly Glu Asp  Gly Asn Asp
    1175                 1180                 1185

atc ttc  ctg cag gac gac gag  acc gtc agc gat gac  atc gac ggc       3609
Ile Phe  Leu Gln Asp Asp Glu  Thr Val Ser Asp Asp  Ile Asp Gly
    1190                 1195                 1200

ggc gcg  ggg ctg gac acc gtc  gac tac tcc gcc atg  atc cat cca       3654
Gly Ala  Gly Leu Asp Thr Val  Asp Tyr Ser Ala Met  Ile His Pro
    1205                 1210                 1215

ggc agg  atc gtt gcg ccg cat  gaa tac ggc ttc ggg  atc gag gcg       3699
Gly Arg  Ile Val Ala Pro His  Glu Tyr Gly Phe Gly  Ile Glu Ala
    1220                 1225                 1230
```

```
gac ctg  tcc agg gaa tgg gtg  cgc aag gcg tcc gcg  ctg ggc gtg        3744
Asp Leu  Ser Arg Glu Trp Val  Arg Lys Ala Ser Ala  Leu Gly Val
    1235                 1240                 1245

gac tat  tac gat aat gtc cgc  aat gtc gaa aac gtc  atc ggt acg        3789
Asp Tyr  Tyr Asp Asn Val Arg  Asn Val Glu Asn Val  Ile Gly Thr
    1250                 1255                 1260

agc atg  aag gat gtg ctc atc  ggc gac gcg caa gcc  aat acc ctg        3834
Ser Met  Lys Asp Val Leu Ile  Gly Asp Ala Gln Ala  Asn Thr Leu
    1265                 1270                 1275

atg ggc  cag ggc ggc gac gat  acc gtg cgc ggc ggc  gac ggc gat        3879
Met Gly  Gln Gly Gly Asp Asp  Thr Val Arg Gly Gly  Asp Gly Asp
    1280                 1285                 1290

gat ctg  ctg ttc ggc ggc gac  ggc aac gac atg ctg  tat ggc gac        3924
Asp Leu  Leu Phe Gly Gly Asp  Gly Asn Asp Met Leu  Tyr Gly Asp
    1295                 1300                 1305

gcc ggc  aac gac acc ctc tac  ggg ggg ctg ggc gac  gat acc ctt        3969
Ala Gly  Asn Asp Thr Leu Tyr  Gly Gly Leu Gly Asp  Asp Thr Leu
    1310                 1315                 1320

gaa ggc  ggc gcg ggc aac gat  tgg ttc ggc cag acg  cag gcg cgc        4014
Glu Gly  Gly Ala Gly Asn Asp  Trp Phe Gly Gln Thr  Gln Ala Arg
    1325                 1330                 1335

gag cat  gac gtg ctg cgc ggc  gga gat ggg gtg gat  acc gtc gat        4059
Glu His  Asp Val Leu Arg Gly  Gly Asp Gly Val Asp  Thr Val Asp
    1340                 1345                 1350

tac agc  cag acc ggc gcg cat  gcc ggc att gcc gcg  ggt cgc atc        4104
Tyr Ser  Gln Thr Gly Ala His  Ala Gly Ile Ala Ala  Gly Arg Ile
    1355                 1360                 1365

ggg ctg  ggc atc ctg gct gac  ctg ggc gcc ggc cgc  gtc gac aag        4149
Gly Leu  Gly Ile Leu Ala Asp  Leu Gly Ala Gly Arg  Val Asp Lys
    1370                 1375                 1380

ctg ggc  gag gcc ggc agc agc  gcc tac gat acg gtt  tcc ggt atc        4194
Leu Gly  Glu Ala Gly Ser Ser  Ala Tyr Asp Thr Val  Ser Gly Ile
    1385                 1390                 1395

gag aac  gtg gtg ggc acg gaa  ctg gcc gac cgc atc  acg ggc gat        4239
Glu Asn  Val Val Gly Thr Glu  Leu Ala Asp Arg Ile  Thr Gly Asp
    1400                 1405                 1410

gcg cag  gcc aac gtg ctg cgc  ggc gcg ggt ggc gcc  gac gtg ctt        4284
Ala Gln  Ala Asn Val Leu Arg  Gly Ala Gly Gly Ala  Asp Val Leu
    1415                 1420                 1425

gcg ggc  ggc gag ggc gac gat  gtg ctg ctg ggc ggc  gac ggc gac        4329
Ala Gly  Gly Glu Gly Asp Asp  Val Leu Leu Gly Gly  Asp Gly Asp
    1430                 1435                 1440

gac cag  ctg tcg ggc gac gcc  gga cgc gat cgc ttg  tac ggc gaa        4374
Asp Gln  Leu Ser Gly Asp Ala  Gly Arg Asp Arg Leu  Tyr Gly Glu
    1445                 1450                 1455

gcc ggt  gac gac tgg ttc ttc  cag gat gcc gcc aat  gcc ggc aat        4419
Ala Gly  Asp Asp Trp Phe Phe  Gln Asp Ala Ala Asn  Ala Gly Asn
```

```
    1460                1465                1470

ctg ctc  gac ggc ggc gac ggc  cgc gat acc gtg gat  ttc agc ggc        4464
Leu Leu  Asp Gly Gly Asp Gly  Arg Asp Thr Val Asp  Phe Ser Gly
    1475                1480                1485

ccg ggc  cgg ggc ctc gac gcc  ggc gca aag ggc gta  ttc ctg agc        4509
Pro Gly  Arg Gly Leu Asp Ala  Gly Ala Lys Gly Val  Phe Leu Ser
    1490                1495                1500

ttg ggc  aag ggg ttc gcc agc  ctg atg gac gaa ccc  gaa acc agc        4554
Leu Gly  Lys Gly Phe Ala Ser  Leu Met Asp Glu Pro  Glu Thr Ser
    1505                1510                1515

aac gtg  ttg cgc aat atc gag  aac gcc gtg ggc agc  gcg cgt gat        4599
Asn Val  Leu Arg Asn Ile Glu  Asn Ala Val Gly Ser  Ala Arg Asp
    1520                1525                1530

gac gtg  ctg atc ggc gac gca  ggc gcc aac gtc ctc  aat ggc ctg        4644
Asp Val  Leu Ile Gly Asp Ala  Gly Ala Asn Val Leu  Asn Gly Leu
    1535                1540                1545

gcg ggc  aac gac gtg ctg tcc  ggc ggc gct ggc gac  gat gtg ctg        4689
Ala Gly  Asn Asp Val Leu Ser  Gly Gly Ala Gly Asp  Asp Val Leu
    1550                1555                1560

ctg ggc  gac gag ggc tcg gac  ctg ctc agc ggc gat  gcg ggc aac        4734
Leu Gly  Asp Glu Gly Ser Asp  Leu Leu Ser Gly Asp  Ala Gly Asn
    1565                1570                1575

gac gat  ctg ttc ggc ggg cag  ggc gat gat act tat  ctg ttc ggg        4779
Asp Asp  Leu Phe Gly Gly Gln  Gly Asp Asp Thr Tyr  Leu Phe Gly
    1580                1585                1590

gtc ggg  tac ggg cac gac acg  atc tac gaa tcg ggc  ggc ggc cat        4824
Val Gly  Tyr Gly His Asp Thr  Ile Tyr Glu Ser Gly  Gly Gly His
    1595                1600                1605

gac acc  atc cgc atc aac gcg  ggg gcg gac cag ctg  tgg ttc gcg        4869
Asp Thr  Ile Arg Ile Asn Ala  Gly Ala Asp Gln Leu  Trp Phe Ala
    1610                1615                1620

cgc cag  ggc aac gac ctg gag  atc cgc att ctc ggc  acc gac gat        4914
Arg Gln  Gly Asn Asp Leu Glu  Ile Arg Ile Leu Gly  Thr Asp Asp
    1625                1630                1635

gca ctt  acc gtg cac gac tgg  tat cgc gac gcc gat  cac cgg gtg        4959
Ala Leu  Thr Val His Asp Trp  Tyr Arg Asp Ala Asp  His Arg Val
    1640                1645                1650

gaa atc  atc cat gcc gcc aac  cag gcg gta gac cag  gca ggc atc        5004
Glu Ile  Ile His Ala Ala Asn  Gln Ala Val Asp Gln  Ala Gly Ile
    1655                1660                1665

gaa aag  ctg gtc gag gca atg  gcg cag tat ccg gac  ccc ggc gcg        5049
Glu Lys  Leu Val Glu Ala Met  Ala Gln Tyr Pro Asp  Pro Gly Ala
    1670                1675                1680

gcg gcg  gct gcc ccg ccg gcg  gcg cgc gtg ccg gac  acg ctg atg        5094
Ala Ala  Ala Ala Pro Pro Ala  Ala Arg Val Pro Asp  Thr Leu Met
    1685                1690                1695

cag tcc  ctg gct gtc aac tgg  cgc tga                                 5121
```

Gln Ser Leu Ala Val Asn Trp Arg
    1700                1705

<210> 64
<211> 1706
<212> PRT
<213> Bordetella pertussis (wild type CyaA)

<400> 64

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

```
Val Thr Ser Gly Asp Ser Val Thr Asp Tyr Leu Ala Arg Thr Arg Arg
    210                 215                 220

Ala Ala Ser Glu Ala Thr Gly Gly Leu Asp Arg Glu Arg Ile Asp Leu
225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly
305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Ser Gly Arg Ser Lys Phe Ser Pro Asp Val Leu Glu Thr Val Pro
    370                 375                 380

Ala Ser Pro Gly Leu Arg Arg Pro Ser Leu Gly Ala Val Glu Arg Gln
385                 390                 395                 400

Asp Ser Gly Tyr Asp Ser Leu Asp Gly Val Gly Ser Arg Ser Phe Ser
                405                 410                 415

Leu Gly Glu Val Ser Asp Met Ala Ala Val Glu Ala Ala Glu Leu Glu
            420                 425                 430

Met Thr Arg Gln Val Leu His Ala Gly Ala Arg Gln Asp Asp Ala Glu
        435                 440                 445

Pro Gly Val Ser Gly Ala Ser Ala His Trp Gly Gln Arg Ala Leu Gln
    450                 455                 460
```

```
Gly Ala Gln Ala Val Ala Ala Ala Gln Arg Leu Val His Ala Ile Ala
465                 470                 475                 480

Leu Met Thr Gln Phe Gly Arg Ala Gly Ser Thr Asn Thr Pro Gln Glu
                485                 490                 495

Ala Ala Ser Leu Ser Ala Ala Val Phe Gly Leu Gly Glu Ala Ser Ser
            500                 505                 510

Ala Val Ala Glu Thr Val Ser Gly Phe Phe Arg Gly Ser Ser Arg Trp
        515                 520                 525

Ala Gly Gly Phe Gly Val Ala Gly Gly Ala Met Ala Leu Gly Gly Gly
    530                 535                 540

Ile Ala Ala Ala Val Gly Ala Gly Met Ser Leu Thr Asp Asp Ala Pro
545                 550                 555                 560

Ala Gly Gln Lys Ala Ala Ala Gly Ala Glu Ile Ala Leu Gln Leu Thr
                565                 570                 575

Gly Gly Thr Val Glu Leu Ala Ser Ser Ile Ala Leu Ala Leu Ala Ala
            580                 585                 590

Ala Arg Gly Val Thr Ser Gly Leu Gln Val Ala Gly Ala Ser Ala Gly
        595                 600                 605

Ala Ala Ala Gly Ala Leu Ala Ala Ala Leu Ser Pro Met Glu Ile Tyr
    610                 615                 620

Gly Leu Val Gln Gln Ser His Tyr Ala Asp Gln Leu Asp Lys Leu Ala
625                 630                 635                 640

Gln Glu Ser Ser Ala Tyr Gly Tyr Glu Gly Asp Ala Leu Leu Ala Gln
                645                 650                 655

Leu Tyr Arg Asp Lys Thr Ala Ala Glu Gly Ala Val Ala Gly Val Ser
            660                 665                 670

Ala Val Leu Ser Thr Val Gly Ala Ala Val Ser Ile Ala Ala Ala Ala
        675                 680                 685

Ser Val Val Gly Ala Pro Val Ala Val Val Thr Ser Leu Leu Thr Gly
    690                 695                 700

Ala Leu Asn Gly Ile Leu Arg Gly Val Gln Gln Pro Ile Ile Glu Lys
705                 710                 715                 720
```

Leu Ala Asn Asp Tyr Ala Arg Lys Ile Asp Glu Leu Gly Gly Pro Gln
725 730 735

Ala Tyr Phe Glu Lys Asn Leu Gln Ala Arg His Glu Gln Leu Ala Asn
740 745 750

Ser Asp Gly Leu Arg Lys Met Leu Ala Asp Leu Gln Ala Gly Trp Asn
755 760 765

Ala Ser Ser Val Ile Gly Val Gln Thr Thr Glu Ile Ser Lys Ser Ala
770 775 780

Leu Glu Leu Ala Ala Ile Thr Gly Asn Ala Asp Asn Leu Lys Ser Val
785 790 795 800

Asp Val Phe Val Asp Arg Phe Val Gln Gly Glu Arg Val Ala Gly Gln
805 810 815

Pro Val Val Leu Asp Val Ala Ala Gly Gly Ile Asp Ile Ala Ser Arg
820 825 830

Lys Gly Glu Arg Pro Ala Leu Thr Phe Ile Thr Pro Leu Ala Ala Pro
835 840 845

Gly Glu Glu Gln Arg Arg Arg Thr Lys Thr Gly Lys Ser Glu Phe Thr
850 855 860

Thr Phe Val Glu Ile Val Gly Lys Gln Asp Arg Trp Arg Ile Arg Asp
865 870 875 880

Gly Ala Ala Asp Thr Thr Ile Asp Leu Ala Lys Val Val Ser Gln Leu
885 890 895

Val Asp Ala Asn Gly Val Leu Lys His Ser Ile Lys Leu Asp Val Ile
900 905 910

Gly Gly Asp Gly Asp Asp Val Val Leu Ala Asn Ala Ser Arg Ile His
915 920 925

Tyr Asp Gly Gly Ala Gly Thr Asn Thr Val Ser Tyr Ala Ala Leu Gly
930 935 940

Arg Gln Asp Ser Ile Thr Val Ser Ala Asp Gly Glu Arg Phe Asn Val
945 950 955 960

Arg Lys Gln Leu Asn Asn Ala Asn Val Tyr Arg Glu Gly Val Ala Thr

965 970 975

Gln Thr Thr Ala Tyr Gly Lys Arg Thr Glu Asn Val Gln Tyr Arg His
980 985 990

Val Glu Leu Ala Arg Val Gly Gln Leu Val Glu Val Asp Thr Leu Glu
995 1000 1005

His Val Gln His Ile Ile Gly Gly Ala Gly Asn Asp Ser Ile Thr
1010 1015 1020

Gly Asn Ala His Asp Asn Phe Leu Ala Gly Gly Ser Gly Asp Asp
1025 1030 1035

Arg Leu Asp Gly Gly Ala Gly Asn Asp Thr Leu Val Gly Gly Glu
1040 1045 1050

Gly Gln Asn Thr Val Ile Gly Gly Ala Gly Asp Asp Val Phe Leu
1055 1060 1065

Gln Asp Leu Gly Val Trp Ser Asn Gln Leu Asp Gly Gly Ala Gly
1070 1075 1080

Val Asp Thr Val Lys Tyr Asn Val His Gln Pro Ser Glu Glu Arg
1085 1090 1095

Leu Glu Arg Met Gly Asp Thr Gly Ile His Ala Asp Leu Gln Lys
1100 1105 1110

Gly Thr Val Glu Lys Trp Pro Ala Leu Asn Leu Phe Ser Val Asp
1115 1120 1125

His Val Lys Asn Ile Glu Asn Leu His Gly Ser Arg Leu Asn Asp
1130 1135 1140

Arg Ile Ala Gly Asp Asp Gln Asp Asn Glu Leu Trp Gly His Asp
1145 1150 1155

Gly Asn Asp Thr Ile Arg Gly Arg Gly Gly Asp Asp Ile Leu Arg
1160 1165 1170

Gly Gly Leu Gly Leu Asp Thr Leu Tyr Gly Glu Asp Gly Asn Asp
1175 1180 1185

Ile Phe Leu Gln Asp Asp Glu Thr Val Ser Asp Asp Ile Asp Gly
1190 1195 1200

```
Gly Ala  Gly Leu Asp Thr Val  Asp Tyr Ser Ala Met  Ile His Pro
    1205                 1210                 1215

Gly Arg  Ile Val Ala Pro His  Glu Tyr Gly Phe Gly  Ile Glu Ala
    1220                 1225                 1230

Asp Leu  Ser Arg Glu Trp Val  Arg Lys Ala Ser Ala  Leu Gly Val
    1235                 1240                 1245

Asp Tyr  Tyr Asp Asn Val Arg  Asn Val Glu Asn Val  Ile Gly Thr
    1250                 1255                 1260

Ser Met  Lys Asp Val Leu Ile  Gly Asp Ala Gln Ala  Asn Thr Leu
    1265                 1270                 1275

Met Gly  Gln Gly Gly Asp Asp  Thr Val Arg Gly Gly  Asp Gly Asp
    1280                 1285                 1290

Asp Leu  Leu Phe Gly Gly Asp  Gly Asn Asp Met Leu  Tyr Gly Asp
    1295                 1300                 1305

Ala Gly  Asn Asp Thr Leu Tyr  Gly Gly Leu Gly Asp  Asp Thr Leu
    1310                 1315                 1320

Glu Gly  Gly Ala Gly Asn Asp  Trp Phe Gly Gln Thr  Gln Ala Arg
    1325                 1330                 1335

Glu His  Asp Val Leu Arg Gly  Gly Asp Gly Val Asp  Thr Val Asp
    1340                 1345                 1350

Tyr Ser  Gln Thr Gly Ala His  Ala Gly Ile Ala Ala  Gly Arg Ile
    1355                 1360                 1365

Gly Leu  Gly Ile Leu Ala Asp  Leu Gly Ala Gly Arg  Val Asp Lys
    1370                 1375                 1380

Leu Gly  Glu Ala Gly Ser Ser  Ala Tyr Asp Thr Val  Ser Gly Ile
    1385                 1390                 1395

Glu Asn  Val Val Gly Thr Glu  Leu Ala Asp Arg Ile  Thr Gly Asp
    1400                 1405                 1410

Ala Gln  Ala Asn Val Leu Arg  Gly Ala Gly Gly Ala  Asp Val Leu
    1415                 1420                 1425

Ala Gly  Gly Glu Gly Asp Asp  Val Leu Leu Gly Gly  Asp Gly Asp
    1430                 1435                 1440
```

Asp Gln Leu Ser Gly Asp Ala Gly Arg Asp Arg Leu Tyr Gly Glu
1445 1450 1455

Ala Gly Asp Asp Trp Phe Phe Gln Asp Ala Ala Asn Ala Gly Asn
1460 1465 1470

Leu Leu Asp Gly Gly Asp Gly Arg Asp Thr Val Asp Phe Ser Gly
1475 1480 1485

Pro Gly Arg Gly Leu Asp Ala Gly Ala Lys Gly Val Phe Leu Ser
1490 1495 1500

Leu Gly Lys Gly Phe Ala Ser Leu Met Asp Glu Pro Glu Thr Ser
1505 1510 1515

Asn Val Leu Arg Asn Ile Glu Asn Ala Val Gly Ser Ala Arg Asp
1520 1525 1530

Asp Val Leu Ile Gly Asp Ala Gly Ala Asn Val Leu Asn Gly Leu
1535 1540 1545

Ala Gly Asn Asp Val Leu Ser Gly Gly Ala Gly Asp Asp Val Leu
1550 1555 1560

Leu Gly Asp Glu Gly Ser Asp Leu Leu Ser Gly Asp Ala Gly Asn
1565 1570 1575

Asp Asp Leu Phe Gly Gly Gln Gly Asp Asp Thr Tyr Leu Phe Gly
1580 1585 1590

Val Gly Tyr Gly His Asp Thr Ile Tyr Glu Ser Gly Gly Gly His
1595 1600 1605

Asp Thr Ile Arg Ile Asn Ala Gly Ala Asp Gln Leu Trp Phe Ala
1610 1615 1620

Arg Gln Gly Asn Asp Leu Glu Ile Arg Ile Leu Gly Thr Asp Asp
1625 1630 1635

Ala Leu Thr Val His Asp Trp Tyr Arg Asp Ala Asp His Arg Val
1640 1645 1650

Glu Ile Ile His Ala Ala Asn Gln Ala Val Asp Gln Ala Gly Ile
1655 1660 1665

Glu Lys Leu Val Glu Ala Met Ala Gln Tyr Pro Asp Pro Gly Ala
1670 1675 1680

```
Ala Ala  Ala Ala Pro Pro Ala  Ala Arg Val Pro Asp  Thr Leu Met
    1685                1690                1695

Gln Ser  Leu Ala Val Asn Trp  Arg
    1700                1705

<210>  65
<211>  5131
<212>  DNA
<213>  Artificial Sequence

<220>
<223>  Optimised CyAA

<400>  65
catatgcagc agtcccatca ggccggttac gcaaacgcag ccgatcgtga atctggtatc      60

ccggcagcgg ttctggatgg tattaaggca gttgccaaag agaagaacgc taccctgatg     120

ttccgtctgg tcaacccaca ctctacttcc ctgatcgcgg aaggtgtggc gaccaaaggc     180

ctgggtgtgc acgccaaaag cagcgactgg ggtctgcagg caggttatat tccggtgaac     240

ccgaatctgt ctaaactgtt cggtcgtgcc ccggaggtga tcgcacgtgc agacaacgat     300

gtcaactctt ctctggcgca tggtcacacc gcggttgatc tgaccctgtc caaagaacgc     360

ctggattacc tgcgtcaggc gggcctggtg acgggcatgg ccgacggtgt tgtagcgagc     420

aaccacgcgg gttatgaaca atttgagttt cgcgttaaag agacctctga cggtcgttac     480

gcggtgcagt accgccgcaa aggtggcgat gactttgaag ctgtcaaagt gatcggtaac     540

gctgctggaa ttccgctgac cgcggatatc gacatgtttg ccatcatgcc tcacctgtcc     600

aactttcgcg attccgcacg cagctctgtg acgtctggtg attccgtcac tgactacctg     660

gctcgtaccc gtcgtgcggc ctctgaagcc actggtggtc tggatcgtga acgcattgac     720

ctgctgtgga aaatcgcccg tgccggtgcg cgctctgctg ttggtaccga agcacgccgc     780

caatttcgtt acgacggcga tatgaacatc ggtgtgatca cggattttga actggaagtt     840

cgcaatgccc tgaaccgtcg tgcacacgcg gtcggcgcgc aggacgttgt tcagcacggt     900

acggagcaga ataacccgtt cccagaagcg gacgaaaaga tctttgttgt gtctgccacc     960

ggtgaatccc agatgctgac tcgtggccag ctgaaagaat acatcggcca acagcgtggc    1020

gaaggctacg ttttctacga gaatcgtgct tacggcgttg cgggcaaatc cctgttcgac    1080

gatggtctgg gcgctgcacc gggcgttccg agcggccgtt ctaaattcag cccggacgta    1140

ctggaaactg tgccggcttc cccgggcctg cgccgtccgt ccctgggcgc agtcgaacgt    1200

caggactccg gctacgattc cctggatggc gttggctccc gctccttctc cctgggcgag    1260

gtttctgaca tggctgcggt ggaagcggca gagctggaaa tgacccgtca agtgctgcac    1320
```

gcgggcgcac gtcaggacga tgctgagccg ggcgtgtccg gtgcgtctgc acactggggt 1380
caacgtgccc tgcagggtgc tcaagctgtc gcggcagcgc agcgtctggt acatgcgatc 1440
gcactgatga cccagttcgg tcgcgcaggt tctaccaaca ctcctcagga agcagctagc 1500
ctgtctgctg ctgttttcgg tctgggcgaa gcgtcttctg cggttgcaga aaccgtgagc 1560
ggtttcttcc gtggttctag ccgctgggcg ggtggttttg gtgtagcagg tggcgctatg 1620
gccctgggtg gcggtatcgc ggctgctgtc ggcgcaggta tgagcctgac cgacgatgct 1680
cctgcgggcc agaaagccgc agctggcgct gaaatcgcgc tgcagctgac tggtggtacc 1740
gttgaactgg ctagctctat cgcgctggct ctggcagcgg cacgtggcgt gacttctggc 1800
ctgcaagtcg ccggtgcctc tgcgggcgct gctgcgggcg ctctggcagc tgcactgtcc 1860
cccatggaaa tctacggtct ggtacagcag tctcactacg cagaccagct ggataaactg 1920
gcgcaggagt cttctgcata cggttacgaa ggcgacgcac tgctggcgca gctgtatcgt 1980
gacaaaactg ctgctgaagg tgctgtggca ggcgtttctg cggtactgtc taccgttggc 2040
gctgcggttt ctattgcggc tgccgcatcc gttgtaggtg cgccggttgc tgtagttacc 2100
tctctgctga ctggtgcact gaacggtatc ctgcgtggtg ttcagcagcc tattatcgaa 2160
aaactggcga atgattatgc ccgtaaaatc gacgaactgg gcggtccgca ggcttatttt 2220
gaaaaaaacc tgcaggcgcg ccacgaacag ctggcaaaca gcgacggcct gcgcaaaatg 2280
ctggcagacc tgcaagctgg ttggaacgcg tcttctgtga tcggcgtgca gaccaccgaa 2340
attagcaaat ctgcgctgga actggctgca attactggca acgcggataa cctgaaaagc 2400
gttgatgttt ttgtcgatcg cttcgttcag ggcgagcgcg ttgctggtca gccggttgtt 2460
ctggacgttg cggcaggcgg catcgacatc gctagccgca agggcgagcg tccggctctg 2520
actttcatta ccccgctggc agctccgggt gaagaacagc gtcgccgtac caagactggt 2580
aaaagcgagt tcaccacctt cgtggaaatc gtaggtaaac aggaccgctg gcgtattcgc 2640
gacggtgcgg cggacaccac catcgacctg gcaaaagttg tttcccaact ggtagatgct 2700
aacggcgtgc tgaaacattc tattaaactg gatgtaatcg gcggtgacgg cgatgacgtt 2760
gtactggcga acgcgtctcg tatccactat gacggcggcg caggtaccaa cacggtttcc 2820
tacgcagcgc tgggccgcca ggattccatc actgttagcg ccgatggcga acgtttcaac 2880
gtgcgtaaac agctgaataa cgcaaacgtc tatcgtgagg gtgtagctac ccagaccacc 2940
gcgtacggta agcgtaccga aaacgttcag tatcgtcacg tggaactggc acgtgtgggt 3000
cagctggtag aggtggatac gctggaacac gtgcagcaca tcatcggtgg cgccggtaac 3060
gattccatta ctggtaacgc tcacgacaac ttcctggcag gtggttctgg tgatgatcgt 3120
ctggacggtg gtgcgggtaa cgatacgctg gtaggcggcg aaggtcagaa caccgtaatt 3180
ggtggtgccg gtgacgacgt tttcctgcag gatctgggcg tttggagcaa ccagctggat 3240

ggcggtgccg gtgttgacac ggttaagtat aacgttcacc agccgtctga agagcgcctg 3300
gaacgtatgg gtgacactgg tattcatgcc gatctgcaga aaggcactgt tgaaaaatgg 3360
cctgcgctga acctgttctc tgtggaccat gttaagaaca ttgaaaacct gcatggctct 3420
cgcctgaacg accgtatcgc tggtgacgac caggataacg aactgtgggg tcatgacggc 3480
aatgatacca ttcgcggtcg tggcggcgac gatattctgc gtggcggtct gggcctggat 3540
accctgtatg gtgaagatgg taacgatatt ttcctgcaag acgatgaaac ggtatctgat 3600
gacatcgacg gtggtgcagg cctggacacc gtagattaca gcgctatgat ccatccgggt 3660
cgtatcgtag ctccgcacga gtacggcttc ggtatcgagg cggacctgtc tcgtgaatgg 3720
gttcgtaaag cgtccgcgct gggtgtggac tactacgata acgttcgtaa cgtggaaaac 3780
gtgatcggta cctccatgaa agacgtgctg attggcgacg cacaggccaa cactctgatg 3840
ggccagggcg gtgatgacac ggttcgcggc ggcgacggtg atgacctgct gttcggtggt 3900
gacggcaacg acatgctgta cggcgacgcg ggcaacgaca ccctgtacgg cggtctgggt 3960
gacgacactc tggaaggtgg tgcaggtaac gactggttcg gccaaaccca ggcacgcgaa 4020
cacgacgtgc tgcgtggtgg cgacggcgta gacaccgtgg attactccca aactggcgct 4080
cacgcgggta tcgcggccgg tcgtatcggt ctgggcattc tggccgatct gggtgccggc 4140
cgtgtcgaca aactgggtga ggctggctct agcgcctatg atactgtttc cggcatcgaa 4200
aacgtggtag gcactgaact ggcagatcgt atcacgggtg acgcgcaagc gaacgttctg 4260
cgtggtgctg gcggtgcaga cgtgctggcg ggtggtgaag gcgacgacgt actgctgggc 4320
ggtgacggtg acgatcagct gagcggtgat gcgggtcgtg accgtctgta cggtgaagca 4380
ggtgacgatt ggttcttcca ggacgctgct aacgctggca acctgctgga cggcggcgat 4440
ggccgtgaca ctgttgactt cagcggtccg ggtcgcggtc tggacgcagg cgcgaaaggc 4500
gttttcctga gcctgggtaa gggtttcgca tctctgatgg acgaaccaga aaccagcaat 4560
gtcctgcgta acatcgaaaa cgctgtcggt tccgcacgcg atgatgttct gattggcgat 4620
gctggtgcca atgttctgaa cggtctggcg ggtaatgatg tactgtctgg tggcgctggt 4680
gatgatgtcc tgctgggtga tgagggcagc gatctgctgt ccggcgatgc cggcaacgac 4740
gatctgttcg gtggccaagg tgatgatact tacctgttcg gcgttggcta tggtcatgac 4800
accatctatg agtctggtgg cggccacgat acgattcgta ttaatgcagg tgctgaccaa 4860
ctgtggtttg cacgtcaggg taacgatctg gaaattcgca tcctgggtac tgatgacgct 4920
ctgaccgtac acgactggta ccgcgatgcg gatcaccgcg tagaaatcat ccatgcggct 4980
aaccaagctg tagaccaggc tggtattgaa aaactggtag aagcgatggc acagtatccg 5040
gacccaggtg cggctgctgc agcacctcca gcggctcgtg tgccggatac cctgatgcag 5100

agcctggccg tcaattggcg ttaatggatc c 5131

<210> 66
<211> 5382
<212> DNA
<213> Artificial Sequence

<220>
<223> E7-CyaA HPV16

<220>
<221> CDS
<222> (1)..(5382)

<220>
<221> misc_feature
<222> (685)..(850)
<223> E7 protein fragment

<220>
<221> misc_feature
<222> (1123)..(1207)
<223> E7 protein fragment

<400> 66
```
atg cag caa tcg cat cag gct ggt tac gca aac gcc gcc gac cgg gag       48
Met Gln Gln Ser His Gln Ala Gly Tyr Ala Asn Ala Ala Asp Arg Glu
1               5                   10                  15

tct ggc atc ccc gca gcc gta ctc gat ggc atc aag gcc gtg gcg aag       96
Ser Gly Ile Pro Ala Ala Val Leu Asp Gly Ile Lys Ala Val Ala Lys
            20                  25                  30

gaa aaa aac gcc aca ttg atg ttc cgc ctg gtc aac ccc cat tcc acc      144
Glu Lys Asn Ala Thr Leu Met Phe Arg Leu Val Asn Pro His Ser Thr
        35                  40                  45

agc ctg att gcc gaa ggg gtg gcc acc aaa gga ttg ggc gtg cac gcc      192
Ser Leu Ile Ala Glu Gly Val Ala Thr Lys Gly Leu Gly Val His Ala
    50                  55                  60

aag tcg tcc gat tgg ggg ttg cag gcg ggc tac att ccc gtc aac ccg      240
Lys Ser Ser Asp Trp Gly Leu Gln Ala Gly Tyr Ile Pro Val Asn Pro
65                  70                  75                  80

aat ctt tcc aaa ctg ttc ggc cgt gcg ccc gag gtg atc gcg cgg gcc      288
Asn Leu Ser Lys Leu Phe Gly Arg Ala Pro Glu Val Ile Ala Arg Ala
                85                  90                  95

gac aac gac gtc aac agc agc ctg gcg cat ggc cat acc gcg gtc gac      336
Asp Asn Asp Val Asn Ser Ser Leu Ala His Gly His Thr Ala Val Asp
            100                 105                 110

ctg acg ctg tcg aaa gag cgg ctt gac tat ctg cgg caa gcg ggc ctg      384
Leu Thr Leu Ser Lys Glu Arg Leu Asp Tyr Leu Arg Gln Ala Gly Leu
        115                 120                 125

gtc acc ggc atg gcc gat ggc gtg gtc gcg agc aac cac gca ggc tac      432
Val Thr Gly Met Ala Asp Gly Val Val Ala Ser Asn His Ala Gly Tyr
    130                 135                 140
```

```
gag cag ttc gag ttt cgc gtg aag gaa acc tcg gac ggg cgc tat gcc        480
Glu Gln Phe Glu Phe Arg Val Lys Glu Thr Ser Asp Gly Arg Tyr Ala
145                 150                 155                 160

gtg cag tat cgc cgc aag ggc ggc gac gat ttc gag gcg gtc aag gtg        528
Val Gln Tyr Arg Arg Lys Gly Gly Asp Asp Phe Glu Ala Val Lys Val
                165                 170                 175

atc ggc aat gcc gcc ggt att cca ctg acg gcg gat ctg cag atc gac        576
Ile Gly Asn Ala Ala Gly Ile Pro Leu Thr Ala Asp Leu Gln Ile Asp
            180                 185                 190

atg ttc gcc att atg ccg cat ctg tcc aac ttc cgc gac tcg gcg cgc        624
Met Phe Ala Ile Met Pro His Leu Ser Asn Phe Arg Asp Ser Ala Arg
        195                 200                 205

agt tcg gtg acc agc ggc gat tcg gtg acc gat tac ctg gcg cgt acg        672
Ser Ser Val Thr Ser Gly Asp Ser Val Thr Asp Tyr Leu Ala Arg Thr
    210                 215                 220

cgt cgt gct agc ggt caa gca gaa ccg gac aga gcc cac tat aat atc        720
Arg Arg Ala Ser Gly Gln Ala Glu Pro Asp Arg Ala His Tyr Asn Ile
225                 230                 235                 240

gtc aca ttt tgc tgt aaa tgc gac tca act ctc cgt ttg tgt gta cag        768
Val Thr Phe Cys Cys Lys Cys Asp Ser Thr Leu Arg Leu Cys Val Gln
                245                 250                 255

agc acc cat gtg gac atc cgc acc ctg gag gat tta ctg atg ggt act        816
Ser Thr His Val Asp Ile Arg Thr Leu Glu Asp Leu Leu Met Gly Thr
            260                 265                 270

ctg ggg att gtt tgt ccg att tgc tct caa aaa cct ggt acc cgc gca        864
Leu Gly Ile Val Cys Pro Ile Cys Ser Gln Lys Pro Gly Thr Arg Ala
        275                 280                 285

cgc ctt aag ttg ttg tgg aaa atc gct cgc gcc ggc gcc cgt tcc gca        912
Arg Leu Lys Leu Leu Trp Lys Ile Ala Arg Ala Gly Ala Arg Ser Ala
    290                 295                 300

gtg ggc acc gag gcg cgt cgc cag ttc cgc tac gac ggc gac atg aat        960
Val Gly Thr Glu Ala Arg Arg Gln Phe Arg Tyr Asp Gly Asp Met Asn
305                 310                 315                 320

atc ggc gtg atc acc gat ttc gag ctg gaa gtg cgc aat gcg ctg aac       1008
Ile Gly Val Ile Thr Asp Phe Glu Leu Glu Val Arg Asn Ala Leu Asn
                325                 330                 335

agg cgg gcg cac gcc gtc ggc gcg cag gac gtg gtc cag cat ggc act       1056
Arg Arg Ala His Ala Val Gly Ala Gln Asp Val Val Gln His Gly Thr
            340                 345                 350

gag cag aac aat cct ttc ccg gag gca gat gag aag att ttc gtc gta       1104
Glu Gln Asn Asn Pro Phe Pro Glu Ala Asp Glu Lys Ile Phe Val Val
        355                 360                 365

tcg gcc acc ggt aaa cgt atg cac ggc gat act ccg acg ctt cat gaa       1152
Ser Ala Thr Gly Lys Arg Met His Gly Asp Thr Pro Thr Leu His Glu
    370                 375                 380

tac atg ttg gat ctg cag cca gaa acg acc gac tta tat tgc tac gag       1200
Tyr Met Leu Asp Leu Gln Pro Glu Thr Thr Asp Leu Tyr Cys Tyr Glu
385                 390                 395                 400
```

```
cag ctg aac aaa cgt act agt gaa agc cag atg ctc acg cgc ggg caa        1248
Gln Leu Asn Lys Arg Thr Ser Glu Ser Gln Met Leu Thr Arg Gly Gln
                405                 410                 415

ctg aag gaa tac att ggc cag cag cgc ggc gag ggc tat gtc ttc tac        1296
Leu Lys Glu Tyr Ile Gly Gln Gln Arg Gly Glu Gly Tyr Val Phe Tyr
            420                 425                 430

gag aac cgt gca tac ggc gtg gcg ggg aaa agc ctg ttc gac gat ggg        1344
Glu Asn Arg Ala Tyr Gly Val Ala Gly Lys Ser Leu Phe Asp Asp Gly
        435                 440                 445

ctg gga gcc gcg ccc ggc gtg ccg agc gga cgt tcg aag ttc tcg ccg        1392
Leu Gly Ala Ala Pro Gly Val Pro Ser Gly Arg Ser Lys Phe Ser Pro
    450                 455                 460

gat gta ctg gaa acg gtg ccg gcg tca ccc gga ttg cgg cgg ccg tcg        1440
Asp Val Leu Glu Thr Val Pro Ala Ser Pro Gly Leu Arg Arg Pro Ser
465                 470                 475                 480

ctg ggc gca gtg gaa cgc cag gat tcc ggc tat gac agc ctt gat ggg        1488
Leu Gly Ala Val Glu Arg Gln Asp Ser Gly Tyr Asp Ser Leu Asp Gly
                485                 490                 495

gtg gga tcg cga tcg ttc tcg ttg ggc gag gtg tcc gac atg gcc gcc        1536
Val Gly Ser Arg Ser Phe Ser Leu Gly Glu Val Ser Asp Met Ala Ala
            500                 505                 510

gtg gaa gcg gcg gaa ctg gaa atg acc cgg caa gtc ttg cac gcc ggg        1584
Val Glu Ala Ala Glu Leu Glu Met Thr Arg Gln Val Leu His Ala Gly
        515                 520                 525

gcg cgg cag gac gat gcc gag ccg ggc gtg agc ggt gcg tcg gcg cac        1632
Ala Arg Gln Asp Asp Ala Glu Pro Gly Val Ser Gly Ala Ser Ala His
    530                 535                 540

tgg ggg cag cgg gcg ctg cag ggc gcc cag gcg gtg gcg gcg gcg cag        1680
Trp Gly Gln Arg Ala Leu Gln Gly Ala Gln Ala Val Ala Ala Ala Gln
545                 550                 555                 560

cgg ctg gtt cat gcc att gcc ctg atg acg caa ttc ggc cgg gcc ggt        1728
Arg Leu Val His Ala Ile Ala Leu Met Thr Gln Phe Gly Arg Ala Gly
                565                 570                 575

tcc acc aac acg ccg cag gaa gcg gcc tcg ttg tcg gcg gcc gtg ttc        1776
Ser Thr Asn Thr Pro Gln Glu Ala Ala Ser Leu Ser Ala Ala Val Phe
            580                 585                 590

ggc ttg ggc gag gcc agc agc gcc gtg gcc gaa acc gtg agc ggt ttt        1824
Gly Leu Gly Glu Ala Ser Ser Ala Val Ala Glu Thr Val Ser Gly Phe
        595                 600                 605

ttc cgc ggg tct tcg cgc tgg gcc ggc ggt ttc ggc gtg gct ggc ggc        1872
Phe Arg Gly Ser Ser Arg Trp Ala Gly Gly Phe Gly Val Ala Gly Gly
    610                 615                 620

gcg atg gcg ctg gga ggc ggc atc gcc gcg gcc gtt ggc gcc ggg atg        1920
Ala Met Ala Leu Gly Gly Gly Ile Ala Ala Ala Val Gly Ala Gly Met
625                 630                 635                 640

tcg ttg acc gat gac gcg ccg gcc gga cag aag gcc gcc gcc ggc gcc        1968
Ser Leu Thr Asp Asp Ala Pro Ala Gly Gln Lys Ala Ala Ala Gly Ala
```

```
                645                 650                 655

gag atc gcg ctg cag ttg aca ggt gga acg gtc gag ctg gct tct tcc        2016
Glu Ile Ala Leu Gln Leu Thr Gly Gly Thr Val Glu Leu Ala Ser Ser
            660                 665                 670

atc gcg ttg gcg ctg gcc gcg gcg cgc ggc gtg acc agc ggc ttg cag        2064
Ile Ala Leu Ala Leu Ala Ala Ala Arg Gly Val Thr Ser Gly Leu Gln
        675                 680                 685

gtg gcc ggg gcg tcg gcc ggg gcg gct gcc ggc gca ttg gcc gcg gcg        2112
Val Ala Gly Ala Ser Ala Gly Ala Ala Ala Gly Ala Leu Ala Ala Ala
    690                 695                 700

ctc agt ccc atg gag atc tac ggc ctg gtg cag caa tcg cac tat gcg        2160
Leu Ser Pro Met Glu Ile Tyr Gly Leu Val Gln Gln Ser His Tyr Ala
705                 710                 715                 720

gat cag ctg gac aag ctg gcg cag gaa tcg agc gca tac ggt tac gag        2208
Asp Gln Leu Asp Lys Leu Ala Gln Glu Ser Ser Ala Tyr Gly Tyr Glu
                725                 730                 735

ggc gac gcc ttg ctg gcc cag ctg tat cgc gac aag acg gcc gcc gag        2256
Gly Asp Ala Leu Leu Ala Gln Leu Tyr Arg Asp Lys Thr Ala Ala Glu
            740                 745                 750

ggc gcc gtc gcc ggc gtc tcc gcc gtc ctg agc acg gtg ggg gcg gcg        2304
Gly Ala Val Ala Gly Val Ser Ala Val Leu Ser Thr Val Gly Ala Ala
        755                 760                 765

gtg tcg atc gcc gcg gcg gcc agc gtg gta ggg gcc ccg gtg gcg gtg        2352
Val Ser Ile Ala Ala Ala Ala Ser Val Val Gly Ala Pro Val Ala Val
    770                 775                 780

gtc act tcc ttg ctg acc ggg gct ctc aac ggc atc ctg cgc ggc gtg        2400
Val Thr Ser Leu Leu Thr Gly Ala Leu Asn Gly Ile Leu Arg Gly Val
785                 790                 795                 800

cag cag ccc atc atc gaa aag ctg gcc aac gat tac gct cgc aag atc        2448
Gln Gln Pro Ile Ile Glu Lys Leu Ala Asn Asp Tyr Ala Arg Lys Ile
                805                 810                 815

gac gag ctg ggc ggg ccg caa gcg tac ttc gag aaa aac ctg cag gcg        2496
Asp Glu Leu Gly Gly Pro Gln Ala Tyr Phe Glu Lys Asn Leu Gln Ala
            820                 825                 830

cgt cac gaa caa ctg gcc aat tcg gac ggc cta cgg aaa atg ctg gcc        2544
Arg His Glu Gln Leu Ala Asn Ser Asp Gly Leu Arg Lys Met Leu Ala
        835                 840                 845

gac ctg cag gcc ggt tgg aac gcc agc agc gtg atc ggg gtg cag acg        2592
Asp Leu Gln Ala Gly Trp Asn Ala Ser Ser Val Ile Gly Val Gln Thr
    850                 855                 860

aca gag atc tcc aag tcg gcg ctc gaa ctg gcc gcc att acc ggc aac        2640
Thr Glu Ile Ser Lys Ser Ala Leu Glu Leu Ala Ala Ile Thr Gly Asn
865                 870                 875                 880

gcg gac aac ctg aaa tcc gtc gac gtg ttc gtg gac cgc ttc gtc cag        2688
Ala Asp Asn Leu Lys Ser Val Asp Val Phe Val Asp Arg Phe Val Gln
                885                 890                 895

ggc gag cgg gtg gcc ggc cag ccg gtg gtc ctc gac gtc gcc gcc ggc        2736
```

```
Gly Glu Arg Val Ala Gly Gln Pro Val Val Leu Asp Val Ala Ala Gly
            900                 905                 910

ggc atc gat atc gcc agc cgc aag ggc gag cgg ccg gcg ctg acg ttc      2784
Gly Ile Asp Ile Ala Ser Arg Lys Gly Glu Arg Pro Ala Leu Thr Phe
        915                 920                 925

atc acg ccg ctg gcc gcg cca gga gaa gag cag cgc cgg cgc acg aaa      2832
Ile Thr Pro Leu Ala Ala Pro Gly Glu Glu Gln Arg Arg Arg Thr Lys
    930                 935                 940

acg ggc aag agc gaa ttc acc aca ttc gtc gag atc gtg ggc aag cag      2880
Thr Gly Lys Ser Glu Phe Thr Thr Phe Val Glu Ile Val Gly Lys Gln
945                 950                 955                 960

gac cgc tgg cgc atc cgg gac ggc gcg gcc gac acc acc atc gat ctg      2928
Asp Arg Trp Arg Ile Arg Asp Gly Ala Ala Asp Thr Thr Ile Asp Leu
                965                 970                 975

gcc aag gtg gtg tcg caa ctg gtc gac gcc aat ggc gtg ctc aag cac      2976
Ala Lys Val Val Ser Gln Leu Val Asp Ala Asn Gly Val Leu Lys His
            980                 985                 990

agc atc aaa ctg gat gtg atc ggc  gga gat ggc gat gac  gtc gtg ctt    3024
Ser Ile Lys Leu Asp Val Ile Gly  Gly Asp Gly Asp Asp  Val Val Leu
        995                 1000                1005

gcc aat  gct tcg cgc atc cat  tat gac ggc ggc gcg  ggc acc aac      3069
Ala Asn  Ala Ser Arg Ile His  Tyr Asp Gly Gly Ala  Gly Thr Asn
    1010                 1015                 1020

acg gtc  agc tat gcc gcc ctg  ggt cga cag gat tcc  att acc gtg      3114
Thr Val  Ser Tyr Ala Ala Leu  Gly Arg Gln Asp Ser  Ile Thr Val
    1025                 1030                 1035

tcc gcc  gac ggg gaa cgt ttc  aac gtg cgc aag cag  ttg aac aac      3159
Ser Ala  Asp Gly Glu Arg Phe  Asn Val Arg Lys Gln  Leu Asn Asn
    1040                 1045                 1050

gcc aac  gtg tat cgc gaa ggc  gtg gct acc cag aca  acc gcc tac      3204
Ala Asn  Val Tyr Arg Glu Gly  Val Ala Thr Gln Thr  Thr Ala Tyr
    1055                 1060                 1065

ggc aag  cgc acg gag aat gtc  caa tac cgc cat gtc  gag ctg gcc      3249
Gly Lys  Arg Thr Glu Asn Val  Gln Tyr Arg His Val  Glu Leu Ala
    1070                 1075                 1080

cgt gtc  ggg caa ctg gtg gag  gtc gac acg ctc gag  cat gtg cag      3294
Arg Val  Gly Gln Leu Val Glu  Val Asp Thr Leu Glu  His Val Gln
    1085                 1090                 1095

cac atc  atc ggc ggg gcc ggc  aac gat tcg atc acc  ggc aat gcg      3339
His Ile  Ile Gly Gly Ala Gly  Asn Asp Ser Ile Thr  Gly Asn Ala
    1100                 1105                 1110

cac gac  aac ttc cta gcc ggc  ggg tcg ggc gac gac  agg ctg gat      3384
His Asp  Asn Phe Leu Ala Gly  Gly Ser Gly Asp Asp  Arg Leu Asp
    1115                 1120                 1125

ggc ggc  gcc ggc aac gac acc  ctg gtt ggc ggc gag  ggc caa aac      3429
Gly Gly  Ala Gly Asn Asp Thr  Leu Val Gly Gly Glu  Gly Gln Asn
    1130                 1135                 1140
```

```
acg gtc  atc ggc ggc gcc ggc  gac gac gta ttc ctg  cag gac ctg        3474
Thr Val  Ile Gly Gly Ala Gly  Asp Asp Val Phe Leu  Gln Asp Leu
    1145                 1150                 1155

ggg gta  tgg agc aac cag ctc  gat ggc ggc gcg ggc  gtc gat acc        3519
Gly Val  Trp Ser Asn Gln Leu  Asp Gly Gly Ala Gly  Val Asp Thr
    1160                 1165                 1170

gtg aag  tac aac gtg cac cag  cct tcc gag gag cgc  ctc gaa cgc        3564
Val Lys  Tyr Asn Val His Gln  Pro Ser Glu Glu Arg  Leu Glu Arg
    1175                 1180                 1185

atg ggc  gac acg ggc atc cat  gcc gat ctt caa aag  ggc acg gtc        3609
Met Gly  Asp Thr Gly Ile His  Ala Asp Leu Gln Lys  Gly Thr Val
    1190                 1195                 1200

gag aag  tgg ccg gcc ctg aac  ctg ttc agc gtc gac  cat gtc aag        3654
Glu Lys  Trp Pro Ala Leu Asn  Leu Phe Ser Val Asp  His Val Lys
    1205                 1210                 1215

aat atc  gag aat ctg cac ggc  tcc cgc cta aac gac  cgc atc gcc        3699
Asn Ile  Glu Asn Leu His Gly  Ser Arg Leu Asn Asp  Arg Ile Ala
    1220                 1225                 1230

ggc gac  gac cag gac aac gag  ctc tgg ggc cac gat  ggc aac gac        3744
Gly Asp  Asp Gln Asp Asn Glu  Leu Trp Gly His Asp  Gly Asn Asp
    1235                 1240                 1245

acg ata  cgc ggc cgg ggc ggc  gac gac atc ctg cgc  ggc ggc ctg        3789
Thr Ile  Arg Gly Arg Gly Gly  Asp Asp Ile Leu Arg  Gly Gly Leu
    1250                 1255                 1260

ggc ctg  gac acg ctg tat ggc  gag gac ggc aac gac  atc ttc ctg        3834
Gly Leu  Asp Thr Leu Tyr Gly  Glu Asp Gly Asn Asp  Ile Phe Leu
    1265                 1270                 1275

cag gac  gac gag acc gtc agc  gat gac atc gac ggc  ggc gcg ggg        3879
Gln Asp  Asp Glu Thr Val Ser  Asp Asp Ile Asp Gly  Gly Ala Gly
    1280                 1285                 1290

ctg gac  acc gtc gac tac tcc  gcc atg atc cat cca  ggc agg atc        3924
Leu Asp  Thr Val Asp Tyr Ser  Ala Met Ile His Pro  Gly Arg Ile
    1295                 1300                 1305

gtt gcg  ccg cat gaa tac ggc  ttc ggg atc gag gcg  gac ctg tcc        3969
Val Ala  Pro His Glu Tyr Gly  Phe Gly Ile Glu Ala  Asp Leu Ser
    1310                 1315                 1320

agg gaa  tgg gtg cgc aag gcg  tcc gcg ctg ggc gtg  gac tat tac        4014
Arg Glu  Trp Val Arg Lys Ala  Ser Ala Leu Gly Val  Asp Tyr Tyr
    1325                 1330                 1335

gat aat  gtc cgc aat gtc gaa  aac gtc atc ggt acg  agc atg aag        4059
Asp Asn  Val Arg Asn Val Glu  Asn Val Ile Gly Thr  Ser Met Lys
    1340                 1345                 1350

gat gtg  ctc atc ggc gac gcg  caa gcc aat acc ctg  atg ggc cag        4104
Asp Val  Leu Ile Gly Asp Ala  Gln Ala Asn Thr Leu  Met Gly Gln
    1355                 1360                 1365

ggc ggc  gac gat acc gtg cgc  ggc ggc gac ggc gat  gat ctg ctg        4149
Gly Gly  Asp Asp Thr Val Arg  Gly Gly Asp Gly Asp  Asp Leu Leu
    1370                 1375                 1380
```

```
ttc ggc  ggc gac ggc aac gac  atg ctg tat ggc gac  gcc ggc aac          4194
Phe Gly  Gly Asp Gly Asn Asp  Met Leu Tyr Gly Asp  Ala Gly Asn
    1385                 1390                 1395

gac acc  ctc tac ggg ggg ctg  ggc gac gat acc ctt  gaa ggc ggc          4239
Asp Thr  Leu Tyr Gly Gly Leu  Gly Asp Asp Thr Leu  Glu Gly Gly
    1400                 1405                 1410

gcg ggc  aac gat tgg ttc ggc  cag acg cag gcg cgc  gag cat gac          4284
Ala Gly  Asn Asp Trp Phe Gly  Gln Thr Gln Ala Arg  Glu His Asp
    1415                 1420                 1425

gtg ctg  cgc ggc gga gat ggg  gtg gat acc gtc gat  tac agc cag          4329
Val Leu  Arg Gly Gly Asp Gly  Val Asp Thr Val Asp  Tyr Ser Gln
    1430                 1435                 1440

acc ggc  gcg cat gcc ggc att  gcc gcg ggt cgc atc  ggg ctg ggc          4374
Thr Gly  Ala His Ala Gly Ile  Ala Ala Gly Arg Ile  Gly Leu Gly
    1445                 1450                 1455

atc ctg  gct gac ctg ggc gcc  ggc cgc gtc gac aag  ctg ggc gag          4419
Ile Leu  Ala Asp Leu Gly Ala  Gly Arg Val Asp Lys  Leu Gly Glu
    1460                 1465                 1470

gcc ggc  agc agc gcc tac gat  acg gtt tcc ggt atc  gag aac gtg          4464
Ala Gly  Ser Ser Ala Tyr Asp  Thr Val Ser Gly Ile  Glu Asn Val
    1475                 1480                 1485

gtg ggc  acg gaa ctg gcc gac  cgc atc acg ggc gat  gcg cag gcc          4509
Val Gly  Thr Glu Leu Ala Asp  Arg Ile Thr Gly Asp  Ala Gln Ala
    1490                 1495                 1500

aac gtg  ctg cgc ggc gcg ggt  ggc gcc gac gtg ctt  gcg ggc ggc          4554
Asn Val  Leu Arg Gly Ala Gly  Gly Ala Asp Val Leu  Ala Gly Gly
    1505                 1510                 1515

gag ggc  gac gat gtg ctg ctg  ggc ggc gac ggc gac  gac cag ctg          4599
Glu Gly  Asp Asp Val Leu Leu  Gly Gly Asp Gly Asp  Asp Gln Leu
    1520                 1525                 1530

tcg ggc  gac gcc gga cgc gat  cgc ttg tac ggc gaa  gcc ggt gac          4644
Ser Gly  Asp Ala Gly Arg Asp  Arg Leu Tyr Gly Glu  Ala Gly Asp
    1535                 1540                 1545

gac tgg  ttc ttc cag gat gcc  gcc aat gcc ggc aat  ctg ctc gac          4689
Asp Trp  Phe Phe Gln Asp Ala  Ala Asn Ala Gly Asn  Leu Leu Asp
    1550                 1555                 1560

ggc ggc  gac ggc cgc gat acc  gtg gat ttc agc ggc  ccg ggc cgg          4734
Gly Gly  Asp Gly Arg Asp Thr  Val Asp Phe Ser Gly  Pro Gly Arg
    1565                 1570                 1575

ggc ctc  gac gcc ggc gca aag  ggc gta ttc ctg agc  ttg ggc aag          4779
Gly Leu  Asp Ala Gly Ala Lys  Gly Val Phe Leu Ser  Leu Gly Lys
    1580                 1585                 1590

ggg ttc  gcc agc ctg atg gac  gaa ccc gaa acc agc  aac gtg ttg          4824
Gly Phe  Ala Ser Leu Met Asp  Glu Pro Glu Thr Ser  Asn Val Leu
    1595                 1600                 1605

cgc aat  atc gag aac gcc gtg  ggc agc gcg cgt gat  gac gtg ctg          4869
Arg Asn  Ile Glu Asn Ala Val  Gly Ser Ala Arg Asp  Asp Val Leu
```

```
    1610                1615                1620

atc ggc  gac gca ggc gcc aac  gtc ctc aat ggc ctg  gcg ggc aac        4914
Ile Gly  Asp Ala Gly Ala Asn  Val Leu Asn Gly Leu  Ala Gly Asn
    1625                1630                1635

gac gtg  ctg tcc ggc ggc gct  ggc gac gat gtg ctg  ctg ggc gac        4959
Asp Val  Leu Ser Gly Gly Ala  Gly Asp Asp Val Leu  Leu Gly Asp
    1640                1645                1650

gag ggc  tcg gac ctg ctc agc  ggc gat gcg ggc aac  gac gat ctg        5004
Glu Gly  Ser Asp Leu Leu Ser  Gly Asp Ala Gly Asn  Asp Asp Leu
    1655                1660                1665

ttc ggc  ggg cag ggc gat gat  act tat ctg ttc ggg  gtc ggg tac        5049
Phe Gly  Gly Gln Gly Asp Asp  Thr Tyr Leu Phe Gly  Val Gly Tyr
    1670                1675                1680

ggg cac  gac acg atc tac gaa  tcg ggc ggc ggc cat  gac acc atc        5094
Gly His  Asp Thr Ile Tyr Glu  Ser Gly Gly Gly His  Asp Thr Ile
    1685                1690                1695

cgc atc  aac gcg ggg gcg gac  cag ctg tgg ttc gcg  cgc cag ggc        5139
Arg Ile  Asn Ala Gly Ala Asp  Gln Leu Trp Phe Ala  Arg Gln Gly
    1700                1705                1710

aac gac  ctg gag atc cgc att  ctc ggc acc gac gat  gca ctt acc        5184
Asn Asp  Leu Glu Ile Arg Ile  Leu Gly Thr Asp Asp  Ala Leu Thr
    1715                1720                1725

gtg cac  gac tgg tat cgc gac  gcc gat cac cgg gtg  gaa atc atc        5229
Val His  Asp Trp Tyr Arg Asp  Ala Asp His Arg Val  Glu Ile Ile
    1730                1735                1740

cat gcc  gcc aac cag gcg gta  gac cag gca ggc atc  gaa aag ctg        5274
His Ala  Ala Asn Gln Ala Val  Asp Gln Ala Gly Ile  Glu Lys Leu
    1745                1750                1755

gtc gag  gca atg gcg cag tat  ccg gac ccc ggc gcg  gcg gcg gct        5319
Val Glu  Ala Met Ala Gln Tyr  Pro Asp Pro Gly Ala  Ala Ala Ala
    1760                1765                1770

gcc ccg  ccg gcg gcg cgc gtg  ccg gac acg ctg atg  cag tcc ctg        5364
Ala Pro  Pro Ala Ala Arg Val  Pro Asp Thr Leu Met  Gln Ser Leu
    1775                1780                1785

gct gtc  aac tgg cgc tga                                              5382
Ala Val  Asn Trp Arg
    1790


<210>  67
<211>  1793
<212>  PRT
<213>  Artificial Sequence

<220>
<223>  Synthetic Construct

<400>  67

Met Gln Gln Ser His Gln Ala Gly Tyr Ala Asn Ala Ala Asp Arg Glu
1               5                   10                  15
```

```
Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Leu Gln Ile Asp
            180                 185                 190

Met Phe Ala Ile Met Pro His Leu Ser Asn Phe Arg Asp Ser Ala Arg
        195                 200                 205

Ser Ser Val Thr Ser Gly Asp Ser Val Thr Asp Tyr Leu Ala Arg Thr
    210                 215                 220

Arg Arg Ala Ser Gly Gln Ala Glu Pro Asp Arg Ala His Tyr Asn Ile
225                 230                 235                 240

Val Thr Phe Cys Cys Lys Cys Asp Ser Thr Leu Arg Leu Cys Val Gln
                245                 250                 255

Ser Thr His Val Asp Ile Arg Thr Leu Glu Asp Leu Leu Met Gly Thr
```

```
            260                 265                 270

Leu Gly Ile Val Cys Pro Ile Cys Ser Gln Lys Pro Gly Thr Arg Ala
        275                 280                 285

Arg Leu Lys Leu Leu Trp Lys Ile Ala Arg Ala Gly Ala Arg Ser Ala
    290                 295                 300

Val Gly Thr Glu Ala Arg Arg Gln Phe Arg Tyr Asp Gly Asp Met Asn
305                 310                 315                 320

Ile Gly Val Ile Thr Asp Phe Glu Leu Glu Val Arg Asn Ala Leu Asn
                325                 330                 335

Arg Arg Ala His Ala Val Gly Ala Gln Asp Val Val Gln His Gly Thr
            340                 345                 350

Glu Gln Asn Asn Pro Phe Pro Glu Ala Asp Glu Lys Ile Phe Val Val
        355                 360                 365

Ser Ala Thr Gly Lys Arg Met His Gly Asp Thr Pro Thr Leu His Glu
    370                 375                 380

Tyr Met Leu Asp Leu Gln Pro Glu Thr Thr Asp Leu Tyr Cys Tyr Glu
385                 390                 395                 400

Gln Leu Asn Lys Arg Thr Ser Glu Ser Gln Met Leu Thr Arg Gly Gln
                405                 410                 415

Leu Lys Glu Tyr Ile Gly Gln Gln Arg Gly Glu Gly Tyr Val Phe Tyr
            420                 425                 430

Glu Asn Arg Ala Tyr Gly Val Ala Gly Lys Ser Leu Phe Asp Asp Gly
        435                 440                 445

Leu Gly Ala Ala Pro Gly Val Pro Ser Gly Arg Ser Lys Phe Ser Pro
    450                 455                 460

Asp Val Leu Glu Thr Val Pro Ala Ser Pro Gly Leu Arg Arg Pro Ser
465                 470                 475                 480

Leu Gly Ala Val Glu Arg Gln Asp Ser Gly Tyr Asp Ser Leu Asp Gly
                485                 490                 495

Val Gly Ser Arg Ser Phe Ser Leu Gly Glu Val Ser Asp Met Ala Ala
            500                 505                 510
```

```
Val Glu Ala Ala Glu Leu Glu Met Thr Arg Gln Val Leu His Ala Gly
        515                 520                 525

Ala Arg Gln Asp Asp Ala Glu Pro Gly Val Ser Gly Ala Ser Ala His
    530                 535                 540

Trp Gly Gln Arg Ala Leu Gln Gly Ala Gln Ala Val Ala Ala Ala Gln
545                 550                 555                 560

Arg Leu Val His Ala Ile Ala Leu Met Thr Gln Phe Gly Arg Ala Gly
                565                 570                 575

Ser Thr Asn Thr Pro Gln Glu Ala Ala Ser Leu Ser Ala Ala Val Phe
            580                 585                 590

Gly Leu Gly Glu Ala Ser Ser Ala Val Ala Glu Thr Val Ser Gly Phe
        595                 600                 605

Phe Arg Gly Ser Ser Arg Trp Ala Gly Gly Phe Gly Val Ala Gly Gly
    610                 615                 620

Ala Met Ala Leu Gly Gly Gly Ile Ala Ala Ala Val Gly Ala Gly Met
625                 630                 635                 640

Ser Leu Thr Asp Asp Ala Pro Ala Gly Gln Lys Ala Ala Ala Gly Ala
                645                 650                 655

Glu Ile Ala Leu Gln Leu Thr Gly Gly Thr Val Glu Leu Ala Ser Ser
            660                 665                 670

Ile Ala Leu Ala Leu Ala Ala Ala Arg Gly Val Thr Ser Gly Leu Gln
        675                 680                 685

Val Ala Gly Ala Ser Ala Gly Ala Ala Ala Gly Ala Leu Ala Ala Ala
    690                 695                 700

Leu Ser Pro Met Glu Ile Tyr Gly Leu Val Gln Gln Ser His Tyr Ala
705                 710                 715                 720

Asp Gln Leu Asp Lys Leu Ala Gln Glu Ser Ser Ala Tyr Gly Tyr Glu
                725                 730                 735

Gly Asp Ala Leu Leu Ala Gln Leu Tyr Arg Asp Lys Thr Ala Ala Glu
            740                 745                 750

Gly Ala Val Ala Gly Val Ser Ala Val Leu Ser Thr Val Gly Ala Ala
        755                 760                 765
```

```
Val Ser Ile Ala Ala Ala Ala Ser Val Val Gly Ala Pro Val Ala Val
    770                 775                 780

Val Thr Ser Leu Leu Thr Gly Ala Leu Asn Gly Ile Leu Arg Gly Val
785                 790                 795                 800

Gln Gln Pro Ile Ile Glu Lys Leu Ala Asn Asp Tyr Ala Arg Lys Ile
                805                 810                 815

Asp Glu Leu Gly Gly Pro Gln Ala Tyr Phe Glu Lys Asn Leu Gln Ala
            820                 825                 830

Arg His Glu Gln Leu Ala Asn Ser Asp Gly Leu Arg Lys Met Leu Ala
        835                 840                 845

Asp Leu Gln Ala Gly Trp Asn Ala Ser Ser Val Ile Gly Val Gln Thr
    850                 855                 860

Thr Glu Ile Ser Lys Ser Ala Leu Glu Leu Ala Ala Ile Thr Gly Asn
865                 870                 875                 880

Ala Asp Asn Leu Lys Ser Val Asp Val Phe Val Asp Arg Phe Val Gln
                885                 890                 895

Gly Glu Arg Val Ala Gly Gln Pro Val Val Leu Asp Val Ala Ala Gly
            900                 905                 910

Gly Ile Asp Ile Ala Ser Arg Lys Gly Glu Arg Pro Ala Leu Thr Phe
        915                 920                 925

Ile Thr Pro Leu Ala Ala Pro Gly Glu Glu Gln Arg Arg Arg Thr Lys
    930                 935                 940

Thr Gly Lys Ser Glu Phe Thr Thr Phe Val Glu Ile Val Gly Lys Gln
945                 950                 955                 960

Asp Arg Trp Arg Ile Arg Asp Gly Ala Ala Asp Thr Thr Ile Asp Leu
                965                 970                 975

Ala Lys Val Val Ser Gln Leu Val Asp Ala Asn Gly Val Leu Lys His
            980                 985                 990

Ser Ile Lys Leu Asp Val Ile Gly  Gly Asp Gly Asp Asp  Val Val Leu
        995                 1000                1005

Ala Asn  Ala Ser Arg Ile His  Tyr Asp Gly Gly Ala  Gly Thr Asn
    1010                1015                1020
```

Thr Val Ser Tyr Ala Ala Leu Gly Arg Gln Asp Ser Ile Thr Val
1025 1030 1035

Ser Ala Asp Gly Glu Arg Phe Asn Val Arg Lys Gln Leu Asn Asn
1040 1045 1050

Ala Asn Val Tyr Arg Glu Gly Val Ala Thr Gln Thr Thr Ala Tyr
1055 1060 1065

Gly Lys Arg Thr Glu Asn Val Gln Tyr Arg His Val Glu Leu Ala
1070 1075 1080

Arg Val Gly Gln Leu Val Glu Val Asp Thr Leu Glu His Val Gln
1085 1090 1095

His Ile Ile Gly Gly Ala Gly Asn Asp Ser Ile Thr Gly Asn Ala
1100 1105 1110

His Asp Asn Phe Leu Ala Gly Gly Ser Gly Asp Asp Arg Leu Asp
1115 1120 1125

Gly Gly Ala Gly Asn Asp Thr Leu Val Gly Gly Glu Gly Gln Asn
1130 1135 1140

Thr Val Ile Gly Gly Ala Gly Asp Asp Val Phe Leu Gln Asp Leu
1145 1150 1155

Gly Val Trp Ser Asn Gln Leu Asp Gly Gly Ala Gly Val Asp Thr
1160 1165 1170

Val Lys Tyr Asn Val His Gln Pro Ser Glu Glu Arg Leu Glu Arg
1175 1180 1185

Met Gly Asp Thr Gly Ile His Ala Asp Leu Gln Lys Gly Thr Val
1190 1195 1200

Glu Lys Trp Pro Ala Leu Asn Leu Phe Ser Val Asp His Val Lys
1205 1210 1215

Asn Ile Glu Asn Leu His Gly Ser Arg Leu Asn Asp Arg Ile Ala
1220 1225 1230

Gly Asp Asp Gln Asp Asn Glu Leu Trp Gly His Asp Gly Asn Asp
1235 1240 1245

Thr Ile Arg Gly Arg Gly Gly Asp Asp Ile Leu Arg Gly Gly Leu

1250 1255 1260

Gly Leu Asp Thr Leu Tyr Gly Glu Asp Gly Asn Asp Ile Phe Leu
1265 1270 1275

Gln Asp Asp Glu Thr Val Ser Asp Asp Ile Asp Gly Gly Ala Gly
1280 1285 1290

Leu Asp Thr Val Asp Tyr Ser Ala Met Ile His Pro Gly Arg Ile
1295 1300 1305

Val Ala Pro His Glu Tyr Gly Phe Gly Ile Glu Ala Asp Leu Ser
1310 1315 1320

Arg Glu Trp Val Arg Lys Ala Ser Ala Leu Gly Val Asp Tyr Tyr
1325 1330 1335

Asp Asn Val Arg Asn Val Glu Asn Val Ile Gly Thr Ser Met Lys
1340 1345 1350

Asp Val Leu Ile Gly Asp Ala Gln Ala Asn Thr Leu Met Gly Gln
1355 1360 1365

Gly Gly Asp Asp Thr Val Arg Gly Gly Asp Gly Asp Asp Leu Leu
1370 1375 1380

Phe Gly Gly Asp Gly Asn Asp Met Leu Tyr Gly Asp Ala Gly Asn
1385 1390 1395

Asp Thr Leu Tyr Gly Gly Leu Gly Asp Asp Thr Leu Glu Gly Gly
1400 1405 1410

Ala Gly Asn Asp Trp Phe Gly Gln Thr Gln Ala Arg Glu His Asp
1415 1420 1425

Val Leu Arg Gly Gly Asp Gly Val Asp Thr Val Asp Tyr Ser Gln
1430 1435 1440

Thr Gly Ala His Ala Gly Ile Ala Ala Gly Arg Ile Gly Leu Gly
1445 1450 1455

Ile Leu Ala Asp Leu Gly Ala Gly Arg Val Asp Lys Leu Gly Glu
1460 1465 1470

Ala Gly Ser Ser Ala Tyr Asp Thr Val Ser Gly Ile Glu Asn Val
1475 1480 1485

Val Gly Thr Glu Leu Ala Asp Arg Ile Thr Gly Asp Ala Gln Ala
1490 1495 1500

Asn Val Leu Arg Gly Ala Gly Gly Ala Asp Val Leu Ala Gly Gly
1505 1510 1515

Glu Gly Asp Asp Val Leu Leu Gly Gly Asp Gly Asp Asp Gln Leu
1520 1525 1530

Ser Gly Asp Ala Gly Arg Asp Arg Leu Tyr Gly Glu Ala Gly Asp
1535 1540 1545

Asp Trp Phe Phe Gln Asp Ala Ala Asn Ala Gly Asn Leu Leu Asp
1550 1555 1560

Gly Gly Asp Gly Arg Asp Thr Val Asp Phe Ser Gly Pro Gly Arg
1565 1570 1575

Gly Leu Asp Ala Gly Ala Lys Gly Val Phe Leu Ser Leu Gly Lys
1580 1585 1590

Gly Phe Ala Ser Leu Met Asp Glu Pro Glu Thr Ser Asn Val Leu
1595 1600 1605

Arg Asn Ile Glu Asn Ala Val Gly Ser Ala Arg Asp Asp Val Leu
1610 1615 1620

Ile Gly Asp Ala Gly Ala Asn Val Leu Asn Gly Leu Ala Gly Asn
1625 1630 1635

Asp Val Leu Ser Gly Gly Ala Gly Asp Asp Val Leu Leu Gly Asp
1640 1645 1650

Glu Gly Ser Asp Leu Leu Ser Gly Asp Ala Gly Asn Asp Asp Leu
1655 1660 1665

Phe Gly Gly Gln Gly Asp Asp Thr Tyr Leu Phe Gly Val Gly Tyr
1670 1675 1680

Gly His Asp Thr Ile Tyr Glu Ser Gly Gly Gly His Asp Thr Ile
1685 1690 1695

Arg Ile Asn Ala Gly Ala Asp Gln Leu Trp Phe Ala Arg Gln Gly
1700 1705 1710

Asn Asp Leu Glu Ile Arg Ile Leu Gly Thr Asp Asp Ala Leu Thr
1715 1720 1725

Val His Asp Trp Tyr Arg Asp Ala Asp His Arg Val Glu Ile Ile
1730 1735 1740

His Ala Ala Asn Gln Ala Val Asp Gln Ala Gly Ile Glu Lys Leu
1745 1750 1755

Val Glu Ala Met Ala Gln Tyr Pro Asp Pro Gly Ala Ala Ala Ala
1760 1765 1770

Ala Pro Pro Ala Ala Arg Val Pro Asp Thr Leu Met Gln Ser Leu
1775 1780 1785

Ala Val Asn Trp Arg
1790

<210> 68
<211> 5409
<212> DNA
<213> Artificial Sequence

<220>
<223> E7-CyaA HPV18

<220>
<221> CDS
<222> (1)..(5409)

<220>
<221> misc_feature
<222> (685)..(871)
<223> E7 protein fragment

<220>
<221> misc_feature
<222> (1141)..(1234)
<223> E7 protein fragment

<400> 68
atg cag caa tcg cat cag gct ggt tac gca aac gcc gcc gac cgg gag 48
Met Gln Gln Ser His Gln Ala Gly Tyr Ala Asn Ala Ala Asp Arg Glu
1 5 10 15

tct ggc atc ccc gca gcc gta ctc gat ggc atc aag gcc gtg gcg aag 96
Ser Gly Ile Pro Ala Ala Val Leu Asp Gly Ile Lys Ala Val Ala Lys
20 25 30

gaa aaa aac gcc aca ttg atg ttc cgc ctg gtc aac ccc cat tcc acc 144
Glu Lys Asn Ala Thr Leu Met Phe Arg Leu Val Asn Pro His Ser Thr
35 40 45

agc ctg att gcc gaa ggg gtg gcc acc aaa gga ttg ggc gtg cac gcc 192
Ser Leu Ile Ala Glu Gly Val Ala Thr Lys Gly Leu Gly Val His Ala
50 55 60

aag tcg tcc gat tgg ggg ttg cag gcg ggc tac att ccc gtc aac ccg 240
Lys Ser Ser Asp Trp Gly Leu Gln Ala Gly Tyr Ile Pro Val Asn Pro

```
65                  70                  75                  80

aat ctt tcc aaa ctg ttc ggc cgt gcg ccc gag gtg atc gcg cgg gcc       288
Asn Leu Ser Lys Leu Phe Gly Arg Ala Pro Glu Val Ile Ala Arg Ala
                85                  90                  95

gac aac gac gtc aac agc agc ctg gcg cat ggc cat acc gcg gtc gac       336
Asp Asn Asp Val Asn Ser Ser Leu Ala His Gly His Thr Ala Val Asp
            100                 105                 110

ctg acg ctg tcg aaa gag cgg ctt gac tat ctg cgg caa gcg ggc ctg       384
Leu Thr Leu Ser Lys Glu Arg Leu Asp Tyr Leu Arg Gln Ala Gly Leu
        115                 120                 125

gtc acc ggc atg gcc gat ggc gtg gtc gcg agc aac cac gca ggc tac       432
Val Thr Gly Met Ala Asp Gly Val Val Ala Ser Asn His Ala Gly Tyr
    130                 135                 140

gag cag ttc gag ttt cgc gtg aag gaa acc tcg gac ggg cgc tat gcc       480
Glu Gln Phe Glu Phe Arg Val Lys Glu Thr Ser Asp Gly Arg Tyr Ala
145                 150                 155                 160

gtg cag tat cgc cgc aag ggc ggc gac gat ttc gag gcg gtc aag gtg       528
Val Gln Tyr Arg Arg Lys Gly Gly Asp Asp Phe Glu Ala Val Lys Val
                165                 170                 175

atc ggc aat gcc gcc ggt att cca ctg acg gcg gat ctg cag atc gac       576
Ile Gly Asn Ala Ala Gly Ile Pro Leu Thr Ala Asp Leu Gln Ile Asp
            180                 185                 190

atg ttc gcc att atg ccg cat ctg tcc aac ttc cgc gac tcg gcg cgc       624
Met Phe Ala Ile Met Pro His Leu Ser Asn Phe Arg Asp Ser Ala Arg
        195                 200                 205

agt tcg gtg acc agc ggc gat tcg gtg acc gat tac ctg gcg cgt acg       672
Ser Ser Val Thr Ser Gly Asp Ser Val Thr Asp Tyr Leu Ala Arg Thr
    210                 215                 220

cgt cgt gct agc ggt gtg aat cac caa cat ctg ccg gcg cgc cgt gct       720
Arg Arg Ala Ser Gly Val Asn His Gln His Leu Pro Ala Arg Arg Ala
225                 230                 235                 240

gaa ccg cag cgc cat acc atg tta tgt atg tgt tgc aag tgt gag gcg       768
Glu Pro Gln Arg His Thr Met Leu Cys Met Cys Cys Lys Cys Glu Ala
                245                 250                 255

cgt atc gag ctg gtc gtt gaa tct agc gct gat gat ctg cgt gcg ttt       816
Arg Ile Glu Leu Val Val Glu Ser Ser Ala Asp Asp Leu Arg Ala Phe
            260                 265                 270

caa cag ctg ttc ctg aac act tta tcc ttc gta tgc cca tgg tgc gcc       864
Gln Gln Leu Phe Leu Asn Thr Leu Ser Phe Val Cys Pro Trp Cys Ala
        275                 280                 285

agc cag cag ggt acc cgc gca cgc ctt aag ttg ttg tgg aaa atc gct       912
Ser Gln Gln Gly Thr Arg Ala Arg Leu Lys Leu Leu Trp Lys Ile Ala
    290                 295                 300

cgc gcc ggc gcc cgt tcc gca gtg ggc acc gag gcg cgt cgc cag ttc       960
Arg Ala Gly Ala Arg Ser Ala Val Gly Thr Glu Ala Arg Arg Gln Phe
305                 310                 315                 320

cgc tac gac ggc gac atg aat atc ggc gtg atc acc gat ttc gag ctg      1008
```

```
Arg Tyr Asp Gly Asp Met Asn Ile Gly Val Ile Thr Asp Phe Glu Leu
                325                 330                 335

gaa gtg cgc aat gcg ctg aac agg cgg gcg cac gcc gtc ggc gcg cag       1056
Glu Val Arg Asn Ala Leu Asn Arg Arg Ala His Ala Val Gly Ala Gln
            340                 345                 350

gac gtg gtc cag cat ggc act gag cag aac aat cct ttc ccg gag gca       1104
Asp Val Val Gln His Gly Thr Glu Gln Asn Asn Pro Phe Pro Glu Ala
        355                 360                 365

gat gag aag att ttc gtc gta tcg gcc acc ggt aaa cgt atg cat ggt       1152
Asp Glu Lys Ile Phe Val Val Ser Ala Thr Gly Lys Arg Met His Gly
    370                 375                 380

ccg aaa gcc act ctg caa gat att gtt ctg cac ttg gaa ccg caa aat       1200
Pro Lys Ala Thr Leu Gln Asp Ile Val Leu His Leu Glu Pro Gln Asn
385                 390                 395                 400

gaa att cca gtt gac ctg ctg tgt cat gaa caa ttg aaa cgt act agt       1248
Glu Ile Pro Val Asp Leu Leu Cys His Glu Gln Leu Lys Arg Thr Ser
                405                 410                 415

gaa agc cag atg ctc acg cgc ggg caa ctg aag gaa tac att ggc cag       1296
Glu Ser Gln Met Leu Thr Arg Gly Gln Leu Lys Glu Tyr Ile Gly Gln
            420                 425                 430

cag cgc ggc gag ggc tat gtc ttc tac gag aac cgt gca tac ggc gtg       1344
Gln Arg Gly Glu Gly Tyr Val Phe Tyr Glu Asn Arg Ala Tyr Gly Val
        435                 440                 445

gcg ggg aaa agc ctg ttc gac gat ggg ctg gga gcc gcg ccc ggc gtg       1392
Ala Gly Lys Ser Leu Phe Asp Asp Gly Leu Gly Ala Ala Pro Gly Val
    450                 455                 460

ccg agc gga cgt tcg aag ttc tcg ccg gat gta ctg gaa acg gtg ccg       1440
Pro Ser Gly Arg Ser Lys Phe Ser Pro Asp Val Leu Glu Thr Val Pro
465                 470                 475                 480

gcg tca ccc gga ttg cgg cgg ccg tcg ctg ggc gca gtg gaa cgc cag       1488
Ala Ser Pro Gly Leu Arg Arg Pro Ser Leu Gly Ala Val Glu Arg Gln
                485                 490                 495

gat tcc ggc tat gac agc ctt gat ggg gtg gga tcg cga tcg ttc tcg       1536
Asp Ser Gly Tyr Asp Ser Leu Asp Gly Val Gly Ser Arg Ser Phe Ser
            500                 505                 510

ttg ggc gag gtg tcc gac atg gcc gcc gtg gaa gcg gcg gaa ctg gaa       1584
Leu Gly Glu Val Ser Asp Met Ala Ala Val Glu Ala Ala Glu Leu Glu
        515                 520                 525

atg acc cgg caa gtc ttg cac gcc ggg gcg cgg cag gac gat gcc gag       1632
Met Thr Arg Gln Val Leu His Ala Gly Ala Arg Gln Asp Asp Ala Glu
    530                 535                 540

ccg ggc gtg agc ggt gcg tcg gcg cac tgg ggg cag cgg gcg ctg cag       1680
Pro Gly Val Ser Gly Ala Ser Ala His Trp Gly Gln Arg Ala Leu Gln
545                 550                 555                 560

ggc gcc cag gcg gtg gcg gcg gcg cag cgg ctg gtt cat gcc att gcc       1728
Gly Ala Gln Ala Val Ala Ala Ala Gln Arg Leu Val His Ala Ile Ala
                565                 570                 575
```

```
ctg atg acg caa ttc ggc cgg gcc ggt tcc acc aac acg ccg cag gaa        1776
Leu Met Thr Gln Phe Gly Arg Ala Gly Ser Thr Asn Thr Pro Gln Glu
            580                 585                 590

gcg gcc tcg ttg tcg gcg gcc gtg ttc ggc ttg ggc gag gcc agc agc        1824
Ala Ala Ser Leu Ser Ala Ala Val Phe Gly Leu Gly Glu Ala Ser Ser
        595                 600                 605

gcc gtg gcc gaa acc gtg agc ggt ttt ttc cgc ggg tct tcg cgc tgg        1872
Ala Val Ala Glu Thr Val Ser Gly Phe Phe Arg Gly Ser Ser Arg Trp
    610                 615                 620

gcc ggc ggt ttc ggc gtg gct ggc ggc gcg atg gcg ctg gga ggc ggc        1920
Ala Gly Gly Phe Gly Val Ala Gly Gly Ala Met Ala Leu Gly Gly Gly
625                 630                 635                 640

atc gcc gcg gcc gtt ggc gcc ggg atg tcg ttg acc gat gac gcg ccg        1968
Ile Ala Ala Ala Val Gly Ala Gly Met Ser Leu Thr Asp Asp Ala Pro
                645                 650                 655

gcc gga cag aag gcc gcc gcc ggc gcc gag atc gcg ctg cag ttg aca        2016
Ala Gly Gln Lys Ala Ala Ala Gly Ala Glu Ile Ala Leu Gln Leu Thr
            660                 665                 670

ggt gga acg gtc gag ctg gct tct tcc atc gcg ttg gcg ctg gcc gcg        2064
Gly Gly Thr Val Glu Leu Ala Ser Ser Ile Ala Leu Ala Leu Ala Ala
        675                 680                 685

gcg cgc ggc gtg acc agc ggc ttg cag gtg gcc ggg gcg tcg gcc ggg        2112
Ala Arg Gly Val Thr Ser Gly Leu Gln Val Ala Gly Ala Ser Ala Gly
    690                 695                 700

gcg gct gcc ggc gca ttg gcc gcg gcg ctc agt ccc atg gag atc tac        2160
Ala Ala Ala Gly Ala Leu Ala Ala Ala Leu Ser Pro Met Glu Ile Tyr
705                 710                 715                 720

ggc ctg gtg cag caa tcg cac tat gcg gat cag ctg gac aag ctg gcg        2208
Gly Leu Val Gln Gln Ser His Tyr Ala Asp Gln Leu Asp Lys Leu Ala
                725                 730                 735

cag gaa tcg agc gca tac ggt tac gag ggc gac gcc ttg ctg gcc cag        2256
Gln Glu Ser Ser Ala Tyr Gly Tyr Glu Gly Asp Ala Leu Leu Ala Gln
            740                 745                 750

ctg tat cgc gac aag acg gcc gcc gag ggc gcc gtc gcc ggc gtc tcc        2304
Leu Tyr Arg Asp Lys Thr Ala Ala Glu Gly Ala Val Ala Gly Val Ser
        755                 760                 765

gcc gtc ctg agc acg gtg ggg gcg gcg gtg tcg atc gcc gcg gcg gcc        2352
Ala Val Leu Ser Thr Val Gly Ala Ala Val Ser Ile Ala Ala Ala Ala
    770                 775                 780

agc gtg gta ggg gcc ccg gtg gcg gtg gtc act tcc ttg ctg acc ggg        2400
Ser Val Val Gly Ala Pro Val Ala Val Val Thr Ser Leu Leu Thr Gly
785                 790                 795                 800

gct ctc aac ggc atc ctg cgc ggc gtg cag cag ccc atc atc gaa aag        2448
Ala Leu Asn Gly Ile Leu Arg Gly Val Gln Gln Pro Ile Ile Glu Lys
                805                 810                 815

ctg gcc aac gat tac gct cgc aag atc gac gag ctg ggc ggg ccg caa        2496
Leu Ala Asn Asp Tyr Ala Arg Lys Ile Asp Glu Leu Gly Gly Pro Gln
            820                 825                 830
```

```
gcg tac ttc gag aaa aac ctg cag gcg cgt cac gaa caa ctg gcc aat        2544
Ala Tyr Phe Glu Lys Asn Leu Gln Ala Arg His Glu Gln Leu Ala Asn
        835                 840                 845

tcg gac ggc cta cgg aaa atg ctg gcc gac ctg cag gcc ggt tgg aac        2592
Ser Asp Gly Leu Arg Lys Met Leu Ala Asp Leu Gln Ala Gly Trp Asn
    850                 855                 860

gcc agc agc gtg atc ggg gtg cag acg aca gag atc tcc aag tcg gcg        2640
Ala Ser Ser Val Ile Gly Val Gln Thr Thr Glu Ile Ser Lys Ser Ala
865                 870                 875                 880

ctc gaa ctg gcc gcc att acc ggc aac gcg gac aac ctg aaa tcc gtc        2688
Leu Glu Leu Ala Ala Ile Thr Gly Asn Ala Asp Asn Leu Lys Ser Val
                885                 890                 895

gac gtg ttc gtg gac cgc ttc gtc cag ggc gag cgg gtg gcc ggc cag        2736
Asp Val Phe Val Asp Arg Phe Val Gln Gly Glu Arg Val Ala Gly Gln
            900                 905                 910

ccg gtg gtc ctc gac gtc gcc gcc ggc ggc atc gat atc gcc agc cgc        2784
Pro Val Val Leu Asp Val Ala Ala Gly Gly Ile Asp Ile Ala Ser Arg
        915                 920                 925

aag ggc gag cgg ccg gcg ctg acg ttc atc acg ccg ctg gcc gcg cca        2832
Lys Gly Glu Arg Pro Ala Leu Thr Phe Ile Thr Pro Leu Ala Ala Pro
    930                 935                 940

gga gaa gag cag cgc cgg cgc acg aaa acg ggc aag agc gaa ttc acc        2880
Gly Glu Glu Gln Arg Arg Arg Thr Lys Thr Gly Lys Ser Glu Phe Thr
945                 950                 955                 960

aca ttc gtc gag atc gtg ggc aag cag gac cgc tgg cgc atc cgg gac        2928
Thr Phe Val Glu Ile Val Gly Lys Gln Asp Arg Trp Arg Ile Arg Asp
                965                 970                 975

ggc gcg gcc gac acc acc atc gat ctg gcc aag gtg gtg tcg caa ctg        2976
Gly Ala Ala Asp Thr Thr Ile Asp Leu Ala Lys Val Val Ser Gln Leu
            980                 985                 990

gtc gac gcc aat ggc gtg ctc aag  cac agc atc aaa ctg  gat gtg atc      3024
Val Asp Ala Asn Gly Val Leu Lys  His Ser Ile Lys Leu  Asp Val Ile
        995                 1000                1005

ggc gga  gat ggc gat gac gtc  gtg ctt gcc aat gct  tcg cgc atc         3069
Gly Gly  Asp Gly Asp Asp Val  Val Leu Ala Asn Ala  Ser Arg Ile
    1010                 1015                 1020

cat tat  gac ggc ggc gcg ggc  acc aac acg gtc agc  tat gcc gcc         3114
His Tyr  Asp Gly Gly Ala Gly  Thr Asn Thr Val Ser  Tyr Ala Ala
    1025                 1030                 1035

ctg ggt  cga cag gat tcc att  acc gtg tcc gcc gac  ggg gaa cgt         3159
Leu Gly  Arg Gln Asp Ser Ile  Thr Val Ser Ala Asp  Gly Glu Arg
    1040                 1045                 1050

ttc aac  gtg cgc aag cag ttg  aac aac gcc aac gtg  tat cgc gaa         3204
Phe Asn  Val Arg Lys Gln Leu  Asn Asn Ala Asn Val  Tyr Arg Glu
    1055                 1060                 1065

ggc gtg  gct acc cag aca acc  gcc tac ggc aag cgc  acg gag aat         3249
Gly Val  Ala Thr Gln Thr Thr  Ala Tyr Gly Lys Arg  Thr Glu Asn
```

```
    1070                1075                1080

gtc caa  tac cgc cat gtc gag  ctg gcc cgt gtc ggg  caa ctg gtg        3294
Val Gln  Tyr Arg His Val Glu  Leu Ala Arg Val Gly  Gln Leu Val
    1085                1090                1095

gag gtc  gac acg ctc gag cat  gtg cag cac atc atc  ggc ggg gcc        3339
Glu Val  Asp Thr Leu Glu His  Val Gln His Ile Ile  Gly Gly Ala
    1100                1105                1110

ggc aac  gat tcg atc acc ggc  aat gcg cac gac aac  ttc cta gcc        3384
Gly Asn  Asp Ser Ile Thr Gly  Asn Ala His Asp Asn  Phe Leu Ala
    1115                1120                1125

ggc ggg  tcg ggc gac gac agg  ctg gat ggc ggc gcc  ggc aac gac        3429
Gly Gly  Ser Gly Asp Asp Arg  Leu Asp Gly Gly Ala  Gly Asn Asp
    1130                1135                1140

acc ctg  gtt ggc ggc gag ggc  caa aac acg gtc atc  ggc ggc gcc        3474
Thr Leu  Val Gly Gly Glu Gly  Gln Asn Thr Val Ile  Gly Gly Ala
    1145                1150                1155

ggc gac  gac gta ttc ctg cag  gac ctg ggg gta tgg  agc aac cag        3519
Gly Asp  Asp Val Phe Leu Gln  Asp Leu Gly Val Trp  Ser Asn Gln
    1160                1165                1170

ctc gat  ggc ggc gcg ggc gtc  gat acc gtg aag tac  aac gtg cac        3564
Leu Asp  Gly Gly Ala Gly Val  Asp Thr Val Lys Tyr  Asn Val His
    1175                1180                1185

cag cct  tcc gag gag cgc ctc  gaa cgc atg ggc gac  acg ggc atc        3609
Gln Pro  Ser Glu Glu Arg Leu  Glu Arg Met Gly Asp  Thr Gly Ile
    1190                1195                1200

cat gcc  gat ctt caa aag ggc  acg gtc gag aag tgg  ccg gcc ctg        3654
His Ala  Asp Leu Gln Lys Gly  Thr Val Glu Lys Trp  Pro Ala Leu
    1205                1210                1215

aac ctg  ttc agc gtc gac cat  gtc aag aat atc gag  aat ctg cac        3699
Asn Leu  Phe Ser Val Asp His  Val Lys Asn Ile Glu  Asn Leu His
    1220                1225                1230

ggc tcc  cgc cta aac gac cgc  atc gcc ggc gac gac  cag gac aac        3744
Gly Ser  Arg Leu Asn Asp Arg  Ile Ala Gly Asp Asp  Gln Asp Asn
    1235                1240                1245

gag ctc  tgg ggc cac gat ggc  aac gac acg ata cgc  ggc cgg ggc        3789
Glu Leu  Trp Gly His Asp Gly  Asn Asp Thr Ile Arg  Gly Arg Gly
    1250                1255                1260

ggc gac  gac atc ctg cgc ggc  ggc ctg ggc ctg gac  acg ctg tat        3834
Gly Asp  Asp Ile Leu Arg Gly  Gly Leu Gly Leu Asp  Thr Leu Tyr
    1265                1270                1275

ggc gag  gac ggc aac gac atc  ttc ctg cag gac gac  gag acc gtc        3879
Gly Glu  Asp Gly Asn Asp Ile  Phe Leu Gln Asp Asp  Glu Thr Val
    1280                1285                1290

agc gat  gac atc gac ggc ggc  gcg ggg ctg gac acc  gtc gac tac        3924
Ser Asp  Asp Ile Asp Gly Gly  Ala Gly Leu Asp Thr  Val Asp Tyr
    1295                1300                1305

tcc gcc  atg atc cat cca ggc  agg atc gtt gcg ccg  cat gaa tac        3969
```

```
Ser Ala  Met Ile His Pro Gly  Arg Ile Val Ala Pro  His Glu Tyr
    1310                 1315                 1320

ggc ttc  ggg atc gag gcg gac  ctg tcc agg gaa tgg  gtg cgc aag        4014
Gly Phe  Gly Ile Glu Ala Asp  Leu Ser Arg Glu Trp  Val Arg Lys
    1325                 1330                 1335

gcg tcc  gcg ctg ggc gtg gac  tat tac gat aat gtc  cgc aat gtc        4059
Ala Ser  Ala Leu Gly Val Asp  Tyr Tyr Asp Asn Val  Arg Asn Val
    1340                 1345                 1350

gaa aac  gtc atc ggt acg agc  atg aag gat gtg ctc  atc ggc gac        4104
Glu Asn  Val Ile Gly Thr Ser  Met Lys Asp Val Leu  Ile Gly Asp
    1355                 1360                 1365

gcg caa  gcc aat acc ctg atg  ggc cag ggc ggc gac  gat acc gtg        4149
Ala Gln  Ala Asn Thr Leu Met  Gly Gln Gly Gly Asp  Asp Thr Val
    1370                 1375                 1380

cgc ggc  ggc gac ggc gat gat  ctg ctg ttc ggc ggc  gac ggc aac        4194
Arg Gly  Gly Asp Gly Asp Asp  Leu Leu Phe Gly Gly  Asp Gly Asn
    1385                 1390                 1395

gac atg  ctg tat ggc gac gcc  ggc aac gac acc ctc  tac ggg ggg        4239
Asp Met  Leu Tyr Gly Asp Ala  Gly Asn Asp Thr Leu  Tyr Gly Gly
    1400                 1405                 1410

ctg ggc  gac gat acc ctt gaa  ggc ggc gcg ggc aac  gat tgg ttc        4284
Leu Gly  Asp Asp Thr Leu Glu  Gly Gly Ala Gly Asn  Asp Trp Phe
    1415                 1420                 1425

ggc cag  acg cag gcg cgc gag  cat gac gtg ctg cgc  ggc gga gat        4329
Gly Gln  Thr Gln Ala Arg Glu  His Asp Val Leu Arg  Gly Gly Asp
    1430                 1435                 1440

ggg gtg  gat acc gtc gat tac  agc cag acc ggc gcg  cat gcc ggc        4374
Gly Val  Asp Thr Val Asp Tyr  Ser Gln Thr Gly Ala  His Ala Gly
    1445                 1450                 1455

att gcc  gcg ggt cgc atc ggg  ctg ggc atc ctg gct  gac ctg ggc        4419
Ile Ala  Ala Gly Arg Ile Gly  Leu Gly Ile Leu Ala  Asp Leu Gly
    1460                 1465                 1470

gcc ggc  cgc gtc gac aag ctg  ggc gag gcc ggc agc  agc gcc tac        4464
Ala Gly  Arg Val Asp Lys Leu  Gly Glu Ala Gly Ser  Ser Ala Tyr
    1475                 1480                 1485

gat acg  gtt tcc ggt atc gag  aac gtg gtg ggc acg  gaa ctg gcc        4509
Asp Thr  Val Ser Gly Ile Glu  Asn Val Val Gly Thr  Glu Leu Ala
    1490                 1495                 1500

gac cgc  atc acg ggc gat gcg  cag gcc aac gtg ctg  cgc ggc gcg        4554
Asp Arg  Ile Thr Gly Asp Ala  Gln Ala Asn Val Leu  Arg Gly Ala
    1505                 1510                 1515

ggt ggc  gcc gac gtg ctt gcg  ggc ggc gag ggc gac  gat gtg ctg        4599
Gly Gly  Ala Asp Val Leu Ala  Gly Gly Glu Gly Asp  Asp Val Leu
    1520                 1525                 1530

ctg ggc  ggc gac ggc gac gac  cag ctg tcg ggc gac  gcc gga cgc        4644
Leu Gly  Gly Asp Gly Asp Asp  Gln Leu Ser Gly Asp  Ala Gly Arg
    1535                 1540                 1545
```

```
gat cgc ttg tac ggc gaa gcc ggt gac gac tgg ttc ttc cag gat      4689
Asp Arg Leu Tyr Gly Glu Ala Gly Asp Asp Trp Phe Phe Gln Asp
    1550                1555                1560

gcc gcc aat gcc ggc aat ctg ctc gac ggc ggc gac ggc cgc gat      4734
Ala Ala Asn Ala Gly Asn Leu Leu Asp Gly Gly Asp Gly Arg Asp
    1565                1570                1575

acc gtg gat ttc agc ggc ccg ggc cgg ggc ctc gac gcc ggc gca      4779
Thr Val Asp Phe Ser Gly Pro Gly Arg Gly Leu Asp Ala Gly Ala
    1580                1585                1590

aag ggc gta ttc ctg agc ttg ggc aag ggg ttc gcc agc ctg atg      4824
Lys Gly Val Phe Leu Ser Leu Gly Lys Gly Phe Ala Ser Leu Met
    1595                1600                1605

gac gaa ccc gaa acc agc aac gtg ttg cgc aat atc gag aac gcc      4869
Asp Glu Pro Glu Thr Ser Asn Val Leu Arg Asn Ile Glu Asn Ala
    1610                1615                1620

gtg ggc agc gcg cgt gat gac gtg ctg atc ggc gac gca ggc gcc      4914
Val Gly Ser Ala Arg Asp Asp Val Leu Ile Gly Asp Ala Gly Ala
    1625                1630                1635

aac gtc ctc aat ggc ctg gcg ggc aac gac gtg ctg tcc ggc ggc      4959
Asn Val Leu Asn Gly Leu Ala Gly Asn Asp Val Leu Ser Gly Gly
    1640                1645                1650

gct ggc gac gat gtg ctg ctg ggc gac gag ggc tcg gac ctg ctc      5004
Ala Gly Asp Asp Val Leu Leu Gly Asp Glu Gly Ser Asp Leu Leu
    1655                1660                1665

agc ggc gat gcg ggc aac gac gat ctg ttc ggc ggg cag ggc gat      5049
Ser Gly Asp Ala Gly Asn Asp Asp Leu Phe Gly Gly Gln Gly Asp
    1670                1675                1680

gat act tat ctg ttc ggg gtc ggg tac ggg cac gac acg atc tac      5094
Asp Thr Tyr Leu Phe Gly Val Gly Tyr Gly His Asp Thr Ile Tyr
    1685                1690                1695

gaa tcg ggc ggc ggc cat gac acc atc cgc atc aac gcg ggg gcg      5139
Glu Ser Gly Gly Gly His Asp Thr Ile Arg Ile Asn Ala Gly Ala
    1700                1705                1710

gac cag ctg tgg ttc gcg cgc cag ggc aac gac ctg gag atc cgc      5184
Asp Gln Leu Trp Phe Ala Arg Gln Gly Asn Asp Leu Glu Ile Arg
    1715                1720                1725

att ctc ggc acc gac gat gca ctt acc gtg cac gac tgg tat cgc      5229
Ile Leu Gly Thr Asp Asp Ala Leu Thr Val His Asp Trp Tyr Arg
    1730                1735                1740

gac gcc gat cac cgg gtg gaa atc atc cat gcc gcc aac cag gcg      5274
Asp Ala Asp His Arg Val Glu Ile Ile His Ala Ala Asn Gln Ala
    1745                1750                1755

gta gac cag gca ggc atc gaa aag ctg gtc gag gca atg gcg cag      5319
Val Asp Gln Ala Gly Ile Glu Lys Leu Val Glu Ala Met Ala Gln
    1760                1765                1770

tat ccg gac ccc ggc gcg gcg gcg gct gcc ccg ccg gcg gcg cgc      5364
Tyr Pro Asp Pro Gly Ala Ala Ala Ala Ala Pro Pro Ala Ala Arg
    1775                1780                1785
```

```
gtg ccg  gac acg ctg atg cag  tcc ctg gct gtc aac  tgg cgc tga        5409
Val Pro  Asp Thr Leu Met Gln  Ser Leu Ala Val Asn  Trp Arg
    1790                1795                1800
```

<210>  69
<211>  1802
<212>  PRT
<213>  Artificial Sequence

<220>
<223>  Synthetic Construct

<400>  69

```
Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Leu Gln Ile Asp
            180                 185                 190
```

Met Phe Ala Ile Met Pro His Leu Ser Asn Phe Arg Asp Ser Ala Arg
195 200 205

Ser Ser Val Thr Ser Gly Asp Ser Val Thr Asp Tyr Leu Ala Arg Thr
210 215 220

Arg Arg Ala Ser Gly Val Asn His Gln His Leu Pro Ala Arg Arg Ala
225 230 235 240

Glu Pro Gln Arg His Thr Met Leu Cys Met Cys Cys Lys Cys Glu Ala
245 250 255

Arg Ile Glu Leu Val Val Glu Ser Ser Ala Asp Asp Leu Arg Ala Phe
260 265 270

Gln Gln Leu Phe Leu Asn Thr Leu Ser Phe Val Cys Pro Trp Cys Ala
275 280 285

Ser Gln Gln Gly Thr Arg Ala Arg Leu Lys Leu Leu Trp Lys Ile Ala
290 295 300

Arg Ala Gly Ala Arg Ser Ala Val Gly Thr Glu Ala Arg Arg Gln Phe
305 310 315 320

Arg Tyr Asp Gly Asp Met Asn Ile Gly Val Ile Thr Asp Phe Glu Leu
325 330 335

Glu Val Arg Asn Ala Leu Asn Arg Arg Ala His Ala Val Gly Ala Gln
340 345 350

Asp Val Val Gln His Gly Thr Glu Gln Asn Asn Pro Phe Pro Glu Ala
355 360 365

Asp Glu Lys Ile Phe Val Val Ser Ala Thr Gly Lys Arg Met His Gly
370 375 380

Pro Lys Ala Thr Leu Gln Asp Ile Val Leu His Leu Glu Pro Gln Asn
385 390 395 400

Glu Ile Pro Val Asp Leu Leu Cys His Glu Gln Leu Lys Arg Thr Ser
405 410 415

Glu Ser Gln Met Leu Thr Arg Gly Gln Leu Lys Glu Tyr Ile Gly Gln
420 425 430

Gln Arg Gly Glu Gly Tyr Val Phe Tyr Glu Asn Arg Ala Tyr Gly Val
435 440 445

```
Ala Gly Lys Ser Leu Phe Asp Asp Gly Leu Gly Ala Ala Pro Gly Val
    450                 455                 460

Pro Ser Gly Arg Ser Lys Phe Ser Pro Asp Val Leu Glu Thr Val Pro
465                 470                 475                 480

Ala Ser Pro Gly Leu Arg Arg Pro Ser Leu Gly Ala Val Glu Arg Gln
                485                 490                 495

Asp Ser Gly Tyr Asp Ser Leu Asp Gly Val Gly Ser Arg Ser Phe Ser
            500                 505                 510

Leu Gly Glu Val Ser Asp Met Ala Ala Val Glu Ala Ala Glu Leu Glu
        515                 520                 525

Met Thr Arg Gln Val Leu His Ala Gly Ala Arg Gln Asp Asp Ala Glu
    530                 535                 540

Pro Gly Val Ser Gly Ala Ser Ala His Trp Gly Gln Arg Ala Leu Gln
545                 550                 555                 560

Gly Ala Gln Ala Val Ala Ala Ala Gln Arg Leu Val His Ala Ile Ala
                565                 570                 575

Leu Met Thr Gln Phe Gly Arg Ala Gly Ser Thr Asn Thr Pro Gln Glu
            580                 585                 590

Ala Ala Ser Leu Ser Ala Ala Val Phe Gly Leu Gly Glu Ala Ser Ser
        595                 600                 605

Ala Val Ala Glu Thr Val Ser Gly Phe Phe Arg Gly Ser Ser Arg Trp
    610                 615                 620

Ala Gly Gly Phe Gly Val Ala Gly Gly Ala Met Ala Leu Gly Gly Gly
625                 630                 635                 640

Ile Ala Ala Ala Val Gly Ala Gly Met Ser Leu Thr Asp Asp Ala Pro
                645                 650                 655

Ala Gly Gln Lys Ala Ala Ala Gly Ala Glu Ile Ala Leu Gln Leu Thr
            660                 665                 670

Gly Gly Thr Val Glu Leu Ala Ser Ser Ile Ala Leu Ala Leu Ala Ala
        675                 680                 685

Ala Arg Gly Val Thr Ser Gly Leu Gln Val Ala Gly Ala Ser Ala Gly
```

690 695 700

Ala Ala Ala Gly Ala Leu Ala Ala Ala Leu Ser Pro Met Glu Ile Tyr
705 710 715 720

Gly Leu Val Gln Gln Ser His Tyr Ala Asp Gln Leu Asp Lys Leu Ala
725 730 735

Gln Glu Ser Ser Ala Tyr Gly Tyr Glu Gly Asp Ala Leu Leu Ala Gln
740 745 750

Leu Tyr Arg Asp Lys Thr Ala Ala Glu Gly Ala Val Ala Gly Val Ser
755 760 765

Ala Val Leu Ser Thr Val Gly Ala Ala Val Ser Ile Ala Ala Ala Ala
770 775 780

Ser Val Val Gly Ala Pro Val Ala Val Val Thr Ser Leu Leu Thr Gly
785 790 795 800

Ala Leu Asn Gly Ile Leu Arg Gly Val Gln Gln Pro Ile Ile Glu Lys
805 810 815

Leu Ala Asn Asp Tyr Ala Arg Lys Ile Asp Glu Leu Gly Gly Pro Gln
820 825 830

Ala Tyr Phe Glu Lys Asn Leu Gln Ala Arg His Glu Gln Leu Ala Asn
835 840 845

Ser Asp Gly Leu Arg Lys Met Leu Ala Asp Leu Gln Ala Gly Trp Asn
850 855 860

Ala Ser Ser Val Ile Gly Val Gln Thr Thr Glu Ile Ser Lys Ser Ala
865 870 875 880

Leu Glu Leu Ala Ala Ile Thr Gly Asn Ala Asp Asn Leu Lys Ser Val
885 890 895

Asp Val Phe Val Asp Arg Phe Val Gln Gly Glu Arg Val Ala Gly Gln
900 905 910

Pro Val Val Leu Asp Val Ala Ala Gly Gly Ile Asp Ile Ala Ser Arg
915 920 925

Lys Gly Glu Arg Pro Ala Leu Thr Phe Ile Thr Pro Leu Ala Ala Pro
930 935 940

Gly Glu Glu Gln Arg Arg Arg Thr Lys Thr Gly Lys Ser Glu Phe Thr
945                 950                 955                 960

Thr Phe Val Glu Ile Val Gly Lys Gln Asp Arg Trp Arg Ile Arg Asp
                965                 970                 975

Gly Ala Ala Asp Thr Thr Ile Asp Leu Ala Lys Val Val Ser Gln Leu
            980                 985                 990

Val Asp Ala Asn Gly Val Leu Lys His Ser Ile Lys Leu Asp Val Ile
        995                 1000                1005

Gly Gly Asp Gly Asp Asp Val Val Leu Ala Asn Ala Ser Arg Ile
    1010                1015                1020

His Tyr Asp Gly Gly Ala Gly Thr Asn Thr Val Ser Tyr Ala Ala
    1025                1030                1035

Leu Gly Arg Gln Asp Ser Ile Thr Val Ser Ala Asp Gly Glu Arg
    1040                1045                1050

Phe Asn Val Arg Lys Gln Leu Asn Asn Ala Asn Val Tyr Arg Glu
    1055                1060                1065

Gly Val Ala Thr Gln Thr Thr Ala Tyr Gly Lys Arg Thr Glu Asn
    1070                1075                1080

Val Gln Tyr Arg His Val Glu Leu Ala Arg Val Gly Gln Leu Val
    1085                1090                1095

Glu Val Asp Thr Leu Glu His Val Gln His Ile Ile Gly Gly Ala
    1100                1105                1110

Gly Asn Asp Ser Ile Thr Gly Asn Ala His Asp Asn Phe Leu Ala
    1115                1120                1125

Gly Gly Ser Gly Asp Asp Arg Leu Asp Gly Gly Ala Gly Asn Asp
    1130                1135                1140

Thr Leu Val Gly Gly Glu Gly Gln Asn Thr Val Ile Gly Gly Ala
    1145                1150                1155

Gly Asp Asp Val Phe Leu Gln Asp Leu Gly Val Trp Ser Asn Gln
    1160                1165                1170

Leu Asp Gly Gly Ala Gly Val Asp Thr Val Lys Tyr Asn Val His
    1175                1180                1185

```
Gln Pro  Ser Glu Glu Arg Leu  Glu Arg Met Gly Asp  Thr Gly Ile
    1190                 1195                 1200

His Ala  Asp Leu Gln Lys Gly  Thr Val Glu Lys Trp  Pro Ala Leu
    1205                 1210                 1215

Asn Leu  Phe Ser Val Asp His  Val Lys Asn Ile Glu  Asn Leu His
    1220                 1225                 1230

Gly Ser  Arg Leu Asn Asp Arg  Ile Ala Gly Asp Asp  Gln Asp Asn
    1235                 1240                 1245

Glu Leu  Trp Gly His Asp Gly  Asn Asp Thr Ile Arg  Gly Arg Gly
    1250                 1255                 1260

Gly Asp  Asp Ile Leu Arg Gly  Gly Leu Gly Leu Asp  Thr Leu Tyr
    1265                 1270                 1275

Gly Glu  Asp Gly Asn Asp Ile  Phe Leu Gln Asp Asp  Glu Thr Val
    1280                 1285                 1290

Ser Asp  Asp Ile Asp Gly Gly  Ala Gly Leu Asp Thr  Val Asp Tyr
    1295                 1300                 1305

Ser Ala  Met Ile His Pro Gly  Arg Ile Val Ala Pro  His Glu Tyr
    1310                 1315                 1320

Gly Phe  Gly Ile Glu Ala Asp  Leu Ser Arg Glu Trp  Val Arg Lys
    1325                 1330                 1335

Ala Ser  Ala Leu Gly Val Asp  Tyr Tyr Asp Asn Val  Arg Asn Val
    1340                 1345                 1350

Glu Asn  Val Ile Gly Thr Ser  Met Lys Asp Val Leu  Ile Gly Asp
    1355                 1360                 1365

Ala Gln  Ala Asn Thr Leu Met  Gly Gln Gly Gly Asp  Asp Thr Val
    1370                 1375                 1380

Arg Gly  Gly Asp Gly Asp Asp  Leu Leu Phe Gly Gly  Asp Gly Asn
    1385                 1390                 1395

Asp Met  Leu Tyr Gly Asp Ala  Gly Asn Asp Thr Leu  Tyr Gly Gly
    1400                 1405                 1410

Leu Gly  Asp Asp Thr Leu Glu  Gly Gly Ala Gly Asn  Asp Trp Phe
    1415                 1420                 1425
```

Gly Gln Thr Gln Ala Arg Glu His Asp Val Leu Arg Gly Gly Asp
1430 1435 1440

Gly Val Asp Thr Val Asp Tyr Ser Gln Thr Gly Ala His Ala Gly
1445 1450 1455

Ile Ala Ala Gly Arg Ile Gly Leu Gly Ile Leu Ala Asp Leu Gly
1460 1465 1470

Ala Gly Arg Val Asp Lys Leu Gly Glu Ala Gly Ser Ser Ala Tyr
1475 1480 1485

Asp Thr Val Ser Gly Ile Glu Asn Val Val Gly Thr Glu Leu Ala
1490 1495 1500

Asp Arg Ile Thr Gly Asp Ala Gln Ala Asn Val Leu Arg Gly Ala
1505 1510 1515

Gly Gly Ala Asp Val Leu Ala Gly Gly Glu Gly Asp Asp Val Leu
1520 1525 1530

Leu Gly Gly Asp Gly Asp Asp Gln Leu Ser Gly Asp Ala Gly Arg
1535 1540 1545

Asp Arg Leu Tyr Gly Glu Ala Gly Asp Asp Trp Phe Phe Gln Asp
1550 1555 1560

Ala Ala Asn Ala Gly Asn Leu Leu Asp Gly Gly Asp Gly Arg Asp
1565 1570 1575

Thr Val Asp Phe Ser Gly Pro Gly Arg Gly Leu Asp Ala Gly Ala
1580 1585 1590

Lys Gly Val Phe Leu Ser Leu Gly Lys Gly Phe Ala Ser Leu Met
1595 1600 1605

Asp Glu Pro Glu Thr Ser Asn Val Leu Arg Asn Ile Glu Asn Ala
1610 1615 1620

Val Gly Ser Ala Arg Asp Asp Val Leu Ile Gly Asp Ala Gly Ala
1625 1630 1635

Asn Val Leu Asn Gly Leu Ala Gly Asn Asp Val Leu Ser Gly Gly
1640 1645 1650

Ala Gly Asp Asp Val Leu Leu Gly Asp Glu Gly Ser Asp Leu Leu

```
    1655                1660                1665

Ser Gly  Asp Ala Gly Asn Asp  Asp Leu Phe Gly Gly  Gln Gly Asp
    1670                1675                1680

Asp Thr  Tyr Leu Phe Gly Val  Gly Tyr Gly His Asp  Thr Ile Tyr
    1685                1690                1695

Glu Ser  Gly Gly Gly His Asp  Thr Ile Arg Ile Asn  Ala Gly Ala
    1700                1705                1710

Asp Gln  Leu Trp Phe Ala Arg  Gln Gly Asn Asp Leu  Glu Ile Arg
    1715                1720                1725

Ile Leu  Gly Thr Asp Asp Ala  Leu Thr Val His Asp  Trp Tyr Arg
    1730                1735                1740

Asp Ala  Asp His Arg Val Glu  Ile Ile His Ala Ala  Asn Gln Ala
    1745                1750                1755

Val Asp  Gln Ala Gly Ile Glu  Lys Leu Val Glu Ala  Met Ala Gln
    1760                1765                1770

Tyr Pro  Asp Pro Gly Ala Ala  Ala Ala Ala Pro Pro  Ala Ala Arg
    1775                1780                1785

Val Pro  Asp Thr Leu Met Gln  Ser Leu Ala Val Asn  Trp Arg
    1790                1795                1800


<210>  70
<211>  4512
<212>  DNA
<213>  Artificial Sequence

<220>
<223>  Nucleotide sequence encoding polypeptides 1-183 + 387-1706 of
       CyaA


<220>
<221>  CDS
<222>  (1)..(4512)

<400>  70
atg cag cag tcc cat cag gcc ggt tac gca aac gca gcc gat cgt gaa        48
Met Gln Gln Ser His Gln Ala Gly Tyr Ala Asn Ala Ala Asp Arg Glu
1               5                   10                  15

tct ggt atc ccg gca gcg gtt ctg gat ggt att aag gca gtt gcc aaa        96
Ser Gly Ile Pro Ala Ala Val Leu Asp Gly Ile Lys Ala Val Ala Lys
            20                  25                  30

gag aag aac gct acc ctg atg ttc cgt ctg gtc aac cca cac tct act       144
```

```
Glu Lys Asn Ala Thr Leu Met Phe Arg Leu Val Asn Pro His Ser Thr
        35                  40                  45

tcc ctg atc gcg gaa ggt gtg gcg acc aaa ggc ctg ggt gtg cac gcc      192
Ser Leu Ile Ala Glu Gly Val Ala Thr Lys Gly Leu Gly Val His Ala
    50                  55                  60

aaa agc agc gac tgg ggt ctg cag gca ggt tat att ccg gtg aac ccg      240
Lys Ser Ser Asp Trp Gly Leu Gln Ala Gly Tyr Ile Pro Val Asn Pro
65                  70                  75                  80

aat ctg tct aaa ctg ttc ggt cgt gcc ccg gag gtg atc gca cgt gca      288
Asn Leu Ser Lys Leu Phe Gly Arg Ala Pro Glu Val Ile Ala Arg Ala
                85                  90                  95

gac aac gat gtc aac tct tct ctg gcg cat ggt cac acc gcg gtt gat      336
Asp Asn Asp Val Asn Ser Ser Leu Ala His Gly His Thr Ala Val Asp
            100                 105                 110

ctg acc ctg tcc aaa gaa cgc ctg gat tac ctg cgt cag gcg ggc ctg      384
Leu Thr Leu Ser Lys Glu Arg Leu Asp Tyr Leu Arg Gln Ala Gly Leu
        115                 120                 125

gtg acg ggc atg gcc gac ggt gtt gta gcg agc aac cac gcg ggt tat      432
Val Thr Gly Met Ala Asp Gly Val Val Ala Ser Asn His Ala Gly Tyr
    130                 135                 140

gaa caa ttt gag ttt cgc gtt aaa gag acc tct gac ggt cgt tac gcg      480
Glu Gln Phe Glu Phe Arg Val Lys Glu Thr Ser Asp Gly Arg Tyr Ala
145                 150                 155                 160

gtg cag tac cgc cgc aaa ggt ggc gat gac ttt gaa gct gtc aaa gtg      528
Val Gln Tyr Arg Arg Lys Gly Gly Asp Asp Phe Glu Ala Val Lys Val
                165                 170                 175

atc ggt aac gct gct gga att ccg ggc ctg cgc cgt ccg tcc ctg ggc      576
Ile Gly Asn Ala Ala Gly Ile Pro Gly Leu Arg Arg Pro Ser Leu Gly
            180                 185                 190

gca gtc gaa cgt cag gac tcc ggc tac gat tcc ctg gat ggc gtt ggc      624
Ala Val Glu Arg Gln Asp Ser Gly Tyr Asp Ser Leu Asp Gly Val Gly
        195                 200                 205

tcc cgc tcc ttc tcc ctg ggc gag gtt tct gac atg gct gcg gtg gaa      672
Ser Arg Ser Phe Ser Leu Gly Glu Val Ser Asp Met Ala Ala Val Glu
    210                 215                 220

gcg gca gag ctg gaa atg acc cgt caa gtg ctg cac gcg ggc gca cgt      720
Ala Ala Glu Leu Glu Met Thr Arg Gln Val Leu His Ala Gly Ala Arg
225                 230                 235                 240

cag gac gat gct gag ccg ggc gtg tcc ggt gcg tct gca cac tgg ggt      768
Gln Asp Asp Ala Glu Pro Gly Val Ser Gly Ala Ser Ala His Trp Gly
                245                 250                 255

caa cgt gcc ctg cag ggt gct caa gct gtc gcg gca gcg cag cgt ctg      816
Gln Arg Ala Leu Gln Gly Ala Gln Ala Val Ala Ala Ala Gln Arg Leu
            260                 265                 270

gta cat gcg atc gca ctg atg acc cag ttc ggt cgc gca ggt tct acc      864
Val His Ala Ile Ala Leu Met Thr Gln Phe Gly Arg Ala Gly Ser Thr
        275                 280                 285
```

```
aac act cct cag gaa gca gct agc ctg tct gct gct gtt ttc ggt ctg       912
Asn Thr Pro Gln Glu Ala Ala Ser Leu Ser Ala Ala Val Phe Gly Leu
    290                 295                 300

ggc gaa gcg tct tct gcg gtt gca gaa acc gtg agc ggt ttc ttc cgt       960
Gly Glu Ala Ser Ser Ala Val Ala Glu Thr Val Ser Gly Phe Phe Arg
305                 310                 315                 320

ggt tct agc cgc tgg gcg ggt ggt ttt ggt gta gca ggt ggc gct atg      1008
Gly Ser Ser Arg Trp Ala Gly Gly Phe Gly Val Ala Gly Gly Ala Met
                325                 330                 335

gcc ctg ggt ggc ggt atc gcg gct gct gtc ggc gca ggt atg agc ctg      1056
Ala Leu Gly Gly Gly Ile Ala Ala Ala Val Gly Ala Gly Met Ser Leu
            340                 345                 350

acc gac gat gct cct gcg ggc cag aaa gcc gca gct ggc gct gaa atc      1104
Thr Asp Asp Ala Pro Ala Gly Gln Lys Ala Ala Ala Gly Ala Glu Ile
        355                 360                 365

gcg ctg cag ctg act ggt ggt acc gtt gaa ctg gct agc tct atc gcg      1152
Ala Leu Gln Leu Thr Gly Gly Thr Val Glu Leu Ala Ser Ser Ile Ala
    370                 375                 380

ctg gct ctg gca gcg gca cgt ggc gtg act tct ggc ctg caa gtc gcc      1200
Leu Ala Leu Ala Ala Ala Arg Gly Val Thr Ser Gly Leu Gln Val Ala
385                 390                 395                 400

ggt gcc tct gcg ggc gct gct gcg ggc gct ctg gca gct gca ctg tcc      1248
Gly Ala Ser Ala Gly Ala Ala Ala Gly Ala Leu Ala Ala Ala Leu Ser
                405                 410                 415

ccc atg gaa atc tac ggt ctg gta cag cag tct cac tac gca gac cag      1296
Pro Met Glu Ile Tyr Gly Leu Val Gln Gln Ser His Tyr Ala Asp Gln
            420                 425                 430

ctg gat aaa ctg gcg cag gag tct tct gca tac ggt tac gaa ggc gac      1344
Leu Asp Lys Leu Ala Gln Glu Ser Ser Ala Tyr Gly Tyr Glu Gly Asp
        435                 440                 445

gca ctg ctg gcg cag ctg tat cgt gac aaa act gct gct gaa ggt gct      1392
Ala Leu Leu Ala Gln Leu Tyr Arg Asp Lys Thr Ala Ala Glu Gly Ala
    450                 455                 460

gtg gca ggc gtt tct gcg gta ctg tct acc gtt ggc gct gcg gtt tct      1440
Val Ala Gly Val Ser Ala Val Leu Ser Thr Val Gly Ala Ala Val Ser
465                 470                 475                 480

att gcg gct gcc gca tcc gtt gta ggt gcg ccg gtt gct gta gtt acc      1488
Ile Ala Ala Ala Ala Ser Val Val Gly Ala Pro Val Ala Val Val Thr
                485                 490                 495

tct ctg ctg act ggt gca ctg aac ggt atc ctg cgt ggt gtt cag cag      1536
Ser Leu Leu Thr Gly Ala Leu Asn Gly Ile Leu Arg Gly Val Gln Gln
            500                 505                 510

cct att atc gaa aaa ctg gcg aat gat tat gcc cgt aaa atc gac gaa      1584
Pro Ile Ile Glu Lys Leu Ala Asn Asp Tyr Ala Arg Lys Ile Asp Glu
        515                 520                 525

ctg ggc ggt ccg cag gct tat ttt gaa aaa aac ctg cag gcg cgc cac      1632
Leu Gly Gly Pro Gln Ala Tyr Phe Glu Lys Asn Leu Gln Ala Arg His
    530                 535                 540
```

```
gaa cag ctg gca aac agc gac ggc ctg cgc aaa atg ctg gca gac ctg      1680
Glu Gln Leu Ala Asn Ser Asp Gly Leu Arg Lys Met Leu Ala Asp Leu
545                 550                 555                 560

caa gct ggt tgg aac gcg tct tct gtg atc ggc gtg cag acc acc gaa      1728
Gln Ala Gly Trp Asn Ala Ser Ser Val Ile Gly Val Gln Thr Thr Glu
                565                 570                 575

att agc aaa tct gcg ctg gaa ctg gct gca att act ggc aac gcg gat      1776
Ile Ser Lys Ser Ala Leu Glu Leu Ala Ala Ile Thr Gly Asn Ala Asp
            580                 585                 590

aac ctg aaa agc gtt gat gtt ttt gtc gat cgc ttc gtt cag ggc gag      1824
Asn Leu Lys Ser Val Asp Val Phe Val Asp Arg Phe Val Gln Gly Glu
        595                 600                 605

cgc gtt gct ggt cag ccg gtt gtt ctg gac gtt gcg gca ggc ggc atc      1872
Arg Val Ala Gly Gln Pro Val Val Leu Asp Val Ala Ala Gly Gly Ile
    610                 615                 620

gac atc gct agc cgc aag ggc gag cgt ccg gct ctg act ttc att acc      1920
Asp Ile Ala Ser Arg Lys Gly Glu Arg Pro Ala Leu Thr Phe Ile Thr
625                 630                 635                 640

ccg ctg gca gct ccg ggt gaa gaa cag cgt cgc cgt acc aag act ggt      1968
Pro Leu Ala Ala Pro Gly Glu Glu Gln Arg Arg Arg Thr Lys Thr Gly
                645                 650                 655

aaa agc gag ttc acc acc ttc gtg gaa atc gta ggt aaa cag gac cgc      2016
Lys Ser Glu Phe Thr Thr Phe Val Glu Ile Val Gly Lys Gln Asp Arg
            660                 665                 670

tgg cgt att cgc gac ggt gcg gcg gac acc acc atc gac ctg gca aaa      2064
Trp Arg Ile Arg Asp Gly Ala Ala Asp Thr Thr Ile Asp Leu Ala Lys
        675                 680                 685

gtt gtt tcc caa ctg gta gat gct aac ggc gtg ctg aaa cat tct att      2112
Val Val Ser Gln Leu Val Asp Ala Asn Gly Val Leu Lys His Ser Ile
    690                 695                 700

aaa ctg gat gta atc ggc ggt gac ggc gat gac gtt gta ctg gcg aac      2160
Lys Leu Asp Val Ile Gly Gly Asp Gly Asp Asp Val Val Leu Ala Asn
705                 710                 715                 720

gcg tct cgt atc cac tat gac ggc ggc gca ggt acc aac acg gtt tcc      2208
Ala Ser Arg Ile His Tyr Asp Gly Gly Ala Gly Thr Asn Thr Val Ser
                725                 730                 735

tac gca gcg ctg ggc cgc cag gat tcc atc act gtt agc gcc gat ggc      2256
Tyr Ala Ala Leu Gly Arg Gln Asp Ser Ile Thr Val Ser Ala Asp Gly
            740                 745                 750

gaa cgt ttc aac gtg cgt aaa cag ctg aat aac gca aac gtc tat cgt      2304
Glu Arg Phe Asn Val Arg Lys Gln Leu Asn Asn Ala Asn Val Tyr Arg
        755                 760                 765

gag ggt gta gct acc cag acc acc gcg tac ggt aag cgt acc gaa aac      2352
Glu Gly Val Ala Thr Gln Thr Thr Ala Tyr Gly Lys Arg Thr Glu Asn
    770                 775                 780

gtt cag tat cgt cac gtg gaa ctg gca cgt gtg ggt cag ctg gta gag      2400
Val Gln Tyr Arg His Val Glu Leu Ala Arg Val Gly Gln Leu Val Glu
```

```
785                 790                 795                 800

gtg gat acg ctg gaa cac gtg cag cac atc atc ggt ggc gcc ggt aac       2448
Val Asp Thr Leu Glu His Val Gln His Ile Ile Gly Gly Ala Gly Asn
                805                 810                 815

gat tcc att act ggt aac gct cac gac aac ttc ctg gca ggt ggt tct       2496
Asp Ser Ile Thr Gly Asn Ala His Asp Asn Phe Leu Ala Gly Gly Ser
            820                 825                 830

ggt gat gat cgt ctg gac ggt ggt gcg ggt aac gat acg ctg gta ggc       2544
Gly Asp Asp Arg Leu Asp Gly Gly Ala Gly Asn Asp Thr Leu Val Gly
        835                 840                 845

ggc gaa ggt cag aac acc gta att ggt ggt gcc ggt gac gac gtt ttc       2592
Gly Glu Gly Gln Asn Thr Val Ile Gly Gly Ala Gly Asp Asp Val Phe
    850                 855                 860

ctg cag gat ctg ggc gtt tgg agc aac cag ctg gat ggc ggt gcc ggt       2640
Leu Gln Asp Leu Gly Val Trp Ser Asn Gln Leu Asp Gly Gly Ala Gly
865                 870                 875                 880

gtt gac acg gtt aag tat aac gtt cac cag ccg tct gaa gag cgc ctg       2688
Val Asp Thr Val Lys Tyr Asn Val His Gln Pro Ser Glu Glu Arg Leu
                885                 890                 895

gaa cgt atg ggt gac act ggt att cat gcc gat ctg cag aaa ggc act       2736
Glu Arg Met Gly Asp Thr Gly Ile His Ala Asp Leu Gln Lys Gly Thr
            900                 905                 910

gtt gaa aaa tgg cct gcg ctg aac ctg ttc tct gtg gac cat gtt aag       2784
Val Glu Lys Trp Pro Ala Leu Asn Leu Phe Ser Val Asp His Val Lys
        915                 920                 925

aac att gaa aac ctg cat ggc tct cgc ctg aac gac cgt atc gct ggt       2832
Asn Ile Glu Asn Leu His Gly Ser Arg Leu Asn Asp Arg Ile Ala Gly
    930                 935                 940

gac gac cag gat aac gaa ctg tgg ggt cat gac ggc aat gat acc att       2880
Asp Asp Gln Asp Asn Glu Leu Trp Gly His Asp Gly Asn Asp Thr Ile
945                 950                 955                 960

cgc ggt cgt ggc ggc gac gat att ctg cgt ggc ggt ctg ggc ctg gat       2928
Arg Gly Arg Gly Gly Asp Asp Ile Leu Arg Gly Gly Leu Gly Leu Asp
                965                 970                 975

acc ctg tat ggt gaa gat ggt aac gat att ttc ctg caa gac gat gaa       2976
Thr Leu Tyr Gly Glu Asp Gly Asn Asp Ile Phe Leu Gln Asp Asp Glu
            980                 985                 990

acg gta tct gat gac atc gac ggt  ggt gca ggc ctg gac  acc gta gat     3024
Thr Val Ser Asp Asp Ile Asp Gly  Gly Ala Gly Leu Asp  Thr Val Asp
        995                 1000                1005

tac agc  gct atg atc cat ccg  ggt cgt atc gta gct  ccg cac gag       3069
Tyr Ser  Ala Met Ile His Pro  Gly Arg Ile Val Ala  Pro His Glu
    1010                1015                1020

tac ggc  ttc ggt atc gag gcg  gac ctg tct cgt gaa  tgg gtt cgt       3114
Tyr Gly  Phe Gly Ile Glu Ala  Asp Leu Ser Arg Glu  Trp Val Arg
    1025                1030                1035

aaa gcg  tcc gcg ctg ggt gtg  gac tac tac gat aac  gtt cgt aac       3159
```

```
Lys Ala  Ser Ala Leu Gly Val  Asp Tyr Tyr Asp Asn  Val Arg Asn
    1040                 1045                 1050

gtg gaa  aac gtg atc ggt acc  tcc atg aaa gac gtg  ctg att ggc       3204
Val Glu  Asn Val Ile Gly Thr  Ser Met Lys Asp Val  Leu Ile Gly
    1055                 1060                 1065

gac gca  cag gcc aac act ctg  atg ggc cag ggc ggt  gat gac acg       3249
Asp Ala  Gln Ala Asn Thr Leu  Met Gly Gln Gly Gly  Asp Asp Thr
    1070                 1075                 1080

gtt cgc  ggc ggc gac ggt gat  gac ctg ctg ttc ggt  ggt gac ggc       3294
Val Arg  Gly Gly Asp Gly Asp  Asp Leu Leu Phe Gly  Gly Asp Gly
    1085                 1090                 1095

aac gac  atg ctg tac ggc gac  gcg ggc aac gac acc  ctg tac ggc       3339
Asn Asp  Met Leu Tyr Gly Asp  Ala Gly Asn Asp Thr  Leu Tyr Gly
    1100                 1105                 1110

ggt ctg  ggt gac gac act ctg  gaa ggt ggt gca ggt  aac gac tgg       3384
Gly Leu  Gly Asp Asp Thr Leu  Glu Gly Gly Ala Gly  Asn Asp Trp
    1115                 1120                 1125

ttc ggc  caa acc cag gca cgc  gaa cac gac gtg ctg  cgt ggt ggc       3429
Phe Gly  Gln Thr Gln Ala Arg  Glu His Asp Val Leu  Arg Gly Gly
    1130                 1135                 1140

gac ggc  gta gac acc gtg gat  tac tcc caa act ggc  gct cac gcg       3474
Asp Gly  Val Asp Thr Val Asp  Tyr Ser Gln Thr Gly  Ala His Ala
    1145                 1150                 1155

ggt atc  gcg gcc ggt cgt atc  ggt ctg ggc att ctg  gcc gat ctg       3519
Gly Ile  Ala Ala Gly Arg Ile  Gly Leu Gly Ile Leu  Ala Asp Leu
    1160                 1165                 1170

ggt gcc  ggc cgt gtc gac aaa  ctg ggt gag gct ggc  tct agc gcc       3564
Gly Ala  Gly Arg Val Asp Lys  Leu Gly Glu Ala Gly  Ser Ser Ala
    1175                 1180                 1185

tat gat  act gtt tcc ggc atc  gaa aac gtg gta ggc  act gaa ctg       3609
Tyr Asp  Thr Val Ser Gly Ile  Glu Asn Val Val Gly  Thr Glu Leu
    1190                 1195                 1200

gca gat  cgt atc acg ggt gac  gcg caa gcg aac gtt  ctg cgt ggt       3654
Ala Asp  Arg Ile Thr Gly Asp  Ala Gln Ala Asn Val  Leu Arg Gly
    1205                 1210                 1215

gct ggc  ggt gca gac gtg ctg  gcg ggt ggt gaa ggc  gac gac gta       3699
Ala Gly  Gly Ala Asp Val Leu  Ala Gly Gly Glu Gly  Asp Asp Val
    1220                 1225                 1230

ctg ctg  ggc ggt gac ggt gac  gat cag ctg agc ggt  gat gcg ggt       3744
Leu Leu  Gly Gly Asp Gly Asp  Asp Gln Leu Ser Gly  Asp Ala Gly
    1235                 1240                 1245

cgt gac  cgt ctg tac ggt gaa  gca ggt gac gat tgg  ttc ttc cag       3789
Arg Asp  Arg Leu Tyr Gly Glu  Ala Gly Asp Asp Trp  Phe Phe Gln
    1250                 1255                 1260

gac gct  gct aac gct ggc aac  ctg ctg gac ggc ggc  gat ggc cgt       3834
Asp Ala  Ala Asn Ala Gly Asn  Leu Leu Asp Gly Gly  Asp Gly Arg
    1265                 1270                 1275
```

```
gac act  gtt gac ttc agc ggt  ccg ggt cgc ggt ctg  gac gca ggc        3879
Asp Thr  Val Asp Phe Ser Gly  Pro Gly Arg Gly Leu  Asp Ala Gly
    1280                 1285                 1290

gcg aaa  ggc gtt ttc ctg agc  ctg ggt aag ggt ttc  gca tct ctg        3924
Ala Lys  Gly Val Phe Leu Ser  Leu Gly Lys Gly Phe  Ala Ser Leu
    1295                 1300                 1305

atg gac  gaa cca gaa acc agc  aat gtc ctg cgt aac  atc gaa aac        3969
Met Asp  Glu Pro Glu Thr Ser  Asn Val Leu Arg Asn  Ile Glu Asn
    1310                 1315                 1320

gct gtc  ggt tcc gca cgc gat  gat gtt ctg att ggc  gat gct ggt        4014
Ala Val  Gly Ser Ala Arg Asp  Asp Val Leu Ile Gly  Asp Ala Gly
    1325                 1330                 1335

gcc aat  gtt ctg aac ggt ctg  gcg ggt aat gat gta  ctg tct ggt        4059
Ala Asn  Val Leu Asn Gly Leu  Ala Gly Asn Asp Val  Leu Ser Gly
    1340                 1345                 1350

ggc gct  ggt gat gat gtc ctg  ctg ggt gat gag ggc  agc gat ctg        4104
Gly Ala  Gly Asp Asp Val Leu  Leu Gly Asp Glu Gly  Ser Asp Leu
    1355                 1360                 1365

ctg tcc  ggc gat gcc ggc aac  gac gat ctg ttc ggt  ggc caa ggt        4149
Leu Ser  Gly Asp Ala Gly Asn  Asp Asp Leu Phe Gly  Gly Gln Gly
    1370                 1375                 1380

gat gat  act tac ctg ttc ggc  gtt ggc tat ggt cat  gac acc atc        4194
Asp Asp  Thr Tyr Leu Phe Gly  Val Gly Tyr Gly His  Asp Thr Ile
    1385                 1390                 1395

tat gag  tct ggt ggc ggc cac  gat acg att cgt att  aat gca ggt        4239
Tyr Glu  Ser Gly Gly Gly His  Asp Thr Ile Arg Ile  Asn Ala Gly
    1400                 1405                 1410

gct gac  caa ctg tgg ttt gca  cgt cag ggt aac gat  ctg gaa att        4284
Ala Asp  Gln Leu Trp Phe Ala  Arg Gln Gly Asn Asp  Leu Glu Ile
    1415                 1420                 1425

cgc atc  ctg ggt act gat gac  gct ctg acc gta cac  gac tgg tac        4329
Arg Ile  Leu Gly Thr Asp Asp  Ala Leu Thr Val His  Asp Trp Tyr
    1430                 1435                 1440

cgc gat  gcg gat cac cgc gta  gaa atc atc cat gcg  gct aac caa        4374
Arg Asp  Ala Asp His Arg Val  Glu Ile Ile His Ala  Ala Asn Gln
    1445                 1450                 1455

gct gta  gac cag gct ggt att  gaa aaa ctg gta gaa  gcg atg gca        4419
Ala Val  Asp Gln Ala Gly Ile  Glu Lys Leu Val Glu  Ala Met Ala
    1460                 1465                 1470

cag tat  ccg gac cca ggt gcg  gct gct gca gca cct  cca gcg gct        4464
Gln Tyr  Pro Asp Pro Gly Ala  Ala Ala Ala Ala Pro  Pro Ala Ala
    1475                 1480                 1485

cgt gtg  ccg gat acc ctg atg  cag agc ctg gcc gtc  aat tgg cgt        4509
Arg Val  Pro Asp Thr Leu Met  Gln Ser Leu Ala Val  Asn Trp Arg
    1490                 1495                 1500

taa                                                                   4512
```

<210> 71
<211> 1503
<212> PRT
<213> Artificial Sequence

<220>
<223> Synthetic Construct

<400> 71

```
Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Gly Leu Arg Arg Pro Ser Leu Gly
            180                 185                 190

Ala Val Glu Arg Gln Asp Ser Gly Tyr Asp Ser Leu Asp Gly Val Gly
        195                 200                 205

Ser Arg Ser Phe Ser Leu Gly Glu Val Ser Asp Met Ala Ala Val Glu
```

```
    210                 215                 220

Ala Ala Glu Leu Glu Met Thr Arg Gln Val Leu His Ala Gly Ala Arg
225                 230                 235                 240

Gln Asp Asp Ala Glu Pro Gly Val Ser Gly Ala Ser Ala His Trp Gly
                245                 250                 255

Gln Arg Ala Leu Gln Gly Ala Gln Ala Val Ala Ala Ala Gln Arg Leu
            260                 265                 270

Val His Ala Ile Ala Leu Met Thr Gln Phe Gly Arg Ala Gly Ser Thr
        275                 280                 285

Asn Thr Pro Gln Glu Ala Ala Ser Leu Ser Ala Ala Val Phe Gly Leu
    290                 295                 300

Gly Glu Ala Ser Ser Ala Val Ala Glu Thr Val Ser Gly Phe Phe Arg
305                 310                 315                 320

Gly Ser Ser Arg Trp Ala Gly Gly Phe Gly Val Ala Gly Gly Ala Met
                325                 330                 335

Ala Leu Gly Gly Gly Ile Ala Ala Ala Val Gly Ala Gly Met Ser Leu
            340                 345                 350

Thr Asp Asp Ala Pro Ala Gly Gln Lys Ala Ala Ala Gly Ala Glu Ile
        355                 360                 365

Ala Leu Gln Leu Thr Gly Gly Thr Val Glu Leu Ala Ser Ser Ile Ala
    370                 375                 380

Leu Ala Leu Ala Ala Ala Arg Gly Val Thr Ser Gly Leu Gln Val Ala
385                 390                 395                 400

Gly Ala Ser Ala Gly Ala Ala Ala Gly Ala Leu Ala Ala Ala Leu Ser
                405                 410                 415

Pro Met Glu Ile Tyr Gly Leu Val Gln Gln Ser His Tyr Ala Asp Gln
            420                 425                 430

Leu Asp Lys Leu Ala Gln Glu Ser Ser Ala Tyr Gly Tyr Glu Gly Asp
        435                 440                 445

Ala Leu Leu Ala Gln Leu Tyr Arg Asp Lys Thr Ala Ala Glu Gly Ala
    450                 455                 460
```

```
Val Ala Gly Val Ser Ala Val Leu Ser Thr Val Gly Ala Ala Val Ser
465                 470                 475                 480

Ile Ala Ala Ala Ala Ser Val Val Gly Ala Pro Val Ala Val Val Thr
                485                 490                 495

Ser Leu Leu Thr Gly Ala Leu Asn Gly Ile Leu Arg Gly Val Gln Gln
            500                 505                 510

Pro Ile Ile Glu Lys Leu Ala Asn Asp Tyr Ala Arg Lys Ile Asp Glu
        515                 520                 525

Leu Gly Gly Pro Gln Ala Tyr Phe Glu Lys Asn Leu Gln Ala Arg His
    530                 535                 540

Glu Gln Leu Ala Asn Ser Asp Gly Leu Arg Lys Met Leu Ala Asp Leu
545                 550                 555                 560

Gln Ala Gly Trp Asn Ala Ser Ser Val Ile Gly Val Gln Thr Thr Glu
                565                 570                 575

Ile Ser Lys Ser Ala Leu Glu Leu Ala Ala Ile Thr Gly Asn Ala Asp
            580                 585                 590

Asn Leu Lys Ser Val Asp Val Phe Val Asp Arg Phe Val Gln Gly Glu
        595                 600                 605

Arg Val Ala Gly Gln Pro Val Val Leu Asp Val Ala Ala Gly Gly Ile
    610                 615                 620

Asp Ile Ala Ser Arg Lys Gly Glu Arg Pro Ala Leu Thr Phe Ile Thr
625                 630                 635                 640

Pro Leu Ala Ala Pro Gly Glu Glu Gln Arg Arg Arg Thr Lys Thr Gly
                645                 650                 655

Lys Ser Glu Phe Thr Thr Phe Val Glu Ile Val Gly Lys Gln Asp Arg
            660                 665                 670

Trp Arg Ile Arg Asp Gly Ala Ala Asp Thr Thr Ile Asp Leu Ala Lys
        675                 680                 685

Val Val Ser Gln Leu Val Asp Ala Asn Gly Val Leu Lys His Ser Ile
    690                 695                 700

Lys Leu Asp Val Ile Gly Gly Asp Gly Asp Asp Val Val Leu Ala Asn
705                 710                 715                 720
```

```
Ala Ser Arg Ile His Tyr Asp Gly Gly Ala Gly Thr Asn Thr Val Ser
                725                 730                 735

Tyr Ala Ala Leu Gly Arg Gln Asp Ser Ile Thr Val Ser Ala Asp Gly
            740                 745                 750

Glu Arg Phe Asn Val Arg Lys Gln Leu Asn Asn Ala Asn Val Tyr Arg
        755                 760                 765

Glu Gly Val Ala Thr Gln Thr Thr Ala Tyr Gly Lys Arg Thr Glu Asn
    770                 775                 780

Val Gln Tyr Arg His Val Glu Leu Ala Arg Val Gly Gln Leu Val Glu
785                 790                 795                 800

Val Asp Thr Leu Glu His Val Gln His Ile Ile Gly Gly Ala Gly Asn
                805                 810                 815

Asp Ser Ile Thr Gly Asn Ala His Asp Asn Phe Leu Ala Gly Gly Ser
            820                 825                 830

Gly Asp Asp Arg Leu Asp Gly Gly Ala Gly Asn Asp Thr Leu Val Gly
        835                 840                 845

Gly Glu Gly Gln Asn Thr Val Ile Gly Gly Ala Gly Asp Asp Val Phe
    850                 855                 860

Leu Gln Asp Leu Gly Val Trp Ser Asn Gln Leu Asp Gly Gly Ala Gly
865                 870                 875                 880

Val Asp Thr Val Lys Tyr Asn Val His Gln Pro Ser Glu Glu Arg Leu
                885                 890                 895

Glu Arg Met Gly Asp Thr Gly Ile His Ala Asp Leu Gln Lys Gly Thr
            900                 905                 910

Val Glu Lys Trp Pro Ala Leu Asn Leu Phe Ser Val Asp His Val Lys
        915                 920                 925

Asn Ile Glu Asn Leu His Gly Ser Arg Leu Asn Asp Arg Ile Ala Gly
    930                 935                 940

Asp Asp Gln Asp Asn Glu Leu Trp Gly His Asp Gly Asn Asp Thr Ile
945                 950                 955                 960

Arg Gly Arg Gly Gly Asp Asp Ile Leu Arg Gly Gly Leu Gly Leu Asp
                965                 970                 975
```

Thr Leu Tyr Gly Glu Asp Gly Asn Asp Ile Phe Leu Gln Asp Asp Glu
980 985 990

Thr Val Ser Asp Asp Ile Asp Gly Gly Ala Gly Leu Asp Thr Val Asp
995 1000 1005

Tyr Ser Ala Met Ile His Pro Gly Arg Ile Val Ala Pro His Glu
1010 1015 1020

Tyr Gly Phe Gly Ile Glu Ala Asp Leu Ser Arg Glu Trp Val Arg
1025 1030 1035

Lys Ala Ser Ala Leu Gly Val Asp Tyr Tyr Asp Asn Val Arg Asn
1040 1045 1050

Val Glu Asn Val Ile Gly Thr Ser Met Lys Asp Val Leu Ile Gly
1055 1060 1065

Asp Ala Gln Ala Asn Thr Leu Met Gly Gln Gly Gly Asp Asp Thr
1070 1075 1080

Val Arg Gly Gly Asp Gly Asp Asp Leu Leu Phe Gly Gly Asp Gly
1085 1090 1095

Asn Asp Met Leu Tyr Gly Asp Ala Gly Asn Asp Thr Leu Tyr Gly
1100 1105 1110

Gly Leu Gly Asp Asp Thr Leu Glu Gly Gly Ala Gly Asn Asp Trp
1115 1120 1125

Phe Gly Gln Thr Gln Ala Arg Glu His Asp Val Leu Arg Gly Gly
1130 1135 1140

Asp Gly Val Asp Thr Val Asp Tyr Ser Gln Thr Gly Ala His Ala
1145 1150 1155

Gly Ile Ala Ala Gly Arg Ile Gly Leu Gly Ile Leu Ala Asp Leu
1160 1165 1170

Gly Ala Gly Arg Val Asp Lys Leu Gly Glu Ala Gly Ser Ser Ala
1175 1180 1185

Tyr Asp Thr Val Ser Gly Ile Glu Asn Val Val Gly Thr Glu Leu
1190 1195 1200

Ala Asp Arg Ile Thr Gly Asp Ala Gln Ala Asn Val Leu Arg Gly

1205 1210 1215

Ala Gly Gly Ala Asp Val Leu Ala Gly Gly Glu Gly Asp Asp Val
1220 1225 1230

Leu Leu Gly Gly Asp Gly Asp Asp Gln Leu Ser Gly Asp Ala Gly
1235 1240 1245

Arg Asp Arg Leu Tyr Gly Glu Ala Gly Asp Asp Trp Phe Phe Gln
1250 1255 1260

Asp Ala Ala Asn Ala Gly Asn Leu Leu Asp Gly Gly Asp Gly Arg
1265 1270 1275

Asp Thr Val Asp Phe Ser Gly Pro Gly Arg Gly Leu Asp Ala Gly
1280 1285 1290

Ala Lys Gly Val Phe Leu Ser Leu Gly Lys Gly Phe Ala Ser Leu
1295 1300 1305

Met Asp Glu Pro Glu Thr Ser Asn Val Leu Arg Asn Ile Glu Asn
1310 1315 1320

Ala Val Gly Ser Ala Arg Asp Asp Val Leu Ile Gly Asp Ala Gly
1325 1330 1335

Ala Asn Val Leu Asn Gly Leu Ala Gly Asn Asp Val Leu Ser Gly
1340 1345 1350

Gly Ala Gly Asp Asp Val Leu Leu Gly Asp Glu Gly Ser Asp Leu
1355 1360 1365

Leu Ser Gly Asp Ala Gly Asn Asp Asp Leu Phe Gly Gly Gln Gly
1370 1375 1380

Asp Asp Thr Tyr Leu Phe Gly Val Gly Tyr Gly His Asp Thr Ile
1385 1390 1395

Tyr Glu Ser Gly Gly Gly His Asp Thr Ile Arg Ile Asn Ala Gly
1400 1405 1410

Ala Asp Gln Leu Trp Phe Ala Arg Gln Gly Asn Asp Leu Glu Ile
1415 1420 1425

Arg Ile Leu Gly Thr Asp Asp Ala Leu Thr Val His Asp Trp Tyr
1430 1435 1440

Arg Asp Ala Asp His Arg Val Glu Ile Ile His Ala Ala Asn Gln
1445 1450 1455

Ala Val Asp Gln Ala Gly Ile Glu Lys Leu Val Glu Ala Met Ala
1460 1465 1470

Gln Tyr Pro Asp Pro Gly Ala Ala Ala Ala Ala Pro Pro Ala Ala
1475 1480 1485

Arg Val Pro Asp Thr Leu Met Gln Ser Leu Ala Val Asn Trp Arg
1490 1495 1500

<210> 72
<211> 4842
<212> DNA
<213> Artificial Sequence

<220>
<223> Nucleotide sequence encoding polypeptide 1-227 + 321-1706 of CyaA

<220>
<221> CDS
<222> (1)..(4842)

<400> 72
atg cag cag tcc cat cag gcc ggt tac gca aac gca gcc gat cgt gaa 48
Met Gln Gln Ser His Gln Ala Gly Tyr Ala Asn Ala Ala Asp Arg Glu
1 5 10 15

tct ggt atc ccg gca gcg gtt ctg gat ggt att aag gca gtt gcc aaa 96
Ser Gly Ile Pro Ala Ala Val Leu Asp Gly Ile Lys Ala Val Ala Lys
20 25 30

gag aag aac gct acc ctg atg ttc cgt ctg gtc aac cca cac tct act 144
Glu Lys Asn Ala Thr Leu Met Phe Arg Leu Val Asn Pro His Ser Thr
35 40 45

tcc ctg atc gcg gaa ggt gtg gcg acc aaa ggc ctg ggt gtg cac gcc 192
Ser Leu Ile Ala Glu Gly Val Ala Thr Lys Gly Leu Gly Val His Ala
50 55 60

aaa agc agc gac tgg ggt ctg cag gca ggt tat att ccg gtg aac ccg 240
Lys Ser Ser Asp Trp Gly Leu Gln Ala Gly Tyr Ile Pro Val Asn Pro
65 70 75 80

aat ctg tct aaa ctg ttc ggt cgt gcc ccg gag gtg atc gca cgt gca 288
Asn Leu Ser Lys Leu Phe Gly Arg Ala Pro Glu Val Ile Ala Arg Ala
85 90 95

gac aac gat gtc aac tct tct ctg gcg cat ggt cac acc gcg gtt gat 336
Asp Asn Asp Val Asn Ser Ser Leu Ala His Gly His Thr Ala Val Asp
100 105 110

ctg acc ctg tcc aaa gaa cgc ctg gat tac ctg cgt cag gcg ggc ctg 384
Leu Thr Leu Ser Lys Glu Arg Leu Asp Tyr Leu Arg Gln Ala Gly Leu
115 120 125

gtg acg ggc atg gcc gac ggt gtt gta gcg agc aac cac gcg ggt tat 432

```
Val Thr Gly Met Ala Asp Gly Val Val Ala Ser Asn His Ala Gly Tyr
    130                 135                 140

gaa caa ttt gag ttt cgc gtt aaa gag acc tct gac ggt cgt tac gcg      480
Glu Gln Phe Glu Phe Arg Val Lys Glu Thr Ser Asp Gly Arg Tyr Ala
145                 150                 155                 160

gtg cag tac cgc cgc aaa ggt ggc gat gac ttt gaa gct gtc aaa gtg      528
Val Gln Tyr Arg Arg Lys Gly Gly Asp Asp Phe Glu Ala Val Lys Val
                165                 170                 175

atc ggt aac gct gct gga att ccg ctg acc gcg gat atc gac atg ttt      576
Ile Gly Asn Ala Ala Gly Ile Pro Leu Thr Ala Asp Ile Asp Met Phe
            180                 185                 190

gcc atc atg cct cac ctg tcc aac ttt cgc gat tcc gca cgc agc tct      624
Ala Ile Met Pro His Leu Ser Asn Phe Arg Asp Ser Ala Arg Ser Ser
        195                 200                 205

gtg acg tct ggt gat tcc gtc act gac tac ctg gct cgt acc cgt cgt      672
Val Thr Ser Gly Asp Ser Val Thr Asp Tyr Leu Ala Arg Thr Arg Arg
    210                 215                 220

gcg gcc tct gaa tcc cag atg ctg act cgt ggc cag ctg aaa gaa tac      720
Ala Ala Ser Glu Ser Gln Met Leu Thr Arg Gly Gln Leu Lys Glu Tyr
225                 230                 235                 240

atc ggc caa cag cgt ggc gaa ggc tac gtt ttc tac gag aat cgt gct      768
Ile Gly Gln Gln Arg Gly Glu Gly Tyr Val Phe Tyr Glu Asn Arg Ala
                245                 250                 255

tac ggc gtt gcg ggc aaa tcc ctg ttc gac gat ggt ctg ggc gct gca      816
Tyr Gly Val Ala Gly Lys Ser Leu Phe Asp Asp Gly Leu Gly Ala Ala
            260                 265                 270

ccg ggc gtt ccg agc ggc cgt tct aaa ttc agc ccg gac gta ctg gaa      864
Pro Gly Val Pro Ser Gly Arg Ser Lys Phe Ser Pro Asp Val Leu Glu
        275                 280                 285

act gtg ccg gct tcc ccg ggc ctg cgc cgt ccg tcc ctg ggc gca gtc      912
Thr Val Pro Ala Ser Pro Gly Leu Arg Arg Pro Ser Leu Gly Ala Val
    290                 295                 300

gaa cgt cag gac tcc ggc tac gat tcc ctg gat ggc gtt ggc tcc cgc      960
Glu Arg Gln Asp Ser Gly Tyr Asp Ser Leu Asp Gly Val Gly Ser Arg
305                 310                 315                 320

tcc ttc tcc ctg ggc gag gtt tct gac atg gct gcg gtg gaa gcg gca     1008
Ser Phe Ser Leu Gly Glu Val Ser Asp Met Ala Ala Val Glu Ala Ala
                325                 330                 335

gag ctg gaa atg acc cgt caa gtg ctg cac gcg ggc gca cgt cag gac     1056
Glu Leu Glu Met Thr Arg Gln Val Leu His Ala Gly Ala Arg Gln Asp
            340                 345                 350

gat gct gag ccg ggc gtg tcc ggt gcg tct gca cac tgg ggt caa cgt     1104
Asp Ala Glu Pro Gly Val Ser Gly Ala Ser Ala His Trp Gly Gln Arg
        355                 360                 365

gcc ctg cag ggt gct caa gct gtc gcg gca gcg cag cgt ctg gta cat     1152
Ala Leu Gln Gly Ala Gln Ala Val Ala Ala Ala Gln Arg Leu Val His
    370                 375                 380
```

```
gcg atc gca ctg atg acc cag ttc ggt cgc gca ggt tct acc aac act        1200
Ala Ile Ala Leu Met Thr Gln Phe Gly Arg Ala Gly Ser Thr Asn Thr
385                 390                 395                 400

cct cag gaa gca gct agc ctg tct gct gct gtt ttc ggt ctg ggc gaa        1248
Pro Gln Glu Ala Ala Ser Leu Ser Ala Ala Val Phe Gly Leu Gly Glu
                405                 410                 415

gcg tct tct gcg gtt gca gaa acc gtg agc ggt ttc ttc cgt ggt tct        1296
Ala Ser Ser Ala Val Ala Glu Thr Val Ser Gly Phe Phe Arg Gly Ser
            420                 425                 430

agc cgc tgg gcg ggt ggt ttt ggt gta gca ggt ggc gct atg gcc ctg        1344
Ser Arg Trp Ala Gly Gly Phe Gly Val Ala Gly Gly Ala Met Ala Leu
        435                 440                 445

ggt ggc ggt atc gcg gct gct gtc ggc gca ggt atg agc ctg acc gac        1392
Gly Gly Gly Ile Ala Ala Ala Val Gly Ala Gly Met Ser Leu Thr Asp
    450                 455                 460

gat gct cct gcg ggc cag aaa gcc gca gct ggc gct gaa atc gcg ctg        1440
Asp Ala Pro Ala Gly Gln Lys Ala Ala Ala Gly Ala Glu Ile Ala Leu
465                 470                 475                 480

cag ctg act ggt ggt acc gtt gaa ctg gct agc tct atc gcg ctg gct        1488
Gln Leu Thr Gly Gly Thr Val Glu Leu Ala Ser Ser Ile Ala Leu Ala
                485                 490                 495

ctg gca gcg gca cgt ggc gtg act tct ggc ctg caa gtc gcc ggt gcc        1536
Leu Ala Ala Ala Arg Gly Val Thr Ser Gly Leu Gln Val Ala Gly Ala
            500                 505                 510

tct gcg ggc gct gct gcg ggc gct ctg gca gct gca ctg tcc ccc atg        1584
Ser Ala Gly Ala Ala Ala Gly Ala Leu Ala Ala Ala Leu Ser Pro Met
        515                 520                 525

gaa atc tac ggt ctg gta cag cag tct cac tac gca gac cag ctg gat        1632
Glu Ile Tyr Gly Leu Val Gln Gln Ser His Tyr Ala Asp Gln Leu Asp
    530                 535                 540

aaa ctg gcg cag gag tct tct gca tac ggt tac gaa ggc gac gca ctg        1680
Lys Leu Ala Gln Glu Ser Ser Ala Tyr Gly Tyr Glu Gly Asp Ala Leu
545                 550                 555                 560

ctg gcg cag ctg tat cgt gac aaa act gct gct gaa ggt gct gtg gca        1728
Leu Ala Gln Leu Tyr Arg Asp Lys Thr Ala Ala Glu Gly Ala Val Ala
                565                 570                 575

ggc gtt tct gcg gta ctg tct acc gtt ggc gct gcg gtt tct att gcg        1776
Gly Val Ser Ala Val Leu Ser Thr Val Gly Ala Ala Val Ser Ile Ala
            580                 585                 590

gct gcc gca tcc gtt gta ggt gcg ccg gtt gct gta gtt acc tct ctg        1824
Ala Ala Ala Ser Val Val Gly Ala Pro Val Ala Val Val Thr Ser Leu
        595                 600                 605

ctg act ggt gca ctg aac ggt atc ctg cgt ggt gtt cag cag cct att        1872
Leu Thr Gly Ala Leu Asn Gly Ile Leu Arg Gly Val Gln Gln Pro Ile
    610                 615                 620

atc gaa aaa ctg gcg aat gat tat gcc cgt aaa atc gac gaa ctg ggc        1920
Ile Glu Lys Leu Ala Asn Asp Tyr Ala Arg Lys Ile Asp Glu Leu Gly
625                 630                 635                 640
```

```
ggt ccg cag gct tat ttt gaa aaa aac ctg cag gcg cgc cac gaa cag       1968
Gly Pro Gln Ala Tyr Phe Glu Lys Asn Leu Gln Ala Arg His Glu Gln
                645                 650                 655

ctg gca aac agc gac ggc ctg cgc aaa atg ctg gca gac ctg caa gct       2016
Leu Ala Asn Ser Asp Gly Leu Arg Lys Met Leu Ala Asp Leu Gln Ala
            660                 665                 670

ggt tgg aac gcg tct tct gtg atc ggc gtg cag acc acc gaa att agc       2064
Gly Trp Asn Ala Ser Ser Val Ile Gly Val Gln Thr Thr Glu Ile Ser
        675                 680                 685

aaa tct gcg ctg gaa ctg gct gca att act ggc aac gcg gat aac ctg       2112
Lys Ser Ala Leu Glu Leu Ala Ala Ile Thr Gly Asn Ala Asp Asn Leu
    690                 695                 700

aaa agc gtt gat gtt ttt gtc gat cgc ttc gtt cag ggc gag cgc gtt       2160
Lys Ser Val Asp Val Phe Val Asp Arg Phe Val Gln Gly Glu Arg Val
705                 710                 715                 720

gct ggt cag ccg gtt gtt ctg gac gtt gcg gca ggc ggc atc gac atc       2208
Ala Gly Gln Pro Val Val Leu Asp Val Ala Ala Gly Gly Ile Asp Ile
                725                 730                 735

gct agc cgc aag ggc gag cgt ccg gct ctg act ttc att acc ccg ctg       2256
Ala Ser Arg Lys Gly Glu Arg Pro Ala Leu Thr Phe Ile Thr Pro Leu
            740                 745                 750

gca gct ccg ggt gaa gaa cag cgt cgc cgt acc aag act ggt aaa agc       2304
Ala Ala Pro Gly Glu Glu Gln Arg Arg Arg Thr Lys Thr Gly Lys Ser
        755                 760                 765

gag ttc acc acc ttc gtg gaa atc gta ggt aaa cag gac cgc tgg cgt       2352
Glu Phe Thr Thr Phe Val Glu Ile Val Gly Lys Gln Asp Arg Trp Arg
    770                 775                 780

att cgc gac ggt gcg gcg gac acc acc atc gac ctg gca aaa gtt gtt       2400
Ile Arg Asp Gly Ala Ala Asp Thr Thr Ile Asp Leu Ala Lys Val Val
785                 790                 795                 800

tcc caa ctg gta gat gct aac ggc gtg ctg aaa cat tct att aaa ctg       2448
Ser Gln Leu Val Asp Ala Asn Gly Val Leu Lys His Ser Ile Lys Leu
                805                 810                 815

gat gta atc ggc ggt gac ggc gat gac gtt gta ctg gcg aac gcg tct       2496
Asp Val Ile Gly Gly Asp Gly Asp Asp Val Val Leu Ala Asn Ala Ser
            820                 825                 830

cgt atc cac tat gac ggc ggc gca ggt acc aac acg gtt tcc tac gca       2544
Arg Ile His Tyr Asp Gly Gly Ala Gly Thr Asn Thr Val Ser Tyr Ala
        835                 840                 845

gcg ctg ggc cgc cag gat tcc atc act gtt agc gcc gat ggc gaa cgt       2592
Ala Leu Gly Arg Gln Asp Ser Ile Thr Val Ser Ala Asp Gly Glu Arg
    850                 855                 860

ttc aac gtg cgt aaa cag ctg aat aac gca aac gtc tat cgt gag ggt       2640
Phe Asn Val Arg Lys Gln Leu Asn Asn Ala Asn Val Tyr Arg Glu Gly
865                 870                 875                 880

gta gct acc cag acc acc gcg tac ggt aag cgt acc gaa aac gtt cag       2688
Val Ala Thr Gln Thr Thr Ala Tyr Gly Lys Arg Thr Glu Asn Val Gln
```

```
                885                 890                 895

tat cgt cac gtg gaa ctg gca cgt gtg ggt cag ctg gta gag gtg gat        2736
Tyr Arg His Val Glu Leu Ala Arg Val Gly Gln Leu Val Glu Val Asp
            900                 905                 910

acg ctg gaa cac gtg cag cac atc atc ggt ggc gcc ggt aac gat tcc        2784
Thr Leu Glu His Val Gln His Ile Ile Gly Gly Ala Gly Asn Asp Ser
        915                 920                 925

att act ggt aac gct cac gac aac ttc ctg gca ggt ggt tct ggt gat        2832
Ile Thr Gly Asn Ala His Asp Asn Phe Leu Ala Gly Gly Ser Gly Asp
    930                 935                 940

gat cgt ctg gac ggt ggt gcg ggt aac gat acg ctg gta ggc ggc gaa        2880
Asp Arg Leu Asp Gly Gly Ala Gly Asn Asp Thr Leu Val Gly Gly Glu
945                 950                 955                 960

ggt cag aac acc gta att ggt ggt gcc ggt gac gac gtt ttc ctg cag        2928
Gly Gln Asn Thr Val Ile Gly Gly Ala Gly Asp Asp Val Phe Leu Gln
                965                 970                 975

gat ctg ggc gtt tgg agc aac cag ctg gat ggc ggt gcc ggt gtt gac        2976
Asp Leu Gly Val Trp Ser Asn Gln Leu Asp Gly Gly Ala Gly Val Asp
            980                 985                 990

acg gtt aag tat aac gtt cac cag  ccg tct gaa gag cgc  ctg gaa cgt      3024
Thr Val Lys Tyr Asn Val His Gln  Pro Ser Glu Glu Arg  Leu Glu Arg
        995                 1000                1005

atg ggt  gac act ggt att cat  gcc gat ctg cag aaa  ggc act gtt        3069
Met Gly  Asp Thr Gly Ile His  Ala Asp Leu Gln Lys  Gly Thr Val
    1010                 1015                 1020

gaa aaa  tgg cct gcg ctg aac  ctg ttc tct gtg gac  cat gtt aag        3114
Glu Lys  Trp Pro Ala Leu Asn  Leu Phe Ser Val Asp  His Val Lys
    1025                 1030                 1035

aac att  gaa aac ctg cat ggc  tct cgc ctg aac gac  cgt atc gct        3159
Asn Ile  Glu Asn Leu His Gly  Ser Arg Leu Asn Asp  Arg Ile Ala
    1040                 1045                 1050

ggt gac  gac cag gat aac gaa  ctg tgg ggt cat gac  ggc aat gat        3204
Gly Asp  Asp Gln Asp Asn Glu  Leu Trp Gly His Asp  Gly Asn Asp
    1055                 1060                 1065

acc att  cgc ggt cgt ggc ggc  gac gat att ctg cgt  ggc ggt ctg        3249
Thr Ile  Arg Gly Arg Gly Gly  Asp Asp Ile Leu Arg  Gly Gly Leu
    1070                 1075                 1080

ggc ctg  gat acc ctg tat ggt  gaa gat ggt aac gat  att ttc ctg        3294
Gly Leu  Asp Thr Leu Tyr Gly  Glu Asp Gly Asn Asp  Ile Phe Leu
    1085                 1090                 1095

caa gac  gat gaa acg gta tct  gat gac atc gac ggt  ggt gca ggc        3339
Gln Asp  Asp Glu Thr Val Ser  Asp Asp Ile Asp Gly  Gly Ala Gly
    1100                 1105                 1110

ctg gac  acc gta gat tac agc  gct atg atc cat ccg  ggt cgt atc        3384
Leu Asp  Thr Val Asp Tyr Ser  Ala Met Ile His Pro  Gly Arg Ile
    1115                 1120                 1125

gta gct  ccg cac gag tac ggc  ttc ggt atc gag gcg  gac ctg tct        3429
```

```
Val Ala Pro His Glu Tyr Gly Phe Gly Ile Glu Ala Asp Leu Ser
    1130                1135                1140

cgt gaa tgg gtt cgt aaa gcg tcc gcg ctg ggt gtg gac tac tac      3474
Arg Glu Trp Val Arg Lys Ala Ser Ala Leu Gly Val Asp Tyr Tyr
    1145                1150                1155

gat aac gtt cgt aac gtg gaa aac gtg atc ggt acc tcc atg aaa      3519
Asp Asn Val Arg Asn Val Glu Asn Val Ile Gly Thr Ser Met Lys
    1160                1165                1170

gac gtg ctg att ggc gac gca cag gcc aac act ctg atg ggc cag      3564
Asp Val Leu Ile Gly Asp Ala Gln Ala Asn Thr Leu Met Gly Gln
    1175                1180                1185

ggc ggt gat gac acg gtt cgc ggc ggc gac ggt gat gac ctg ctg      3609
Gly Gly Asp Asp Thr Val Arg Gly Gly Asp Gly Asp Asp Leu Leu
    1190                1195                1200

ttc ggt ggt gac ggc aac gac atg ctg tac ggc gac gcg ggc aac      3654
Phe Gly Gly Asp Gly Asn Asp Met Leu Tyr Gly Asp Ala Gly Asn
    1205                1210                1215

gac acc ctg tac ggc ggt ctg ggt gac gac act ctg gaa ggt ggt      3699
Asp Thr Leu Tyr Gly Gly Leu Gly Asp Asp Thr Leu Glu Gly Gly
    1220                1225                1230

gca ggt aac gac tgg ttc ggc caa acc cag gca cgc gaa cac gac      3744
Ala Gly Asn Asp Trp Phe Gly Gln Thr Gln Ala Arg Glu His Asp
    1235                1240                1245

gtg ctg cgt ggt ggc gac ggc gta gac acc gtg gat tac tcc caa      3789
Val Leu Arg Gly Gly Asp Gly Val Asp Thr Val Asp Tyr Ser Gln
    1250                1255                1260

act ggc gct cac gcg ggt atc gcg gcc ggt cgt atc ggt ctg ggc      3834
Thr Gly Ala His Ala Gly Ile Ala Ala Gly Arg Ile Gly Leu Gly
    1265                1270                1275

att ctg gcc gat ctg ggt gcc ggc cgt gtc gac aaa ctg ggt gag      3879
Ile Leu Ala Asp Leu Gly Ala Gly Arg Val Asp Lys Leu Gly Glu
    1280                1285                1290

gct ggc tct agc gcc tat gat act gtt tcc ggc atc gaa aac gtg      3924
Ala Gly Ser Ser Ala Tyr Asp Thr Val Ser Gly Ile Glu Asn Val
    1295                1300                1305

gta ggc act gaa ctg gca gat cgt atc acg ggt gac gcg caa gcg      3969
Val Gly Thr Glu Leu Ala Asp Arg Ile Thr Gly Asp Ala Gln Ala
    1310                1315                1320

aac gtt ctg cgt ggt gct ggc ggt gca gac gtg ctg gcg ggt ggt      4014
Asn Val Leu Arg Gly Ala Gly Gly Ala Asp Val Leu Ala Gly Gly
    1325                1330                1335

gaa ggc gac gac gta ctg ctg ggc ggt gac ggt gac gat cag ctg      4059
Glu Gly Asp Asp Val Leu Leu Gly Gly Asp Gly Asp Asp Gln Leu
    1340                1345                1350

agc ggt gat gcg ggt cgt gac cgt ctg tac ggt gaa gca ggt gac      4104
Ser Gly Asp Ala Gly Arg Asp Arg Leu Tyr Gly Glu Ala Gly Asp
    1355                1360                1365
```

```
gat tgg  ttc ttc cag gac gct  gct aac gct ggc aac  ctg ctg gac         4149
Asp Trp  Phe Phe Gln Asp Ala  Ala Asn Ala Gly Asn  Leu Leu Asp
    1370                 1375                 1380

ggc ggc  gat ggc cgt gac act  gtt gac ttc agc ggt  ccg ggt cgc         4194
Gly Gly  Asp Gly Arg Asp Thr  Val Asp Phe Ser Gly  Pro Gly Arg
    1385                 1390                 1395

ggt ctg  gac gca ggc gcg aaa  ggc gtt ttc ctg agc  ctg ggt aag         4239
Gly Leu  Asp Ala Gly Ala Lys  Gly Val Phe Leu Ser  Leu Gly Lys
    1400                 1405                 1410

ggt ttc  gca tct ctg atg gac  gaa cca gaa acc agc  aat gtc ctg         4284
Gly Phe  Ala Ser Leu Met Asp  Glu Pro Glu Thr Ser  Asn Val Leu
    1415                 1420                 1425

cgt aac  atc gaa aac gct gtc  ggt tcc gca cgc gat  gat gtt ctg         4329
Arg Asn  Ile Glu Asn Ala Val  Gly Ser Ala Arg Asp  Asp Val Leu
    1430                 1435                 1440

att ggc  gat gct ggt gcc aat  gtt ctg aac ggt ctg  gcg ggt aat         4374
Ile Gly  Asp Ala Gly Ala Asn  Val Leu Asn Gly Leu  Ala Gly Asn
    1445                 1450                 1455

gat gta  ctg tct ggt ggc gct  ggt gat gat gtc ctg  ctg ggt gat         4419
Asp Val  Leu Ser Gly Gly Ala  Gly Asp Asp Val Leu  Leu Gly Asp
    1460                 1465                 1470

gag ggc  agc gat ctg ctg tcc  ggc gat gcc ggc aac  gac gat ctg         4464
Glu Gly  Ser Asp Leu Leu Ser  Gly Asp Ala Gly Asn  Asp Asp Leu
    1475                 1480                 1485

ttc ggt  ggc caa ggt gat gat  act tac ctg ttc ggc  gtt ggc tat         4509
Phe Gly  Gly Gln Gly Asp Asp  Thr Tyr Leu Phe Gly  Val Gly Tyr
    1490                 1495                 1500

ggt cat  gac acc atc tat gag  tct ggt ggc ggc cac  gat acg att         4554
Gly His  Asp Thr Ile Tyr Glu  Ser Gly Gly Gly His  Asp Thr Ile
    1505                 1510                 1515

cgt att  aat gca ggt gct gac  caa ctg tgg ttt gca  cgt cag ggt         4599
Arg Ile  Asn Ala Gly Ala Asp  Gln Leu Trp Phe Ala  Arg Gln Gly
    1520                 1525                 1530

aac gat  ctg gaa att cgc atc  ctg ggt act gat gac  gct ctg acc         4644
Asn Asp  Leu Glu Ile Arg Ile  Leu Gly Thr Asp Asp  Ala Leu Thr
    1535                 1540                 1545

gta cac  gac tgg tac cgc gat  gcg gat cac cgc gta  gaa atc atc         4689
Val His  Asp Trp Tyr Arg Asp  Ala Asp His Arg Val  Glu Ile Ile
    1550                 1555                 1560

cat gcg  gct aac caa gct gta  gac cag gct ggt att  gaa aaa ctg         4734
His Ala  Ala Asn Gln Ala Val  Asp Gln Ala Gly Ile  Glu Lys Leu
    1565                 1570                 1575

gta gaa  gcg atg gca cag tat  ccg gac cca ggt gcg  gct gct gca         4779
Val Glu  Ala Met Ala Gln Tyr  Pro Asp Pro Gly Ala  Ala Ala Ala
    1580                 1585                 1590

gca cct  cca gcg gct cgt gtg  ccg gat acc ctg atg  cag agc ctg         4824
Ala Pro  Pro Ala Ala Arg Val  Pro Asp Thr Leu Met  Gln Ser Leu
    1595                 1600                 1605
```

```
gcc gtc  aat tgg cgt taa                                          4842
Ala Val  Asn Trp Arg
    1610


<210>  73
<211>  1613
<212>  PRT
<213>  Artificial Sequence

<220>
<223>  Synthetic Construct

<400>  73

Met Gln Gln Ser His Gln Ala Gly Tyr Ala Asn Ala Ala Asp Arg Glu
1               5                   10                  15


Ser Gly Ile Pro Ala Ala Val Leu Asp Gly Ile Lys Ala Val Ala Lys
            20                  25                  30


Glu Lys Asn Ala Thr Leu Met Phe Arg Leu Val Asn Pro His Ser Thr
        35                  40                  45


Ser Leu Ile Ala Glu Gly Val Ala Thr Lys Gly Leu Gly Val His Ala
    50                  55                  60


Lys Ser Ser Asp Trp Gly Leu Gln Ala Gly Tyr Ile Pro Val Asn Pro
65                  70                  75                  80


Asn Leu Ser Lys Leu Phe Gly Arg Ala Pro Glu Val Ile Ala Arg Ala
                85                  90                  95


Asp Asn Asp Val Asn Ser Ser Leu Ala His Gly His Thr Ala Val Asp
            100                 105                 110


Leu Thr Leu Ser Lys Glu Arg Leu Asp Tyr Leu Arg Gln Ala Gly Leu
        115                 120                 125


Val Thr Gly Met Ala Asp Gly Val Val Ala Ser Asn His Ala Gly Tyr
    130                 135                 140


Glu Gln Phe Glu Phe Arg Val Lys Glu Thr Ser Asp Gly Arg Tyr Ala
145                 150                 155                 160


Val Gln Tyr Arg Arg Lys Gly Gly Asp Asp Phe Glu Ala Val Lys Val
                165                 170                 175


Ile Gly Asn Ala Ala Gly Ile Pro Leu Thr Ala Asp Ile Asp Met Phe
            180                 185                 190
```

```
Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ser Gln Met Leu Thr Arg Gly Gln Leu Lys Glu Tyr
225                 230                 235                 240

Ile Gly Gln Gln Arg Gly Glu Gly Tyr Val Phe Tyr Glu Asn Arg Ala
                245                 250                 255

Tyr Gly Val Ala Gly Lys Ser Leu Phe Asp Asp Gly Leu Gly Ala Ala
            260                 265                 270

Pro Gly Val Pro Ser Gly Arg Ser Lys Phe Ser Pro Asp Val Leu Glu
        275                 280                 285

Thr Val Pro Ala Ser Pro Gly Leu Arg Arg Pro Ser Leu Gly Ala Val
    290                 295                 300

Glu Arg Gln Asp Ser Gly Tyr Asp Ser Leu Asp Gly Val Gly Ser Arg
305                 310                 315                 320

Ser Phe Ser Leu Gly Glu Val Ser Asp Met Ala Ala Val Glu Ala Ala
                325                 330                 335

Glu Leu Glu Met Thr Arg Gln Val Leu His Ala Gly Ala Arg Gln Asp
            340                 345                 350

Asp Ala Glu Pro Gly Val Ser Gly Ala Ser Ala His Trp Gly Gln Arg
        355                 360                 365

Ala Leu Gln Gly Ala Gln Ala Val Ala Ala Ala Gln Arg Leu Val His
    370                 375                 380

Ala Ile Ala Leu Met Thr Gln Phe Gly Arg Ala Gly Ser Thr Asn Thr
385                 390                 395                 400

Pro Gln Glu Ala Ala Ser Leu Ser Ala Ala Val Phe Gly Leu Gly Glu
                405                 410                 415

Ala Ser Ser Ala Val Ala Glu Thr Val Ser Gly Phe Phe Arg Gly Ser
            420                 425                 430

Ser Arg Trp Ala Gly Gly Phe Gly Val Ala Gly Gly Ala Met Ala Leu
        435                 440                 445
```

Gly Gly Gly Ile Ala Ala Ala Val Gly Ala Gly Met Ser Leu Thr Asp
450 455 460

Asp Ala Pro Ala Gly Gln Lys Ala Ala Ala Gly Ala Glu Ile Ala Leu
465 470 475 480

Gln Leu Thr Gly Gly Thr Val Glu Leu Ala Ser Ser Ile Ala Leu Ala
485 490 495

Leu Ala Ala Ala Arg Gly Val Thr Ser Gly Leu Gln Val Ala Gly Ala
500 505 510

Ser Ala Gly Ala Ala Ala Gly Ala Leu Ala Ala Ala Leu Ser Pro Met
515 520 525

Glu Ile Tyr Gly Leu Val Gln Gln Ser His Tyr Ala Asp Gln Leu Asp
530 535 540

Lys Leu Ala Gln Glu Ser Ser Ala Tyr Gly Tyr Glu Gly Asp Ala Leu
545 550 555 560

Leu Ala Gln Leu Tyr Arg Asp Lys Thr Ala Ala Glu Gly Ala Val Ala
565 570 575

Gly Val Ser Ala Val Leu Ser Thr Val Gly Ala Ala Val Ser Ile Ala
580 585 590

Ala Ala Ala Ser Val Val Gly Ala Pro Val Ala Val Val Thr Ser Leu
595 600 605

Leu Thr Gly Ala Leu Asn Gly Ile Leu Arg Gly Val Gln Gln Pro Ile
610 615 620

Ile Glu Lys Leu Ala Asn Asp Tyr Ala Arg Lys Ile Asp Glu Leu Gly
625 630 635 640

Gly Pro Gln Ala Tyr Phe Glu Lys Asn Leu Gln Ala Arg His Glu Gln
645 650 655

Leu Ala Asn Ser Asp Gly Leu Arg Lys Met Leu Ala Asp Leu Gln Ala
660 665 670

Gly Trp Asn Ala Ser Ser Val Ile Gly Val Gln Thr Thr Glu Ile Ser
675 680 685

Lys Ser Ala Leu Glu Leu Ala Ala Ile Thr Gly Asn Ala Asp Asn Leu

690 695 700

Lys Ser Val Asp Val Phe Val Asp Arg Phe Val Gln Gly Glu Arg Val
705 710 715 720

Ala Gly Gln Pro Val Val Leu Asp Val Ala Ala Gly Gly Ile Asp Ile
725 730 735

Ala Ser Arg Lys Gly Glu Arg Pro Ala Leu Thr Phe Ile Thr Pro Leu
740 745 750

Ala Ala Pro Gly Glu Glu Gln Arg Arg Arg Thr Lys Thr Gly Lys Ser
755 760 765

Glu Phe Thr Thr Phe Val Glu Ile Val Gly Lys Gln Asp Arg Trp Arg
770 775 780

Ile Arg Asp Gly Ala Ala Asp Thr Thr Ile Asp Leu Ala Lys Val Val
785 790 795 800

Ser Gln Leu Val Asp Ala Asn Gly Val Leu Lys His Ser Ile Lys Leu
805 810 815

Asp Val Ile Gly Gly Asp Gly Asp Asp Val Val Leu Ala Asn Ala Ser
820 825 830

Arg Ile His Tyr Asp Gly Gly Ala Gly Thr Asn Thr Val Ser Tyr Ala
835 840 845

Ala Leu Gly Arg Gln Asp Ser Ile Thr Val Ser Ala Asp Gly Glu Arg
850 855 860

Phe Asn Val Arg Lys Gln Leu Asn Asn Ala Asn Val Tyr Arg Glu Gly
865 870 875 880

Val Ala Thr Gln Thr Thr Ala Tyr Gly Lys Arg Thr Glu Asn Val Gln
885 890 895

Tyr Arg His Val Glu Leu Ala Arg Val Gly Gln Leu Val Glu Val Asp
900 905 910

Thr Leu Glu His Val Gln His Ile Ile Gly Gly Ala Gly Asn Asp Ser
915 920 925

Ile Thr Gly Asn Ala His Asp Asn Phe Leu Ala Gly Gly Ser Gly Asp
930 935 940

Asp Arg Leu Asp Gly Gly Ala Gly Asn Asp Thr Leu Val Gly Gly Glu
945 950 955 960

Gly Gln Asn Thr Val Ile Gly Gly Ala Gly Asp Asp Val Phe Leu Gln
965 970 975

Asp Leu Gly Val Trp Ser Asn Gln Leu Asp Gly Gly Ala Gly Val Asp
980 985 990

Thr Val Lys Tyr Asn Val His Gln Pro Ser Glu Glu Arg Leu Glu Arg
995 1000 1005

Met Gly Asp Thr Gly Ile His Ala Asp Leu Gln Lys Gly Thr Val
1010 1015 1020

Glu Lys Trp Pro Ala Leu Asn Leu Phe Ser Val Asp His Val Lys
1025 1030 1035

Asn Ile Glu Asn Leu His Gly Ser Arg Leu Asn Asp Arg Ile Ala
1040 1045 1050

Gly Asp Asp Gln Asp Asn Glu Leu Trp Gly His Asp Gly Asn Asp
1055 1060 1065

Thr Ile Arg Gly Arg Gly Gly Asp Asp Ile Leu Arg Gly Gly Leu
1070 1075 1080

Gly Leu Asp Thr Leu Tyr Gly Glu Asp Gly Asn Asp Ile Phe Leu
1085 1090 1095

Gln Asp Asp Glu Thr Val Ser Asp Asp Ile Asp Gly Gly Ala Gly
1100 1105 1110

Leu Asp Thr Val Asp Tyr Ser Ala Met Ile His Pro Gly Arg Ile
1115 1120 1125

Val Ala Pro His Glu Tyr Gly Phe Gly Ile Glu Ala Asp Leu Ser
1130 1135 1140

Arg Glu Trp Val Arg Lys Ala Ser Ala Leu Gly Val Asp Tyr Tyr
1145 1150 1155

Asp Asn Val Arg Asn Val Glu Asn Val Ile Gly Thr Ser Met Lys
1160 1165 1170

Asp Val Leu Ile Gly Asp Ala Gln Ala Asn Thr Leu Met Gly Gln
1175 1180 1185

Gly Gly Asp Asp Thr Val Arg Gly Gly Asp Gly Asp Asp Leu Leu
1190 1195 1200

Phe Gly Gly Asp Gly Asn Asp Met Leu Tyr Gly Asp Ala Gly Asn
1205 1210 1215

Asp Thr Leu Tyr Gly Gly Leu Gly Asp Asp Thr Leu Glu Gly Gly
1220 1225 1230

Ala Gly Asn Asp Trp Phe Gly Gln Thr Gln Ala Arg Glu His Asp
1235 1240 1245

Val Leu Arg Gly Gly Asp Gly Val Asp Thr Val Asp Tyr Ser Gln
1250 1255 1260

Thr Gly Ala His Ala Gly Ile Ala Ala Gly Arg Ile Gly Leu Gly
1265 1270 1275

Ile Leu Ala Asp Leu Gly Ala Gly Arg Val Asp Lys Leu Gly Glu
1280 1285 1290

Ala Gly Ser Ser Ala Tyr Asp Thr Val Ser Gly Ile Glu Asn Val
1295 1300 1305

Val Gly Thr Glu Leu Ala Asp Arg Ile Thr Gly Asp Ala Gln Ala
1310 1315 1320

Asn Val Leu Arg Gly Ala Gly Gly Ala Asp Val Leu Ala Gly Gly
1325 1330 1335

Glu Gly Asp Asp Val Leu Leu Gly Gly Asp Gly Asp Asp Gln Leu
1340 1345 1350

Ser Gly Asp Ala Gly Arg Asp Arg Leu Tyr Gly Glu Ala Gly Asp
1355 1360 1365

Asp Trp Phe Phe Gln Asp Ala Ala Asn Ala Gly Asn Leu Leu Asp
1370 1375 1380

Gly Gly Asp Gly Arg Asp Thr Val Asp Phe Ser Gly Pro Gly Arg
1385 1390 1395

Gly Leu Asp Ala Gly Ala Lys Gly Val Phe Leu Ser Leu Gly Lys
1400 1405 1410

Gly Phe Ala Ser Leu Met Asp Glu Pro Glu Thr Ser Asn Val Leu
1415 1420 1425

Arg Asn Ile Glu Asn Ala Val Gly Ser Ala Arg Asp Asp Val Leu
1430 1435 1440

Ile Gly Asp Ala Gly Ala Asn Val Leu Asn Gly Leu Ala Gly Asn
1445 1450 1455

Asp Val Leu Ser Gly Gly Ala Gly Asp Asp Val Leu Leu Gly Asp
1460 1465 1470

Glu Gly Ser Asp Leu Leu Ser Gly Asp Ala Gly Asn Asp Asp Leu
1475 1480 1485

Phe Gly Gly Gln Gly Asp Asp Thr Tyr Leu Phe Gly Val Gly Tyr
1490 1495 1500

Gly His Asp Thr Ile Tyr Glu Ser Gly Gly Gly His Asp Thr Ile
1505 1510 1515

Arg Ile Asn Ala Gly Ala Asp Gln Leu Trp Phe Ala Arg Gln Gly
1520 1525 1530

Asn Asp Leu Glu Ile Arg Ile Leu Gly Thr Asp Asp Ala Leu Thr
1535 1540 1545

Val His Asp Trp Tyr Arg Asp Ala Asp His Arg Val Glu Ile Ile
1550 1555 1560

His Ala Ala Asn Gln Ala Val Asp Gln Ala Gly Ile Glu Lys Leu
1565 1570 1575

Val Glu Ala Met Ala Gln Tyr Pro Asp Pro Gly Ala Ala Ala Ala
1580 1585 1590

Ala Pro Pro Ala Ala Arg Val Pro Asp Thr Leu Met Gln Ser Leu
1595 1600 1605

Ala Val Asn Trp Arg
1610

<210> 74
<211> 98
<212> PRT
<213> Artificial Sequence

<220>
<223> Protein sequence of E7 of HPV31

<400> 74

Met Arg Gly Glu Thr Pro Thr Leu Gln Asp Tyr Val Leu Asp Leu Gln
1               5                   10                  15

Pro Glu Ala Thr Asp Leu Tyr Cys Tyr Glu Gln Leu Pro Asp Ser Ser
            20                  25                  30

Asp Glu Glu Asp Val Ile Asp Ser Pro Ala Gly Gln Ala Lys Pro Asp
        35                  40                  45

Thr Ser Asn Tyr Asn Ile Val Thr Phe Cys Cys Gln Cys Glu Ser Thr
    50                  55                  60

Leu Arg Leu Cys Val Gln Ser Thr Gln Val Asp Ile Arg Ile Leu Gln
65                  70                  75                  80

Glu Leu Leu Met Gly Ser Phe Gly Ile Val Cys Pro Asn Cys Ser Thr
                85                  90                  95

Arg Leu

<210>  75
<211>  97
<212>  PRT
<213>  Artificial Sequence

<220>
<223>  Protein sequence of E7 of HPV33

<400>  75

Met Arg Gly His Lys Pro Thr Leu Lys Glu Tyr Ile Leu Asp Leu Tyr
1               5                   10                  15

Pro Glu Pro Thr Asp Leu Tyr Cys Tyr Glu Gln Leu Ser Asp Ser Ser
            20                  25                  30

Asp Glu Asp Glu Gly Leu Asp Arg Pro Asp Gly Gln Ala Gln Pro Ala
        35                  40                  45

Thr Ala Asp Tyr Tyr Ile Val Thr Cys Cys His Thr Cys Asn Thr Thr
    50                  55                  60

Val Arg Leu Cys Val Asn Ser Thr Ala Ser Asp Leu Arg Thr Ile Gln
65                  70                  75                  80

Gln Leu Leu Met Gly Thr Val Asn Ile Val Cys Pro Ser Cys Ala Gln
                85                  90                  95

Leu

<210> 76
<211> 106
<212> PRT
<213> Artificial Sequence

<220>
<223> Protein sequence of E7 of HPV45

<400> 76

Met His Gly Pro Arg Glu Thr Leu Gln Glu Ile Val Leu His Leu Glu
1 5 10 15

Pro Gln Asn Glu Leu Asp Pro Val Asp Leu Leu Cys Tyr Glu Gln Leu
20 25 30

Ser Glu Ser Glu Glu Glu Asn Asp Glu Ala Asp Gly Val Ser His Ala
35 40 45

Gln Leu Pro Ala Arg Arg Ala Glu Pro Gln Arg His Lys Ile Leu Cys
50 55 60

Val Cys Cys Lys Cys Asp Gly Arg Ile Glu Leu Thr Val Glu Ser Ser
65 70 75 80

Ala Glu Asp Leu Arg Thr Leu Gln Gln Leu Phe Leu Ser Thr Leu Ser
85 90 95

Phe Val Cys Pro Trp Cys Ala Thr Asn Gln
100 105

<210> 77
<211> 99
<212> PRT
<213> Artificial Sequence

<220>
<223> Protein sequence of E7 of HPV52

<400> 77

Met Arg Gly Asp Lys Ala Thr Ile Lys Asp Tyr Ile Leu Asp Leu Gln
1 5 10 15

Pro Glu Thr Thr Asp Leu His Cys Tyr Glu Gln Leu Gly Asp Ser Ser
20 25 30

Asp Glu Glu Asp Thr Asp Gly Val Asp Arg Pro Asp Gly Gln Ala Glu
35 40 45

```
Gln Ala Thr Ser Asn Tyr Tyr Ile Val Thr Tyr Cys His Ser Cys Asp
    50                  55                  60

Ser Thr Leu Arg Leu Cys Ile His Ser Thr Ala Thr Asp Leu Arg Thr
65                  70                  75                  80

Leu Gln Gln Met Leu Leu Gly Thr Leu Gln Val Val Cys Pro Gly Cys
                85                  90                  95

Ala Arg Leu



<210>  78
<211>  99
<212>  PRT
<213>  Artificial Sequence

<220>
<223>  Modified version of protein sequence of E7 of HPV52

<400>  78

Met Arg Gly Asp Lys Ala Thr Ile Lys Asp Tyr Ile Leu Asp Leu Gln
1               5                   10                  15

Pro Glu Thr Thr Asp Leu His Cys Tyr Glu Gln Leu Gly Asp Ser Ser
            20                  25                  30

Asp Glu Glu Asp Thr Asp Gly Val Asp Arg Pro Asp Gly Gln Ala Glu
        35                  40                  45

Gln Ala Thr Ser Asn Tyr Tyr Ile Val Thr Tyr Cys His Ser Cys Asp
    50                  55                  60

Ser Thr Leu Arg Leu Cys Ile His Ser Thr Ala Thr Asp Leu Arg Thr
65                  70                  75                  80

Leu Gln Gln Leu Leu Met Gly Thr Leu Gln Val Val Cys Pro Gly Cys
                85                  90                  95

Ala Arg Leu



<210>  79
<211>  98
<212>  PRT
<213>  Artificial Sequence

<220>
<223>  Protein sequence of E7 of HPV 58
```

<400> 79

```
Met Arg Gly Asn Asn Pro Thr Leu Arg Glu Tyr Ile Leu Asp Leu His
1               5                   10                  15

Pro Glu Pro Thr Asp Leu Phe Cys Tyr Glu Gln Leu Cys Asp Ser Ser
            20                  25                  30

Asp Glu Asp Glu Ile Gly Leu Asp Arg Pro Asp Gly Gln Ala Gln Pro
        35                  40                  45

Ala Thr Ala Asn Tyr Tyr Ile Val Thr Cys Cys Tyr Thr Cys Asp Thr
    50                  55                  60

Thr Val Arg Leu Cys Ile Asn Ser Thr Thr Thr Asp Val Arg Thr Leu
65                  70                  75                  80

Gln Gln Leu Leu Met Gly Thr Cys Thr Ile Val Cys Pro Ser Cys Ala
                85                  90                  95

Gln Gln
```

<210> 80
<211> 828
<212> DNA
<213> Artificial Sequence

<220>
<223> Polynucleotide for antigen E7 of HPV16, HPV18 and HPV45 acidic-deleted

<220>
<221> CDS
<222> (1)..(828)

<400> 80

```
ggt cag gcg gaa ccg gat cgc gcg cac tac aac atc gtt act ttt tgt        48
Gly Gln Ala Glu Pro Asp Arg Ala His Tyr Asn Ile Val Thr Phe Cys
1               5                   10                  15

tgc aaa tgt gat tcc act ctg cgc ctg tgt gtg caa tcc acc cac gta        96
Cys Lys Cys Asp Ser Thr Leu Arg Leu Cys Val Gln Ser Thr His Val
            20                  25                  30

gac att cgc act ctg gaa gac ctg ctg atg ggt acc ctt ggt att gtt       144
Asp Ile Arg Thr Leu Glu Asp Leu Leu Met Gly Thr Leu Gly Ile Val
        35                  40                  45

tgt ccg att tgc tcc cag aag ccg gcg tct ggc gtt aac cac caa cac       192
Cys Pro Ile Cys Ser Gln Lys Pro Ala Ser Gly Val Asn His Gln His
    50                  55                  60

ctg ccg gcg cgt cgc gca gag ccg cag cgt cac acc atg ctg tgc atg       240
Leu Pro Ala Arg Arg Ala Glu Pro Gln Arg His Thr Met Leu Cys Met
```

```
65                  70                  75                  80

tgc tgt aaa tgt gag gcc cgt atc gag ctg gtc gtt gag tct agc gcc        288
Cys Cys Lys Cys Glu Ala Arg Ile Glu Leu Val Val Glu Ser Ser Ala
                85                  90                  95

gac gat ctg cgt gcg ttt cag cag ctg ttc ctg aac acg ctg agc ttc        336
Asp Asp Leu Arg Ala Phe Gln Gln Leu Phe Leu Asn Thr Leu Ser Phe
            100                 105                 110

gtt tgc ccg tgg tgt gct agc cag cag ggc gtg tct cac gct cag ctg        384
Val Cys Pro Trp Cys Ala Ser Gln Gln Gly Val Ser His Ala Gln Leu
        115                 120                 125

ccg gca cgt cgt gct gag cct cag cgt cac aaa atc ctg tgc gtg tgc        432
Pro Ala Arg Arg Ala Glu Pro Gln Arg His Lys Ile Leu Cys Val Cys
    130                 135                 140

tgc aaa tgc gat ggc cgc atc gaa ctg acc gtg gaa tct agc gca gaa        480
Cys Lys Cys Asp Gly Arg Ile Glu Leu Thr Val Glu Ser Ser Ala Glu
145                 150                 155                 160

gac ctg cgt acg ctg cag caa ctg ttc ctg agc acg ctg tcc ttc gta        528
Asp Leu Arg Thr Leu Gln Gln Leu Phe Leu Ser Thr Leu Ser Phe Val
                165                 170                 175

tgc cct tgg tgc gca act aat cag atg cac ggt gac acc ccg acc ctg        576
Cys Pro Trp Cys Ala Thr Asn Gln Met His Gly Asp Thr Pro Thr Leu
            180                 185                 190

cac gaa tac atg ctg gac ctg cag cca gaa acc acc gat ctg tac tgt        624
His Glu Tyr Met Leu Asp Leu Gln Pro Glu Thr Thr Asp Leu Tyr Cys
        195                 200                 205

tac gaa cag ctg aac atg cat ggc cca aaa gct act ctg cag gat atc        672
Tyr Glu Gln Leu Asn Met His Gly Pro Lys Ala Thr Leu Gln Asp Ile
    210                 215                 220

gtc ctg cat ctg gaa cca cag aac gaa atc ccg gta gat ctg ctg tgc        720
Val Leu His Leu Glu Pro Gln Asn Glu Ile Pro Val Asp Leu Leu Cys
225                 230                 235                 240

cat gag cag ctg atg cat ggt ccg cgt gaa acc ctg cag gaa atc gtt        768
His Glu Gln Leu Met His Gly Pro Arg Glu Thr Leu Gln Glu Ile Val
                245                 250                 255

ctg cac ctg gaa ccg caa aac gaa ctg gac ccg gtt gac ctg ctg tgc        816
Leu His Leu Glu Pro Gln Asn Glu Leu Asp Pro Val Asp Leu Leu Cys
            260                 265                 270

tat gaa cag ctg                                                        828
Tyr Glu Gln Leu
        275


<210>  81
<211>  276
<212>  PRT
<213>  Artificial Sequence

<220>
<223>  Synthetic Construct
```

<400> 81

```
Gly Gln Ala Glu Pro Asp Arg Ala His Tyr Asn Ile Val Thr Phe Cys
1               5                   10                  15

Cys Lys Cys Asp Ser Thr Leu Arg Leu Cys Val Gln Ser Thr His Val
            20                  25                  30

Asp Ile Arg Thr Leu Glu Asp Leu Leu Met Gly Thr Leu Gly Ile Val
        35                  40                  45

Cys Pro Ile Cys Ser Gln Lys Pro Ala Ser Gly Val Asn His Gln His
    50                  55                  60

Leu Pro Ala Arg Arg Ala Glu Pro Gln Arg His Thr Met Leu Cys Met
65                  70                  75                  80

Cys Cys Lys Cys Glu Ala Arg Ile Glu Leu Val Val Glu Ser Ser Ala
                85                  90                  95

Asp Asp Leu Arg Ala Phe Gln Gln Leu Phe Leu Asn Thr Leu Ser Phe
            100                 105                 110

Val Cys Pro Trp Cys Ala Ser Gln Gln Gly Val Ser His Ala Gln Leu
        115                 120                 125

Pro Ala Arg Arg Ala Glu Pro Gln Arg His Lys Ile Leu Cys Val Cys
    130                 135                 140

Cys Lys Cys Asp Gly Arg Ile Glu Leu Thr Val Glu Ser Ser Ala Glu
145                 150                 155                 160

Asp Leu Arg Thr Leu Gln Gln Leu Phe Leu Ser Thr Leu Ser Phe Val
                165                 170                 175

Cys Pro Trp Cys Ala Thr Asn Gln Met His Gly Asp Thr Pro Thr Leu
            180                 185                 190

His Glu Tyr Met Leu Asp Leu Gln Pro Glu Thr Thr Asp Leu Tyr Cys
        195                 200                 205

Tyr Glu Gln Leu Asn Met His Gly Pro Lys Ala Thr Leu Gln Asp Ile
    210                 215                 220

Val Leu His Leu Glu Pro Gln Asn Glu Ile Pro Val Asp Leu Leu Cys
225                 230                 235                 240

His Glu Gln Leu Met His Gly Pro Arg Glu Thr Leu Gln Glu Ile Val
```

```
                245                 250                 255

Leu His Leu Glu Pro Gln Asn Glu Leu Asp Pro Val Asp Leu Leu Cys
            260                 265                 270

Tyr Glu Gln Leu
        275


<210>  82
<211>  933
<212>  DNA
<213>  Artificial Sequence

<220>
<223>  Polynucleotide for antigen E7 of HPV16, HPV18 and HPV45 full


<220>
<221>  CDS
<222>  (1)..(933)

<400>  82
gaa gac gaa atc gac ggc cct gcg ggc cag gct gaa cca gat cgt gct        48
Glu Asp Glu Ile Asp Gly Pro Ala Gly Gln Ala Glu Pro Asp Arg Ala
1               5                   10                  15

cac tac aac atc gta act ttt tgc tgt aag tgc gat agc act ctg cgt        96
His Tyr Asn Ile Val Thr Phe Cys Cys Lys Cys Asp Ser Thr Leu Arg
            20                  25                  30

ctg tgc gta cag tct act cac gtt gat atc cgc act ctg gaa gat ctg       144
Leu Cys Val Gln Ser Thr His Val Asp Ile Arg Thr Leu Glu Asp Leu
        35                  40                  45

ctg atg ggt acc ctg ggt atc gtc tgc cca atc tgc tct caa aag cct       192
Leu Met Gly Thr Leu Gly Ile Val Cys Pro Ile Cys Ser Gln Lys Pro
    50                  55                  60

gct tct ggt gtt aac cat cag cac ctg ccg gct cgt cgc gct gaa cca       240
Ala Ser Gly Val Asn His Gln His Leu Pro Ala Arg Arg Ala Glu Pro
65                  70                  75                  80

cag cgt cat acc atg ctg tgc atg tgt tgc aaa tgc gag gct cgc atc       288
Gln Arg His Thr Met Leu Cys Met Cys Cys Lys Cys Glu Ala Arg Ile
                85                  90                  95

gaa ctg gtt gtt gaa tcc agc gct gac gac ctg cgt gcg ttt cag caa       336
Glu Leu Val Val Glu Ser Ser Ala Asp Asp Leu Arg Ala Phe Gln Gln
            100                 105                 110

ctg ttc ctg aac acg ctg tct ttt gtt tgt ccg tgg tgt gcc tcc cag       384
Leu Phe Leu Asn Thr Leu Ser Phe Val Cys Pro Trp Cys Ala Ser Gln
        115                 120                 125

cag gag aac gat gaa gcg gat ggc gtg tcc cac gcg cag ctg ccg gca       432
Gln Glu Asn Asp Glu Ala Asp Gly Val Ser His Ala Gln Leu Pro Ala
    130                 135                 140

cgt cgt gca gaa ccg cag cgc cac aag att ctg tgc gtt tgc tgt aaa       480
Arg Arg Ala Glu Pro Gln Arg His Lys Ile Leu Cys Val Cys Cys Lys
```

```
145                 150                 155                 160

tgt gat ggc cgt atc gaa ctg act gtg gaa tcc tcc gcg gaa gat ctg           528
Cys Asp Gly Arg Ile Glu Leu Thr Val Glu Ser Ser Ala Glu Asp Leu
                165                 170                 175

cgt acg ctg cag cag ctg ttc ctg tct acc ctg tct ttc gtg tgc ccg           576
Arg Thr Leu Gln Gln Leu Phe Leu Ser Thr Leu Ser Phe Val Cys Pro
            180                 185                 190

tgg tgc gcc acc aat caa atg cac ggc gac acc ccg acc ctg cac gaa           624
Trp Cys Ala Thr Asn Gln Met His Gly Asp Thr Pro Thr Leu His Glu
        195                 200                 205

tac atg ctg gac ctg cag ccg gaa acc acg gat ctg tat tgt tat gaa           672
Tyr Met Leu Asp Leu Gln Pro Glu Thr Thr Asp Leu Tyr Cys Tyr Glu
    210                 215                 220

cag ctg aac gat agc agc gag gaa atg cac ggt ccg aaa gca act ctg           720
Gln Leu Asn Asp Ser Ser Glu Glu Met His Gly Pro Lys Ala Thr Leu
225                 230                 235                 240

cag gac att gtg ctg cat ctg gag cca cag aac gaa atc ccg gtt gat           768
Gln Asp Ile Val Leu His Leu Glu Pro Gln Asn Glu Ile Pro Val Asp
                245                 250                 255

ctg ctg tgc cac gag caa ctg agc gat tcc gaa gag gaa aac gac gaa           816
Leu Leu Cys His Glu Gln Leu Ser Asp Ser Glu Glu Glu Asn Asp Glu
            260                 265                 270

att gat atg cat ggt ccg cgc gag acc ctg caa gaa atc gtc ctg cac           864
Ile Asp Met His Gly Pro Arg Glu Thr Leu Gln Glu Ile Val Leu His
        275                 280                 285

ctg gaa ccg caa aac gaa ctg gac cct gta gac ctg ctg tgc tac gaa           912
Leu Glu Pro Gln Asn Glu Leu Asp Pro Val Asp Leu Leu Cys Tyr Glu
    290                 295                 300

cag ctg tcc gaa tct gaa gaa                                               933
Gln Leu Ser Glu Ser Glu Glu
305                 310


<210>  83
<211>  311
<212>  PRT
<213>  Artificial Sequence

<220>
<223>  Synthetic Construct

<400>  83

Glu Asp Glu Ile Asp Gly Pro Ala Gly Gln Ala Glu Pro Asp Arg Ala
1               5                   10                  15


His Tyr Asn Ile Val Thr Phe Cys Cys Lys Cys Asp Ser Thr Leu Arg
            20                  25                  30


Leu Cys Val Gln Ser Thr His Val Asp Ile Arg Thr Leu Glu Asp Leu
        35                  40                  45
```

```
Leu Met Gly Thr Leu Gly Ile Val Cys Pro Ile Cys Ser Gln Lys Pro
    50                  55                  60

Ala Ser Gly Val Asn His Gln His Leu Pro Ala Arg Arg Ala Glu Pro
65                  70                  75                  80

Gln Arg His Thr Met Leu Cys Met Cys Cys Lys Cys Glu Ala Arg Ile
                85                  90                  95

Glu Leu Val Val Glu Ser Ser Ala Asp Asp Leu Arg Ala Phe Gln Gln
            100                 105                 110

Leu Phe Leu Asn Thr Leu Ser Phe Val Cys Pro Trp Cys Ala Ser Gln
        115                 120                 125

Gln Glu Asn Asp Glu Ala Asp Gly Val Ser His Ala Gln Leu Pro Ala
    130                 135                 140

Arg Arg Ala Glu Pro Gln Arg His Lys Ile Leu Cys Val Cys Cys Lys
145                 150                 155                 160

Cys Asp Gly Arg Ile Glu Leu Thr Val Glu Ser Ser Ala Glu Asp Leu
                165                 170                 175

Arg Thr Leu Gln Gln Leu Phe Leu Ser Thr Leu Ser Phe Val Cys Pro
            180                 185                 190

Trp Cys Ala Thr Asn Gln Met His Gly Asp Thr Pro Thr Leu His Glu
        195                 200                 205

Tyr Met Leu Asp Leu Gln Pro Glu Thr Thr Asp Leu Tyr Cys Tyr Glu
    210                 215                 220

Gln Leu Asn Asp Ser Ser Glu Glu Met His Gly Pro Lys Ala Thr Leu
225                 230                 235                 240

Gln Asp Ile Val Leu His Leu Glu Pro Gln Asn Glu Ile Pro Val Asp
                245                 250                 255

Leu Leu Cys His Glu Gln Leu Ser Asp Ser Glu Glu Glu Asn Asp Glu
            260                 265                 270

Ile Asp Met His Gly Pro Arg Glu Thr Leu Gln Glu Ile Val Leu His
        275                 280                 285

Leu Glu Pro Gln Asn Glu Leu Asp Pro Val Asp Leu Leu Cys Tyr Glu
```

290 295 300

Gln Leu Ser Glu Ser Glu Glu
305 310

<210> 84
<211> 1080
<212> DNA
<213> Artificial Sequence

<220>
<223> Polynucleotide for antigen E7 of HPV16, 18, 33 and 45 acidic-deleted

<220>
<221> CDS
<222> (1)..(1080)

<400> 84
ggt cag gcg gaa ccg gat cgc gcg cac tac aac atc gtt act ttt tgt 48
Gly Gln Ala Glu Pro Asp Arg Ala His Tyr Asn Ile Val Thr Phe Cys
1 5 10 15

tgc aaa tgt gat tcc act ctg cgc ctg tgt gtg caa tcc acc cac gta 96
Cys Lys Cys Asp Ser Thr Leu Arg Leu Cys Val Gln Ser Thr His Val
20 25 30

gac atc cgc act ctg gaa gac ctg ctg atg ggt acc ctg ggt att gtt 144
Asp Ile Arg Thr Leu Glu Asp Leu Leu Met Gly Thr Leu Gly Ile Val
35 40 45

tgt ccg att tgc tcc cag aag ccg gcg tct ggc gtt aac cac caa cac 192
Cys Pro Ile Cys Ser Gln Lys Pro Ala Ser Gly Val Asn His Gln His
50 55 60

ctg ccg gcg cgt cgc gca gag ccg cag cgt cac acc atg ctg tgc atg 240
Leu Pro Ala Arg Arg Ala Glu Pro Gln Arg His Thr Met Leu Cys Met
65 70 75 80

tgc tgt aaa tgt gag gcc cgt att gaa ctg gtc gtt gag tct agc gcc 288
Cys Cys Lys Cys Glu Ala Arg Ile Glu Leu Val Val Glu Ser Ser Ala
85 90 95

gac gat ctg cgt gcg ttt cag cag ctg ttc ctg aac acg ctg agc ttc 336
Asp Asp Leu Arg Ala Phe Gln Gln Leu Phe Leu Asn Thr Leu Ser Phe
100 105 110

gtt tgc ccg tgg tgt gct agc cag cag ggc gtg tct cac gct cag ctg 384
Val Cys Pro Trp Cys Ala Ser Gln Gln Gly Val Ser His Ala Gln Leu
115 120 125

ccg gca cgt cgt gct gag cct cag cgt cac aaa atc ctg tgc gtg tgc 432
Pro Ala Arg Arg Ala Glu Pro Gln Arg His Lys Ile Leu Cys Val Cys
130 135 140

tgc aaa tgc gat ggc cgc atc gaa ctg acc gtg gaa tct agc gca gaa 480
Cys Lys Cys Asp Gly Arg Ile Glu Leu Thr Val Glu Ser Ser Ala Glu
145 150 155 160

gac ctg cgt acg ctg cag caa ctg ttc ctg agc acg ctg tcc ttc gta 528

```
Asp Leu Arg Thr Leu Gln Gln Leu Phe Leu Ser Thr Leu Ser Phe Val
                165                 170                 175

tgc cct tgg tgc gca act aat cag ggt cag gcg caa ccg gcc acg gcg       576
Cys Pro Trp Cys Ala Thr Asn Gln Gly Gln Ala Gln Pro Ala Thr Ala
            180                 185                 190

gac tac tat atc gtg acc tgt tgc cac acc tgc aac act acc gtt cgc       624
Asp Tyr Tyr Ile Val Thr Cys Cys His Thr Cys Asn Thr Thr Val Arg
        195                 200                 205

ttg tgt gtc aat agc acc gct tcc gat ctg cgt acc att caa caa ctg       672
Leu Cys Val Asn Ser Thr Ala Ser Asp Leu Arg Thr Ile Gln Gln Leu
    210                 215                 220

ctg atg ggt acc gtt aac atc gtg tgc ccg agc tgc gca cag ctg atg       720
Leu Met Gly Thr Val Asn Ile Val Cys Pro Ser Cys Ala Gln Leu Met
225                 230                 235                 240

cac ggt gac acc ccg acc ctg cac gaa tac atg ctg gac ctg cag cca       768
His Gly Asp Thr Pro Thr Leu His Glu Tyr Met Leu Asp Leu Gln Pro
                245                 250                 255

gaa acc acc gat ctg tac tgt tac gaa cag ctg aac atg cat ggc cca       816
Glu Thr Thr Asp Leu Tyr Cys Tyr Glu Gln Leu Asn Met His Gly Pro
            260                 265                 270

aaa gct act ctg cag gat atc gtc ctg cat ctg gaa cca cag aac gaa       864
Lys Ala Thr Leu Gln Asp Ile Val Leu His Leu Glu Pro Gln Asn Glu
        275                 280                 285

atc ccg gta gat ctg ctg tgc cat gag cag ctg atg cat ggt ccg cgt       912
Ile Pro Val Asp Leu Leu Cys His Glu Gln Leu Met His Gly Pro Arg
    290                 295                 300

gaa acc ctg cag gaa atc gtt ctg cac ctg gaa ccg caa aac gaa ctg       960
Glu Thr Leu Gln Glu Ile Val Leu His Leu Glu Pro Gln Asn Glu Leu
305                 310                 315                 320

gac ccg gtt gac ctg ctg tgc tat gaa cag ctg atg cgt ggc cat aag      1008
Asp Pro Val Asp Leu Leu Cys Tyr Glu Gln Leu Met Arg Gly His Lys
                325                 330                 335

ccg acg ctg aaa gag tat att ctg gac ctg tac cca gag ccg acg gat      1056
Pro Thr Leu Lys Glu Tyr Ile Leu Asp Leu Tyr Pro Glu Pro Thr Asp
            340                 345                 350

ttg tac tgt tat gaa cag ctg agc                                      1080
Leu Tyr Cys Tyr Glu Gln Leu Ser
        355                 360


<210>  85
<211>  360
<212>  PRT
<213>  Artificial Sequence

<220>
<223>  Synthetic Construct

<400>  85

Gly Gln Ala Glu Pro Asp Arg Ala His Tyr Asn Ile Val Thr Phe Cys
```

```
1               5                   10                  15

Cys Lys Cys Asp Ser Thr Leu Arg Leu Cys Val Gln Ser Thr His Val
            20                  25                  30

Asp Ile Arg Thr Leu Glu Asp Leu Leu Met Gly Thr Leu Gly Ile Val
        35                  40                  45

Cys Pro Ile Cys Ser Gln Lys Pro Ala Ser Gly Val Asn His Gln His
    50                  55                  60

Leu Pro Ala Arg Arg Ala Glu Pro Gln Arg His Thr Met Leu Cys Met
65                  70                  75                  80

Cys Cys Lys Cys Glu Ala Arg Ile Glu Leu Val Val Glu Ser Ser Ala
                85                  90                  95

Asp Asp Leu Arg Ala Phe Gln Gln Leu Phe Leu Asn Thr Leu Ser Phe
            100                 105                 110

Val Cys Pro Trp Cys Ala Ser Gln Gln Gly Val Ser His Ala Gln Leu
        115                 120                 125

Pro Ala Arg Arg Ala Glu Pro Gln Arg His Lys Ile Leu Cys Val Cys
    130                 135                 140

Cys Lys Cys Asp Gly Arg Ile Glu Leu Thr Val Glu Ser Ser Ala Glu
145                 150                 155                 160

Asp Leu Arg Thr Leu Gln Gln Leu Phe Leu Ser Thr Leu Ser Phe Val
                165                 170                 175

Cys Pro Trp Cys Ala Thr Asn Gln Gly Gln Ala Gln Pro Ala Thr Ala
            180                 185                 190

Asp Tyr Tyr Ile Val Thr Cys Cys His Thr Cys Asn Thr Thr Val Arg
        195                 200                 205

Leu Cys Val Asn Ser Thr Ala Ser Asp Leu Arg Thr Ile Gln Gln Leu
    210                 215                 220

Leu Met Gly Thr Val Asn Ile Val Cys Pro Ser Cys Ala Gln Leu Met
225                 230                 235                 240

His Gly Asp Thr Pro Thr Leu His Glu Tyr Met Leu Asp Leu Gln Pro
                245                 250                 255
```

```
Glu Thr Thr Asp Leu Tyr Cys Tyr Glu Gln Leu Asn Met His Gly Pro
            260                 265                 270

Lys Ala Thr Leu Gln Asp Ile Val Leu His Leu Glu Pro Gln Asn Glu
        275                 280                 285

Ile Pro Val Asp Leu Leu Cys His Glu Gln Leu Met His Gly Pro Arg
    290                 295                 300

Glu Thr Leu Gln Glu Ile Val Leu His Leu Glu Pro Gln Asn Glu Leu
305                 310                 315                 320

Asp Pro Val Asp Leu Leu Cys Tyr Glu Gln Leu Met Arg Gly His Lys
                325                 330                 335

Pro Thr Leu Lys Glu Tyr Ile Leu Asp Leu Tyr Pro Glu Pro Thr Asp
            340                 345                 350

Leu Tyr Cys Tyr Glu Gln Leu Ser
        355                 360


<210>  86
<211>  1224
<212>  DNA
<213>  Artificial Sequence

<220>
<223>  Polynucleotide for antigen E7 of HPV16, 18, 33 and 45 full


<220>
<221>  CDS
<222>  (1)..(1224)

<400>  86
gaa gac gaa atc gac ggc cct gcg ggc cag gct gaa cca gat cgt gct       48
Glu Asp Glu Ile Asp Gly Pro Ala Gly Gln Ala Glu Pro Asp Arg Ala
1               5                   10                  15

cac tac aac atc gta act ttt tgc tgt aag tgc gat agc act ctg cgt       96
His Tyr Asn Ile Val Thr Phe Cys Cys Lys Cys Asp Ser Thr Leu Arg
            20                  25                  30

ctg tgc gta cag tct act cac gtt gat atc cgc act ctg gaa gat ctg      144
Leu Cys Val Gln Ser Thr His Val Asp Ile Arg Thr Leu Glu Asp Leu
        35                  40                  45

ctg atg ggt acc ctg ggt atc gtc tgc cca atc tgc tct caa aag cct      192
Leu Met Gly Thr Leu Gly Ile Val Cys Pro Ile Cys Ser Gln Lys Pro
    50                  55                  60

gct tct ggt gtt aac cat cag cac ctg ccg gct cgt cgc gct gaa cca      240
Ala Ser Gly Val Asn His Gln His Leu Pro Ala Arg Arg Ala Glu Pro
65                  70                  75                  80

cag cgt cat acc atg ctg tgc atg tgt tgc aaa tgc gag gct cgc atc      288
```

```
Gln Arg His Thr Met Leu Cys Met Cys Cys Lys Cys Glu Ala Arg Ile
                85                  90                  95

gaa ctg gtt gtt gaa tcc agc gct gac gac ctg cgt gcg ttt cag caa        336
Glu Leu Val Val Glu Ser Ser Ala Asp Asp Leu Arg Ala Phe Gln Gln
            100                 105                 110

ctg ttc ctg aac acg ctg tct ttt gtt tgt ccg tgg tgt gcc tcc cag        384
Leu Phe Leu Asn Thr Leu Ser Phe Val Cys Pro Trp Cys Ala Ser Gln
        115                 120                 125

cag gag aac gat gaa gcg gat ggc gtg tcc cac gcg cag ctg ccg gca        432
Gln Glu Asn Asp Glu Ala Asp Gly Val Ser His Ala Gln Leu Pro Ala
    130                 135                 140

cgt cgt gca gaa ccg cag cgc cac aag att ctg tgc gtt tgc tgt aaa        480
Arg Arg Ala Glu Pro Gln Arg His Lys Ile Leu Cys Val Cys Cys Lys
145                 150                 155                 160

tgt gat ggc cgt atc gaa ctg act gtg gaa tcc tcc gcg gaa gat ctg        528
Cys Asp Gly Arg Ile Glu Leu Thr Val Glu Ser Ser Ala Glu Asp Leu
                165                 170                 175

cgt acg ctg cag cag ctg ttc ctg tct acc ctg tct ttc gtg tgc ccg        576
Arg Thr Leu Gln Gln Leu Phe Leu Ser Thr Leu Ser Phe Val Cys Pro
            180                 185                 190

tgg tgc gcc acc aat caa tcg gat gaa gat gaa ggt ctg gat cgt ccg        624
Trp Cys Ala Thr Asn Gln Ser Asp Glu Asp Glu Gly Leu Asp Arg Pro
        195                 200                 205

gat ggt caa gcg cag ccg gcg act gca gat tac tat atc gtc act tgt        672
Asp Gly Gln Ala Gln Pro Ala Thr Ala Asp Tyr Tyr Ile Val Thr Cys
    210                 215                 220

tgc cac acc tgc aat acc acc gtg cgt ctg tgt gtg aac agc acg gcg        720
Cys His Thr Cys Asn Thr Thr Val Arg Leu Cys Val Asn Ser Thr Ala
225                 230                 235                 240

agc gac ctg cgc acg atc caa cag ttg ttg atg ggc acc gtg aat atc        768
Ser Asp Leu Arg Thr Ile Gln Gln Leu Leu Met Gly Thr Val Asn Ile
                245                 250                 255

gtc tgc cca agc tgt gct cag ttg atg cac ggc gac acc ccg acc ctg        816
Val Cys Pro Ser Cys Ala Gln Leu Met His Gly Asp Thr Pro Thr Leu
            260                 265                 270

cac gaa tac atg ctg gac ctg cag ccg gaa acc acg gat ctg tat tgt        864
His Glu Tyr Met Leu Asp Leu Gln Pro Glu Thr Thr Asp Leu Tyr Cys
        275                 280                 285

tat gaa cag ctg aac gat agc agc gag gaa atg cac ggt ccg aaa gca        912
Tyr Glu Gln Leu Asn Asp Ser Ser Glu Glu Met His Gly Pro Lys Ala
    290                 295                 300

act ctg cag gac att gtg ctg cat ctg gag cca cag aac gaa atc ccg        960
Thr Leu Gln Asp Ile Val Leu His Leu Glu Pro Gln Asn Glu Ile Pro
305                 310                 315                 320

gtt gat ctg ctg tgc cac gag caa ctg agc gat tcc gaa gag gaa aac       1008
Val Asp Leu Leu Cys His Glu Gln Leu Ser Asp Ser Glu Glu Glu Asn
                325                 330                 335
```

```
gac gaa att gat atg cat ggt ccg cgc gag acc ctg caa gaa atc gtc      1056
Asp Glu Ile Asp Met His Gly Pro Arg Glu Thr Leu Gln Glu Ile Val
            340                 345                 350

ctg cac ctg gaa ccg caa aac gaa ctg gac cct gta gac ctg ctg tgc      1104
Leu His Leu Glu Pro Gln Asn Glu Leu Asp Pro Val Asp Leu Leu Cys
        355                 360                 365

tac gaa cag ctg tcc gaa tct gaa gaa atg cgt ggt cac aaa ccg acg      1152
Tyr Glu Gln Leu Ser Glu Ser Glu Glu Met Arg Gly His Lys Pro Thr
    370                 375                 380

ctg aaa gag tac atc ctg gac ctg tac ccg gaa cct acg gat ctg tac      1200
Leu Lys Glu Tyr Ile Leu Asp Leu Tyr Pro Glu Pro Thr Asp Leu Tyr
385                 390                 395                 400

tgt tat gaa caa ctg agc gat agc                                      1224
Cys Tyr Glu Gln Leu Ser Asp Ser
                405


<210>  87
<211>  408
<212>  PRT
<213>  Artificial Sequence

<220>
<223>  Synthetic Construct

<400>  87

Glu Asp Glu Ile Asp Gly Pro Ala Gly Gln Ala Glu Pro Asp Arg Ala
1               5                   10                  15


His Tyr Asn Ile Val Thr Phe Cys Cys Lys Cys Asp Ser Thr Leu Arg
            20                  25                  30


Leu Cys Val Gln Ser Thr His Val Asp Ile Arg Thr Leu Glu Asp Leu
        35                  40                  45


Leu Met Gly Thr Leu Gly Ile Val Cys Pro Ile Cys Ser Gln Lys Pro
    50                  55                  60


Ala Ser Gly Val Asn His Gln His Leu Pro Ala Arg Arg Ala Glu Pro
65                  70                  75                  80


Gln Arg His Thr Met Leu Cys Met Cys Cys Lys Cys Glu Ala Arg Ile
                85                  90                  95


Glu Leu Val Val Glu Ser Ser Ala Asp Asp Leu Arg Ala Phe Gln Gln
            100                 105                 110


Leu Phe Leu Asn Thr Leu Ser Phe Val Cys Pro Trp Cys Ala Ser Gln
        115                 120                 125
```

```
Gln Glu Asn Asp Glu Ala Asp Gly Val Ser His Ala Gln Leu Pro Ala
    130                 135                 140

Arg Arg Ala Glu Pro Gln Arg His Lys Ile Leu Cys Val Cys Cys Lys
145                 150                 155                 160

Cys Asp Gly Arg Ile Glu Leu Thr Val Glu Ser Ser Ala Glu Asp Leu
                165                 170                 175

Arg Thr Leu Gln Gln Leu Phe Leu Ser Thr Leu Ser Phe Val Cys Pro
            180                 185                 190

Trp Cys Ala Thr Asn Gln Ser Asp Glu Asp Glu Gly Leu Asp Arg Pro
        195                 200                 205

Asp Gly Gln Ala Gln Pro Ala Thr Ala Asp Tyr Tyr Ile Val Thr Cys
    210                 215                 220

Cys His Thr Cys Asn Thr Thr Val Arg Leu Cys Val Asn Ser Thr Ala
225                 230                 235                 240

Ser Asp Leu Arg Thr Ile Gln Gln Leu Leu Met Gly Thr Val Asn Ile
                245                 250                 255

Val Cys Pro Ser Cys Ala Gln Leu Met His Gly Asp Thr Pro Thr Leu
            260                 265                 270

His Glu Tyr Met Leu Asp Leu Gln Pro Glu Thr Thr Asp Leu Tyr Cys
        275                 280                 285

Tyr Glu Gln Leu Asn Asp Ser Ser Glu Glu Met His Gly Pro Lys Ala
    290                 295                 300

Thr Leu Gln Asp Ile Val Leu His Leu Glu Pro Gln Asn Glu Ile Pro
305                 310                 315                 320

Val Asp Leu Leu Cys His Glu Gln Leu Ser Asp Ser Glu Glu Glu Asn
                325                 330                 335

Asp Glu Ile Asp Met His Gly Pro Arg Glu Thr Leu Gln Glu Ile Val
            340                 345                 350

Leu His Leu Glu Pro Gln Asn Glu Leu Asp Pro Val Asp Leu Leu Cys
        355                 360                 365

Tyr Glu Gln Leu Ser Glu Ser Glu Glu Met Arg Gly His Lys Pro Thr
    370                 375                 380
```

```
Leu Lys Glu Tyr Ile Leu Asp Leu Tyr Pro Glu Pro Thr Asp Leu Tyr
385                 390                 395                 400


Cys Tyr Glu Gln Leu Ser Asp Ser
                405


<210>  88
<211>  759
<212>  DNA
<213>  Artificial Sequence

<220>
<223>  Polynucleotide for antigen E7 of HPV31, 52 and 58 acidic-deleted


<220>
<221>  CDS
<222>  (1)..(759)

<400>  88
gcg ggt caa gcc aaa ccg gat acc tcc aac tac aac att gtg acc ttc        48
Ala Gly Gln Ala Lys Pro Asp Thr Ser Asn Tyr Asn Ile Val Thr Phe
1               5                   10                  15

tgt tgt caa tgc gag agc acg ctg cgt ctg tgt gtt caa agc acc cag        96
Cys Cys Gln Cys Glu Ser Thr Leu Arg Leu Cys Val Gln Ser Thr Gln
            20                  25                  30

gtt gac att cgt atc ctg caa gaa ctg ctg atg ggc tct ttt ggc atc       144
Val Asp Ile Arg Ile Leu Gln Glu Leu Leu Met Gly Ser Phe Gly Ile
        35                  40                  45

gtc tgc cca aat tgc agc act cgc ctg ggc cag gcg gag cag gcg acg       192
Val Cys Pro Asn Cys Ser Thr Arg Leu Gly Gln Ala Glu Gln Ala Thr
    50                  55                  60

agc aac tac tac att gtc acg tat tgc cat tcc tgt gat agc act ctg       240
Ser Asn Tyr Tyr Ile Val Thr Tyr Cys His Ser Cys Asp Ser Thr Leu
65                  70                  75                  80

cgt ctg tgt atc cac agc acg gca acc gat ctg cgt acc ttg caa caa       288
Arg Leu Cys Ile His Ser Thr Ala Thr Asp Leu Arg Thr Leu Gln Gln
                85                  90                  95

ctg ctg atg ggc acc ctg cag gtg gtt tgt ccg ggt tgc gct cgc ctg       336
Leu Leu Met Gly Thr Leu Gln Val Val Cys Pro Gly Cys Ala Arg Leu
            100                 105                 110

ggc cag gcg cag cca gcg acg gcc aat tac tac atc gtt acc tgt tgc       384
Gly Gln Ala Gln Pro Ala Thr Ala Asn Tyr Tyr Ile Val Thr Cys Cys
        115                 120                 125

tat acg tgc gat act acc gtc cgt ctg tgc att aac agc acc acc acg       432
Tyr Thr Cys Asp Thr Thr Val Arg Leu Cys Ile Asn Ser Thr Thr Thr
    130                 135                 140

gac gtg cgt acc ctg caa cag ctg ctg atg ggt acc tgc acg att gtt       480
Asp Val Arg Thr Leu Gln Gln Leu Leu Met Gly Thr Cys Thr Ile Val
145                 150                 155                 160
```

```
tgt ccg agc tgt gca caa caa atg cgt ggt gaa acg ccg acc ctg cag         528
Cys Pro Ser Cys Ala Gln Gln Met Arg Gly Glu Thr Pro Thr Leu Gln
                165                 170                 175

gat tat gtg ttg gac ttg cag ccg gag gca acg gac ctg tac tgc tat         576
Asp Tyr Val Leu Asp Leu Gln Pro Glu Ala Thr Asp Leu Tyr Cys Tyr
            180                 185                 190

gag cag ctg atg cgt ggt gac aag gcc acc att aaa gat tac atc ctg         624
Glu Gln Leu Met Arg Gly Asp Lys Ala Thr Ile Lys Asp Tyr Ile Leu
        195                 200                 205

gac ttg caa ccg gaa acc acc gac ctg cac tgc tat gag cag ctg ggt         672
Asp Leu Gln Pro Glu Thr Thr Asp Leu His Cys Tyr Glu Gln Leu Gly
    210                 215                 220

atg cgc ggt aat aac ccg acc ttg cgt gag tat atc ttg gac ctg cac         720
Met Arg Gly Asn Asn Pro Thr Leu Arg Glu Tyr Ile Leu Asp Leu His
225                 230                 235                 240

ccg gag ccg acc gat ctg ttc tgt tac gaa cag ctg tgc                     759
Pro Glu Pro Thr Asp Leu Phe Cys Tyr Glu Gln Leu Cys
                245                 250


<210>  89
<211>  253
<212>  PRT
<213>  Artificial Sequence

<220>
<223>  Synthetic Construct

<400>  89

Ala Gly Gln Ala Lys Pro Asp Thr Ser Asn Tyr Asn Ile Val Thr Phe
1               5                   10                  15


Cys Cys Gln Cys Glu Ser Thr Leu Arg Leu Cys Val Gln Ser Thr Gln
            20                  25                  30


Val Asp Ile Arg Ile Leu Gln Glu Leu Leu Met Gly Ser Phe Gly Ile
        35                  40                  45


Val Cys Pro Asn Cys Ser Thr Arg Leu Gly Gln Ala Glu Gln Ala Thr
    50                  55                  60


Ser Asn Tyr Tyr Ile Val Thr Tyr Cys His Ser Cys Asp Ser Thr Leu
65                  70                  75                  80


Arg Leu Cys Ile His Ser Thr Ala Thr Asp Leu Arg Thr Leu Gln Gln
                85                  90                  95


Leu Leu Met Gly Thr Leu Gln Val Val Cys Pro Gly Cys Ala Arg Leu
            100                 105                 110
```

```
Gly Gln Ala Gln Pro Ala Thr Ala Asn Tyr Tyr Ile Val Thr Cys Cys
        115                 120                 125

Tyr Thr Cys Asp Thr Thr Val Arg Leu Cys Ile Asn Ser Thr Thr Thr
    130                 135                 140

Asp Val Arg Thr Leu Gln Gln Leu Leu Met Gly Thr Cys Thr Ile Val
145                 150                 155                 160

Cys Pro Ser Cys Ala Gln Gln Met Arg Gly Glu Thr Pro Thr Leu Gln
                165                 170                 175

Asp Tyr Val Leu Asp Leu Gln Pro Glu Ala Thr Asp Leu Tyr Cys Tyr
            180                 185                 190

Glu Gln Leu Met Arg Gly Asp Lys Ala Thr Ile Lys Asp Tyr Ile Leu
        195                 200                 205

Asp Leu Gln Pro Glu Thr Thr Asp Leu His Cys Tyr Glu Gln Leu Gly
    210                 215                 220

Met Arg Gly Asn Asn Pro Thr Leu Arg Glu Tyr Ile Leu Asp Leu His
225                 230                 235                 240

Pro Glu Pro Thr Asp Leu Phe Cys Tyr Glu Gln Leu Cys
                245                 250


<210>  90
<211>  885
<212>  DNA
<213>  Artificial Sequence

<220>
<223>  Polynucleotide for antigen E7 of HPV31, 52 and 58 full


<220>
<221>  CDS
<222>  (1)..(885)

<400>  90
gat gaa gag gat gtc atc gat tcc cca gca ggc caa gca aag ccg gac        48
Asp Glu Glu Asp Val Ile Asp Ser Pro Ala Gly Gln Ala Lys Pro Asp
1               5                   10                  15

acc agc aat tac aat atc gtg acc ttc tgc tgc caa tgt gag tcc act        96
Thr Ser Asn Tyr Asn Ile Val Thr Phe Cys Cys Gln Cys Glu Ser Thr
            20                  25                  30

ctg cgt ctg tgc gtt cag agc acc cag gtt gac att cgt att ctg caa       144
Leu Arg Leu Cys Val Gln Ser Thr Gln Val Asp Ile Arg Ile Leu Gln
        35                  40                  45

gaa ttg ttg atg ggt agc ttt ggc att gtg tgt ccg aac tgc agc acc       192
```

```
Glu Leu Leu Met Gly Ser Phe Gly Ile Val Cys Pro Asn Cys Ser Thr
    50                  55                  60

cgt ctg agc gat gaa gag gac acc gac ggt gtc gac cgt ccg gac ggc      240
Arg Leu Ser Asp Glu Glu Asp Thr Asp Gly Val Asp Arg Pro Asp Gly
65                  70                  75                  80

caa gct gag cag gcg acc agc aac tac tat atc gtt acg tac tgt cac      288
Gln Ala Glu Gln Ala Thr Ser Asn Tyr Tyr Ile Val Thr Tyr Cys His
                85                  90                  95

agc tgc gat agc act ctg cgc ctg tgt att cat agc acg gcc acc gat      336
Ser Cys Asp Ser Thr Leu Arg Leu Cys Ile His Ser Thr Ala Thr Asp
            100                 105                 110

ctg cgt acc ctg caa caa ctg ctg atg ggt act ctg cag gtt gtg tgc      384
Leu Arg Thr Leu Gln Gln Leu Leu Met Gly Thr Leu Gln Val Val Cys
        115                 120                 125

cca ggt tgt gct cgt ctg tcc gac gag gac gaa atc ggc ctg gac cgt      432
Pro Gly Cys Ala Arg Leu Ser Asp Glu Asp Glu Ile Gly Leu Asp Arg
    130                 135                 140

cct gat ggc cag gcc cag ccg gca acg gcg aac tat tac att gtg acc      480
Pro Asp Gly Gln Ala Gln Pro Ala Thr Ala Asn Tyr Tyr Ile Val Thr
145                 150                 155                 160

tgc tgc tat acg tgc gat acc acg gtc cgt ctg tgc att aac tct acc      528
Cys Cys Tyr Thr Cys Asp Thr Thr Val Arg Leu Cys Ile Asn Ser Thr
                165                 170                 175

acc acg gat gtt cgc acg ctg cag caa ctg ctg atg ggt acc tgt acc      576
Thr Thr Asp Val Arg Thr Leu Gln Gln Leu Leu Met Gly Thr Cys Thr
            180                 185                 190

att gtt tgc ccg tct tgt gcc cag cag atg cgc ggt gaa acg ccg acc      624
Ile Val Cys Pro Ser Cys Ala Gln Gln Met Arg Gly Glu Thr Pro Thr
        195                 200                 205

ctg cag gat tat gtt ctg gac ctg caa ccg gag gcg acc gat ctg tat      672
Leu Gln Asp Tyr Val Leu Asp Leu Gln Pro Glu Ala Thr Asp Leu Tyr
    210                 215                 220

tgc tac gag caa ctg ccg gac agc tcg atg cgt ggt gac aag gcg acg      720
Cys Tyr Glu Gln Leu Pro Asp Ser Ser Met Arg Gly Asp Lys Ala Thr
225                 230                 235                 240

att aag gac tac atc ttg gat ctg cag ccg gaa acc acc gac ctg cac      768
Ile Lys Asp Tyr Ile Leu Asp Leu Gln Pro Glu Thr Thr Asp Leu His
                245                 250                 255

tgc tat gag cag ctg ggt gat tct atg cgc ggt aac aat ccg acc ctg      816
Cys Tyr Glu Gln Leu Gly Asp Ser Met Arg Gly Asn Asn Pro Thr Leu
            260                 265                 270

cgc gag tac att ctg gac ttg cat ccg gaa ccg acg gac ctg ttc tgt      864
Arg Glu Tyr Ile Leu Asp Leu His Pro Glu Pro Thr Asp Leu Phe Cys
        275                 280                 285

tac gag cag ctg tgt gac agc                                          885
Tyr Glu Gln Leu Cys Asp Ser
    290                 295
```

<210> 91
<211> 295
<212> PRT
<213> Artificial Sequence

<220>
<223> Synthetic Construct

<400> 91

```
Asp Glu Glu Asp Val Ile Asp Ser Pro Ala Gly Gln Ala Lys Pro Asp
1               5                   10                  15

Thr Ser Asn Tyr Asn Ile Val Thr Phe Cys Cys Gln Cys Glu Ser Thr
            20                  25                  30

Leu Arg Leu Cys Val Gln Ser Thr Gln Val Asp Ile Arg Ile Leu Gln
        35                  40                  45

Glu Leu Leu Met Gly Ser Phe Gly Ile Val Cys Pro Asn Cys Ser Thr
    50                  55                  60

Arg Leu Ser Asp Glu Glu Asp Thr Asp Gly Val Asp Arg Pro Asp Gly
65                  70                  75                  80

Gln Ala Glu Gln Ala Thr Ser Asn Tyr Tyr Ile Val Thr Tyr Cys His
                85                  90                  95

Ser Cys Asp Ser Thr Leu Arg Leu Cys Ile His Ser Thr Ala Thr Asp
            100                 105                 110

Leu Arg Thr Leu Gln Gln Leu Leu Met Gly Thr Leu Gln Val Val Cys
        115                 120                 125

Pro Gly Cys Ala Arg Leu Ser Asp Glu Asp Glu Ile Gly Leu Asp Arg
    130                 135                 140

Pro Asp Gly Gln Ala Gln Pro Ala Thr Ala Asn Tyr Tyr Ile Val Thr
145                 150                 155                 160

Cys Cys Tyr Thr Cys Asp Thr Thr Val Arg Leu Cys Ile Asn Ser Thr
                165                 170                 175

Thr Thr Asp Val Arg Thr Leu Gln Gln Leu Leu Met Gly Thr Cys Thr
            180                 185                 190

Ile Val Cys Pro Ser Cys Ala Gln Gln Met Arg Gly Glu Thr Pro Thr
        195                 200                 205
```

```
Leu Gln Asp Tyr Val Leu Asp Leu Gln Pro Glu Ala Thr Asp Leu Tyr
    210                 215                 220

Cys Tyr Glu Gln Leu Pro Asp Ser Ser Met Arg Gly Asp Lys Ala Thr
225                 230                 235                 240

Ile Lys Asp Tyr Ile Leu Asp Leu Gln Pro Glu Thr Thr Asp Leu His
                245                 250                 255

Cys Tyr Glu Gln Leu Gly Asp Ser Met Arg Gly Asn Asn Pro Thr Leu
            260                 265                 270

Arg Glu Tyr Ile Leu Asp Leu His Pro Glu Pro Thr Asp Leu Phe Cys
        275                 280                 285

Tyr Glu Gln Leu Cys Asp Ser
    290                 295


<210>  92
<211>  759
<212>  DNA
<213>  Artificial Sequence

<220>
<223>  Polynucleotide for antigen E7 of HPV31, 33 and 52 acidic-deleted


<220>
<221>  CDS
<222>  (1)..(759)

<400>  92
gcg ggt caa gcc aaa ccg gat acc tcc aac tac aac att gtg acc ttc        48
Ala Gly Gln Ala Lys Pro Asp Thr Ser Asn Tyr Asn Ile Val Thr Phe
1               5                   10                  15

tgt tgt caa tgc gag agc acg ctg cgt ctg tgt gtt caa agc acc cag        96
Cys Cys Gln Cys Glu Ser Thr Leu Arg Leu Cys Val Gln Ser Thr Gln
            20                  25                  30

gtt gac att cgt atc ctg caa gaa ctg ctg atg ggc tct ttt ggc atc       144
Val Asp Ile Arg Ile Leu Gln Glu Leu Leu Met Gly Ser Phe Gly Ile
        35                  40                  45

gtc tgc cca aat tgc agc act cgc ctg ggt cag gcg caa ccg gcc acg       192
Val Cys Pro Asn Cys Ser Thr Arg Leu Gly Gln Ala Gln Pro Ala Thr
    50                  55                  60

gcg gac tac tat atc gtg acc tgt tgc cac acc tgc aac act acc gtt       240
Ala Asp Tyr Tyr Ile Val Thr Cys Cys His Thr Cys Asn Thr Thr Val
65                  70                  75                  80

cgc ttg tgt gtc aat agc acc gct tcc gat ctg cgt acc att caa caa       288
Arg Leu Cys Val Asn Ser Thr Ala Ser Asp Leu Arg Thr Ile Gln Gln
                85                  90                  95

ctg ctg atg ggt acc gtt aac atc gtg tgc ccg agc tgc gca cag ctg       336
```

```
Leu Leu Met Gly Thr Val Asn Ile Val Cys Pro Ser Cys Ala Gln Leu
            100                 105                 110

ggc cag gcg gag cag gcg acg agc aac tac tac att gtc acg tat tgc        384
Gly Gln Ala Glu Gln Ala Thr Ser Asn Tyr Tyr Ile Val Thr Tyr Cys
        115                 120                 125

cat tcc tgt gat agc act ctg cgt ctg tgt atc cac agc acg gca acc        432
His Ser Cys Asp Ser Thr Leu Arg Leu Cys Ile His Ser Thr Ala Thr
    130                 135                 140

gat ctg cgt acc ttg caa caa ctg ctg atg ggc acc ctg cag gtg gtt        480
Asp Leu Arg Thr Leu Gln Gln Leu Leu Met Gly Thr Leu Gln Val Val
145                 150                 155                 160

tgt ccg ggt tgc gct cgc ctg atg cgt ggt gaa acg ccg acc ctg cag        528
Cys Pro Gly Cys Ala Arg Leu Met Arg Gly Glu Thr Pro Thr Leu Gln
                165                 170                 175

gat tat gtg ttg gac ttg cag ccg gag gca acg gac ctg tac tgc tat        576
Asp Tyr Val Leu Asp Leu Gln Pro Glu Ala Thr Asp Leu Tyr Cys Tyr
            180                 185                 190

gag cag ctg atg cgt ggc cat aag ccg acg ctg aaa gag tat att ctg        624
Glu Gln Leu Met Arg Gly His Lys Pro Thr Leu Lys Glu Tyr Ile Leu
        195                 200                 205

gac ctg tac cca gag ccg acg gat ttg tac tgt tat gaa cag ctg agc        672
Asp Leu Tyr Pro Glu Pro Thr Asp Leu Tyr Cys Tyr Glu Gln Leu Ser
    210                 215                 220

atg cgt ggt gac aag gcc acc att aaa gat tac atc ctg gac ttg caa        720
Met Arg Gly Asp Lys Ala Thr Ile Lys Asp Tyr Ile Leu Asp Leu Gln
225                 230                 235                 240

ccg gaa acc acc gac ctg cac tgc tat gag cag ctg ggt                    759
Pro Glu Thr Thr Asp Leu His Cys Tyr Glu Gln Leu Gly
                245                 250


<210>  93
<211>  253
<212>  PRT
<213>  Artificial Sequence

<220>
<223>  Synthetic Construct

<400>  93

Ala Gly Gln Ala Lys Pro Asp Thr Ser Asn Tyr Asn Ile Val Thr Phe
1               5                   10                  15


Cys Cys Gln Cys Glu Ser Thr Leu Arg Leu Cys Val Gln Ser Thr Gln
            20                  25                  30


Val Asp Ile Arg Ile Leu Gln Glu Leu Leu Met Gly Ser Phe Gly Ile
        35                  40                  45


Val Cys Pro Asn Cys Ser Thr Arg Leu Gly Gln Ala Gln Pro Ala Thr
```

```
    50                  55                  60

Ala Asp Tyr Tyr Ile Val Thr Cys Cys His Thr Cys Asn Thr Thr Val
65                  70                  75                  80

Arg Leu Cys Val Asn Ser Thr Ala Ser Asp Leu Arg Thr Ile Gln Gln
                85                  90                  95

Leu Leu Met Gly Thr Val Asn Ile Val Cys Pro Ser Cys Ala Gln Leu
            100                 105                 110

Gly Gln Ala Glu Gln Ala Thr Ser Asn Tyr Tyr Ile Val Thr Tyr Cys
        115                 120                 125

His Ser Cys Asp Ser Thr Leu Arg Leu Cys Ile His Ser Thr Ala Thr
    130                 135                 140

Asp Leu Arg Thr Leu Gln Gln Leu Leu Met Gly Thr Leu Gln Val Val
145                 150                 155                 160

Cys Pro Gly Cys Ala Arg Leu Met Arg Gly Glu Thr Pro Thr Leu Gln
                165                 170                 175

Asp Tyr Val Leu Asp Leu Gln Pro Glu Ala Thr Asp Leu Tyr Cys Tyr
            180                 185                 190

Glu Gln Leu Met Arg Gly His Lys Pro Thr Leu Lys Glu Tyr Ile Leu
        195                 200                 205

Asp Leu Tyr Pro Glu Pro Thr Asp Leu Tyr Cys Tyr Glu Gln Leu Ser
    210                 215                 220

Met Arg Gly Asp Lys Ala Thr Ile Lys Asp Tyr Ile Leu Asp Leu Gln
225                 230                 235                 240

Pro Glu Thr Thr Asp Leu His Cys Tyr Glu Gln Leu Gly
                245                 250
```

<210>  94
<211>  882
<212>  DNA
<213>  Artificial Sequence

<220>
<223>  Polynucleotide for antigen E7 of HPV31, 33 and 52 full

<220>
<221>  CDS
<222>  (1)..(882)

<400> 94
gat gaa gag gat gtc atc gat tcc cca gca ggc caa gca aag ccg gac 48
Asp Glu Glu Asp Val Ile Asp Ser Pro Ala Gly Gln Ala Lys Pro Asp
1 5 10 15

acc agc aat tac aat atc gtg acc ttc tgc tgc caa tgt gag tcc act 96
Thr Ser Asn Tyr Asn Ile Val Thr Phe Cys Cys Gln Cys Glu Ser Thr
20 25 30

ctg cgt ctg tgc gtt cag agc acc cag gtt gac att cgt att ctg caa 144
Leu Arg Leu Cys Val Gln Ser Thr Gln Val Asp Ile Arg Ile Leu Gln
35 40 45

gaa ttg ttg atg ggt agc ttt ggc att gtg tgt ccg aac tgc agc acc 192
Glu Leu Leu Met Gly Ser Phe Gly Ile Val Cys Pro Asn Cys Ser Thr
50 55 60

cgt ctg tcg gat gaa gat gaa ggt ctg gat cgt ccg gat ggt caa gcg 240
Arg Leu Ser Asp Glu Asp Glu Gly Leu Asp Arg Pro Asp Gly Gln Ala
65 70 75 80

cag ccg gcg act gca gat tac tat atc gtc act tgt tgc cac acc tgc 288
Gln Pro Ala Thr Ala Asp Tyr Tyr Ile Val Thr Cys Cys His Thr Cys
85 90 95

aat acc acc gtg cgt ctg tgt gtg aac agc acg gcg agc gac ctg cgc 336
Asn Thr Thr Val Arg Leu Cys Val Asn Ser Thr Ala Ser Asp Leu Arg
100 105 110

acg atc caa cag ttg ttg atg ggc acc gtg aat atc gtc tgc cca agc 384
Thr Ile Gln Gln Leu Leu Met Gly Thr Val Asn Ile Val Cys Pro Ser
115 120 125

tgt gct cag ttg agc gat gaa gag gac acc gac ggt gtc gac cgt ccg 432
Cys Ala Gln Leu Ser Asp Glu Glu Asp Thr Asp Gly Val Asp Arg Pro
130 135 140

gac ggc caa gct gag cag gcg acc agc aac tac tat atc gtt acg tac 480
Asp Gly Gln Ala Glu Gln Ala Thr Ser Asn Tyr Tyr Ile Val Thr Tyr
145 150 155 160

tgt cac agc tgc gat agc act ctg cgc ctg tgt att cat agc acg gcc 528
Cys His Ser Cys Asp Ser Thr Leu Arg Leu Cys Ile His Ser Thr Ala
165 170 175

acc gat ctg cgt acc ctg caa caa ctg ctg atg ggt act ctg cag gtt 576
Thr Asp Leu Arg Thr Leu Gln Gln Leu Leu Met Gly Thr Leu Gln Val
180 185 190

gtg tgc cca ggt tgt gct cgt ctg atg cgc ggt gaa acg ccg acc ctg 624
Val Cys Pro Gly Cys Ala Arg Leu Met Arg Gly Glu Thr Pro Thr Leu
195 200 205

cag gat tat gtt ctg gac ctg caa ccg gag gcg acc gat ctg tat tgc 672
Gln Asp Tyr Val Leu Asp Leu Gln Pro Glu Ala Thr Asp Leu Tyr Cys
210 215 220

tac gag caa ctg ccg gac agc tcg atg cgt ggt cac aaa ccg acg ctg 720
Tyr Glu Gln Leu Pro Asp Ser Ser Met Arg Gly His Lys Pro Thr Leu
225 230 235 240

aaa gag tac atc ctg gac ctg tac ccg gaa cct acg gat ctg tac tgt 768

```
Lys Glu Tyr Ile Leu Asp Leu Tyr Pro Glu Pro Thr Asp Leu Tyr Cys
                245                 250                 255

tat gaa caa ctg agc gat agc atg cgt ggt gac aag gcg acg att aag       816
Tyr Glu Gln Leu Ser Asp Ser Met Arg Gly Asp Lys Ala Thr Ile Lys
            260                 265                 270

gac tac atc ttg gat ctg cag ccg gaa acc acc gac ctg cac tgc tat       864
Asp Tyr Ile Leu Asp Leu Gln Pro Glu Thr Thr Asp Leu His Cys Tyr
        275                 280                 285

gag cag ctg ggt gat tct                                               882
Glu Gln Leu Gly Asp Ser
    290


<210>  95
<211>  294
<212>  PRT
<213>  Artificial Sequence

<220>
<223>  Synthetic Construct

<400>  95

Asp Glu Glu Asp Val Ile Asp Ser Pro Ala Gly Gln Ala Lys Pro Asp
1               5                   10                  15


Thr Ser Asn Tyr Asn Ile Val Thr Phe Cys Cys Gln Cys Glu Ser Thr
            20                  25                  30


Leu Arg Leu Cys Val Gln Ser Thr Gln Val Asp Ile Arg Ile Leu Gln
        35                  40                  45


Glu Leu Leu Met Gly Ser Phe Gly Ile Val Cys Pro Asn Cys Ser Thr
    50                  55                  60


Arg Leu Ser Asp Glu Asp Glu Gly Leu Asp Arg Pro Asp Gly Gln Ala
65                  70                  75                  80


Gln Pro Ala Thr Ala Asp Tyr Tyr Ile Val Thr Cys Cys His Thr Cys
                85                  90                  95


Asn Thr Thr Val Arg Leu Cys Val Asn Ser Thr Ala Ser Asp Leu Arg
            100                 105                 110


Thr Ile Gln Gln Leu Leu Met Gly Thr Val Asn Ile Val Cys Pro Ser
        115                 120                 125


Cys Ala Gln Leu Ser Asp Glu Glu Asp Thr Asp Gly Val Asp Arg Pro
    130                 135                 140


Asp Gly Gln Ala Glu Gln Ala Thr Ser Asn Tyr Tyr Ile Val Thr Tyr
```

145 150 155 160

Cys His Ser Cys Asp Ser Thr Leu Arg Leu Cys Ile His Ser Thr Ala
165 170 175

Thr Asp Leu Arg Thr Leu Gln Gln Leu Leu Met Gly Thr Leu Gln Val
180 185 190

Val Cys Pro Gly Cys Ala Arg Leu Met Arg Gly Glu Thr Pro Thr Leu
195 200 205

Gln Asp Tyr Val Leu Asp Leu Gln Pro Glu Ala Thr Asp Leu Tyr Cys
210 215 220

Tyr Glu Gln Leu Pro Asp Ser Ser Met Arg Gly His Lys Pro Thr Leu
225 230 235 240

Lys Glu Tyr Ile Leu Asp Leu Tyr Pro Glu Pro Thr Asp Leu Tyr Cys
245 250 255

Tyr Glu Gln Leu Ser Asp Ser Met Arg Gly Asp Lys Ala Thr Ile Lys
260 265 270

Asp Tyr Ile Leu Asp Leu Gln Pro Glu Thr Thr Asp Leu His Cys Tyr
275 280 285

Glu Gln Leu Gly Asp Ser
290

<210> 96
<211> 1011
<212> DNA
<213> Artificial Sequence

<220>
<223> Polynucleotide for antigen E7 of HPV31, 33, 52 and 58 acidic-deleted

<220>
<221> CDS
<222> (1)..(1011)

<400> 96
gcg ggt caa gcg aag ccg gat acg agc aac tat aac att gtc acg ttc 48
Ala Gly Gln Ala Lys Pro Asp Thr Ser Asn Tyr Asn Ile Val Thr Phe
1 5 10 15

tgc tgc cag tgt gaa agc acc ttg cgt ctg tgt gtc cag agc acc caa 96
Cys Cys Gln Cys Glu Ser Thr Leu Arg Leu Cys Val Gln Ser Thr Gln
20 25 30

gtt gac atc cgt att ctg cag gag ttg ctg atg ggc agc ttt ggc atc 144

```
Val Asp Ile Arg Ile Leu Gln Glu Leu Leu Met Gly Ser Phe Gly Ile
        35                  40                  45

gtg tgc ccg aat tgc agc act cgc ctg ggt cag gca cag ccg gcc acc      192
Val Cys Pro Asn Cys Ser Thr Arg Leu Gly Gln Ala Gln Pro Ala Thr
    50                  55                  60

gct gac tac tac att gtt act tgt tgc cat acc tgc aat acc acg gtc      240
Ala Asp Tyr Tyr Ile Val Thr Cys Cys His Thr Cys Asn Thr Thr Val
65                  70                  75                  80

cgc ctg tgc gtc aat tcc acc gct tcc gac ttg cgt acc att cag caa      288
Arg Leu Cys Val Asn Ser Thr Ala Ser Asp Leu Arg Thr Ile Gln Gln
                85                  90                  95

ctg ctg atg ggc acg gtt aac atc gtt tgc ccg tct tgt gcg cag ctg      336
Leu Leu Met Gly Thr Val Asn Ile Val Cys Pro Ser Cys Ala Gln Leu
            100                 105                 110

ggc caa gcg gaa cag gcg acg agc aat tac tac atc gtg act tac tgt      384
Gly Gln Ala Glu Gln Ala Thr Ser Asn Tyr Tyr Ile Val Thr Tyr Cys
        115                 120                 125

cac tcg tgt gac agc acg ctg cgt ttg tgc att cac agc acc gcg acc      432
His Ser Cys Asp Ser Thr Leu Arg Leu Cys Ile His Ser Thr Ala Thr
    130                 135                 140

gat ctg cgt acg ctg caa caa ttg ctg atg ggt acc ctg cag gtg gtg      480
Asp Leu Arg Thr Leu Gln Gln Leu Leu Met Gly Thr Leu Gln Val Val
145                 150                 155                 160

tgc cct ggt tgc gca cgt ctg ggt caa gct caa ccg gca acc gcg aac      528
Cys Pro Gly Cys Ala Arg Leu Gly Gln Ala Gln Pro Ala Thr Ala Asn
                165                 170                 175

tac tat atc gtt acg tgt tgt tat acg tgt gat acc acc gtt cgt ctg      576
Tyr Tyr Ile Val Thr Cys Cys Tyr Thr Cys Asp Thr Thr Val Arg Leu
            180                 185                 190

tgc atc aac agc acc acc acc gat gtc cgc acg ctg caa caa ctg ctg      624
Cys Ile Asn Ser Thr Thr Thr Asp Val Arg Thr Leu Gln Gln Leu Leu
        195                 200                 205

atg ggt acg tgt acg att gtt tgc ccg agc tgt gcc cag caa atg cgt      672
Met Gly Thr Cys Thr Ile Val Cys Pro Ser Cys Ala Gln Gln Met Arg
    210                 215                 220

ggt gaa acc cca acg ctg cag gac tac gtg ctg gat ttg cag ccg gaa      720
Gly Glu Thr Pro Thr Leu Gln Asp Tyr Val Leu Asp Leu Gln Pro Glu
225                 230                 235                 240

gca acc gac ctg tac tgc tac gag cag ctg atg cgt ggt cac aag ccg      768
Ala Thr Asp Leu Tyr Cys Tyr Glu Gln Leu Met Arg Gly His Lys Pro
                245                 250                 255

acg ctg aaa gag tat atc ttg gac ctg tat ccg gag cct acc gat ctg      816
Thr Leu Lys Glu Tyr Ile Leu Asp Leu Tyr Pro Glu Pro Thr Asp Leu
            260                 265                 270

tat tgc tat gaa cag ctg agc atg cgt ggc gat aag gcg acc atc aaa      864
Tyr Cys Tyr Glu Gln Leu Ser Met Arg Gly Asp Lys Ala Thr Ile Lys
        275                 280                 285
```

```
gac tac att ctg gac ttg cag ccg gaa acc acg gat ctg cat tgc tac           912
Asp Tyr Ile Leu Asp Leu Gln Pro Glu Thr Thr Asp Leu His Cys Tyr
    290                 295                 300

gaa caa ctg ggc atg cgc ggt aac aat ccg acc ctg cgc gag tat atc           960
Glu Gln Leu Gly Met Arg Gly Asn Asn Pro Thr Leu Arg Glu Tyr Ile
305                 310                 315                 320

ctg gat ctg cac cca gag ccg act gac ctg ttc tgc tat gag cag ttg          1008
Leu Asp Leu His Pro Glu Pro Thr Asp Leu Phe Cys Tyr Glu Gln Leu
                325                 330                 335

tgc                                                                      1011
Cys
```

<210> 97
<211> 337
<212> PRT
<213> Artificial Sequence

<220>
<223> Synthetic Construct

<400> 97

```
Ala Gly Gln Ala Lys Pro Asp Thr Ser Asn Tyr Asn Ile Val Thr Phe
1               5                   10                  15

Cys Cys Gln Cys Glu Ser Thr Leu Arg Leu Cys Val Gln Ser Thr Gln
            20                  25                  30

Val Asp Ile Arg Ile Leu Gln Glu Leu Leu Met Gly Ser Phe Gly Ile
        35                  40                  45

Val Cys Pro Asn Cys Ser Thr Arg Leu Gly Gln Ala Gln Pro Ala Thr
    50                  55                  60

Ala Asp Tyr Tyr Ile Val Thr Cys Cys His Thr Cys Asn Thr Thr Val
65                  70                  75                  80

Arg Leu Cys Val Asn Ser Thr Ala Ser Asp Leu Arg Thr Ile Gln Gln
                85                  90                  95

Leu Leu Met Gly Thr Val Asn Ile Val Cys Pro Ser Cys Ala Gln Leu
            100                 105                 110

Gly Gln Ala Glu Gln Ala Thr Ser Asn Tyr Tyr Ile Val Thr Tyr Cys
        115                 120                 125

His Ser Cys Asp Ser Thr Leu Arg Leu Cys Ile His Ser Thr Ala Thr
    130                 135                 140
```

```
Asp Leu Arg Thr Leu Gln Gln Leu Leu Met Gly Thr Leu Gln Val Val
145                 150                 155                 160

Cys Pro Gly Cys Ala Arg Leu Gly Gln Ala Gln Pro Ala Thr Ala Asn
                165                 170                 175

Tyr Tyr Ile Val Thr Cys Cys Tyr Thr Cys Asp Thr Thr Val Arg Leu
            180                 185                 190

Cys Ile Asn Ser Thr Thr Thr Asp Val Arg Thr Leu Gln Gln Leu Leu
        195                 200                 205

Met Gly Thr Cys Thr Ile Val Cys Pro Ser Cys Ala Gln Gln Met Arg
    210                 215                 220

Gly Glu Thr Pro Thr Leu Gln Asp Tyr Val Leu Asp Leu Gln Pro Glu
225                 230                 235                 240

Ala Thr Asp Leu Tyr Cys Tyr Glu Gln Leu Met Arg Gly His Lys Pro
                245                 250                 255

Thr Leu Lys Glu Tyr Ile Leu Asp Leu Tyr Pro Glu Pro Thr Asp Leu
            260                 265                 270

Tyr Cys Tyr Glu Gln Leu Ser Met Arg Gly Asp Lys Ala Thr Ile Lys
        275                 280                 285

Asp Tyr Ile Leu Asp Leu Gln Pro Glu Thr Thr Asp Leu His Cys Tyr
    290                 295                 300

Glu Gln Leu Gly Met Arg Gly Asn Asn Pro Thr Leu Arg Glu Tyr Ile
305                 310                 315                 320

Leu Asp Leu His Pro Glu Pro Thr Asp Leu Phe Cys Tyr Glu Gln Leu
                325                 330                 335

Cys
```

<210>  98
<211>  1176
<212>  DNA
<213>  Artificial Sequence

<220>
<223>  Full polynucleotide for antigen E7 of HPV31, 33, 52 and 58

<220>
<221>  CDS

```
<222>  (1)..(1176)

<400>  98
gat gaa gag gat gtc atc gat tcc cca gca ggc caa gca aag ccg gac        48
Asp Glu Glu Asp Val Ile Asp Ser Pro Ala Gly Gln Ala Lys Pro Asp
1               5                   10                  15

acc agc aat tac aat atc gtg acc ttc tgc tgc caa tgt gag tcc act        96
Thr Ser Asn Tyr Asn Ile Val Thr Phe Cys Cys Gln Cys Glu Ser Thr
            20                  25                  30

ctg cgt ctg tgc gtt cag agc acc cag gtt gac att cgt att ctg caa       144
Leu Arg Leu Cys Val Gln Ser Thr Gln Val Asp Ile Arg Ile Leu Gln
        35                  40                  45

gaa ttg ttg atg ggt agc ttt ggc att gtg tgt ccg aac tgc agc acc       192
Glu Leu Leu Met Gly Ser Phe Gly Ile Val Cys Pro Asn Cys Ser Thr
    50                  55                  60

cgt ctg tcg gat gaa gat gaa ggt ctg gat cgt ccg gat ggt caa gcg       240
Arg Leu Ser Asp Glu Asp Glu Gly Leu Asp Arg Pro Asp Gly Gln Ala
65                  70                  75                  80

cag ccg gcg act gca gat tac tat atc gtc act tgt tgc cac acc tgc       288
Gln Pro Ala Thr Ala Asp Tyr Tyr Ile Val Thr Cys Cys His Thr Cys
                85                  90                  95

aat acc acc gtg cgt ctg tgt gtg aac agc acg gcg agc gac ctg cgc       336
Asn Thr Thr Val Arg Leu Cys Val Asn Ser Thr Ala Ser Asp Leu Arg
            100                 105                 110

acg atc caa cag ttg ttg atg ggc acc gtg aat atc gtc tgc cca agc       384
Thr Ile Gln Gln Leu Leu Met Gly Thr Val Asn Ile Val Cys Pro Ser
        115                 120                 125

tgt gct cag ttg agc gat gaa gag gac acc gac ggt gtc gac cgt ccg       432
Cys Ala Gln Leu Ser Asp Glu Glu Asp Thr Asp Gly Val Asp Arg Pro
    130                 135                 140

gac ggc caa gct gag cag gcg acc agc aac tac tat atc gtt acg tac       480
Asp Gly Gln Ala Glu Gln Ala Thr Ser Asn Tyr Tyr Ile Val Thr Tyr
145                 150                 155                 160

tgt cac agc tgc gat agc act ctg cgc ctg tgt att cat agc acg gcc       528
Cys His Ser Cys Asp Ser Thr Leu Arg Leu Cys Ile His Ser Thr Ala
                165                 170                 175

acc gat ctg cgt acc ctg caa caa ctg ctg atg ggt act ctg cag gtt       576
Thr Asp Leu Arg Thr Leu Gln Gln Leu Leu Met Gly Thr Leu Gln Val
            180                 185                 190

gtg tgc cca ggt tgt gct cgt ctg tcc gac gag gac gaa atc ggc ctg       624
Val Cys Pro Gly Cys Ala Arg Leu Ser Asp Glu Asp Glu Ile Gly Leu
        195                 200                 205

gac cgt cct gat ggc cag gcc cag ccg gca acg gcg aac tat tac att       672
Asp Arg Pro Asp Gly Gln Ala Gln Pro Ala Thr Ala Asn Tyr Tyr Ile
    210                 215                 220

gtg acc tgc tgc tat acg tgc gat acc acg gtc cgt ctg tgc att aac       720
Val Thr Cys Cys Tyr Thr Cys Asp Thr Thr Val Arg Leu Cys Ile Asn
225                 230                 235                 240
```

```
tct acc acc acg gat gtt cgc acg ctg cag caa ctg ctg atg ggt acc      768
Ser Thr Thr Thr Asp Val Arg Thr Leu Gln Gln Leu Leu Met Gly Thr
                245                 250                 255

tgt acc att gtt tgc ccg tct tgt gcc cag cag atg cgc ggt gaa acg      816
Cys Thr Ile Val Cys Pro Ser Cys Ala Gln Gln Met Arg Gly Glu Thr
            260                 265                 270

ccg acc ctg cag gat tat gtt ctg gac ctg caa ccg gag gcg acc gat      864
Pro Thr Leu Gln Asp Tyr Val Leu Asp Leu Gln Pro Glu Ala Thr Asp
        275                 280                 285

ctg tat tgc tac gag caa ctg ccg gac agc tcg atg cgt ggt cac aaa      912
Leu Tyr Cys Tyr Glu Gln Leu Pro Asp Ser Ser Met Arg Gly His Lys
    290                 295                 300

ccg acg ctg aaa gag tac atc ctg gac ctg tac ccg gaa cct acg gat      960
Pro Thr Leu Lys Glu Tyr Ile Leu Asp Leu Tyr Pro Glu Pro Thr Asp
305                 310                 315                 320

ctg tac tgt tat gaa caa ctg agc gat agc atg cgt ggt gac aag gcg     1008
Leu Tyr Cys Tyr Glu Gln Leu Ser Asp Ser Met Arg Gly Asp Lys Ala
                325                 330                 335

acg att aag gac tac atc ttg gat ctg cag ccg gaa acc acc gac ctg     1056
Thr Ile Lys Asp Tyr Ile Leu Asp Leu Gln Pro Glu Thr Thr Asp Leu
            340                 345                 350

cac tgc tat gag cag ctg ggt gat tct atg cgc ggt aac aat ccg acc     1104
His Cys Tyr Glu Gln Leu Gly Asp Ser Met Arg Gly Asn Asn Pro Thr
        355                 360                 365

ctg cgc gag tac att ctg gac ttg cat ccg gaa ccg acg gac ctg ttc     1152
Leu Arg Glu Tyr Ile Leu Asp Leu His Pro Glu Pro Thr Asp Leu Phe
    370                 375                 380

tgt tac gag cag ctg tgt gac agc                                     1176
Cys Tyr Glu Gln Leu Cys Asp Ser
385                 390


<210>  99
<211>  392
<212>  PRT
<213>  Artificial Sequence

<220>
<223>  Synthetic Construct

<400>  99

Asp Glu Glu Asp Val Ile Asp Ser Pro Ala Gly Gln Ala Lys Pro Asp
1               5                   10                  15


Thr Ser Asn Tyr Asn Ile Val Thr Phe Cys Cys Gln Cys Glu Ser Thr
            20                  25                  30


Leu Arg Leu Cys Val Gln Ser Thr Gln Val Asp Ile Arg Ile Leu Gln
        35                  40                  45
```

```
Glu Leu Leu Met Gly Ser Phe Gly Ile Val Cys Pro Asn Cys Ser Thr
    50                  55                  60

Arg Leu Ser Asp Glu Asp Glu Gly Leu Asp Arg Pro Asp Gly Gln Ala
65                  70                  75                  80

Gln Pro Ala Thr Ala Asp Tyr Tyr Ile Val Thr Cys Cys His Thr Cys
                85                  90                  95

Asn Thr Thr Val Arg Leu Cys Val Asn Ser Thr Ala Ser Asp Leu Arg
            100                 105                 110

Thr Ile Gln Gln Leu Leu Met Gly Thr Val Asn Ile Val Cys Pro Ser
        115                 120                 125

Cys Ala Gln Leu Ser Asp Glu Glu Asp Thr Asp Gly Val Asp Arg Pro
    130                 135                 140

Asp Gly Gln Ala Glu Gln Ala Thr Ser Asn Tyr Tyr Ile Val Thr Tyr
145                 150                 155                 160

Cys His Ser Cys Asp Ser Thr Leu Arg Leu Cys Ile His Ser Thr Ala
                165                 170                 175

Thr Asp Leu Arg Thr Leu Gln Gln Leu Leu Met Gly Thr Leu Gln Val
            180                 185                 190

Val Cys Pro Gly Cys Ala Arg Leu Ser Asp Glu Asp Glu Ile Gly Leu
        195                 200                 205

Asp Arg Pro Asp Gly Gln Ala Gln Pro Ala Thr Ala Asn Tyr Tyr Ile
    210                 215                 220

Val Thr Cys Cys Tyr Thr Cys Asp Thr Thr Val Arg Leu Cys Ile Asn
225                 230                 235                 240

Ser Thr Thr Thr Asp Val Arg Thr Leu Gln Gln Leu Leu Met Gly Thr
                245                 250                 255

Cys Thr Ile Val Cys Pro Ser Cys Ala Gln Gln Met Arg Gly Glu Thr
            260                 265                 270

Pro Thr Leu Gln Asp Tyr Val Leu Asp Leu Gln Pro Glu Ala Thr Asp
        275                 280                 285

Leu Tyr Cys Tyr Glu Gln Leu Pro Asp Ser Ser Met Arg Gly His Lys
    290                 295                 300
```

```
Pro Thr Leu Lys Glu Tyr Ile Leu Asp Leu Tyr Pro Glu Pro Thr Asp
305                 310                 315                 320

Leu Tyr Cys Tyr Glu Gln Leu Ser Asp Ser Met Arg Gly Asp Lys Ala
                325                 330                 335

Thr Ile Lys Asp Tyr Ile Leu Asp Leu Gln Pro Glu Thr Thr Asp Leu
            340                 345                 350

His Cys Tyr Glu Gln Leu Gly Asp Ser Met Arg Gly Asn Asn Pro Thr
        355                 360                 365

Leu Arg Glu Tyr Ile Leu Asp Leu His Pro Glu Pro Thr Asp Leu Phe
    370                 375                 380

Cys Tyr Glu Gln Leu Cys Asp Ser
385                 390
```

## Description

## FIELD OF THE INVENTION:

The invention relates to an immunotherapeutic vaccine for the treatment of infection by HPV16 or HPV18 or both HPV16 and HPV18 when the treated subject is selected from the group of human subjects infected with HPV16 or HPV18 or both (HPV16 and/or HPV18) and having no premalignant genital lesions related to said HPV(s), in particular no premalignant cervical lesions, and especially having no cancer related to said HPV infections, in particular no carcinomas related to said HPV infection.

The active ingredient of the therapeutic vaccine comprises the E7 protein of determined HPVs, in particular comprises the E7 protein of HPV16 and HPV18, especially as HPV16 E7-CyaA and HPV18 E7-CyaA fusion proteins and said active ingredient is adjuvanted by a topical adjuvant administered to the same dermal tissue. The active ingredient of the therapeutic vaccine may further comprise additional E7 proteins from different HPV alpha types.

The invention concerns accordingly the treatment of existing infection by HPV16 or HPV18 or both HPV16 and HPV18, for the prevention of the onset of premalignant ano-genital, in particular genital, in particular cervical lesions or for the control of the progression toward genital lesions, in particular cervical lesions, of moderate to high-grade, such as cervical intraepithelial neoplasia of grade 2 or 3. It also concerns the treatment of existing infection by HPV16 or HPV18 or both, for the prevention or the control of the progression toward HPV-related cancers, especially carcinomas. It may further concern the treatment of infections by other HPVs as will be described herein.

The invention also relates to the vaccine formulation comprising said active ingredient and adjuvant.

## BACKGROUND OF THE INVENTION:

The development of diagnostic tests for human papillomavirus (HPV) has allowed women at risk for cervical intraepithelial neoplasia (CIN) and cervical cancer to be identified before lesions appear.[1] However, HPV-infected women who are clinically asymptomatic and for example have normal cytology or mild abnormalities currently have no treatment options other than watchful waiting for clearance or, in the event that a high-grade lesion or cancer appears, surgery with possible adjunct chemotherapy.[2] Although surgical excision is often successful for high-grade lesions and cervical cancer, it can lead to infertility, pregnancy problems, incontinence, sexual dysfunction, and occasionally life-threatening haemorrhages.[3]

Although HPV infections detected in women by a positive HPV test, could be transient infections that clear spontaneously within 8 to 12 months [5], they could also be persistent infections that last for more than 12 months likely because they escaped the immune response leading to spontaneous clearance [9]. Also, this population includes women who have recurrent infections, in particular after a lesion previously treated by standard procedures [19, 20]. Subjects with such persistent infections or recurrent infections after treatment of their lesions are at higher risk of cervical neoplasia and cancer.

HPV16 and 18 infections are the most persistent HPV subtypes consequently leading to the highest risk of progression to high grade lesions and causing more than 70% of cervical cancers. With the availability of new HPV diagnostic tests used to genotype HPV16/18 in primary screening, more and more HPV16/18 infected women are identified before they have developed high grade lesions.

HPV16 and HPV18 types have been described as more likely to lead to high grade neoplasia and cervical cancer than other oncogenic HPV types. In fact, it was calculated that, gone undetected, women 30 years or older, infected with these two types and with normal cytology have an increased risk of developing high grade lesions and cancer versus non-infected women: estimated relative risk for a CIN3 or worse in HPV16 positive *versus* HPV negative women: 42,0; estimated relative risk for a CIN3 or worse in HPV18 positive *versus* HPV negative women: 20,5; estimated relative risk for a CIN3 or worse in HPV16 and HPV18 positive *versus* HPV negative women: 35,0 [38,39].

A therapeutic vaccine that would offer the possibility of treating HPV-infected subjects, before moderate or high-grade lesions appear and before local immunosuppression and viral escape mechanisms develop[4], would bring a clear benefit to current cervical cancer management by avoiding disease transmission, more densely spaced follow-up screenings, surgery of neoplasia and cancer treatments. To control existing infections and progression toward malignancies, a therapeutic vaccine must induce T cell-mediated immunity against HPV-infected cells. Most therapeutic vaccines in development target the HPV E7 protein and, in some cases, also the E6 protein. These onco-proteins are expressed at very low levels during the early phase of the viral cycle when no lesions have formed[40] and are often detectable only by indirect measurement methods but become directly detectable once lesions appear. It has been shown that the E6 and E7 onco-proteins are required for cellular transformation, and are present in all cervical cancers and precursor lesions.[5-7] Several candidate therapeutic HPV vaccines have shown promise in treating ano-genital high-grade intraepithelial neoplasia. However, these vaccines, intended for women who already have high-grade lesions and hence detectable levels of E7 expression, haven't reached efficacy levels comparable to current standard of care.

The inventors performed a systematic review of PubMed for articles in English describing the results of human clinical trials on HPV therapeutic vaccines for the treatment or prevention of high-grade cervical lesions or cervical cancer. Articles that met or might have met the selection criteria were selected for extraction of the following information: name of the candidate therapeutic vaccine; type of vaccine (fusion protein, DNA vaccines, etc.); disease target and stage; clinical efficacy, immunogenicity, and viral clearance. Review articles were inspected for possible additional references, which were then subjected to the initial selection process. Finally, for all candidate vaccines identified, PubMed was searched for any additional articles that met the selection criteria.

17 candidate HPV vaccines were identified for which the results of human clinical trials have been published (see Table 2). A variety of approaches have been tested, including a chimeric virus-like particle, a dendritic cell vaccine, fusion proteins, peptides (or mixtures of peptides), an attenuated virus, and recombinant vaccinia viruses. All have been tested in women who already have high-grade cervical lesions or cancer. In all but one case, the publications described phase 1 or 2 trial results. Most of the candidate vaccines have been shown to induce T-cell immunity and a few have shown some promising evidence of disease control or viral clearance, but in most cases, the studies were small and did not include control groups, precluding any statistical analysis.

## SUMARY OF THE INVENTION:

The invention provides for the first time the means suitable for use in immunotherapeutic vaccination of HPV-infected subjects before appearance of ano-genital neoplasia with clinical morbidity. Such subjects are currently not eligible for treatments disclosed in the art but rather fulfil distinct criteria especially distinct clinical profiles.

The invention accordingly provides compounds and protocols for the treatment for human subjects with existing infection by HPV16 or by HPV18 or by both HPV16 and HPV18 who can be clinically asymptomatic or bearing such said infection(s) associated with undetermined or mild epithelial abnormalities or borderline abnormalities.

Accordingly, the invention concerns an active ingredient which is a composition comprising a polypeptide consisting of the HPV16E7 and the HPV18E7 proteins, as defined herein (full-length E7 or portions thereof) wherein said E7 protein is recombined with catalytically inactive adenylate cyclase of *Bordetella pertussis* (CyaA) (designated E7-CyaA) for use in the immunotherapeutic treatment of existing infection by HPV16 or by HPV18 or by both in saidsubject, in particular female subjects when the infection is clinically asymptomatic or is associated with mild epithelial abnormalities or undetermined or borderline abnormalities. The invention also relates to a method for the immunotherapeutic treatment of said subjects with the herein defined active ingredient.

In particular, in said use or said method, the active ingredient is associated in an administration regime, with a topical adjuvant enhancing the E7 antigen-specific T-cell immune response when said response is induced by the active ingredient of the composition defined herein.

The composition of the invention, illustrated in particular with ProCervix in the Examples, is the first therapeutic vaccine against HPV16 and 18 infections before lesions occur in women at high risk of developing high grade cervical lesions or cancer.

In contrast with all other therapeutic HPV vaccines targeting onco-proteins E7 and/or E6, which are intended for subjects with cervical high-grade lesions or cervical cancer caused by HPV16 and/or HPV 18 infection and which usually have directly detectable levels of said onco-proteins present in said dysplasia,the composition defined herein (and ProCervix in particular) is suitable to treat women without any clinical morbitidy and without directly detectable or very weak expression of E7 onco-protein. As such it is an unconventional use because the purpose of the invention is treating before the occurrence of high-grade neoplasia or cancer rather than watchfully waiting for such lesions to develop and clinical morbidity occurs. It also contrasts with the art which assumes that an antigen-specific immune response of killer T lymphocytes requires that the targeted antigen (E7) should be present (for all practical matters detectable) on the targeted infected cells in order for said killer T lymphocytes to be able to induce cell death of the targeted infected cells, leading to viral clearance and hence to abortion of the transformation process.

The composition defined herein, in particular as illustrated with ProCervix, provides good safety and tolerance of the therapeutic HPV vaccine with a protocol compliant with otherwise healthy subjects. Treatment of infected women as proposed herein, leads to viral clearance in a majority of subjects, and substantially reduces transmission of the HPV virus to sexual partners, unlike therapeutics that are only used once high-grade lesions have already appeared, indicating that the viral genome has integrated into the human genome. At this later stage, the viral lifecycle has long been completed (including viral shedding required for transmission). The composition is thus formulated and used as an immunotherapeutic vaccine.

According to the invention, the eligible subjects, after having been diagnosed either for transient or persistent infection by HPV16 or by HPV18 or by both are eligible for the treatment if they have a clinically asymptomatic infection or an infection associated with mild epithelial abnormalities or undetermined or borderline abnormalities. In addition, subjects who are diagnosed with recurrent infection by HPV16 or HPV18 or both, following regression of lesions or clearance of a previous infection or following treated or cured previous infection by HPV16 or HPV18 or both, are also eligible for treatment..

According to the invention an eligible female subject may in particular have been diagnosed as having a normal cervical cytology (tested usually by PAP smears) or having low-grade lesion such as low-grade squamous intraepithelial lesion (LSIL) or Atypical Squamous Cells of Undetermined Significance (ASC-UC or ASCUS) or cervical intraepithelial neoplasia of type 1 (CIN1).

The proposed adjuvanted immunotherapeutic vaccine is provided for the treatment of the infection in order to achieve prevention of HPV16 and/or HPV18 induced epithelial diseases and malignancies, including induced ano-genital, in particular cervical lesions, especially of moderate to high grade lesions, or in order to achieve the prevention of HPV16- and/or HPV18-induced local immunosuppression or of the development of viral escape mechanisms.

In a particular embodiment, the invention also concerns the proposed adjuvanted immunotherapeutic vaccine for use in the treatment as defined herein to achieve viral clearance of HPV16 or HPV18 or both.

In a particular embodiment, the invention also concerns the proposed adjuvanted immunotherapeutic vaccine for use in the treatment as defined herein to achieve regression of undetermined or mild epithelial abnormalities or borderline abnormalities associated with HPV16 or HPV18 or both.

In a particular embodiment, the invention also concerns the proposed adjuvanted immunotherapeutic vaccine for use in the treatment as defined herein in order to achieve the prevention of HPV16- and/or HPV18-induced local immunosuppression or of the development of viral escape mechanisms.

In a particular embodiment of the treatment according to the invention, the administration regime associates the active ingredient and a topical adjuvant which is imiquimod, such as 5% imiquimod cream.

In a particular embodiment, the invention also concerns a composition containing the active ingredient for use as immunotherapeutic vaccine in the treatment defined herein with a topical adjuvant, wherein the administration regime requires that both components are administered separately to the same tissue, in particular to the dermis.

In a particular embodiment the invention concerns the active ingredient in the immunotherapeutic vaccine associated with an adjuvant when the active ingredient and adjuvant are administered via different routes and/or according to different time schedule and may be administered sequentially.

The invention also concerns the composition as active ingredient for use in the treatment defined herein wherein the composition is administered intradermally.

The invention also concerns the composition as active ingredient for use in the treatment defined herein wherein the composition and the topical adjuvant are administered at a site which is not the natural site of the targeted infection and in particular is not the site of the HPV-associated clinical pathology in particular the site of possible or detected lesion.

According to the invention, the administered dose of the composition is in the range of 100 to 1200$\mu$g.

The invention thus concerns the composition for the treatment as defined herein, in association with a topical adjuvant wherein the composition is administered as a single dose of the active ingredient or alternatively as more than one dose, in particular 2 or 3 doses to achieve the treatment.

When multiple doses are administered, the second and possibly further doses are administered at intervals(s) of a few weeks (2, 3, 4, 5, advantageously 6, or 7) to several months (2, 4, 6, 8, 10, 12 months).

In a particular embodiment, 2 doses are administered within an interval of 5 to 7, especially 6 weeks. In another embodiment said 2 doses are followed by administration of a third dose after at least 6 months of the second dose, advantageously after 6 months.

In another embodiment, composition may be administered as a single or as a multiple-dose in a prime-boost regime wherein in said prime-boost regime the priming administration and the boosting administration are homologous (same active ingredient or vaccine) and separated by more than 6 weeks and in particular more than 6 months.

The invention also relates to a combination of (i) the composition as active ingredient in accordance with the definition provided herein and (ii) a topical adjuvant of the T-cell immune response for use in the treatment for human subjects diagnosed for clinically asymptomatic existing infection by HPV16 or by HPV18 or by both HPV16 and HPV18 or with such infection associated with mild epithelial abnormalities or undetermined or borderline abnormalities.

In a particular embodiment of the invention, the composition as active ingredient of the immunotherapeutic vaccine comprises the E7-CyaA of HPV16 and the E7-CyaA of HPV18 that contain respectively E7 polypeptides each of them consisting of 2 fragments derived from (i) the sequence of SEQ ID No.61 wherein amino acid residues 30 to 42 of the full-length E7 have been deleted giving rise to a fragment having residues 1-29 and a fragment having resiudes 43-98 of said sequence and (ii) the sequence of SEQ ID N.62 wherein amino acid residues 32 to 42 of the full-length E7 have been deleted giving rise to a fragment having residues 1-29 and a fragment having residues 43-105 of said sequence, in particular in equal quantities. These E7-CyaA proteins derived from E7 of HPV16 and E7 of HPV18 are the active ingredient of ProCervix according to the designation used in the Examples.

In another embodiment of the invention, the composition as active ingredient of the immunotherapeutic vaccine consists of the E7-CyaA of HPV16 and the E7-CyaA of HPV18 that contain respectively E7 polypeptides each of them consisting of 2 fragments derived from (i) the sequences of SEQ ID No.61 wherein amino acid residues 30 to 42 of the full-length E7 have been deleted giving rise to a fragment having residues 1-29 and a fragment having resiudes 43-98 of said E7 and of SEQ ID No.62 wherein amino acid residues 32 to 42 of the full-length E7 have been deleted giving rise to a fragment having residues 1-29 and a fragment having residues 43-105 of said E7, in particular in equal quantities.

In a further embodiment said composition as active ingredient of the immunotherapeutic vaccine, comprises or consists in the E7-CyaA of HPV16 and the E7-CyaA of HPV18 that contain respectively E7 polypeptide sequences of HPV16 and HPV18 and have the sequence of SEQ ID No.67 and SEQ ID No.69.

The invention also relates to the composition for use in the treatment of a subject who is, in addition or alternatively, infected with at least one of the HPV selected among other HPV types classified as carcinogenic by IARC, such as among HPV31, HPV33, HPV35, HPV39, HPV45, HPV51, HPV52 HPV56, HPV58 and HPV59, wherein said composition further contains one or more different E7-CyaA protein(s) ofHPVs selected among HPV31, HPV33, HPV35, HPV39, HPV45, HPV51, HPV52, HPV56, HPV58and HPV59 . In such a case the definitions and embodiments disclosed herein in relation to HPV16 or HPV18 infection and treatment apply in a similar manner to the E7-CyaA proteins of HPV31, HPV33, HPV35, HPV39, HPV45, HPV51, HPV52, HPV56, HPV58 and HPV59 to the extent that they are present in the active ingredient of the immunotherapeutic vaccine.

The invention also concerns a topical adjuvant of the T-cell immune response suitable for administration in a human subject, in particular an adjuvant enhancing the E7 antigen-specific T-cell memory response, more particularly a TLR-7 ligand, more particularly imiquimod, for use for administration with a composition as defined herein, and to the same biological tissue especially the dermis, in the treatment of a human subject infected by human papillomavirus of type 16 (HPV16) or 18 (HPV18) or both HPV16 and HPV18, wherein the subject is a female who is positive for detection of HPV16 DNA or HPV18 DNA or both and presenting either with asymptomatic infection or with cervical inconclusive abnormalities (ASCUS) or with mild abnormalities such as low-grade squamous intraepithelial lesions (LSILs) or cervical intraepithelial neoplasia 1 (CIN1).

**BRIEF DESCRIPTION OF THE DRAWINGS**

**Figure 1. Study design**

Nineteen healthy women 18-45 years of age positive for HPV16 and/or HPV18 and with normal cervical cytology were enrolled in the open-label part and 28 were enrolled in the randomised, double-blind part. The open-label part included three cohorts (ProCervix 100 μg solution + imiquimod; ProCervix 600 μg solution + imiquimod; ProCervix 600 μg powder + imiquimod) in which subjects were injected with ProCervix at weeks 0 and 6 and applied imiquimod cream at the injection sites 15 min and 24 h after injections. In the double-blind part, subjects were randomised 2:1:1 to be injected at weeks 0 and 6 of the solution formulation of ProCervix 600 μg + imiquimod cream, the solution formulation of ProCervix 600 μg + placebo cream, or placebo injections + imiquimod cream. A safety review committee reviewed safety and tolerability data from the first two open-label cohorts before deciding whether to proceed with second vaccinations and with the subsequent cohort.

**Figure 2. Subject disposition**

Nineteen healthy women positive for HPV16 and/or HPV18 and with normal cervical cytology were enrolled in the open-label part and 28 were enrolled in the randomised, double-blind part. All subjects completed the study, although seven received only a single vaccination.

**Figure 3. Solicited injection-site reactions**

Solicited injection-site reactions were recorded by subjects for up to 7 days (solution formulation of ProCervix or placebo injection) or 10-14 days (powder formulation of ProCervix) after each injection. Proportions reporting the reactions are provided for the safety population. Abbreviations: PC 100, ProCervix 100 μg; PC 600, ProCervix 600 μg; Im, imiquimod; soln, solution.

**Figure 4. Solicited systemic reactions**

Solicited systemic reactions were recorded by subjects for up to 7 days (solution formulation of ProCervix or placebo injection) or 10-14 days (powder formulation of ProCervix) after each injection. Proportions reporting the reactions are provided for the safety population.

**Figure 5. Viral clearance**

Proportion of subjects with viral clearance over time in control groups (A) and groups receiving a ProCervix + imiquimod cream (B). Mean follow-up was 18·3 months (range, 10·3-29·3) after first vaccination for subjects treated with the solution formulation of ProCervix or placebo and 10·2 months (range, 5·7-12·3) for subjects treated with the powder formulation of ProCervix.

**Figure 6. T cell response**

The T cell response to overlapping 15-mer peptides covering HPV16 or HPV18 E7 was assessed by IFN-γ ELISPOT in PBMC collected at weeks 2, 6, 10, and 26. (A) Proportion of subjects with a T-cell response (i.e., responding to HPV16 E7 peptides, HPV18 E7 peptides, or both). (B) Proportion of subjects with a T-cell response to treatment with the solution or powder formulation of ProCervix 600 μg + imiquimod and responding to HPV16 E7 peptides, HPV18 E7 peptides, or both according to viral clearance status. "Cleared at any time" includes subjects who had recurrence after initial clearance, subjects with sustained clearance, and subjects who cleared HPV but for whom no follow-up results were available. Abbreviations: PC 100, ProCervix 100 μg; PC 600, ProCervix 600 μg; Im, imiquimod. Values are for the safety population.

## DETAILED DESCRIPTION

The invention is directed to a composition comprising the HPV16E7 and the HPV18E7 proteins, wherein said E7 protein is recombined with catalytically inactive adenylate cyclase of *Bordetella pertussis* (CyaA) (designated E7-CyaA) for use as active ingredient of an immunotherapeutic vaccine in the treatment of a human subject infected by human papillomavirus of type 16 (HPV16) or 18 (HPV18) or both HPV16 and HPV18, wherein the subject is a female positive for HPV16 DNA or HPV18 DNA or both and presenting either with clinically asymptomatic infection or with Atypical Squamous Cells of Undetermined Significance (ASCUS) or with mild abnormalities such as low-grade squamous intraepithelial lesions (LSILs) or cervical intraepithelial neoplasia 1 (CIN1).

The invention similarly relates to a method of treatment of a human subject infected by human papillomavirus of type 16 (HPV16) or 18 (HPV18) or both HPV16 and HPV18, wherein the subject is a female positive for HPV16 DNA or HPV18 DNA or both and presenting either with asymptomatic infection or with Atypical Squamous Cells of Undetermined Significance (ASCUS) or with mild abnormalities such as low-grade squamous intraepithelial lesions (LSILs) or cervical intraepithelial neoplasia 1 (CIN1) said method comprising the step of administering to the subject an immunotherapeutic vaccine which comprises, as active ingredient, a composition comprising HPV16E7 and the HPV18E7 proteins, said E7 proteins is recombined to catalytically inactive adenylate cyclase of *Bordetella pertussis* (CyaA) (designated E7-CyaA).

Accordingly the embodiments disclosed hereafter with respect to the use of the composition similarly apply to the above defined method of treating a subject.

The invention also relates to a composition comprising HPV16E7 and the HPV18E7 proteins, each of said E7 proteins is recombined to catalytically inactive adenylate cyclase of *Bordetella pertussis* (CyaA) (designated E7-CyaA) for use as active ingredient in the preparation of an immunotherapeutic vaccine for the treatment of a human subject infected by human papillomavirus of type 16 (HPV16) or 18 (HPV18) or both HPV16 and HPV18, wherein the subject is a female positive for HPV16 DNA or HPV18 DNA or both and presenting either with asymptomatic infection or with Atypical Squamous Cells of Undetermined Significance (ASCUS) or with mild abnormalities such as low-grade squamous intraepithelial lesions (LSILs) or cervical intraepithelial neoplasia 1 (CIN1).

By the term *"female"* it is intended according to the invention either girls from 9 to 15 of age or adult females over 15 of age.

A *"clinically asymptomatic infection by HPV16 or HPV18 or both"* according to the invention qualifies an infection for which the subject could be detected positive for HPV16 or HPV18 or both (this status corresponding either to seropositivity, i.e., presence of antibodies against HPV16 or HPV18 antigens such as Early (E) or Late (L) antigens in a biological sample or to the presence of HPV16 or HPV18 DNA allowing detection and genotyping of at least one HPV16 or HPV18 genome copy /10,000 cells of a biological sample), but the subject would not be diagnosed with clinical symptom associated with high-risk HPV infection. In particular, the patient is considered healthy from a clinical point of view or presenting no clinical morbidity. In a particular embodiment, the subject has been diagnosed as having a normal cervical cytologic examination and possibly a normal colposcopic and histological examination. In a particular embodiment, the infection is productive (i.e., gives rise to the production of viral particles in the host which may be accompanied with induction of anti-L antibodies). In such clinically asymptomatic HPV infection viral infectious particles may be produced but the infection may regress spontaneously within a time line of up to about 24 months. According to the invention, a particular subpopulation of subjects having *"clinically asymptomatic infection by HPY16 or HPY18 or both"* presents with no detectable antibodies against targeted E7 antigen.

*"LSIL"* (low-grade squamous intraepithelial lesions), "*ASCUS*' (Atypical Squamous Cells of Undetermined Significance) also designated as cervical lesions of undetermined significance or borderline abnormalities and *"CIN1"* (cervical intraepithelial neoplasia 1) lesions designate lesions which are lesions of undetermined significance or borderline or low-grade lesions that are not considered as premalignant and are associated with a clinical status defined as healthy or having no clinical morbidity. They distinguish from moderate and high-grade lesions such as CIN2 or CIN3. These lesions or abnormalities are early stage developments of HPV infection that may more often spontaneously regress as compared to premalignant or malignant lesions.

The term *"treatment"* within the context of the invention relates to the therapeutic effect observed following administration of the herein disclosed composition, in particular the adjuvanted composition. Accordingly, treatment may give rise to sustained viral clearance in the subject followed on a period of time of at least 18 months, or may encompass prevention of the onset or development of ano-genital, in particular of genital lesions, in particular cervical lesions, or control of the development of the lesions observed at the time the treatment is initiated in such a way that premalignant or malignant lesions or disease do not appear over a period of time of 18 months or more. In other words, the treatment enables the control of the risk that the subject develops premalignant or malignant lesions (moderate to high grade lesions, in particular cervical lesions) or cancer, associated with infections by HPV16 or HPV18 and, optionally, depending on the active ingredient of the composition by further HPV types as disclosed herein.

According to a particular embodiment of the invention the composition which is the active ingredient of an immunotherapeutic vaccine is used in the prevention of HPV16 and/or HPV18 induced epithelial diseases and malignancies, including induced ano-genital, in particular genital, especially cervical lesions, especially of moderate to high grade lesions, or in the prevention of HPV16 and/or HPV18 induced local immunosuppression or viral escape mechanism development.

The invention concerns in particular the composition for use as active ingredient of an immunotherapeutic vaccine as defined herein, in the prevention of the onset of HPV16 or HPV-18-related ano-genital, especially cervical lesions or in the control of the development of ASCUS or LSILs abnormalities or CIN1 lesion, in particular to prevent progression of infection toward premalignant genital lesion of grade 2 or 3, in particular toward CIN2 or CIN3.

In a particular embodiment, the treatment carried out with immunotherapeutic vaccine containing the composition as defined herein is suitable and effective for viral clearance of HPV16 or HPV18 or HPV16 and HPV18.

The invention relates in particular to such a composition for use as active ingredient of an immunotherapeutic vaccine in the treatment of infection due to HPV16 or HPV18 or both, or to a method of treatment with said composition, wherein the subject is:

- either a female who has been previously diagnosed with transient or with persistent infection by HPV16 or HPV18 or both or,
- a female who has been previously diagnosed with recurrent infection by HPV16 or HPV18 or both following regression or clearance of previous infection or following treated or cured previous infection by HPV16 or HPV18 or both.

Females with *"persistent HPV16/HPV18 infection"* are subjects who have been tested positive for an infection by HPV16 or HPV18 or both at least two times consecutively at twelve months interval or more persistent infection can last for about 18 months or more and in particular for 18 months to many years. Subjects having persistent infection may not show lesions caused by HPV or may show LSIL, ASCUS or other undetermined or low-grade lesions caused by HPV such as cervical intraepithelial neoplasia of grade 1 (CIN1). In such a case, the HPV persists in the basal and para-basal cell layers of the epithelium at some sites of infection driving cell proliferation in a way that could progress to high-grade lesions or to cancer, if not treated.

Females with *"recurrent* HPV16/HPV18 *infection"* are subjects who have been infected by HPV16 or HPV18 or both in the past and have been treated (by surgery or other available treatments) for this infection or subjects who have undergone spontaneous HPV clearance for this past infection and are again diagnosed positive for HPV16 and/or HPV18.

In a more particular embodiment of the composition for use as active ingredient of an immunotherapeutic vaccine in the treatment of infection due to HPV16 or HPV18 or both according to the invention, the subject has been diagnosed as having a normal cervical cytology (tested usually by PAP smears) or having an undetermined lesion or a low-grade lesion such as low-grade squamous intraepithelial lesion (LSIL) or ASC-UC (or ASCUS) or cervical intraepithelial neoplasia of grade 1 (CIN1) when tested by histology analysis of the lesion.

Surprisingly, an increased frequency of HPV clearance was found in subjects with normal cytology vaccinated with ProCervix versus with placebo despite the extremely low level of HPV E7 antigen known to be expressed in infected cells at this early stage of the disease [33]. Taken this low level of expression, it was not predictable that ProCervix could mount an effective cellular immune response because it was considered unlikely that the E7 antigen would be sufficiently clustered on the surface of the HPV infected cell to allow effective binding of T cell receptor complexes of E7 antigen-specific cytotoxic T cells, which can induce death of the detected infected cell.

In addition, ProCervix also induced clearance in women who have long lasting pre-existing infections before vaccination (up to 4,3 years) and in women who had a high grade lesion treated in the past, but had a recurrent infection thereafter (4/4 cases). This is unexpected as these women, had already failed to induce an effective immune response in response to the HPV infection. It is surprizing that vaccination with an antigen to which they had already been exposed was able to overcome immune escape and allow eradicating HPV infected cells.

These results are even more remarkable as the treatment was performed in the upper thigh of the subjects and not locally, at the site of natural infection (cervix uteri).

In view of the surprising results obtained by the inventors, it is pointed out that the composition of the invention may be administered to female subjects at risk of developing a premalignant or a malignant lesion, in particular ano-genital lesion and more particularly cervical lesion, in relation to infection by HPV16 or/and HPV18.

According to a preferred embodiment of the invention, the composition for use as active ingredient of an immunotherapeutic vaccine is used in combination with a topical adjuvant.

The expression *"in combination"* relating to the use of the composition and the adjuvant means that they are both necessary for the full completion of the administration protocol but may be or should be administered separate in time, in particular at the same site of administration.

In a preferred embodiment, the composition for use as active ingredient of an immunotherapeutic vaccine in the treatment of infection due to HPV16 or HPV18 or both, is used in an administration regime that includes the administration of a topical adjuvant provided at or near the site of administration of the composition, wherein said adjuvant is an adjuvant enhancing the E7 antigen-specific T-cell immune response, in particular of the T-cell memory response against the antigen, which is the active ingredient in the therapeutic vaccine and is suitable for administration in a human subject.

The expression *"at or near the site"* relates to the application of the product on the skin of the subject in a zone sufficiently close to the administration point of the active ingredient to enable it to exert its topical adjuvant effect on the immunological response elicited by the active ingredient, to the dermis. In a preferred embodiment, the adjuvant is applied at the injection point of the active ingredient.

According to the invention, the administration of the active ingredient and of the adjuvant is not performed at the site of the body which is naturally targeted by the virus or where the infection by HPV is active and in particular may develop abnormalities or malignancies. Accordingly, the site of the administration of the active ingredient and of the adjuvant is not the genital zone and in particular is not the cervical zone. Rather the administration of the active ingredient and of the adjuvant is performed at a remote site with respect to this zone and for example in the thigh, more particularly in the upper thigh. When multiple-dose administration is performed, the administration sites may be the same for all administration steps or may be different and especially may encompass both thighs of the subject. The adjuvant which is selected for use with the composition in the treatment of the infection by HPV16 or HPV18 or both should increase, favor or stimulate the T-cell immune response against the virus, in particular the T-cell memory response. It may be a TLR ligand, such as a TLR ligand of class 7/8 or a LTR ligand of class 3 (for example Poly-ICLC) or of class 9. In a preferred embodiment, the adjuvant of the T-cell immune response is imiquimod. In another embodiment the adjuvant is from the imidazoquinolines family.

In a particular embodiment of the invention, the adjuvant is imiquimod, in particular imiquimod as a cream, more particularly as 5% imiquimod cream such as the product sold as Aldara™ or 3.75% imiquimod or 2.5% imiquimod (Zyclara™) or generics thereof.

In accordance with a particular embodiment of the invention, the composition for use as active ingredient of an immunotherapeutic vaccine as defined herein is used in the treatment of infection due to HPV16 or HPV18 or both in an administration regime requiring that the composition and the adjuvant are administered to the same biological tissue and in particular sequentially.

The invention also relates, in a particular embodiment, to a composition for use as active ingredient of an immunotherapeutic vaccine in the treatment of infections due to HPV16 or HPV18 or both wherein the administration regime encompasses multiple-dose administration of the topical adjuvant for each dose of composition, in particular separate especially sequential administration in time of 2 or more doses of adjuvant such as imiquimod cream or another suitable adjuvant, especially 5% imiquimod cream, for each dose of composition.

The composition for use as active ingredient of an immunotherapeutic vaccine may be administered intradermally, in particular by intradermal injection, more particularly by intradermal injection in the subject's thigh.

The invention is also directed to composition for use as active ingredient of an immunotherapeutic vaccine as defined herein in the treatment of infection due to HPV16 or HPV18 or both, wherein the dose of the composition which is administered to the subject is in the range of 100μg to 1200μg.

The dose of the composition may be administered as a mono-dose or as multiple doses. In case multiple doses are administered, they may be administered at distinct sites on the subject, either separately or concomitantly in time. When multiple doses are administered they may especially be administered on different days or on different weeks, in particular according to an administration schedule of 1 to 10 weeks, 1 to 7, in particular 1 to 6 or 1 to 5 weeks. In a particular administration regime, multiple doses correspond to 2 doses administered at a 6-week interval.

A single dose of the composition in particular contains 100 μg to 600μg or 600 μg to 1200μg of active ingredient,

especially 600μg. When multiple doses are administered, each of them may contain active ingredient within the above disclosed ranges. In a particular embodiment, multiple doses may contain an equal amount of active ingredient, in particular 600μg for each dose.

In a particular embodiment of the invention, the composition is administered as a single dose of the 600μg active ingredient or alternatively as more than one dose, in particular 2 or 3 doses, in particular within 1 to 6 (±1)weeks.

Alternatively the composition may be administered on a single day and multiple doses, preferentially doses of the 600μg active ingredient, are administered sequentially at different locations, preferentially on the left and right upper thigh.

In a particular embodiment of the invention, the use of the composition as defined herein encompasses the following administration regimen:

- (i) intradermally administering the composition at a site remote from the ano-genital, especially genital area, in particular in one or in both thighs of the subject, wherein administration requires a single dose or a multiple-dose schedule provided the total quantity of the composition administered to the subject is within a range of 100 to 1200μg;

- (ii) separately in time, in particular 10 to 30 minutes after the administration of each dose of the composition, more particularly 15 minutes later, administering a topical adjuvant as defined herein at the site of the intradermal administration of the composition and repeating said administration of topical adjuvant 20 to 36 hours apart, in particular 24 hours later;

- (iii) optionally repeating both primary administration steps (i) and (ii) after a period of 1 to 6 weeks, preferentially 6 weeks plus or minus one week of said primary administration steps of the composition and respectively adjuvant;

- (iv) optionally and either alternatively or cumulatively to (iii), repeating administration steps (i) and (ii) within 6 to 12 months of the primary administration step(s).

As defined above, the *"active ingredient"* of the immunotherapeutic vaccine of the invention, is a composition (i.e. a mixture of compounds) comprising or containing HPV16E7 and the HPV18E7 proteins, possibly as recombined fragments of the native proteins, said E7 proteins being further recombined to catalytically inactive adenylate cyclase of *Bordetella pertussis* (CyaA) and accordingly designated E7-CyaA.

According to the invention the *"E7 protein"* qualifies a polypeptide which is the HPV full-length E7 protein or an antigenic portion thereof, either provided as a continuous sequence of amino acid residues of said full-length or antigenic portion thereof or as fragments inserted in various sites of the CyaA, wherein said fragments, if rejoined in the proper order, would constitute the full-length protein or the said antigenic portion thereof When said E7 protein (including when used as fragments) is a portion of the native (full-length) E7 protein it consists of a sequence having at least 85% or at least 90% of the sequence of the full-length protein, preferably at least 95% of it or more than 98% of it. An *"E7 protein"* may also encompass a variant of the full-length protein as a result of point mutations such as substitutions of less than 5% or less than 3% of the amino acid residues of the full-length protein. Examples of E7 protein according to this definition are provided herein and in the Examples as determined fragments of the native E7 or of a portion thereof, especially as used in ProCervix.

The E7 protein and the E7-CyaA proteins may be obtained by any available technique, including by cloning and expression in a host cell, especially in *E.coli,* in particular, the proteins are the product of the expression of nucleic acid sequences codon-optimized for expression in *E.coli.*

According to a particular embodiment of the composition, the E7 protein is the HPV16 E7 wherein amino acid residues 30-42 of the full-length protein have been deleted. According to another particular embodiment of the composition defined in the invention, the E7 protein is the HPV18 E7 protein wherein residues 32-42 of the full-length protein have been deleted. These particular embodiments are advantageously combined in the ProCervix active ingredient to obtain HPV16 E7-CyaA and HPV18 E7-CyaA.

The composition for use according to the invention may either be restricted to E7-CyaA proteins of HPV16 and HPV18 or may comprise further E7 proteins or further E7-CyaA proteins originating from distinct HPV types. According to an embodiment of the invention, where additional E7 protein(s) is(are) provided for the preparation of the active ingredient said E7 protein(s) is(are) derived from a virus type other than HPV16 or HPV18, selected among the carcinogenic HPV and in particular the mucosal alpha 7 and alpha 9 types of HPVs.

In a particular embodiment, the HPV providing one or several additional E7 protein(s) for use according to the invention are alpha7 HPV and are especially HPV39, HPV45, HPV59 and/or the additional HPV providing an E7 protein for use according to the invention is an alpha9 HPV selected among HPV31, HPV33, HPV35, HPV52 and HPV58.

In a particular embodiment of the invention, the active ingredient of the therapeutic vaccine is a composition comprising fusion proteins of the E7 protein (possibly recombinant E7) with *Bordetella pertussis* CyaA, wherein the E7 are the proteins of the HPV16 and of the HPV18 and of the HPV31, HPV33, HPV52, HPV58 and HPV45.

In such a case, the composition may comprise as many types of E7-CyaA fusion proteins as the number of HPVs represented in the composition.

Alternatively the E7 proteins may be recombined and fused with a single CyaA protein or with multiple CyaA proteins each of them comprising a determined selection of various E7 proteins of selected HPVs. Such chimeric protein(s) may in particular encompass the E7 protein of the HPV16, HPV18, and of any of the HPV31, HPV33, HPV52, HPV58 and HPV45. In an aspect of the invention, the thus obtained chimeric protein(s) may comprise the E7 protein of 3, 4, 5, 6 or 7 HPV types, with the proviso that they comprise the E7 protein of the HPV16 and HPV18. The E7 protein may be present in the chimeric protein as successive C-terminal fragments of the selected HPVs, recombined with the corresponding N-terminal fragments of the selected E7 proteins. Examples thereof have been disclosed in particular in WO 2014/016312 which is incorporated herein by reference for the description of the E7 amino acid sequences and their fusion, including their combination with the CyaA sequence and for their encoding polynucleotides and method of preparation.

In a particular and preferred embodiment, the active ingredient, designated ProCervix, is a composition containing the recombinant HPV16E7 and the recombinant HPV18E7 proteins, each fused to a catalytically inactive CyaA of *Bordetella pertussis.* Advantageously, E7-CyaA of HPV16 and HPV18 are both present in the composition. Said fusion proteins suitable for the preparation of a bivalent immunotherapeutic vaccine have been described in particular in patent application WO 2005/089792 which is incorporated herein by reference. The thus defined active ingredient delivers the E7 antigens to $CD11b^+$ dendritic cells, where they are processed and presented as $CD4^+$ and $CD8^+$ T-cell epitopes to T lymphocytes.

According to the invention the E7 proteins are constituted of fragments (in particular 2 fragments which together would give rise to the E7 protein as defined herein consisting of an antigen portion of full-length E7) which are inserted in determined distinct sites of the CyaA protein.

The amino acid sequence and the nucleotide sequence of HPV16 E7 is disclosed as SEQ ID No.61. The amino acid sequence and the nucleotide sequence of HPV18 E7 is disclosed as SEQ ID No.62.

The amino acid sequence of the CyaA protein is disclosed herein as SEQ ID No.64 and the polynucleotide encoding the same is described as SEQ ID No.63 or as SEQ ID No.65 for an illustrative but not limitative embodiment of the sequence optimized for expression in *E.coli.*

In a particular embodiment where one CyaA contains only one type of E7, the E7 protein (or its fragment) is inserted between amino acids 224-235 and/or between amino acids 319-320 of CyaA; both insertion sites are used in particular when 2 fragments of the E7 are recombined with the CyaA protein. Such particular constructs are those illustrated in the Examples. In a particular embodiment of the invention, the active ingredient of the therapeutic vaccine is the composition described in the Examples provided herein and containing the HPV16 E7-CyaA fusion protein including amino acids 1-29 of HPV16 E7 inserted between amino acids 319-320- of CyaA and amino acids 43-98 of HPV16 E7 inserted between amino acids 224-235 of CyaA in admixture with the HPV18 E7-CyaA fusion protein including amino acid 1-31 of HPV18 E7 inserted between amino acids 319-320 of CyaA and amino acids 43-105 of HPV18 E7 inserted between amino acids 224-235 of CyaA.

In a particular embodiment, equal amounts of said HPV16 E7-CyaA fusion protein and of HPV18 E7-CyaA fusion protein are contained in the therapeutic vaccine of the invention.

E7 and CyaA proteins are disclosed in the art and the preparation of a fusion starting from these proteins is well known from the skilled person. Examples of the preparation of E7-CyaA fusion proteins are disclosed in particular in WO 2005/089792 and in WO2014/016312.

In a particular composition for use according to the invention as active ingredient of an immunotherapeutic vaccine, the E7-CyaA proteins comprise or consist of the E7-CyaA of HPV16 and the E7-CyaA of HPV18 that contain respectively the E7 proteins of SEQ ID No.61 wherein amino acid residues 30 to 42 have been deleted and of SEQ ID No.62 wherein amino acid residues 32 to 42 have been deleted.,

In a further embodiment the composition as active ingredient of an immunotherapeutic vaccine, comprises or consists in the E7-CyaA of HPV16 and the E7-CyaA of HPV18 of sequence SEQ ID NO.67 and SEQ ID NO.69.

Nucleotide sequences coding for E7-CyaA of HPV16 and E7-CyaA of HPV18 are disclosed as SEQ ID No. 66 and 68, respectively.

In a particular embodiment a dose of the composition contains, as active ingredient, 300$\mu$g of E7-CyaA of HPV16 and 300$\mu$g of E7-CyaA of HPV18.

In another embodiment of the invention, the composition for use according to the invention, may be administered to a subject who is further infected with at least one of the HPV selected among at least one of HPV31, HPV33, HPV35, HPV39, HPV45, HPV52, HPV58 or HPV59 or among HPV31, HPV33, HPV52, HPV58 and HPV45, said composition being accordingly for use for the treatment of the subject against one or more of the HPV selected from HPV16, HPV18 or HPV31, HPV33, (HPV35, HPV39, HPV45), HPV52, HPV58 (or HPV59) said composition further containing one or more different E7-CyaA protein(s) of HPVs selected among HPV31, HPV33, HPV35, HPV39, HPV45, HPV52, HPV58 or HPV59 or HPV31, HPV33, HPV52, HPV58 and HPV45 respectively.

The definitions which have been provided herein in relation to HPV16 and HPV18 may obviously be adapted to the other HPV disclosed herein when the latter are relevant to the defined composition.

In such embodiment where the CyaA contains many E7 of different HPV types, the CyaA may be as defined above or may be modified and especially may have undergone deletions in its amino acid sequence. Particular examples suitable for the achievement of the invention that involves CyaA deleted in part are illustrated as SEQ ID No. 71 or as SEQ ID No.73 respectively encoded by SEQ ID No.70 and SEQ ID No.72.

In an embodiment of the invention, the E7 antigens used for the preparation of the E7-CyaA are those having the following amino acid sequences: for HPV16 type (SEQ ID No.61) or fragments thereof resulting from deletion of residues 30 to 42, for HPV18 type (SEQ ID No.62) or fragments thereof resulting from deletion of residues 32 to 42, for HPV31 type (SEQ ID No74), for HPV33 type (SEQ ID No.75), for HPV45 type (SEQ ID No.76), for HPV52 type (SEQ ID No.77) or for HPV58 type (SEQ ID No.79). To remove a naturally human autoimmune epitope identified by the inventors in the E7 protein of the HPV52 type (as set forth in SEQ ID No.77), the amino acid residue in positions 84 and 86 have been substituted (M->L at position 84 and L->M at position 86); this modified sequence of the E7 protein of the HPV52 type is as set forth in SEQ ID No.78.

From the amino acid sequences of these various E7 proteins, recombinant proteins may be obtained which associate the E7 C-terminal fragments of distinct HPV types followed by E7 N-terminal fragments of the same HPV types, wherein the C-terminal/N-terminal pairs are associated in chimeric sequences such as the following:

- for a combination where HPV types are HPV16, HVP18 and HPV45 types, the heterologous polypeptide consists either of the sequence as set forth in SEQ ID No.81, or the sequence as set forth in SEQ ID No.83;
- for a combination where HPV types are HPV16, HVP18, HPV33 and HPV45 types, the heterologous polypeptide consists either of the sequence as set forth in SEQ ID No.85. or the sequence as set forth in SEQ ID No.87;
- for a combination where the above three or four HPV types are further associated with E7 proteins or their fragments as defined herein of additional HPV types, HPV types are HPV31, HVP52 and HPV58 types, and the polypeptide consists either of the sequence as set forth in SEQ ID No.89 or the sequence as set forth in SEQ ID No.91 ; or HPV types are HPV31, HVP33 and HPV52 types, and the polypeptide consists either of the sequence as set forth in SEQ ID No.93 or the sequence as set forth in SEQ ID No.95 or HPV types are HPV31, HPV33, HPV52 and HPV58 types, and the polypeptide consists either of the sequence as set forth in SEQ ID No.97 or the sequence as set forth in SEQ ID No.99.

The invention also concerns a combination of a composition of active ingredient and of a topical adjuvant as defined herein and optionally excipient(s), for use as separately and sequentially administered components in the treatment of infection or disease causally related to HPV16 or HPV18 or both, in a human subject infected by human papillomavirus of type 16 (HPV16) or 18 (HPV18) or both, wherein the subject is selected among females positive for HPV16 DNA or HPV18 DNA or both and presenting either with clinically asymptomatic infection or with cervical undetermined abnormalities (ASCUS) or with mild abnormalities such as low-grade squamous intraepithelial lesions (LSILs) or cervical intraepithelial neoplasia 1 (CIN1).

A particular combination is characterized by the fact that the composition of active ingredient is formulated as a liquid, in particular a solution or a suspension, or formulated as a lyophilized form, in particular a powder and the topical adjuvant is provided in a separate formulation such as a cream.

The invention thus also relates to a kit comprising the composition as active ingredient and the topical adjuvant as defined in the present application wherein said composition is provided as a mono-dose or a multi-dose in a vial or in a syringe and the topical adjuvant is provided in a separate vessel or sachet, said kit further comprising the therapeutic indication that the combination is for use as an immunotherapeutic vaccine in the treatment of a human subject infected by human papillomavirus of type 16 (HPV16) or 18 (HPV18) or both HPV16 and HPV18, wherein the subject is selected among females positive for HPV16 DNA or HPV18 DNA or both and presenting either with asymptomatic infection or with cervical inconclusive abnormalities (ASCUS) or with mild abnormalities such as low-grade squamous intraepithelial lesions (LSILs) or cervical intraepithelial neoplasia 1 (CIN1).

The combination or the kit according thus defined may be further characterized by the fact that the adjuvant is an adjuvant enhancing the E7 antigen-specific T-cell immune response, in particular an adjuvant enhancing the E7 antigen-specific T-cell memory response, such as those defined herein, preferably imiquimod, especially imiquimod 5%.

In a particular embodiment, the combination or the kit thus encompassed by the invention may be (further) characterized by the fact that the composition as active ingredient contains respectively the E7 proteins of SEQ ID No.61 or one or multiple fragment(s) thereof resulting from deletion of residues 30 to 42 and of SEQ ID No.62 or one or multiple fragment(s) thereof resulting from deletion of residues 32 to 42. In particular embodiment the E7-CyaA of HPV16 and the E7-CyaA of HPV18 are respectively the recombinant proteins having SEQ ID No.67 and SEQ ID No.69 in dose range of 100$\mu$g to 1200$\mu$g, in particular 600$\mu$g in total. In a particular embodiment, in said dose each type of E7-CyaA is in equal quantity as the other type(s).

In another particular embodiment, the combination or the kit comprises additional E7-CyaA fusion proteins of different HPVs selected among HPV31, HPV33, HPV35, HPV39, HPV45, HPV52, HPV58 or HPV59, in particular among HPV31, HPV33, HPV52, HPV58 and HPV45, said obtained composition being in dose range of 100μg to 1200μg. The E7 proteins of these HPV have been described in the art and specific examples of these proteins are illustrated in the present application,

The invention also relates to a topical adjuvant of the T-cell immune response suitable for administration in a human subject, in particular of the T-cell memory response, more particularly imiquimod cream, for use for separate, and optionally sequential, administration with a composition as defined herein, in the treatment of a human subject infected by human papillomavirus of type 16 (HPV16) or 18 (HPV18) or both, wherein the subject is a female positive for HPV16 DNA or HPV18 DNA or both and presenting either with asymptomatic infection or with cervical undetermined abnormalities (ASCUS) or with mild abnormalities such as low-grade squamous intraepithelial lesions (LSILs) or cervical intraepithelial neoplasia 1 (CIN1).

All embodiments relating to the composition or to the adjuvant, which are defined herein apply similarly to the use of the adjuvant in combination with the composition.

The preparation of the active ingredient consisting of E7-CyaA is performed according to well-known techniques available in the art especially having recourse to gene synthesis and expression in a host cell as reported in the art (in particular in WO 2005/089792 and WO 2014/016312) using the sequences of cyaA and cyaC genes cloned in a plasmid. The expression of the recombinant E7-CyaA proteins is especially achieved in *E.coli*. In a particular embodiment the nucleotide sequence of the polynucleotide used for expression is optimized for the E7 protein. Optimization is performed using well known methods in the art such as those provided by companies offering these services such as GenCust or GeneArt® Gene Synthesis. Optimization aims at favoring presence in the polynucleotide to be expressed, of codons used by the producing cells (codon adaptation). In particular codon optimization aims at reducing rare codons at a level of 10% or less than 10% in the optimized polynucleotide and/or at increasing higher codons at a level of 20% or more in the obtained polynucleotide. In addition undesired restriction sites may be mutated in order to delete them if they appear as an issue for cloning. Furthermore cryptic internal promoter may be deleted when they are associated with transcription factor-binding sites. In addition sequences may be optimized in order to avoid rho-independent transcription terminator and/or hot spot for recombination such as CHI sites.

For illustration, optimized coding sequences of E7 are in particular disclosed as sequence between positions 685 and 850 (encoding fragment with consecutive residues 1-29 of E7) together with sequence between positions 1123 to 1207 (encoding fragment with consecutive residiues 43-98 of E7) of SEQ ID No.66 for HPV16-E7A30-42 and between positions 685 to 871 (encoding fragment with consecutive residues 1-31 of E7) together with sequence between positions 1141 to 1234 (encoding fragment with consecutive residues 43-105 of E7) of SEQ ID No.68 for HPV18-E7Δ32-42. Coding sequences for the E7 or E7-CyaA constructs are also provided in the present application.

Likewise, the sequence encoding the CyaA protein may be the native sequence of the gene or the optimized sequence for use in the producing cells. For illustration purpose the native and a particular optimized sequences encoding CyaA are disclosed as SEQ ID No.63 and SEQ ID No.65 respectively.

For the preparation of the recombinant E7-CyaA, *E.coli* is especially mentioned as a suitable producing cell.

## EXAMPLES

Therapeutic vaccination against human papillomavirus (HPV) offers the possibility of preventing progression to cervical lesions or cancer by clearing HPV infections. ProCervix is a therapeutic vaccine composed of recombinant HPV16 and HPV18 E7 proteins fused to catalytically inactive *Bordetella pertussis* adenylate cyclase.

Accordingly, the invention relates to and describes a clinical trial in which a therapeutic vaccine was tested in HPV-infected women with normal cervical cytology. Several other therapeutic vaccines are being developed for HPV-infected women who already have CIN or cervical cancer. For the few therapeutic HPV vaccines that have shown some evidence of efficacy in clinical trials, results remain to be confirmed in fully controlled, randomized clinical trials.

The candidate therapeutic vaccine for women infected with HPV16 or HPV18 but with normal cervical cytology or mild abnormalities in the present study is designated ProCervix. ProCervix contains recombinant HPV16 and HPV18 E7 proteins each fused to a catalytically inactive *Bordetella pertussis* adenylate cyclase (CyaA), which delivers the E7 antigens to $CD11b^+$ dendritic cells, where they are processed and presented as $CD4^+$ and $CD8^+$ T-cell epitopes to T lymphocytes.[8, 9] In mice, vaccination with a recombinant CyaA bearing the HPV16 E7 antigen induces an anti-E7 T cell specific immune response and eliminates established HPV16 E7-expressing tumours.[8, 10] Here, are reported of a phase 1 study examining the safety, tolerability, immunogenicity, and viral clearance activity of ProCervix in women infected with HPV16 or HPV18 with normal cervical cytology.

**Methods** - This phase 1 trial examined the tolerability and immunogenicity of ProCervix in women infected with HPV16 or HPV18 with normal cervical cytology (EudraCT No. 2010-018629-21). In the open-label part, subjects received two intradermal vaccinations 6 weeks apart of 100 or 600 μg ProCervix + topical imiquimod cream at the injection site. In the double-blind part, subjects were randomised 2:1:1 to two treatments 6 weeks apart of 600 μg ProCervix + imiquimod cream, 600 μg ProCervix + placebo cream, or placebo injection + imiquimod cream. Endpoints included reactogenicity, adverse events, T-cell response by interferon-γ enzyme-linked immunospot assay, and viral clearance as determined by quantitative PCR.

**Findings** - 47 women were included. There were no dropouts, treatment-related serious adverse events, or dose-limiting toxicities. Local reactions to ProCervix were transient and mostly mild to moderate. The most frequent systemic reactions specific to ProCervix were mild to moderate transient myalgia and fatigue. E7-specific T-cell responses were detected in 16 of 26 subjects receiving 600 μg ProCervix + imiquimod and 1 of 6 receiving placebo + imiquimod. Viral clearance was more frequent with ProCervix 600 μg + imiquimod (19/28) than placebo + imiquimod (3/7). Viral infections recurred less often in the 600 μg ProCervix + imiquimod group (2/13) than in other groups (5 of 8). Cytological abnormalities other than ASC-US occurred in 8 of 40 subjects receiving ProCervix and 5 of 7 receiving placebo.

**Interpretation** - ProCervix plus imiquimod was well-tolerated by women infected with HPV16 or HPV18 with normal cervical cytology. ProCervix also stimulates T-cell responses to E7 and promotes sustained viral clearance.

**I. Material and Methods**

**Study design and objectives**

This phase 1 trial (EudraCT No. 2010-018629-21) was performed at VAXINFECTIO (University of Antwerp, Belgium) between June 10, 2010 and December 31, 2012. The primary objective was to examine the tolerability (including cervical cytology and HPV viral loads) of ProCervix in healthy women infected with HPV16, HPV18, or both and with normal cervical cytology. Secondary objectives were to examine the cellular and humoral immunogenicity of ProCervix. The study was approved by an independent ethics committee (Comité Voor Medische Ethiek, Universitair Ziekenhuis Antwerpen) and complied with Belgian laws, Good Clinical Practice, and the Declaration of Helsinki (October 2008 revision). All subjects participating in the study provided written informed consent.

**Subjects**

Healthy, non-pregnant, non-lactating women 18-45 years of age were considered for enrolment if they were infected with HPV16, HPV18, or both according to two different genotyping tests (see "Virology" below) and had consecutive normal cervical cytology examinations separated by 6 weeks to 12 months and a normal gynaecological examination. The women also had to employ effective contraception for at least one month before vaccination and four months after vaccination and had to have a total $CD4^+$ count ≥500 cells/$mm^3$. They could not have had previous cervical cancer or untreated high-grade histological lesion (CIN2/3); another malignancy; a severe allergy or history of asthma; a previous severe reaction to any of the components of the study vaccine; previously received a prophylactic HPV vaccine; received a vaccine within 4 weeks of receiving the first dose of the study vaccine; or have had any other disease or condition that, in the opinion of the investigator, could have affected the outcome of the trial.

**Study treatments**

ProCervix consists of equal amounts of two recombinant E7-CyaA fusion proteins expressed in and purified from *Escherichia coli.* Solution and powder formulations were prepared and reconstituted before use. Full details are provided in the Supplemental Methods. Topical 5% imiquimod cream (Aldara™, 3M Health Care Limited, Loughborough, UK) was used as an adjuvant.

**Study conduct**

The study comprised an open-label part with three cohorts and a randomised double-blind part (Figure 1). Decisions about whether to proceed with second vaccinations and with subsequent cohorts were made by a safety review committee.

In the open-label part of the study, subjects received intradermal injections into the upper thigh at weeks 0 and 6 of ProCervix in the following cohorts: 100 μg ProCervix solution as five 0-2-ml injections; 600 μg ProCervix solution as five 0·2-ml injections; and 600 μg ProCervix powder as a single 0·2-ml injection. In these three cohorts, injection of ProCervix was followed 15 min and 24 h later by topical application of 5% imiquimod cream (50 mg) at each site of injection for 30 min.

In the randomized, double-blind part of the study, subjects were randomised 2:1:1 to receive five 0·2-ml intradermal injections at weeks 0 and 6 of 600 μg ProCervix solution + 50 mg imiquimod cream, 600 μg ProCervix solution

+ 50 mg placebo cream (Excipial Hydrolotion neutre, Laboratoires SPIRIG SAS, Toulouse, France), or placebo injection (phosphate-buffered saline + 1.32 M urea) + 50 mg 5% imiquimod cream. Imiquimod or placebo cream was topically applied to the sites of injection 15 min and 24 h after vaccination for 30 min. Treatments were randomized using an unstratified computer-generated list containing a three-digit randomisation number and the corresponding treatment group allocation. Treatment assignments were communicated to an unblinded site pharmacist who prepared the injections and creams. Investigators, staff, study monitors, and subjects were blinded to treatment assignment.

Subjects received the second vaccination if the maximum erythema, swelling, and induration after first vaccination was ≤75 mm. They could also receive a second injection if any of these reactions were >75 but ≤100 mm but there were no other local or possibly related systemic grade 3 reactions.

**Safety and tolerability**

Subjects recorded local reactions (pain, tenderness, erythema, swelling, induration, ulceration, scabs, and itching) and systemic reactions (arthralgia, myalgia, headache, fatigue, nausea, fever, rigors) for up to 7 days (solution formulation of ProCervix or placebo injection) or 10-14 days (powder formulation of ProCervix) after each injection in a diary. Treatment-emergent adverse events (TEAEs) were recorded by investigators throughout the study and coded using MedDRA version 14. Severity of TEAEs was graded according to the US Food & Drug Administration Toxicity Grading Scale for Healthy Adult and Adolescent Volunteers Enrolled in Preventive Vaccine Clinical Trials.[11] Investigators assessed the causality of events as not related or as unlikely, possibly, probably, or definitely related to the treatment.

**Immunogenicity**

T-cell responses to HPV16 and HPV18 E7 antigen stimulation were assessed by interferon-γ (IFN-γ) enzyme-linked immunospot assay (ELISpot). Peripheral blood mononuclear cells (PBMC) were stimulated using pools of 15-mer peptides overlapping by 11 amino acids covering the full HPV16 or HPV18 E7 sequences. Serum antibodies to HPV16 E7, HPV18 E7, and CyaA E5 were detected by enzyme-linked immunosorbent assay (ELISA). An immune responder was defined as (i) for subjects with no baseline response, a specific positive response at any post-vaccination time point before week 26; or (ii) for subjects with a baseline response, a two-fold increase in specific positive response. Full details of the ELISA and ELISpot methods are described in the Supplemental Methods.

**Collection of cervical samples and assessment of cervical cytology**

Gynaecological examinations included cervical brush scrapings at the screening visit (3 weeks before the first vaccination) and at weeks 10 and 26. Additional cervical brush scrapings may have been taken before the end of the study. Cervical cells were collected into BD-SurePath (BD Diagnostics - Tripath, Burlington, NC, USA) using a Cervex-Brush Combi (Rovers Medical Devices B.V., KV Oss, The Netherlands). Cervical cytology was assessed in thin-layer cell preparations and according to the 2001 version of the Bethesda System.[12] Cells were collected from remaining samples by density sedimentation at 3000 × g on a BD PrepMate system (BD Diagnostics - Tripath). DNA was isolated from the cell pellet remainder as described by Micalessi et al.[13]

**Virology**

DNA was obtained by automatic nucleic acid preparation using a JANUS Automated Solution Handling System (Perkin-Elmer, Waltham, MA). TaqMan-based multiplex qPCR (Life Technologies, Carlsbad, CA, USA) was performed to detect HPV genotypes including HPV16 and HPV18, with β-globin as a control. Type-specific HPV viral loads were expressed as the number of HPV copies/cell. HPV16/18 positivity (≥ 1 HPV copy/10,000 cells) was confirmed by Qualitative RealTi*me* High Risk HPV test (Abbott Molecular, Des Plaines, IL, USA). Full details of nucleic acid preparation and PCR methods are described in the Methods and Table 3. Clearance was defined as a negative HPV test result and sustained clearance as maintenance of HPV negativity until the end of the study.

**Statistical analysis**

Statistical analysis and calculations were made using Excel 2010 (Microsoft, Redmond, WA, USA) or SAS version 9.2 (SAS Institute, Cary, NC, USA). No formal sample size calculations or statistical tests were performed. Predefined primary and secondary endpoints are detailed in the Supplemental Methods. For analysis, all TEAEs considered by the investigator as possibly, probably, or definitely treatment-related, were grouped as treatment-related.

## II. Results

### Subjects

Nineteen healthy women positive for HPV16, HPV18, or both but with normal cervical cytology were enrolled in the open-label part of the study, and another 28 were enrolled in the randomised, double-blind part (Figure 2). All subjects completed the study, including seven subjects who received only a single vaccination.

Baseline characteristics were similar in all groups (Table 1). HPV16 was detected in 36 subjects and HPV18 in 11 subjects. Ten subjects were co-infected with HPV subtypes other than HPV16 and HPV18. All subjects were NILM at screening, although approximately half (23/47; 49%) had one or more reports of abnormal cytology in the 5 years before the study.

### Safety and tolerability

*TEAEs*

All subjects treated with ProCervix reported TEAEs considered by the investigator to be treatment-related. The most common treatment-related TEAEs were local events at the injection site (discoloration, erythema, pain, induration, swelling, and dryness), although headache and fatigue were also recorded as TEAEs related to treatment. No grade 4 (potentially life-threatening) or 5 (fatal) events were reported. The most frequent grade 3 treatment-related TEAEs were injection-site pain (n=11), injection-site swelling (n=5), and injection-site erythema (n=6). None of the subjects withdrew from the study due to an adverse event. In seven cases, after consulting with the safety review committee, the investigator decided to not give a second vaccination because of concerns that a severe local reaction would occur. A single subject experienced SAEs (syncope and a fall with concussion) considered unrelated to treatment.

Lymphadenopathy was reported by 8 of 40 (20%) subjects treated with ProCervix but by none of 7 the subjects treated with placebo (Figure 1). One case of grade 3 lymphadenopathy was reported by a subject treated with ProCervix 600 μg powder + imiquimod group. The event resolved after treatment with ibuprofen and fusidine cream. All other cases of lymphadenopathy were mild or moderate

*Solicited reactions*

All subjects reported solicited reactions, most of which were mild (grade 1) or moderate (grade 2) (Figures 3 and 4). The frequency, intensity, and duration of solicited reactions did not appear to depend on the vaccine dose or imiquimod adjuvantation. Seventeen subjects, all treated with ProCervix, reported severe (grade 3) solicited reactions. Most of these were at the injection-site (pain [n=11], tenderness [n=8], erythema [n=7], swelling [n=5], itching [n=2], and induration [n=2]), although a few grade 3 systemic reactions were reported (fatigue [n=3], myalgia [n=1]). Seven of the subjects experiencing grade 3 reactions were treated with medication (over-the-counter oral analgesics). In most cases, grade 3 reactions were down-graded to grade 2 within 1 to 2 days (data not shown).

Most of the subjects treated with ProCervix but few treated with placebo reported injection-site reactions (Figure 3). Scabs at the injection site, all grade 1, were reported only by subjects (6 of 19) treated with the combination of ProCervix 600 μg solution and imiquimod. After the first injection, solicited injection-site reactions were most severe after a delay of 2 to 5 days, whereas after the second injection, they were most severe after 0 to 2 days (data not shown). None of the subjects reported ulceration, and none experienced recall of injection-site reactions at the first vaccination site following the second vaccination. Rates of injection-site reactions were similar for subjects treated with the powder and solution formulations of ProCervix 600 μg, although grade 3 reactions were more frequent with the powder than the solution formulation for injection-site pain, erythema, and induration.

In all groups, the most common solicited systemic reactions were headache, myalgia, and fatigue (Figure 4). For several of the solicited systemic reactions, proportions were higher with ProCervix than placebo (52·5% vs. 14·3% for myalgia, 65·0% vs. 57·1% for headache, 72·5% vs. 57·1% for fatigue, 7·5% vs. 0% for fever, and 30·0% vs. 14·3% for rigors). Rates of systemic reactions were similar with the powder and solution formulations of ProCervix 600 μg, and in both cases, none were grade 3 (Figure 4).

### Viral clearance

In the ProCervix + imiquimod groups, the proportion of subjects with viral clearance increased steadily over time (Figure 5). In contrast, in subjects treated with ProCervix + placebo cream or with the placebo injection + imiquimod, the frequency of clearance did not show a clear trend over time. At the last assessment (mean follow-up =18·3 months for ProCervix solution or placebo and 10·7 months for ProCervix Powder), the proportion with viral clearance was higher in subjects treated with ProCervix solution 600 μg + imiquimod group (14/19; 74%) than in subjects treated with ProCervix 100 μg solution + imiquimod (2/5; 40%), ProCervix 600 μg solution + placebo cream (2/7; 29%), or placebo injection + imiquimod (3/7; 43%).

Twenty-one subjects had at least one additional HPV test after an initial clearance of the virus. HPV16 or HPV18 was re-detected in only 2 of 13 subjects (15%) treated with ProCervix 600 μg + imiquimod, whereas in the other groups, HPV16 or HPV18 was re-detected in 5 of 8 subjects (62%).

**Cervical cytology**

Thirteen subjects developed cytological abnormalities excluding ASC-US (8/40 [20%] for ProCervix; 5/7 [71%] for placebo) (Table 2). Two of these subjects, both in the ProCervix 600 μg solution + imiquimod group, had high-grade lesions and underwent conisation. HPV had not been cleared in either of these subjects. No clear differences in cytology were detected between treatment groups.

**Immunogenicity**

*Humoral immune response*

ProCervix did not induce serum anti-E7 antibodies in any of the subjects, but CyaA antibodies were detected in all 40 subjects vaccinated with ProCervix (data not shown). The 7 subjects injected with placebo did not have detectable anti-E7 or anti-CyaA antibodies (data not shown).

*Cellular immune response*

As measured by IFN-γ ELISpot, ProCervix solution induced E7-specific T-cell responses when used in conjunction with imiquimod cream but not with placebo cream (Figure 6A). The effect of ProCervix appeared dose-dependent: when combined with imiquimod, the proportion of T-cell responders was higher for the 600 μg dose ProCervix (61% overall, 61% for the solution formulation, and 62% for the powder formulation) than for the 100 μg dose (40%) or the placebo (17%).

As an exploratory analysis, we examined the T-cell response according to HPV clearance in subjects treated with ProCervix 600 μg + imiquimod (Figure 6B). The percentage of T-cell responders was higher in subjects with sustained HPV clearance (78%) and in subjects who cleared the virus at any time (71%) than in subjects who did not clear the virus (44%).

**Discussion**

This phase 1 study examined the safety and immunogenicity of ProCervix, a therapeutic HPV vaccine containing recombinant E7 of HPV16 and HPV18 fused to catalytically inactive *B. pertussis* CyaA. We report here the first clinical trial results for a therapeutic HPV vaccine in HPV-infected women with normal cervical cytology. The study was conducted in 47 subjects, evaluated two ProCervix formulations, and included both open-label dose-escalation and placebo-controlled randomised double-blind parts.

The study showed that intradermal vaccination with ProCervix is safe and well tolerated for women infected with HPV16 or HPV18. No safety concerns were detected. The first vaccination with ProCervix lead to delayed local reactions at the vaccination site (after 2 to 5 days) that were mostly mild to moderate. These local reactions occurred more quickly after the second vaccination (after 0 to 2 days) but remained mostly mild to moderate. Notable systemic reactions more common with ProCervix than placebo included myalgia, fatigue, and lymphadenopathy. All reactions were transient, and the only interventions needed were oral OTC analgesics and fusidine cream.

The systemic and local reactions to ProCervix were consistent with the pharmacological effects of a vaccine inducing a T-cell response. In most subjects, ProCervix induced a dose-dependent and imiquimod-enhanced E7-specific T-cell response as measured by ELIspot. Such a T-cell-mediated immune response against HPV antigens is important in the control of HPV infection and progression to CIN by sustaining clearance of HPV.[14] ProCervix also induced a humoral response to CyaA in all subjects, but it did not induce anti-E7 antibodies, which is consistent with preclinical animal studies. This is probably because the E7 antigen is fragmented, truncated, and inserted into CyaA.[8]

Effector T-cell responses to HPV in the peripheral blood are known to be weak, require ex vivo sensitization to be detected (e.g. ELISpot following stimulation with E7 in vitro), do not measure the T cell response at the site of infection in the cervix, and do not reliably identify persons whose lesions will regress.[7] Despite these limitations, within the ProCervix 600 μg+ imiquimod group, the T-cell response rate was higher in subjects who cleared HPV than in subjects who did not clear it or in whom HPV recurred. These results suggest that sustained clearance was mediated by an effector T-cell response to E7.

**Conclusion**

The results of this phase 1 study suggest that ProCervix in combination with imiquimod is well-tolerated by women infected with HPV16 or HPV18. Furthermore, by stimulating a T-cell response to E7 and thereby promoting viral clearance, ProCervix might limit progression of HPV16 and HPV18 infections to high-grade lesions and cervical cancer.

**Table 1. Baseline characteristics by treatment**

| Characteristic | Subcategory | ProCervix 100 µg solution + imiquimod N=5 | ProCervix solution 600 µg+ imiquimod N=19 | ProCervix solution 600 µg + placebo N=7 | Placebo injection + imiquimod N=7 | ProCervix 600 µg powder + imiquimod N=9 |
|---|---|---|---|---|---|---|
| Mean age ± standard deviation (y) | - | 29·0 ± 8·9 | 32·3 ± 5·9 | 33·3 ± 10·2 | 32·7 ± 8·7 | 33·2 ± 7·6 |
| Race | Caucasian | 5 (100·0) | 18 (94·7) | 7 (100·0) | 7 (100·0) | 9 (100·0) |
| | Other | 0 (0·0) | 1 (5·3) | 0 (0·0) | 0 (0·0) | 0 (0·0) |
| HPV type, n (%) | HPV16 | 5 (100·0) | 15 (78·9) | 5 (71·4) | 5 (71·4) | 6 (66·7) |
| | HPV18 | 0 (0·0) | 4 (26·3) | 2 (28·6) | 2 (28·6) | 3 (33·3) |
| Co-infection with other HPV types, n (%) | Positive | 1 (20·0) | 6 (31·6) | 0 (0·0) | 1 (14·3) | 2 (22·2) |
| Mean duration of infection (mo) | HPV16 | 19·8 | 12·3 | 12·2 | 15·4 | 13·7 |
| | HPV18 | - | 10·6 | 10·0 | 3·5 | 25·7 |
| Previous cytology, n (%) [a] | Negative (NILM) | 2 (40·0) | 10 (52·6) | 2 (28·6) | 4 (57·1) | 4 (44·4) |
| | ASC-US | 3 (60·0) | 5 (26·3) | 1 (14·3) | 0 (0·0) | 1 (11·1) |
| | LSIL | 0 (0·0) | 4 (21·1) | 2 (28·6) | 0 (0·0) | 3 (33·3) |
| | HSIL | 0 (0·0) | 0 (0·0) | 2 (28·6) | 2 (28·6) | 0 (0·0) |

Values are for the safety population. [a] In the past 5 years

**Table 2. Worst cervical during the study by treatment**

| Measure | Subcategory | ProCervix 100 µg solution + imiquimod N=5 n (%) | ProCervix solution 600 µg+ imiquimod N=19 n (%) | ProCervix solution 600 µg + placebo N=7 n (%) | Placebo injection + imiquimod N=7 n (%) | ProCervix 600 µg powder + imiquimod N=9 n (%) |
|---|---|---|---|---|---|---|
| Mean time to final follow-up (mo) | - | 23·3 | 17·8 | 15·0 | 18·9 | 10·7 |
| Proportion in each cytology category, n (%) | NILM | 2 (40·0) | 6 (31·6) | 3 (42·9) | 2 (28·6) | 4 (44·4) |
| | ASC-US | 2 (40·0) | 9 (47·4) | 4 (57·1) | (0·0) | 2 (22·2) |
| | AGC | 0 (0·0) | 0 (0·0) | 0 (0·0) | 0 (0·0) | 1 (11·1) |
| | LSIL | 1 (20·0) | 2 (10·5) | 0 (0·0) | 5 (71·4) | 0 (0·0) |
| | ASC-H | 0 (0·0) | 1[a] (5·3) | 0 (0·0) | 0 (0·0) | 2 (22·2) |
| | HSIL | 0 (0·0) | 1[b] (5·3) | 0 (0·0) | 0 (0·0) | 0 (0·0) |

Values are the worse cytology reported by each subject and are for the intent-to-treat population.

[a] One subject treated with ProCervix 600 µg solution + imiquimod was ASC-H at 11 months, underwent conisation (CIN3), and had normal cytology and was HPV-negative at the final examination (18 months).

[b] One subject treated with ProCervix 600 µg solution + imiquimod was HSIL at the final examination (18 months) and underwent conisation (CIN2). HPV was not cleared in this subject by the final examination.

SUPPLEMENTAL INFORMATION

**Predefined primary and secondary endpoints**

Primary endpoints were analysed in the safety population, which included all subjects who received any study treatment. The primary endpoints included the incidence, duration, severity, and relationship of TEAEs; the incidence and severity of local reactions; local skin evaluations at the sites of injections on the thigh and reports of systemic symptoms; treatment-emergent changes in vital signs, clinical laboratory tests, and physical examinations; and changes in cervical cytology and cervical HPV virology.

Secondary endpoints were evaluated in the intent-to-treat population, which included all randomized subjects in the double-blind portion and all subjects enrolled in the open-label portion who received any amount of study treatment and who had at least one post-baseline immunological evaluation. The secondary endpoints included treatment-emergent changes in T-cell responses to HPV16 and HPV18 E7 antigen stimulation; and treatment-emergent changes in anti-HPV16 E7, anti-HPV18 E7, and anti-CyaA serum antibody titers.

**Vaccines antigens and formulations**

The HPV16 E7-CyaA fusion protein included amino acids 1-29 of HPV16 E7 inserted between amino acids 319-320 of CyaA and amino acids and 43-98 of HPV16 E7 inserted between amino acids 224-235 of CyaA. The HPV18 E7-CyaA fusion protein included amino acid 1-31 of HPV18 E7 inserted between amino acids 319-320 of CyaA and amino acids 43-105 of HPV18 E7 inserted between amino acids 224-235 of the wild type CyaA. The liquid formulation (solution) of ProCervix (lot no. G091/PRO001/FC001) was provided in sterile, single-use vials and was diluted prior to use to dose either 0.1 mg or 0.6 mg ProCervix in 1 mL of phosphate-buffered saline (PBS) with 1.32 M urea. The lyophilized formulation (powder) of ProCervix (lot no. G152/C16C18/FC001) was provided in single-use vials and was reconstituted with sterile water prior to use to dose 3 mg/mL ProCervix in PBS with 1.32 M urea and 33 g/L trehalose, providing for 0.6 mg ProCervix in 0.2 mL injectable solution.

**ELISpot assay**

Peripheral blood mononuclear cells (PMBC) were isolated from blood samples and stored in liquid nitrogen. 96-well ELISPOT plates (Millipore, Billerica, MA, USA) were coated overnight at 4°C with 4 μg/mL anti-human interferon-γ (IFN-γ) (Thermo Scientific Fisher, Waltham, MA, USA) in PBS. PBMC were thawed, diluted in CTL test medium (CTL, Shaker Heights, OH, USA) + 1% L-glutamine and added to the plates ($4 \times 10^5$/well) along with 50 or 100 μg/mL pooled 15-mer peptides overlapping by 11 amino acids covering respectively the full HPV16 or HPV18 E7 sequences (Bachem, Torrance, CA, USA), medium alone as a negative control, or 5 μg/mL phytohemagglutinin (Sigma Aldrich, St. Louis, MO, USA) as a positive control. After 20 h at 37°C, plates are washed with PBS + 0.1% Tween-20 (PBST). Next, the plates are incubated overnight at 4°C with 1 μg/mL biotinylated anti-human IFN-γ (Thermo Scientific Fisher) in PBST + 1% BSA (PBST-BSA). After washing with PBST, plates were incubated for 2 h at room temperature with 1:16,000 streptavidin-horseradish peroxidase (Becton Dickinson, Franklin Lakes, NJ, USA) in PBST-BSA. Bound antibody was detected with 0.33 mg/mL 3-amino-9-ethyl carbazole (Sigma Aldrich) in 0.1 M acetate buffer. The number of spots measured with an Immunospot Analyzer and Immunospot software (CTL). A specific positive response was defined as:

$$\text{(Mean of X} - \text{standard deviation of X)} - \text{(mean Y} + 2 \times \text{standard deviation of Y)} > 20 \text{ spot-forming cells per } 4 \times 10^5 \text{ PBMC}$$

where X = number of spots for the stimulating antigen and Y = number of spots for the medium control. In addition, the mean of X had to be >3 × mean of Y or Y = 0

A subject was considered to have a specific positive ELISPOT response if:

- the difference between the (mean number of spots - standard deviation) in the presence of the stimulating peptides and the (mean number of spots + 2 × standard deviation) in the presence of medium was > 20 per $4 \times 10^5$ PBMC; or
- the mean of number of spots in the presence of the stimulating peptides was at least 3-fold higher than the mean number of spots in the presence of medium or no spots were detected in the presence of medium.

A subject was considered an immune responder if:

- the subject had a specific positive response at any post-vaccination time point before week 26 in but did not have a specific positive response at week 0; or
- if the subject had a specific response at week 0, a two-fold increase in the specific positive response vs. week 0 at any post-vaccination time point before week 26.

## ELISA

*Capture proteins for the ELISA*

Recombinant CyaA E5 (Genticel, Labege, France) was produced in *Escherichia coli* and purified from inclusion bodies by a two-step procedure that included DEAE-sepharose and phenyl-sepharose chromatography (GE Healthcare, Cleveland, OH, USA). HPV16 E7-HIS (SCIL) and HPV18 E7-HIS (GTP) were produced as described by Mirecka et al.[15] These two His-tag-proteins were expressed in *E. coli* and purified on a metal chelate affinity chromatography column preloaded with $Ni^{2+}$ (GE Healthcare). HPV18 E7-HIS was further purified by size-exclusion chromatography (GE Healthcare) and Endotrap blue chromatography (Hyglos Gmbh, Regensburg, Germany).

*ELISA procedure*

MaxiSorp 96-well plates (Nunc) were coated with 5 μg/mL recombinant HPV16 E7-HIS (SCIL) and HPV18 E7-HIS (GTP) or 0.625 μg/mL CyaA E5 (anti-CyaA ELISA) in 50 mM carbonate-bicarbonate buffer pH 9.6. The plates were then blocked overnight at 4°C in PBST-BSA and then incubated for 1 h at room temperature with serial 2-fold dilutions of serum (starting at 1:500) or standard (pooled anti-CyaA-positive serum, concentration arbitrarily set at 10,000 ELISA units/mL) in PBST-BSA. After washing with PBST, the plates were incubated with 1:5000 peroxidase-conjugated anti-human immunoglobulin (Sigma-Aldrich) in PBST-BSA. After washing in PBST, bound antibody was detected with tetramethylbenzidine and stopped by addition of sulphuric acid. The absorbance at 405 nm was measured with a microtiter plate reader and compared to a standard curve using SoftMax Pro (Molecular Devices, Sunnyvale, CA, USA) to calculate the concentration of antibody. The lower limit of quantitation for the ELISAs was 0.275 ELISA units/mL.

## Cervical cells sampling and density sedimentation for HPV DNA Testing

Cervical cells were collected using the Cervex-Brush Combi (Rovers, Oss, The Netherlands) as recommended in European Union guidelines. [15, 16] After collection, the head of the brush was left in the vial containing SurePath (BD Diagnostics - TriPath, Burlington, NC, USA). The vials were then transported to the Laboratory for Clinical and Molecular Pathology (AML, Sonic Healthcare, Antwerp, Belgium) where all samples were prepared. Prior to density sedimentation, the vial containing the brush head was vortexed and 2.0 mL was removed to be divided for RealTi*me* High Risk (HR) HPV assay (Abbott Molecular, Des Plaines, IL, USA; 0.6 mL) testing and Hybrid Capture II testing (Gaithersburg, MD, USA ;1.4 mL). The remaining 8 mL was subjected to density sedimentation at 3000 g on a BD PrepMate system (BD Diagnostics - Tripath, Burlington, NC USA) to enrich the cell samples. DNA was isolated from the cellular pellet remaining after cytologic processing as described previously.[13]

Isolation of DNA from cervical cells for qPCR HPV testing

DNA was obtained by automatic nucleic acid preparation (JANUS Automated Solution Handling System, Perkin-Elmer, Waltham, MA, USA). First, the optical density at 405 nm (OD405) was measured in solution-based cytology leftovers (Multilabel Counter Victor 3, Perkin-Elmer, Waltham, MA, USA) to determine the volume of cell suspension to be transferred to a 96-deep well block (OD405 ≤ 0.045, 800 μL; 0.046-0.050, 550 μL; 0.051-0.055, 500 μL; 0.056-0.064, 450 μL; ≥ 0.065, 400 μL), the volume of supernatant to be discarded after centrifugation of the block at 2500 rpm for 10 min (Rotanta 460, Hettich, Tuttlingen, Germany) (OD405 ≤ 0.045, 750 μL, 0.046-0.050, 500 μL; 0.051-0.055, 450 μL, 0.056-0.064, 400 μL; ≥ 0.065, 370 μL), and the volume of digestion solution (10 mM Tris, 1 mM EDTA, 200 μ g/mL proteinase K) to be added to the cell pellet (OD405 ≤ 0.085, 50 μL; 0.086-0.09, 100 μL, 0.10-0.34, 150 μL; ≥ 0.35, 200 μL). Each 96-deep well block contained 91 cervical samples and one well of water as a negative quality control for the extraction procedure and subsequent PCR. Digestion was performed for 3 h at 56°C, followed by 10 min at 95°C to inactivate the proteinase K. The DNA extracts were stored at -20°C until qPCR.

## Quantitative PCR (qPCR)

*Set-up*

TaqMan-based qPCR was performed to detect 18 HPV genotypes and β-globin. The HPV primers and probes were directed against HPV6 E6, HPV11 E6, HPV16 E7, HPV18 E7, HPV31 E6, HPV33 E6, HPV35 E6, HPV39 E7, HPV45 E7, HPV51 E7, HPV52 E7, HPV53 E6, HPV56 E7, HPV58 E6, HPV59 E7, HPV66 E6, HPV67 L1, and HPV68 E7. Eight multiplex reactions were performed in 5 μL containing LightCycler 480 Probes Master (Roche Applied Science, Basel, Switzerland), a variable concentration of primers and probes, and 1 μL of template DNA. Table 1 gives an overview of the multiplex PCRs and the sequences and concentrations of primers and fluorescent probes. A JANUS Automated Workstation (Perkin-Elmer, Waltham, MA, USA) was used for solution handling. Two 384-well plates were prepared for each set of 91 sample extracts. The first plate was used for the triplex PCRs and the second plate for the duplex PCRs. The plates were sealed with a Plate Sealer Roboseal (HJ Bioanalytik GmbH, Mönchengladbach, Germany) and placed in a thermocycler (LightCycler 480, Roche Applied Science, Basel, Switzerland) for 45 two-step cycles of 10 s at 95°C and 30 s at 60°C. Each PCR run included 91 sample extracts and a negative control, a positive control, and two (duplex reactions) or three (triplex reactions) calibrators. The positive control contained all HPV types that could be amplified by the HPV primers in the reaction mixture. For each type, the positive control contained $1 \times 10^5$ HPV copies, and the negative control was water that had been subjected to the extraction procedure. The HPV calibrators contained $1 \times 10^6$ copies of a single HPV type. The β-globin calibrator contained 129 ng/μL and the control contained 64.5 ng/μL.

**Table 1. Type-specific primers and probes for qPCR**

| Multiplex PCR | Primers and probes | Sequence (5' → 3') |
|---|---|---|
| 1. HPV67 L1[a] and HPV39 E7 | F33/52//67 L1 | CGTCGCAGGCGTAAACG |
| | R33/52/58/67 L1 | ACAGGAGGCAGGTACAC |
| | HPV33.52.58.67 L1 Probe | AGATGTCCGTGTGGCGG |
| | HPV39E7-FP | CGAGCAATTAGGAGAGTCAGAGG |
| | HPV39E7-RP | TGTGTGACGCTGTGGTTCAT |
| | Probe 39E7 | AACCCGACCATGCAGTT |
| 2. HPV31 E6 and HPV16 E7 | HPV31E6-FP | ACGATTCCACAACATAGGAGGA |
| | HPV31E6-RP | TACACTTGGGTTTCAGTACGAGGT |
| | Probe 31E6 | CTCCAACATGCTATGCAACGTCC |
| | HPV16E7-FP | AGCTCAGAGGAGGAGGATGAA |
| | HPV16E7-RP | GGTTACAATATTGTAATGGGCTC |
| | Probe 16E7 | CAGCTGGACAAGCAGA |
| 3. HPV18 E7 and β-globin | HPV18-153F | CCGACGAGCCGAACCA |
| | HPV18-219R | CTCAATTCTGGCTTCACACTTACAA |
| | HPV18-170T | AACGTCACACAATGTT |
| | B-globine-143F | TGCATTTGACTCCTGAGGAGAA |
| | B-globine-223R | GGGCCTCACCACCAACTTC |
| | B-globine-167T | CTGCCGTTACTGCCCT |
| 4. HPV45 E7, 56 E7 and 68 E7 | HPV45-161F | CGTCGGGCTGGTAGTTGTG |
| | HPV45-286R | ATTGCATTTGGAACCTCAGAATG |
| | HPV45-181T | ATGACTAACTCCATCTGC |
| | HPV56seq 807F | CCAAAGAGGACCTGCGTGTT |
| | HPV56seq 884R | TACTTGATGCGCAGAGTGGG |
| | HPV56seq 828T | TACAACAGCTGCTTATGG |
| | HPV68-FP | ACAACAGCGTCACACAATTCAGT |
| | HPV68-RP | CAGTTCTACGTTCCGCAGGTT |
| | HPV68 Probe | ACTGCAACTAGTAGTAGAAGCGTCGCGGG |
| 5. HPV33 E6, 52 E7 and 58 E6 | HPV33E6-FP | TGTGCGGCGTGTTGGA |
| | HPV33E6-RP | TGGCGTTTTTACACGTCACAGT |
| | Probe 33E6 | CCCGACGTAGAGAAA |
| | HPV52-116F | GTGTGGACCGGCCAGATG |

(continued)

| Multiplex PCR | Primers and probes | Sequence (5' → 3') |
|---|---|---|
| | HPV52-228R | CGTCGCAGTGCTATGAATGC |
| | HPV52-135T | ACAAGCAGAACAAGCC |
| | HPV58 64F | CCACGGACATTGCATGATTTG |
| | HPV58 144R | CTTTTTGCATTCAACGCATTTCA |
| | HPV58-95T | TGGAGACATCTGTGCATGAAATCGAA |
| 6. HPV59 E7, 35 E6 and 6 E6 | HPV59E7-FP | TGTGCTACGAGCAATTACCTGACT |
| | HPV59E7-RP | TGATTAACTCCATCTGGTTCATCTTT |
| | HPV59E7-Probe | CGACTCCGAGAATGA |
| | HPV35E6-FP | AAAAACCGCTGTGTCCAGTTG |
| | HPV35E6-RP | CACCTCGGTTTCTCTACGTGTTG |
| | Probe 35E6 | ATTCCATAACATCGGTGGACGGT |
| | HPV6:476U27 | GTTCATAAAGCTAAATTGTACGTGGAA |
| | HPV6:645L22 | TGTGAATCTTGTCCGTCCACTT |
| | HPV6:529L36-Probe | ACAATATCCTTTAGGGTAACATGTCTTCCAT GCATG |
| 7. HPV51 E7, 53 E6 and 66 E6 | HPV51 358 F | AAAGCAAAAATTGGTGGACGA |
| | HPV51 438 R | TGCCAGCAATTAGCGCATT |
| | HPV51 392 Probe | CATGAAATAGCGGGACGTTGGACG |
| | HPV53 FP | AACGGTTTCACAAAATTTCACATATG |
| | HPV53 RP | TGATTCAGTTGCTGTTGTGTGTCT |
| | HPV53 Probe | ACCGGGTCGTGCCTGACATGC |
| | HPV66 433 F | GTCCGTTAACACCGGAGGAA |
| | HPV66 495 R | CCCGGTCCATGCATATGC |
| | HPV66 454 Probe | AACAATTGCACTGTGAACATAAAAGACGAT TTCATT |
| 8. HPV11 E6 and *Trichomonas vaginalis* | HPV11:475U28 | GCTTCATAAAACTAAATAACCAGTGGAA |
| | HPV11:563L23 | TCAGGAGGCTGCAGGTCTAGTAC |
| | HPV11:644P17 | CGTTGCTTACACTGCTG |
| | TV-FP | CATTGACCACACGGACAAAAAG |
| | TV-RP | CGAAGTGCTCGAATGCGA |
| | TV-Probe | TCATTTCGGATGGTCAAGCAGCCA |

[a] The HPV67 L1 primers were also directed against the L1 region of HPV33, 52 and 58. A triplex qPCR (multiplex qPCR 5 in Table 1) for HPV33 E6, 52 E7 and 58 E6 was performed to confirm the presence/absence of these types in the LBC sample.

*Optimisation*

Ten-fold dilution series of HPV plasmids (1 to 1 × $10^6$ copies) and a two-fold dilution series of female human DNA (Promega, Fitchburg, WI, USA; range: 2-129 ng) were subjected to each qPCR to generate standard curves for determining analytical sensitivity, specificity and efficiency. Plasmids containing HPV16 and HPV18 were purchased from Clonit (Milan, Italy). Plasmids containing HPV types 31, 33, 35, 39, 45, 51, 52, 53, 56, 58, 59, 66, 67 and 68 were kindly supplied by T. Matsukara (National Institute of Infectious Diseases, Tokyo, Japan), A. Lörincz (Digene Corp., Gaithersburg, MD, USA), E. de Villiers (DKFZ, Heidelberg, Germany), and G. Orth (Institute Pasteur, Paris, France) or were prepared by cloning of PCR products of clinical samples, estimating the copy numbers for individual plasmid preparations by spectrophotometry. Each plasmid dilution was completed with 32.14 ng/μL female human DNA (Promega, Fitchburg, WI, USA) to mimic the complex nucleic acid environment present when amplifying genomic DNA. The intra- (repeatability) and inter-run variability (reproducibility) were evaluated using clinical samples with crossing point (Cp)-values across the PCR dynamic range. To perform absolute quantification of the amount of HPV present in an unknown sample, standard curves and calibrators were made to correlate DNA quantity and the Cp value. External standard curves were generated for each HPV type based on 10-fold serial dilutions of HPV plasmids (1 to 1 $\times$ $10^6$ copies). β-globin standard curves were obtained by amplifying a two-fold dilution series of female human DNA (2-129 ng). The HPV copy number (copies/μL) and DNA concentration (ng/μL) were determined from the standard curves. The amount of genomic DNA (ng) present in each sample was divided by the weight of one genome equivalent (6.6 pg/cell) to obtain the number of cells in the sample. The viral load in each sample was expressed as the number of HPV copies per cell.

*Validation*

To assess the validity of a qPCR run, both the Cp value of the calibrator and the concentration of the positive control were evaluated. For each HPV type, the mean Cp value of the calibrator was determined based on repeated measurements in 20 independent qPCR runs. The standard deviation (SD) was determined based on a fixed coefficient of variation (CV) of 2.5%, which was calculated based on 500 measurements for each HPV calibrator in 50 independent qPCR runs. The mean Cp and the 3 $\times$ SD on either side of the mean were plotted on a Levy-Jennings control chart. A PCR run was considered valid when the Cp value of the calibrator fell within the 3 $\times$ SD range of the control chart mean. If the Cp of the calibrator fell outside the 3 $\times$ SD, the concentration of the positive control was evaluated using a fixed CV of 75%, which corresponded to a 0.5 log10 difference in concentration. The average concentration of the positive control for each HPV type was determined based on repeated measurements in 20 independent qPCR runs. The mean concentration and the SD were plotted on a Levy-Jennings control chart. If the concentration of the positive control was out of range, this control was considered invalid.

*Quality control*

Quality assurance and internal quality control steps were included throughout the qPCR workflow, and the different steps of the workflow were performed in strictly separated rooms. In addition, external quality control is performed every 6 months by international quality control programs (United Kingdom National External Quality Assessment Service [UK NEQAS] for Microbiology, London, UK; INSTAND e. V., Düsseldorf, Germany; quality control for molecular diagnostics [QCMD], Glasgow, Scotland, UK]. Since 2008, the Department of Cytopathology and Molecular Diagnostics has acquired an ISO 15189 accreditation granted by the Belgian accreditation body (BELAC) for the performance of the HPV qPCR test.

**Real-time HPV type-specific PCR**

The HPV qPCR test is a clinically validated assay[18] that uses automated sample preparation and DNA extraction combined with real-time PCR technology to detect and quantify 18 different HPV types: HPV6 E6, HPV11 E6, HPV16 E7, HPV18 E7, HPV31 E6, HPV33 E6, HPV35 E6, HPV39 E7, HPV45 E7, HPV51 E7, HPV52 E7, HPV53 E6, HPV56 E7, HPV58 E7, HPV59 E7, HPV66 E6, HPV33, 52, 58, and 67 L1,[6] and HPV68 E7 as previously described by Micalessi et al. [13]

*β-globin real-time quantitative PCR*

A β-globin real-time quantitative PCR was used to assess the DNA quality and to estimate the number of cells in the test sample. [13, 18] The amount of β -globin DNA (ng) present in each sample was divided by the weight of 1 genome equivalent (6.6 pg/cell) and a factor of 2 (because there are two copies of β-globin DNA/genome equivalent) to obtain the number of genome equivalents (cell) in the sample. This β-globin control PCR was considered positive when at least 1000 cells could be measured. [18] The analytical sensitivity of the different type specific HPV qPCRs varied between 1 and 100 HPV copies/reaction. [20] The number of HPV copies was divided by the number of cells to calculate the viral load (HPV copies/cell). A threshold of 0.0001 HPV copies/cell was considered positive.

*Hybrid Capture II test*

The clinical performance of the qPCR for HPV 16, 18, 31, 33, 35, 39, 45, 51, 52, 56, 58, 59 and 68 was conformed with the Hybrid Capture II test (Digene). [20-22] From each BD-SurePath specimen, 1.4 mL of random sample was kept in the vial and stored at 4°C before testing. The samples were batch analysed in a central laboratory (Laborverbund für Medizinische Diagnostik, Heidelberg, Germany) throughout the trial in accordance with the manufacturer's instructions. Samples were considered valid if the ratio of relative light units for the sample to the standard positive control was > 1.

*RealTime High Risk HPV tests*

To confirm HPV 16/18 positivity detected by the qPCR HPV assay, qualitative RealTi*me* High Risk HPV tests (Abbott Molecular, Des Plaines, IL, USA) were performed in accordance with the manufacturer's instructions. The RealTi*me* High Risk HPV is a qualitative in vitro test for the detection of DNA from 14 high-risk HPV genotypes (16, 18, 31, 33, 35, 39, 45, 51, 52, 56, 58, 59, 66, and 68) in clinical specimens.

From each BD-SurePath specimen, 0.6 mL of random sample was transferred to a 5-mL tube and loaded onto an Abbott m2000sp instrument for DNA extraction and PCR plate pipetting. 96-well plates were manually sealed and transferred to a m2000rt instrument (Abbott). The detection of the 14 high-risk (HR) HPV genotypes is achieved using a primer mix targeting a conserved region of HPV genomes (L1) and single-stranded DNA probes. The assay can differentiate between HPV16, HPV18, and non-HPV16/18 genotypes. Internal control amplicons are generated with a primer set targeting an endogenous human β-globin sequence and are detected with the internal control-specific probe. The Abbott RealTime HR HPV assay detects the endogenous human β-globin sequence as sample validity control for cell adequacy, sample extraction, and amplification efficiency. HPV subtypes were considered detected if the cycle number was less than a fixed assay cut-off cycle or as not detected if the cycle number was not generated or was ≥ the assay cut-off cycle. For confirmation of HPV16 and HPV18 positive samples, cycle numbers < 40 were considered positive.

**Cervical cytology reading**

Thin-layer cell preparations with discrete staining for cytological analysis were generated with the BD PrepStain slide processor using 200 μl of cervical cell-enriched fraction. Thin-layer cytology slides were read without knowledge of HPV results. The cytological results were classified according to the Bethesda system 2001[12] as follows: negative for intraepithelial lesion or malignancy (NILM), atypical squamous cells of undetermined significance (ASC-US); atypical squamous cells of undetermined significance, cannot exclude high-grade squamous intraepithelial lesions (ASC-H); atypical glandular cells of undetermined significance (AGC); low-grade squamous intraepithelial lesions (LSIL); or high-grade squamous intraepithelial lesions (HSIL).

**References for the supplemental methods**

## Table 3. Candidate HPV vaccines identified by the systematic review

| Vaccine Type | Candidate vaccine | Target HPV antigen | Disease target | Highest trial phase reported | Increased T-cell immunity | Clinical response | HPV clearance | Reference |
|---|---|---|---|---|---|---|---|---|
| Fusion protein virus-like particles | L1VLP-E7 | HPV16 E7 | CIN2-3 | 2 | 5/23 (22%) | Histological improvement to CIN 1 or normal in 39% for vaccine vs. 25% for placebo | 6/16 (37%) for vaccine vs. 1/7 for placebo (14%) | Kaufmann et al. 2007[23] |
| Dendritic cell vaccines | E7-pulsed autologous dendritic cell vaccine | HPV16/18 E7 | Stage IB or IIA cervical cancer after radical surgery | 1 | 10/10 (100%) | Not reported | Not reported | Santin et al. 2008[24] |
| DNA vaccines | ZYC101a | HPV16/18 E6, E7 | CIN2-3 | 3 | 45/110 (41%) | Disease clearance 43% for vaccine vs. 25% placebo (NS) | Not reported | Garcia et al. 2004[25] |
| Fusion proteins | SGN-00101 (HspE7) | HPV-16 E7 | CIN3 | 2 | Not reported | CR, 13/58 (22.5%); PR, 32 (55%); SD, 11 (19%); progression, 2 (3.5%) | Not reported | Einstein et al. 2007[26] |
| | | | CIN2-3 | 2 | 9/17 (53%) | Complete regression, 7/20 (35%); regression to CIN1, 1 (5%); SD, 11 (55%); progression, 1 (5%) | 4/17 (23%) | Roman et al. 2007[27] |
| | PD-E7 | HPV16 E7 | High-grade CIN | 1/2 | 5/7 (71%) | Not reported | Not reported | Hallez et al. 2004[28] |

| Vaccine Type | Candidate vaccine | Target HPV antigen | Disease target | Highest trial phase reported | Increased T-cell immunity | Clinical response | HPV clearance | Reference |
|---|---|---|---|---|---|---|---|---|
| | E6-E7 | HPV16 E6, E7 | CIN | 1 | 4/9 (44%) | No differences found after vaccination | 7/14 (50%) | Frazer et al. 2004[29] |
| Lipopeptide vaccines | HPV-16 E7 lipopeptide vaccine | HPV16 E7 | Refractory cervical or vaginal cancer | 1 | No significant increase | Clinical responses in 0/12 | Not reported | Steller et al. 1998[30] |
| Live attenuated virus | ADXS11-001 (formerly Lovaxin C or Lm-LLO-E7) | E7 | Advanced invasive cervical carcinoma | 1 | Not reported | SD, 7/13 (54%); progression, 5 (38%); unconfirmed PR, 1 (8%) | Not reported | Maciag et al. 2009[31] |
| Peptide vaccines | Synthetic long-peptide vaccine | HPV16 E6, E7 | HSIL | 2 | 5/5 (100%) | Not reported | Not reported | de Vos van Steenwijk et al. 2012[32] |
| | HPV-16 E7 peptide | HPV-16 E7 | CIN3/VIN3 | 1 | 10/18 (56%) | PR/CR in 9/17 (53%) | 12/18 (67%) | Muderspach et al. 2000[33] |
| | CIGB-228 | HPV16 E7 | CIN 2-3 | Pilot study/not stated | 7/7 (100%) | CR, 4/7; PR, 2 (29%) | 3/7 (43%) | Solares et al. 2011[34] |
| Peptide vaccine | Dual E7 peptide vaccine | HPV16 E7 | Cervical carcinoma | 1/2 | Not reported | Not reported | Not reported | van Driel et al. 1999 |
| Recominant vaccinia virus | MVA-E2 | HPV E2 | CIN2-3 | 2 | 34/34 (100%) | Total elimination of high-grade lesions, 20/34 (59%); 50% decrease in lesions, 11 (32%); reduced to CIN2, 2 (6%); reduced to CIN1, 1 (3%) | 12/34 (35%) vs. 0/20 (50%) for placebo | García-Hernández et al. 2006[35] |
| | TA-HPV | HPV16/18 E6, E7 | Early-stage cervical cancer | Not stated | CTL responses in 4/23 (17%) | Not reported | Not reported | Kaufmann et al. 2002[36] |

| Vaccine Type | Candidate vaccine | Target HPV antigen | Disease target | Highest trial phase reported | Increased T-cell immunity | Clinical response | HPV clearance | Reference |
|---|---|---|---|---|---|---|---|---|
| | TA-HPV | HPV16/18 E6, E7 | Late-stage cervical cancer | 1/2 | 1/3 (33%) | Not reported | Not reported | Borysiewicz et al. 1996[37] |
| | TG4001/R3484 | HPV16 E6, E7 | CIN2-3 | 2 | Not reported | Clinical response in 10/21 (48%) | Median time to clearance of high-risk HPV, 13.5 w | Brun et al. 2011[5] |

Abbreviations: CIN, cervical intraepithelial neoplasia; CR, complete response; HSIL, high-grade cervical squamous intraepithelial lesion; NS, not significant; PR, partial response; SD, stable disease; VIN, vulval intraepithelial neoplasia

**REFERENCES**

1. Kitchener HC, Denton K, Soldan K, Crosbie EJ. Developing role of HPV in cervical cancer prevention. British Medical Journal. 2013; 347: f4781.
2. Willmott LJ, Monk BJ. Cervical cancer therapy: current, future and anti-angiogensis targeted treatment. Expert Review of Anticancer Therapy. 2009; 9(7): 895-903.
3. Stern PL, van der Burg SH, Hampson IN, Broker TR, Fiander A, Lacey CJ, et al. Therapy of human papillomavirus-related disease. Vaccine. 2012; 30 Suppl 5: F71-82.
4. van der Burg SH, Melief CJ. Therapeutic vaccination against human papilloma virus induced malignancies. Current Opinion in Immunology. 2011; 23(2): 252-7.
5. Brun JL, Dalstein V, Leveque J, Mathevet P, Raulic P, Baldauf JJ, et al. Regression of high-grade cervical intraepithelial neoplasia with TG4001 targeted immunotherapy. American Journal of Obstetrics and Gynecology. 2011; 204(2): 169 e1-8.
6. Kawana K, Adachi K, Kojima S, Kozuma S, Fujii T. Therapeutic Human Papillomavirus (HPV) Vaccines: A Novel Approach. Open Virology Journal. 2012; 6: 264-9.
7. Stanley M, Pinto LA, Trimble C. Human papillomavirus vaccines--immune responses. Vaccine. 2012; 30 Suppl 5: F83-7.
8. Preville X, Ladant D, Timmerman B, Leclerc C. Eradication of established tumors by vaccination with recombinant Bordetella pertussis adenylate cyclase carrying the human papillomavirus 16 E7 oncoprotein. Cancer Research. 2005; 65(2): 641-9.
9. Simsova M, Sebo P, Leclerc C. The adenylate cyclase toxin from Bordetella pertussis--a novel promising vehicle for antigen delivery to dendritic cells. International Journal of Medical Microbiology. 2004; 293(7-8): 571-6.
10. Berraondo P, Nouze C, Preville X, Ladant D, Leclerc C. Eradication of large tumors in mice by a tritherapy targeting the innate, adaptive, and regulatory components of the immune system. Cancer Research. 2007; 67(18): 8847-55.
11. Food and Drug Administration Center for Biologics Evaluation and Research. Toxicity Grading Scale for Healthy Adult and Adolescent Volunteers Enrolled in Preventive Vaccine Clinical Trials. Rockville, MD: US Food and Drug Administration; 2007.
12. Solomon D, Davey D, Kurman R, Moriarty A, O'Connor D, Prey M, et al. The 2001 Bethesda System: terminology for reporting results of cervical cytology. Journal of the American Medical Association. 2002; 287(16): 2114-9.
13. Micalessi IM, Boulet GA, Bogers JJ, Benoy IH, Depuydt CE. High-throughput detection, genotyping and quantification of the human papillomavirus using real-time PCR. Clinical Chemistry and Laboratory Medicine. 2012; 50(4): 655-61.
14. Farhat S, Nakagawa M, Moscicki AB. Cell-mediated immune responses to human papillomavirus 16 E6 and E7 antigens as measured by interferon gamma enzyme-linked immunospot in women with cleared or persistent human papillomavirus infection. International Journal of Gynecological Cancer. 2009; 19(4): 508-12.

15. Mirecka EA, Rudolph R, Hey T. Expression and purification of His-tagged HPV16 E7 protein active in pRb binding. Protein expression and purification. 2006; 48(2): 281-91.

16. Arbyn M, Herbert A, Schenck U, Nieminen P, Jordan J, McGoogan E, et al. European guidelines for quality assurance in cervical cancer screening: recommendations for collecting samples for conventional and liquid-based cytology. Cytopathology : official journal of the British Society for Clinical Cytology. 2007; 18(3): 133-9.

17. Depuydt CE, Benoy IH, Bailleul EJ, Vandepitte J, Vereecken AJ, Bogers JJ. Improved endocervical sampling and HPV viral load detection by Cervex-Brush Combi. Cytopathology : official journal of the British Society for Clinical Cytology. 2006; 17(6): 374-81.

18. Depuydt CE, Benoy IH, Beert JF, Criel AM, Bogers JJ, Arbyn M. Clinical validation of a type-specific real-time quantitative human papillomavirus PCR against the performance of hybrid capture 2 for the purpose of cervical cancer screening. Journal of clinical microbiology. 2012; 50(12): 4073-7.

19. Moberg M, Gustavsson I, Gyllensten U. Real-time PCR-based system for simultaneous quantification of human papillomavirus types associated with high risk of cervical cancer. Journal of clinical microbiology. 2003; 41(7): 3221-8.

20. Depuydt CE, Boulet GA, Horvath CA, Benoy IH, Vereecken AJ, Bogers JJ. Comparison of MY09/11 consensus PCR and type-specific PCRs in the detection of oncogenic HPV types. Journal of cellular and molecular medicine. 2007; 11(4): 881-91.

21. Lorincz AT. Hybrid Capture method for detection of human papillomavirus DNA in clinical specimens: a tool for clinical management of equivocal Pap smears and for population screening. The journal of obstetrics and gynaecology research. 1996; 22(6): 629-36.

22. Poljak M, Brencic A, Seme K, Vince A, Marin IJ. Comparative evaluation of first-and second-generation digene hybrid capture assays for detection of human papillomaviruses associated with high or intermediate risk for cervical cancer. Journal of clinical microbiology. 1999; 37(3): 796-7.

23. Kaufmann AM, Nieland JD, Jochmus I, Baur S, Friese K, Gabelsberger J, et al. Vaccination trial with HPV16 L1E7 chimeric virus-like particles in women suffering from high grade cervical intraepithelial neoplasia (CIN 2/3). International journal of cancer Journal international du cancer. 2007; 121(12): 2794-800.

24. Santin AD, Bellone S, Palmieri M, Zanolini A, Ravaggi A, Siegel ER, et al. Human papillomavirus type 16 and 18 E7-pulsed dendritic cell vaccination of stage IB or IIA cervical cancer patients: a phase I escalating-dose trial. Journal of virology. 2008; 82(4): 1968-79.

25. Garcia F, Petry KU, Muderspach L, Gold MA, Braly P, Crum CP, et al. ZYC101a for treatment of high-grade cervical intraepithelial neoplasia: a randomized controlled trial. Obstetrics and gynecology. 2004; 103(2): 317-26.

26. Einstein MH, Kadish AS, Burk RD, Kim MY, Wadler S, Streicher H, et al. Heat shock fusion protein-based immunotherapy for treatment of cervical intraepithelial neoplasia III. Gynecologic oncology. 2007; 106(3): 453-60.

27. Roman LD, Wilczynski S, Muderspach LI, Burnett AF, O'Meara A, Brinkman JA, et al. A phase II study of Hsp-7 (SGN-00101) in women with high-grade cervical intraepithelial neoplasia. Gynecologic oncology. 2007; 106(3): 558-66.

28. Hallez S, Simon P, Maudoux F, Doyen J, Noel JC, Beliard A, et al. Phase I/II trial of immunogenicity of a human papillomavirus (HPV) type 16 E7 protein-based vaccine in women with oncogenic HPV-positive cervical intraepithelial neoplasia. Cancer immunology, immunotherapy : CII. 2004; 53(7): 642-50.

29. Frazer IH, Quinn M, Nicklin JL, Tan J, Perrin LC, Ng P, et al. Phase 1 study of HPV16-specific immunotherapy with E6E7 fusion protein and ISCOMATRIX adjuvant in women with cervical intraepithelial neoplasia. Vaccine. 2004; 23(2): 172-81.

30. Steller MA, Gurski KJ, Murakami M, Daniel RW, Shah KV, Celis E, et al. Cell-mediated immunological responses in cervical and vaginal cancer patients immunized with a lipidated epitope of human papillomavirus type 16 E7. Clinical cancer research : an official journal of the American Association for Cancer Research. 1998; 4(9): 2103-9.

31. Maciag PC, Radulovic S, Rothman J. The first clinical use of a live-attenuated Listeria monocytogenes vaccine: a Phase I safety study of Lm-LLO-E7 in patients with advanced carcinoma of the cervix. Vaccine. 2009; 27(30): 3975-83.

32. de Vos van Steenwijk PJ, Ramwadhdoebe TH, Lowik MJ, van der Minne CE, Berends-van der Meer DM, Fathers LM, et al. A placebo-controlled randomized HPV16 synthetic long-peptide vaccination study in women with high-grade cervical squamous intraepithelial lesions. Cancer immunology, immunotherapy : CII. 2012; 61(9): 1485-92.

33. Muderspach L, Wilczynski S, Roman L, Bade L, Felix J, Small LA, et al. A phase I trial of a human papillomavirus (HPV) peptide vaccine for women with high-grade cervical and vulvar intraepithelial neoplasia who are HPV 16 positive. Clinical cancer research : an official journal of the American Association for Cancer Research. 2000; 6(9): 3406-16.

34. Solares AM, Baladron I, Ramos T, Valenzuela C, Borbon Z, Fanjull S, et al. Safety and Immunogenicity of a Human Papillomavirus Peptide Vaccine (CIGB-228) in Women with High-Grade Cervical Intraepithelial Neoplasia:

First-in-Human, Proof-of-Concept Trial. ISRN obstetrics and gynecology. 2011; 2011: 292951.
35. Garcia-Hernandez E, Gonzalez-Sanchez JL, Andrade-Manzano A, Contreras ML, Padilla S, Guzman CC, et al. Regression of papilloma high-grade lesions (CIN 2 and CIN 3) is stimulated by therapeutic vaccination with MVA E2 recombinant vaccine. Cancer gene therapy. 2006; 13(6): 592-7.
36. Kaufmann AM, Stem PL, Rankin EM, Sommer H, Nuessler V, Schneider A, et al. Safety and immunogenicity of TA-HPV, a recombinant vaccinia virus expressing modified human papillomavirus (HPV)-16 and HPV-18 E6 and E7 genes, in women with progressive cervical cancer. Clinical cancer research : an official journal of the American Association for Cancer Research. 2002; 8(12): 3676-85.
37. Borysiewicz LK, Fiander A, Nimako M, Man S, Wilkinson GW, Westmoreland D, et al. A recombinant vaccinia virus encoding human papillomavirus types 16 and 18, E6 and E7 proteins as immunotherapy for cervical cancer. Lancet. 1996; 347(9014): 1523-7.
38. Wright et al., American Journal of Clinical pathology, 2011; 136:578-586:
39. Khan et al., Journal of the National Cancer Institute, 2005; 97(14):1072-1079
40. Doorbar et al Vaccine 2012, 30S

**Claims**

1. Composition comprising HPV16E7 and the HPV18E7 proteins, said E7 protein is recombined with catalytically inactive adenylate cyclase of *Bordetella pertussis* (CyaA) (designated E7-CyaA) for use as active ingredient of an immunotherapeutic vaccine in the treatment of a human subject infected by human papillomavirus of type 16 (HPV16) or 18 (HPV18) or both HPV16 and HPV18, wherein the subject is a female positive for HPV16 DNA or HPV18 DNA or both and presented with either asymptomatic infection or with cervical undetermined abnormalities (ASCUS) or with mild abnormalities such as low-grade squamous intraepithelial lesions (LSILs) or cervical intraepithelial neoplasia 1 (CIN1).

2. Composition for use according to claim 1, wherein the subject is:

   - either a female who has been previously diagnosed with transient or with persistent infection by HPV16 or HPV18 or both or,
   - a female who has been previously diagnosed with recurrent infection by HPV16 or HPV18 or both following regression or clearance of previous infection or following treated or cured previous infection by HPV16 or HPV18 or both.

3. Composition for use according to claim 1, wherein the subject has been diagnosed as having a normal cervical cytology or having low-grade lesion such as low-grade squamous intraepithelial lesion (LSIL) or ASC-UC or cervical intraepithelial neoplasia of type 1 (CIN1).

4. Composition for use according to any of the preceding claims in the prevention of HPV16 and/or HPV18 induced epithelial diseases and malignancies, including induced ano-genital, in particular genital, more particularly cervical lesions or for the prevention of HPV16 and/or HPV18 induced local immunosuppression or viral escape mechanism development.

5. Composition for use according to any of the preceding claims, in an administration regime that includes the administration of a topical adjuvant, wherein said adjuvant is an adjuvant of the E7-specific T-cell immune response, in particular of the E7-specific T-cell memory response and is suitable for administration in a human subject.

6. Composition for use according to any of the preceding claims in combination with a topical adjuvant.

7. Composition for use according to any of the preceding claims, wherein the composition and the adjuvant are administered separately and sequentially in time and the adjuvant is administered at or near the site of administration of the composition, on the same biological tissue.

**8.** Composition for use according to any of the preceding claims wherein the adjuvant is a TLR-7 ligand, in particular imiquimod, in particular imiquimod as a cream, more particularly as 5% imiquimod cream.

**9.** Composition for use according to any of the preceding claims wherein the administration regime encompasses multiple-dose administration of the topical adjuvant, in particular separate administration in time of 2 or more doses of imiquimod cream.

**10.** Composition for use according to any of the preceding claims, wherein the composition is administered intradermally, in particular by intradermal injection, more particularly by intradermal injection in the subject's thigh for each dose of the composition.

**11.** Composition for use according to any of the preceding claims, wherein the composition and the adjuvant are administered at a site which is not the natural site of the targeted infection or the site of the possible or the detected lesion.

**12.** Composition for use according to any of the preceding claims, in the prevention of the onset of HPV16 or HPV-18-related ano-genital, in particular genital especially cervical lesions or in the control of the development of ASCUS or LSILs abnormalities or CIN1 lesion, in particular to prevent progression of infection toward premalignant genital lesion of type 2 or 3, in particular toward CIN2 or CIN3.

**13.** Composition for use according to any of the preceding claims, for viral clearance of HPV16 or HPV18 or HPV16 and HPV18, or to achieve regression of undetermined or mild epithelial abnormalities or borderline abnormalities associated with HPV16, HPV18 or both.

**14.** Composition for use according to any of the preceding claims, wherein the dose of the composition which is administered to the subject is in the range of 100μg to 1200μg.

**15.** Composition for use according to any of the preceding claims, wherein the composition is administered as a single dose of the active ingredient or alternatively as more than one dose, in particular 2 or 3 doses, in particular within 1 to 6 weeks, or alternatively as a single or as a multiple-dose in a prime-boost regime wherein in said prime-boost regime the priming administration and the boosting homologous administration are separated by more than 6 weeks.

**16.** Composition for use according to any of the preceding claims, wherein said use encompasses the following administration regimen:

- (i) intradermally administering the composition at a site remote from the genital area, wherein administration requires a single dose or a multiple-dose schedule provided the total quantity of the composition administered to the subject is within a range of 100 to 1200μg;
- (ii) separately in time, in particular 10 to 30 minutes after the administration of each dose of the composition, more particularly 15 minutes later, administering a topical adjuvant at the site of the intradermal administration of the composition and repeating said administration of topical adjuvant 20 to 36 hours apart, in particular 24 hours later;
- (iii) optionally repeating both primary administration steps (i) and (ii) within 1 to 6 weeks of said primary administration steps of the composition and respectively adjuvant.
- (iv) optionally and either alternatively or cumulatively to (iii), repeating administration steps (i) and (ii) within 6 to 12 months of the primary administration step(s).

**17.** The composition for use according to any of the preceding claims, wherein the E7-CyaA of HPV16 and the E7-CyaA of HPV18 contain respectively the E7 proteins of (i) SEQ ID No.61 wherein amino acid residues 30 to 42 have been deleted and said E7 protein consists in a fragment having residues 1-29 and a fragment having resiudes 43-98 of said sequence and of (ii) SEQ ID No.62 wherein amino acid residues 32 to 42 have been deleted and said E7 protein consists in a fragment having residues 1-29 and a fragment having residues 43-105 of said sequence , in particular in equal quantities.

**18.** The composition for use as active ingredient of an immunotherapeutic vaccine according to any of the preceding claims, wherein the E7-CyaA of HPV16 and the E7-CyaA of HPV18 are respectively the recombinant proteins having SEQ ID No.67 and SEQ ID No.69.

**19.** The composition for use as active ingredient of an immunotherapeutic vaccine according to any of the preceding claims, wherein the subject is further infected with at least one of the HPV selected among HPV31, HPV33, HPV35, HPV39, HPV45, HPV52, HPV58 or HPV59 or among HPV31, HPV33, HPV52, HPV58 and HPV45, said composition further containing one or more different E7-CyaA protein(s) of HPVs selected HPV31, HPV33, HPV35, HPV39, HPV45, HPV52, HPV58 or HPV59 or among HPV31, HPV33, HPV52, HPV58 and HPV45.

**20.** A combination of a composition of active ingredient and of a topical adjuvant as defined in any of claims 1 to 19 and optionally excipient(s), for use as separately administered components in the treatment of infection or disease causally related to HPV16 or HPV18 or both, in a human subject infected by human papillomavirus of type 16 (HPV16) or 18 (HPV18) or both HPV16 and HPV18, wherein the subject is selected among females positive for HPV16 DNA or HPV18 DNA or both and presenting either with asymptomatic infection or with cervical inconclusive abnormalities (ASCUS) or with mild abnormalities such as low-grade squamous intraepithelial lesions (LSILs) or cervical intraepithelial neoplasia 1 (CIN1).

**21.** A combination according to claim 20, wherein the composition of active ingredient is formulated as a liquid, in particular a solution or a suspension, or as a lyophilized form, in particular a powder and the topical adjuvant is contained in a separate formulation such as a cream.

**22.** A kit comprising the composition as active ingredient and the topical adjuvant as defined in any of claims 1 to 19 wherein said composition is provided as a monodose or a multi-dose in a vial or in a syringe and the topical adjuvant is provided in a separate vessel or sachet, said kit further comprising the therapeutic indication that the combination is for use as an immunotherapeutic vaccine in the treatment of a human subject infected by human papillomavirus of type 16 (HPV16) or 18 (HPV18) or both HPV16 and HPV18, wherein the subject is selected among females positive for HPV16 DNA or HPV18 DNA or both and presenting either with asymptomatic infection or with cervical undetermined abnormalities (ASCUS) or with mild abnormalities such as low-grade squamous intraepithelial lesions (LSILs) or cervical intraepithelial neoplasia 1 (CIN1).

**23.** The combination or the kit according to any of claims 20 to 22 wherein the adjuvant is an adjuvant of the T-cell immune response, in particular an adjuvant of the T-cell memory response, preferably a TLR-7 ligand, preferably imiquimod, especially imiquimod 5%.

**24.** The combination or the kit according to any of claims 20 to 23, wherein the composition as active ingredient contains respectively the E7 proteins of SEQ ID No.61 wherein amino acid residues 30 to 42 have been deleted and of SEQ ID No.62 wherein amino acid residues 32 to 42 have been deleted or in particular the E7-CyaA of HPV16 and the E7-CyaA of HPV18 are respectively the recombinant proteins having SEQ ID No.67 and SEQ ID No.69 in dose range of 100μg to 1200μg, in particular in equal quantities.

**25.** A topical adjuvant of the T-cell immune response suitable for administration in a human subject, in particular of the T-cell memory response, more particularly imiquimod cream, for use for separate, and optionally sequential, administration with a composition as defined in any of claims 1 to 19, in the treatment of a human subject infected by human papillomavirus of type 16 (HPV16) or 18 (HPV18) or both HPV16 and HPV18, wherein the subject is a female positive for HPV16 DNA or HPV18 DNA or both and presenting either with asymptomatic infection or with cervical undetermined (ASCUS) or with mild abnormalities such as low-grade squamous intraepithelial lesions (LSILs) or cervical intraepithelial neoplasia 1 (CIN1).

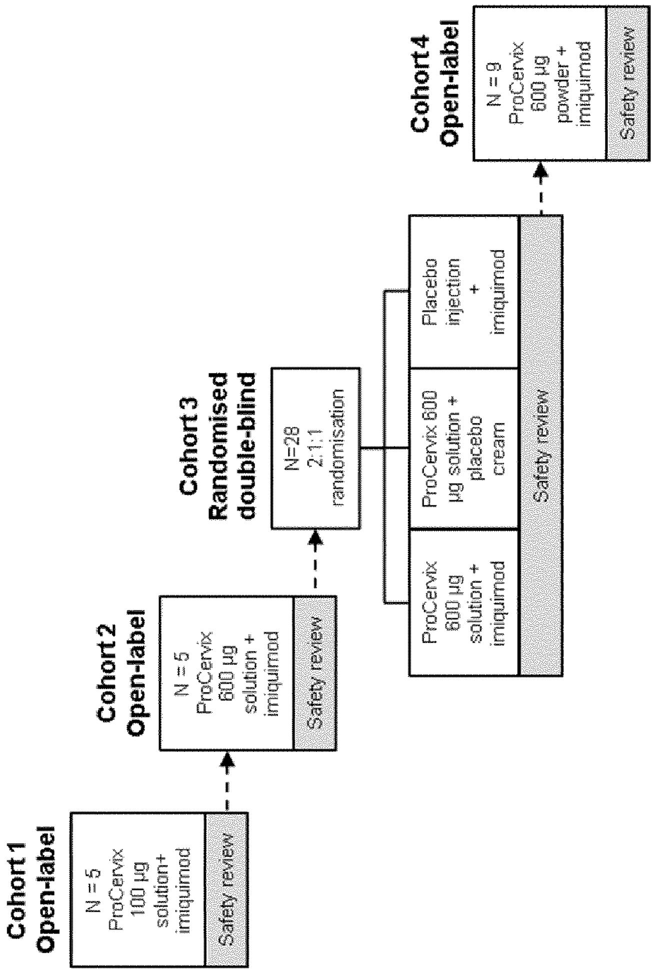
Cohort 1
Open-label
N = 5
ProCervix
100 µg
solution+
imiquimod
Safety review
Cohort 2
Open-label
N = 5
ProCervix
600 µg
solution +
imiquimod
Safety review
Cohort 3
Randomised
double-blind
N=28
2:1:1
randomisation
ProCervix
600 µg
solution +
imiquimod
ProCervix 600
µg solution +
placebo
cream
Placebo
injection
+
imiquimod
Safety review
Cohort 4
Open-label
N = 9
ProCervix
600 µg
powder +
imiquimod
Safety review

FIGURE 1

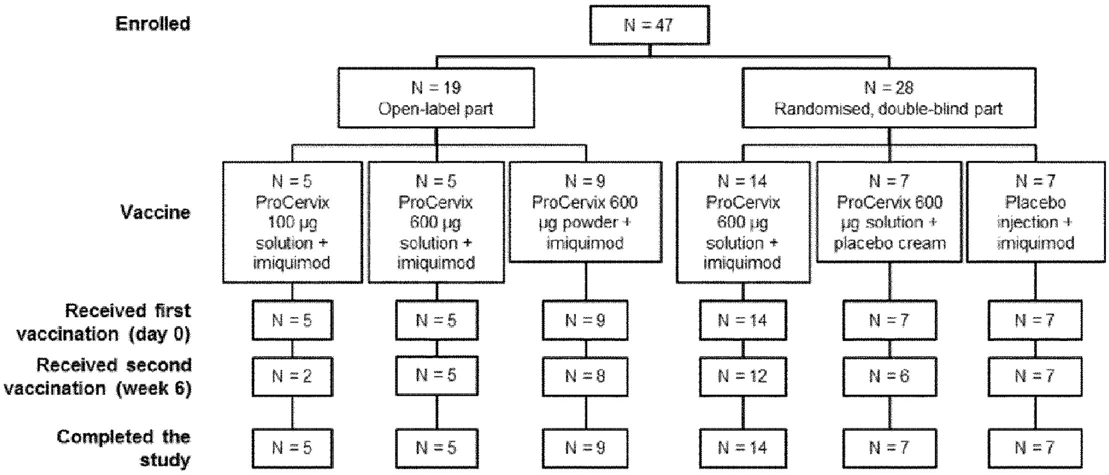
Enrolled
N = 47
N = 19
Open-label part
N = 28
Randomised, double-blind part
Vaccine
N = 5
ProCervix
100 µg
solution +
imiquimod
N = 5
ProCervix
600 µg
solution +
imiquimod
N = 9
ProCervix 600
µg powder +
imiquimod
N = 14
ProCervix
600 µg
solution +
imiquimod
N = 7
ProCervix 600
µg solution +
placebo cream
N = 7
Placebo
injection +
imiquimod
Received first
vaccination (day 0)
N = 5
N = 5
N = 9
N = 14
N = 7
N = 7
Received second
vaccination (week 6)
N = 2
N = 5
N = 8
N = 12
N = 6
N = 7
Completed the
study
N = 5
N = 5
N = 9
N = 14
N = 7
N = 7

# FIGURE 2

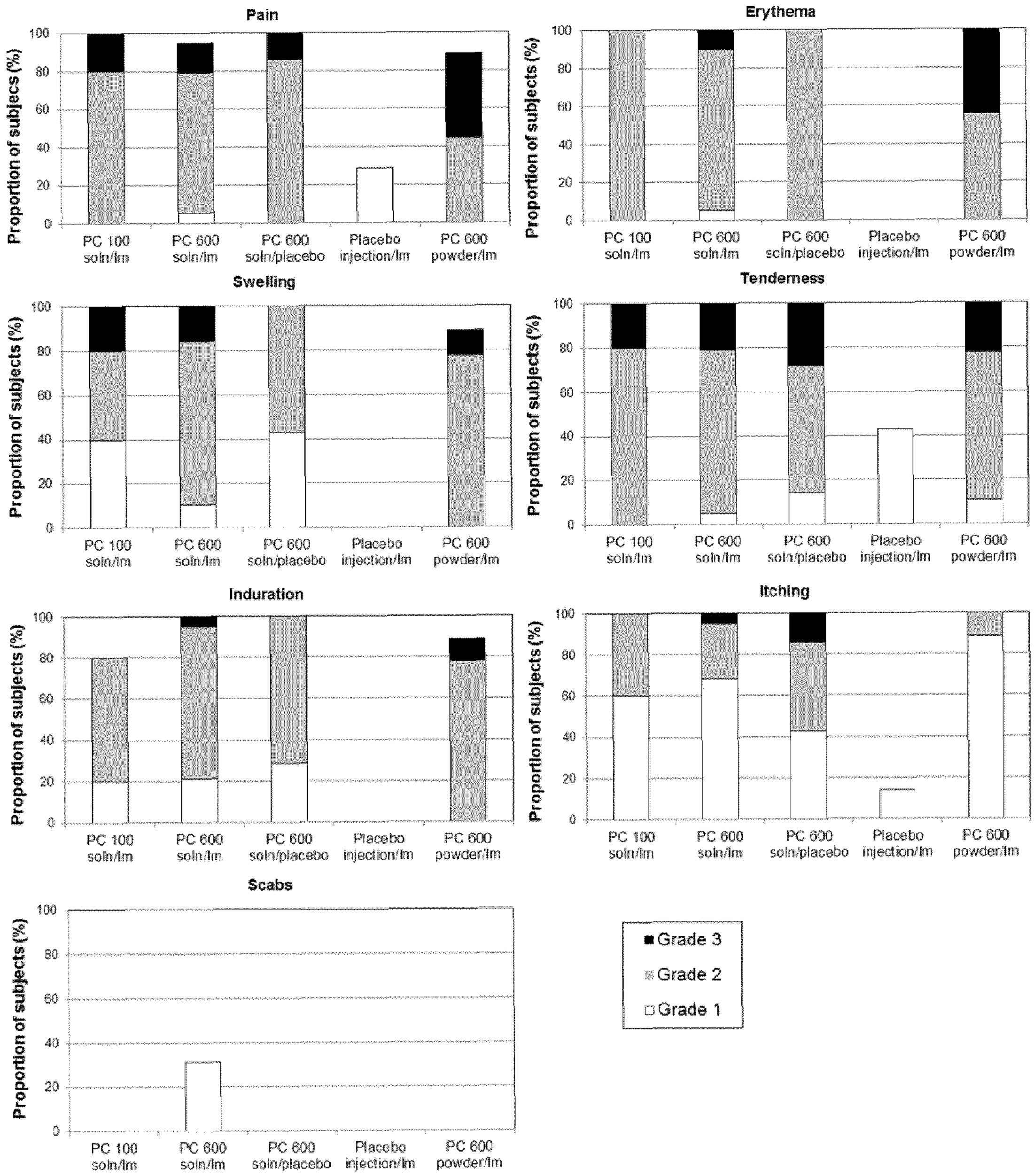
Pain
Erythema
Swelling
Tenderness
Induration
Itching
Scabs
Proportion of subjects (%)
0
20
40
60
80
100
PC 100 soln/Im
PC 600 soln/Im
PC 600 soln/placebo
Placebo injection/Im
PC 600 powder/Im
Grade 3
Grade 2
Grade 1

**FIGURE 3**

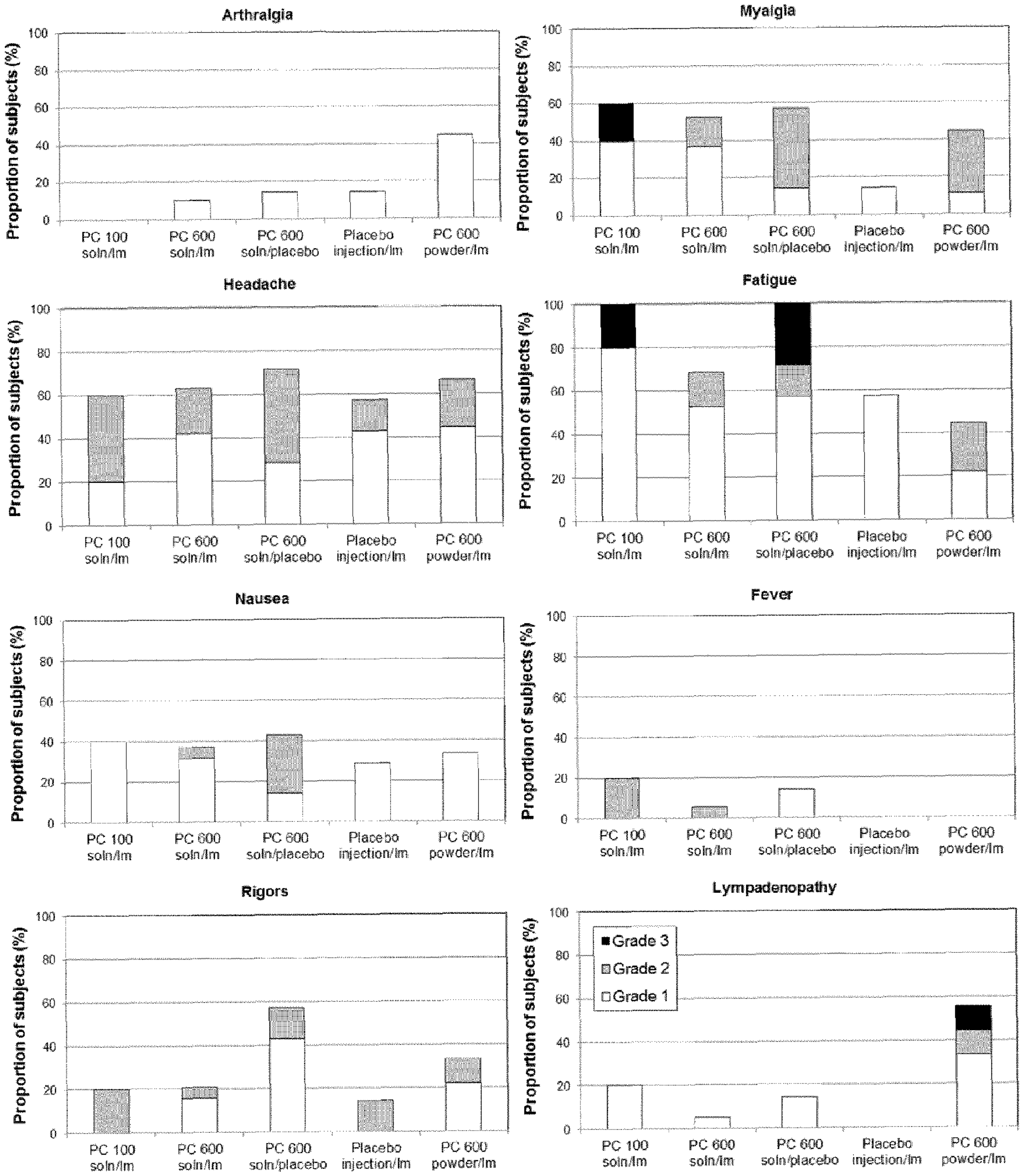

Arthralgia
Myalgia
Headache
Fatigue
Nausea
Fever
Rigors
Lympadenopathy
Proportion of subjects (%)
0
20
40
60
80
100
PC 100 soln/Im
PC 600 soln/Im
PC 600 soln/placebo
Placebo injection/Im
PC 600 powder/Im
Grade 3
Grade 2
Grade 1


FIGURE 4

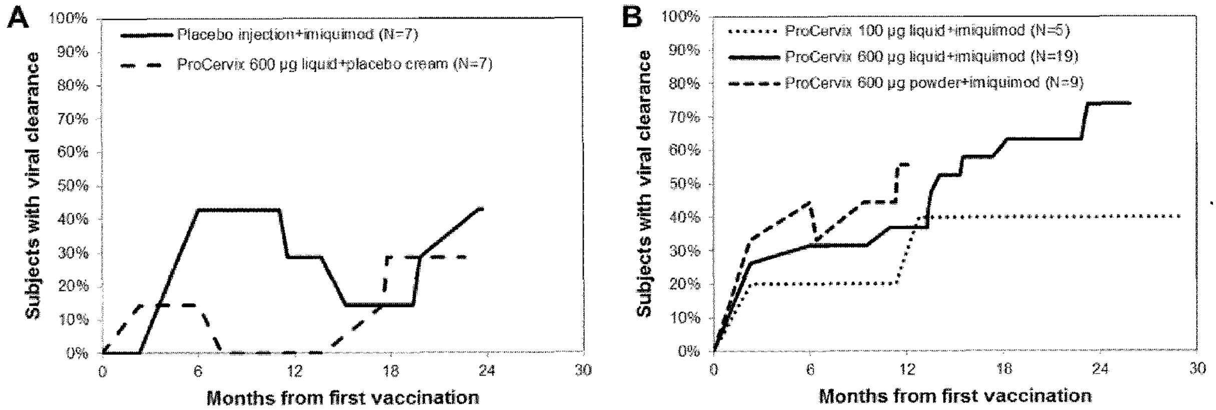
A
Placebo injection+imiquimod (N=7)
ProCervix 600 µg liquid+placebo cream (N=7)
Subjects with viral clearance
100%
90%
80%
70%
60%
50%
40%
30%
20%
10%
0%
0
6
12
18
24
30
Months from first vaccination
B
ProCervix 100 µg liquid+imiquimod (N=5)
ProCervix 600 µg liquid+imiquimod (N=19)
ProCervix 600 µg powder+imiquimod (N=9)
Subjects with viral clearance
Months from first vaccination

**FIGURE 5**

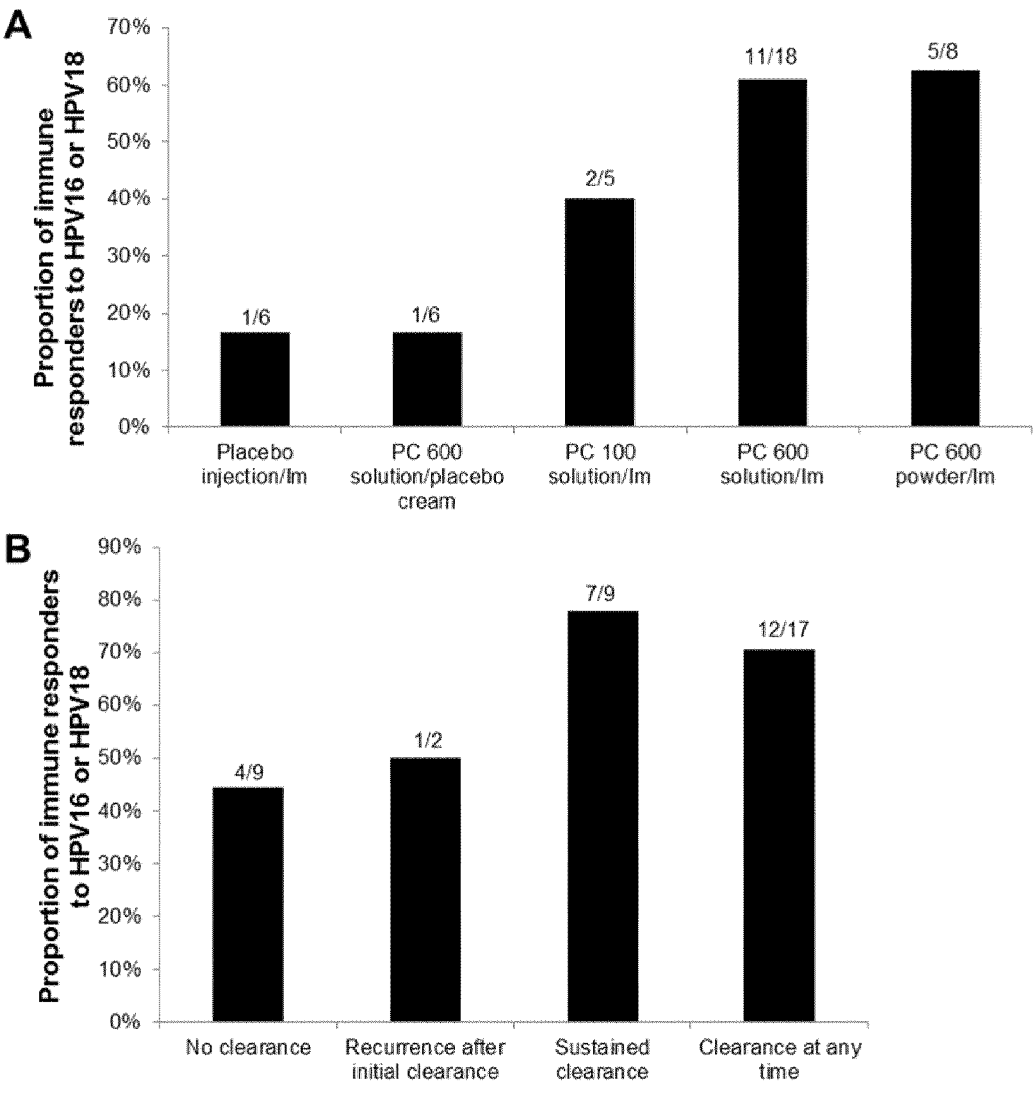
A
Proportion of immune responders to HPV16 or HPV18
70%
60%
50%
40%
30%
20%
10%
0%
1/6
1/6
2/5
11/18
5/8
Placebo injection/Im
PC 600 solution/placebo cream
PC 100 solution/Im
PC 600 solution/Im
PC 600 powder/Im
B
Proportion of immune responders to HPV16 or HPV18
90%
80%
70%
60%
50%
40%
30%
20%
10%
0%
4/9
1/2
7/9
12/17
No clearance
Recurrence after initial clearance
Sustained clearance
Clearance at any time

FIGURE 6

Europäisches Patentamt
European Patent Office
Office européen des brevets

# EUROPEAN SEARCH REPORT

Application Number
EP 14 30 7153

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>Y | WO 2014/016312 A1 (GENTICEL [FR]) 30 January 2014 (2014-01-30)<br>* See e.g. page 52, lines 3-27; page 53, lines 17-27; from line 6 of page 54 to line 22 of page 55; page 58, lines 8-14; pages 79-81; claim 18; SEQ ID NOs 25 and 26 *<br>----- | 22-24<br>1-25 | INV.<br>A61K39/12<br>C07K14/005<br>C07K14/235<br>A61K39/00 |
| Y | WO 03/008649 A1 (UNIV TEXAS [US]; SASTRY K JAGANNADHA [US]; TORTOLERO-LUNA GUILLERMO [U) 30 January 2003 (2003-01-30)<br>* See e.g. page 5, lines 15-20; page 15, lines 5-8; or claim 42 *<br>----- | 1-25 | |
| X<br>Y | WO 2012/101112 A1 (GENTICEL [FR]; EQUERRE MICHAEL [FR]; MOMOT MARIE [FR]; GOUBIER ANNE [F) 2 August 2012 (2012-08-02)<br>* See e.g. page 31, lines 5-7; page 32, line 3; page 33, lines 1-9; page 34, lines 13-17; page 41, line 11; claims 18 and 21 *<br>----- | 22-24<br>1-25 | |
| | | | TECHNICAL FIELDS SEARCHED (IPC) |
| Y | HINTEN F ET AL: "HPV-related (pre)malignancies of the female anogenital tract in renal transplant recipients", CRITICAL REVIEWS IN ONCOLOGY / HEMATOLOGY, vol. 84, no. 2, 2012, pages 161-180, XP028951651, ISSN: 1040-8428, DOI: 10.1016/J.CRITREVONC.2012.02.008<br>* See e.g. page 167 *<br>----- | 1-25 | A61K<br>C07K<br>C12N |

The present search report has been drawn up for all claims

2

| Place of search | Date of completion of the search | Examiner |
|---|---|---|
| Munich | 29 May 2015 | Valcárcel, Rafael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 14 30 7153

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2015

| Patent document cited in search report | Publication date | Patent family member(s) | Publication date |
|---|---|---|---|
| WO 2014016312 A1 | 30-01-2014 | AU 2013295047 A1 | 05-02-2015 |
| | | CA 2879999 A1 | 30-01-2014 |
| | | EP 2689786 A1 | 29-01-2014 |
| | | EP 2874657 A1 | 27-05-2015 |
| | | KR 20150031487 A | 24-03-2015 |
| | | WO 2014016312 A1 | 30-01-2014 |
| WO 03008649 A1 | 30-01-2003 | CN 1556863 A | 22-12-2004 |
| | | EP 1417353 A1 | 12-05-2004 |
| | | HK 1070391 A1 | 18-04-2002 |
| | | JP 2004535816 A | 02-12-2004 |
| | | US 2005048467 A1 | 03-03-2005 |
| | | US 2009053296 A1 | 26-02-2009 |
| | | WO 03008649 A1 | 30-01-2003 |
| WO 2012101112 A1 | 02-08-2012 | AU 2012210612 A1 | 02-05-2013 |
| | | CA 2825470 A1 | 02-08-2012 |
| | | CN 103476424 A | 25-12-2013 |
| | | EP 2478915 A1 | 25-07-2012 |
| | | EP 2667890 A1 | 04-12-2013 |
| | | JP 2014506562 A | 17-03-2014 |
| | | KR 20140034147 A | 19-03-2014 |
| | | US 2014037670 A1 | 06-02-2014 |
| | | WO 2012101112 A1 | 02-08-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014016312 A
- WO 2005089792 A


### Non-patent literature cited in the description


- **KITCHENER HC ; DENTON K ; SOLDAN K ; CROSBIE EJ.** Developing role of HPV in cervical cancer prevention. *British Medical Journal,* 2013, vol. 347, f4781
- **WILLMOTT LJ ; MONK BJ.** Cervical cancer therapy: current, future and anti-angiogensis targeted treatment. *Expert Review of Anticancer Therapy,* 2009, vol. 9 (7), 895-903
- **STERN PL ; VAN DER BURG SH ; HAMPSON IN ; BROKER TR ; FIANDER A ; LACEY CJ et al.** Therapy of human papillomavirus-related disease. *Vaccine,* 2012, vol. 30 (5), F71-82
- **VAN DER BURG SH ; MELIEF CJ.** Therapeutic vaccination against human papilloma virus induced malignancies. *Current Opinion in Immunology,* 2011, vol. 23 (2), 252-7
- **BRUN JL, DALSTEIN V ; LEVEQUE J ; MATHEVET P ; RAULIC P ; BALDAUF JJ et al.** Regression of high-grade cervical intraepithelial neoplasia with TG4001 targeted immunotherapy. *American Journal of Obstetrics and Gynecology,* 2011, vol. 204 (2), 169 e1-8
- **KAWANA K ; ADACHI K ; KOJIMA S ; KOZUMA S ; FUJII T.** Therapeutic Human Papillomavirus (HPV) Vaccines: A Novel Approach. *Open Virology Journal,* 2012, vol. 6, 264-9
- **STANLEY M ; PINTO LA ; TRIMBLE C.** Human papillomavirus vaccines--immune responses. *Vaccine,* 2012, vol. 30 (5), F83-7
- **PREVILLE X ; LADANT D ; TIMMERMAN B ; LECLERC C.** Eradication of established tumors by vaccination with recombinant Bordetella pertussis adenylate cyclase carrying the human papillomavirus 16 E7 oncoprotein. *Cancer Research,* 2005, vol. 65 (2), 641-9
- **SIMSOVA M ; SEBO P ; LECLERC C.** The adenylate cyclase toxin from Bordetella pertussis--a novel promising vehicle for antigen delivery to dendritic cells. *International Journal of Medical Microbiology,* 2004, vol. 293 (7-8), 571-6
- **BERRAONDO P ; NOUZE C ; PREVILLE X ; LADANT D ; LECLERC C.** Eradication of large tumors in mice by a tritherapy targeting the innate, adaptive, and regulatory components of the immune system. *Cancer Research,* 2007, vol. 67 (18), 8847-55
- Toxicity Grading Scale for Healthy Adult and Adolescent Volunteers Enrolled in Preventive Vaccine Clinical Trials. US Food and Drug Administration, 2007
- **SOLOMON D ; DAVEY D ; KURMAN R ; MORIARTY A ; O'CONNOR D ; PREY M et al.** The 2001 Bethesda System: terminology for reporting results of cervical cytology. *Journal of the American Medical Association,* 2002, vol. 287 (16), 2114-9
- **MICALESSI IM ; BOULET GA ; BOGERS JJ ; BENOY IH ; DEPUYDT CE.** High-throughput detection, genotyping and quantification of the human papillomavirus using real-time PCR. *Clinical Chemistry and Laboratory Medicine,* 2012, vol. 50 (4), 655-61
- **FARHAT S ; NAKAGAWA M ; MOSCICKI AB.** Cell-mediated immune responses to human papillomavirus 16 E6 and E7 antigens as measured by interferon gamma enzyme-linked immunospot in women with cleared or persistent human papillomavirus infection. *International Journal of Gynecological Cancer,* 2009, vol. 19 (4), 508-12
- **MIRECKA EA ; RUDOLPH R ; HEY T.** Expression and purification of His-tagged HPV16 E7 protein active in pRb binding. *Protein expression and purification,* 2006, vol. 48 (2), 281-91
- **ARBYN M ; HERBERT A ; SCHENCK U ; NIEMINEN P ; JORDAN J ; MCGOOGAN E et al.** European guidelines for quality assurance in cervical cancer screening: recommendations for collecting samples for conventional and liquid-based cytology. *Cytopathology : official journal of the British Society for Clinical Cytology,* 2007, vol. 18 (3), 133-9
- **DEPUYDT CE ; BENOY IH ; BAILLEUL EJ ; VANDEPITTE J ; VEREECKEN AJ ; BOGERS JJ.** Improved endocervical sampling and HPV viral load detection by Cervex-Brush Combi. *Cytopathology : official journal of the British Society for Clinical Cytology,* 2006, vol. 17 (6), 374-81

- **DEPUYDT CE ; BENOY IH ; BEERT JF ; CRIEL AM ; BOGERS JJ ; ARBYN M.** Clinical validation of a type-specific real-time quantitative human papillomavirus PCR against the performance of hybrid capture 2 for the purpose of cervical cancer screening. *Journal of clinical microbiology,* 2012, vol. 50 (12), 4073-7
- **MOBERG M ; GUSTAVSSON I ; GYLLENSTEN U.** Real-time PCR-based system for simultaneous quantification of human papillomavirus types associated with high risk of cervical cancer. *Journal of clinical microbiology,* 2003, vol. 41 (7), 3221-8
- **DEPUYDT CE ; BOULET GA ; HORVATH CA ; BENOY IH ; VEREECKEN AJ ; BOGERS JJ.** Comparison of MY09/11 consensus PCR and type-specific PCRs in the detection of oncogenic HPV types. *Journal of cellular and molecular medicine,* 2007, vol. 11 (4), 881-91
- **LORINCZ AT.** Hybrid Capture method for detection of human papillomavirus DNA in clinical specimens: a tool for clinical management of equivocal Pap smears and for population screening. *The journal of obstetrics and gynaecology research,* 1996, vol. 22 (6), 629-36
- **POLJAK M ; BRENCIC A ; SEME K ; VINCE A ; MARIN IJ.** Comparative evaluation of first-and second-generation digene hybrid capture assays for detection of human papillomaviruses associated with high or intermediate risk for cervical cancer. *Journal of clinical microbiology,* 1999, vol. 37 (3), 796-7
- **KAUFMANN AM ; NIELAND JD ; JOCHMUS I ; BAUR S ; FRIESE K ; GABELSBERGER J et al.** Vaccination trial with HPV16 L1E7 chimeric virus-like particles in women suffering from high grade cervical intraepithelial neoplasia (CIN 2/3). *International journal of cancer Journal international du cancer,* 2007, vol. 121 (12), 2794-800
- **SANTIN AD ; BELLONE S ; PALMIERI M ; ZANOLINI A ; RAVAGGI A ; SIEGEL ER et al.** Human papillomavirus type 16 and 18 E7-pulsed dendritic cell vaccination of stage IB or IIA cervical cancer patients: a phase I escalating-dose trial. *Journal of virology,* 2008, vol. 82 (4), 1968-79
- **GARCIA F ; PETRY KU ; MUDERSPACH L ; GOLD MA ; BRALY P ; CRUM CP et al.** ZYC101a for treatment of high-grade cervical intraepithelial neoplasia: a randomized controlled trial. *Obstetrics and gynecology,* 2004, vol. 103 (2), 317-26
- **EINSTEIN MH ; KADISH AS ; BURK RD ; KIM MY ; WADLER S ; STREICHER H et al.** Heat shock fusion protein-based immunotherapy for treatment of cervical intraepithelial neoplasia III. *Gynecologic oncology,* 2007, vol. 106 (3), 453-60
- **ROMAN LD ; WILCZYNSKI S ; MUDERSPACH LI ; BURNETT AF ; O'MEARA A ; BRINKMAN JA et al.** A phase II study of Hsp-7 (SGN-00101) in women with high-grade cervical intraepithelial neoplasia. *Gynecologic oncology,* 2007, vol. 106 (3), 558-66
- **HALLEZ S ; SIMON P ; MAUDOUX F ; DOYEN J ; NOEL JC ; BELIARD A et al.** Phase I/II trial of immunogenicity of a human papillomavirus (HPV) type 16 E7 protein-based vaccine in women with oncogenic HPV-positive cervical intraepithelial neoplasia. *Cancer immunology, immunotherapy : CII,* 2004, vol. 53 (7), 642-50
- **FRAZER IH ; QUINN M ; NICKLIN JL ; TAN J ; PERRIN LC ; NG P et al.** Phase 1 study of HPV16-specific immunotherapy with E6E7 fusion protein and ISCOMATRIX adjuvant in women with cervical intraepithelial neoplasia. *Vaccine,* 2004, vol. 23 (2), 172-81
- **STELLER MA ; GURSKI KJ ; MURAKAMI M ; DANIEL RW ; SHAH KV ; CELIS E et al.** Cell-mediated immunological responses in cervical and vaginal cancer patients immunized with a lipidated epitope of human papillomavirus type 16 E7. *Clinical cancer research : an official journal of the American Association for Cancer Research,* 1998, vol. 4 (9), 2103-9
- **MACIAG PC ; RADULOVIC S ; ROTHMAN J.** The first clinical use of a live-attenuated Listeria monocytogenes vaccine: a Phase I safety study of Lm-LLO-E7 in patients with advanced carcinoma of the cervix. *Vaccine,* 2009, vol. 27 (30), 3975-83
- **DE VOS VAN STEENWIJK PJ ; RAMWADHDOEBE TH ; LOWIK MJ ; VAN DER MINNE CE ; BERENDS-VAN DER MEER DM ; FATHERS LM et al.** A placebo-controlled randomized HPV16 synthetic long-peptide vaccination study in women with high-grade cervical squamous intraepithelial lesions. *Cancer immunology, immunotherapy : CII,* 2012, vol. 61 (9), 1485-92
- **MUDERSPACH L ; WILCZYNSKI S ; ROMAN L ; BADE L ; FELIX J ; SMALL LA et al.** A phase I trial of a human papillomavirus (HPV) peptide vaccine for women with high-grade cervical and vulvar intraepithelial neoplasia who are HPV 16 positive. *Clinical cancer research : an official journal of the American Association for Cancer Research,* 2000, vol. 6 (9), 3406-16
- **SOLARES AM ; BALADRON I ; RAMOS T ; VALENZUELA C ; BORBON Z ; FANJULL S et al.** Safety and Immunogenicity of a Human Papillomavirus Peptide Vaccine (CIGB-228) in Women with High-Grade Cervical Intraepithelial Neoplasia: First-in-Human, Proof-of-Concept Trial. *ISRN obstetrics and gynecology. 2011,* 2011, 292951

- **GARCIA-HERNANDEZ E ; GONZALEZ-SANCHEZ JL ; ANDRADE-MANZANO A ; CONTRERAS ML ; PADILLA S ; GUZMAN CC et al.** Regression of papilloma high-grade lesions (CIN 2 and CIN 3) is stimulated by therapeutic vaccination with MVA E2 recombinant vaccine. *Cancer gene therapy,* 2006, vol. 13 (6), 592-7
- **KAUFMANN AM ; STEM PL ; RANKIN EM ; SOMMER H ; NUESSLER V ; SCHNEIDER A et al.** Safety and immunogenicity of TA-HPV, a recombinant vaccinia virus expressing modified human papillomavirus (HPV)-16 and HPV-18 E6 and E7 genes, in women with progressive cervical cancer. *Clinical cancer research : an official journal of the American Association for Cancer Research,* 2002, vol. 8 (12), 3676-85
- **BORYSIEWICZ LK ; FIANDER A ; NIMAKO M ; MAN S ; WILKINSON GW ; WESTMORELAND D et al.** A recombinant vaccinia virus encoding human papillomavirus types 16 and 18, E6 and E7 proteins as immunotherapy for cervical cancer. *Lancet,* 1996, vol. 347 (9014), 1523-7
- **WRIGHT et al.** *American Journal of Clinical pathology,* 2011, vol. 136, 578-586
- **KHAN et al.** *Journal of the National Cancer Institute,* 2005, vol. 97 (14), 1072-1079
- **DOORBAR et al.** *Vaccine,* 2012, 30